(19) Europäisches Patentamt
European Patent Office
Office européen des brevets

(11) **EP 4 278 891 A1**

(12) **EUROPEAN PATENT APPLICATION**

(43) Date of publication:
**22.11.2023 Bulletin 2023/47**

(21) Application number: **22174669.6**

(22) Date of filing: **20.05.2022**

(51) International Patent Classification (IPC):
*A01H 1/04* (2006.01)     *A01H 1/00* (2006.01)
*A01H 5/10* (2018.01)     *A01H 6/20* (2018.01)
*C12Q 1/6895* (2018.01)

(52) Cooperative Patent Classification (CPC):
**A01H 1/045; A01H 1/1245; A01H 5/10; A01H 6/20;
C12Q 1/6895;** C12Q 2600/156

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(83) **Declaration under Rule 32(1) EPC (expert
solution)**

(71) Applicant: **KWS SAAT SE & Co. KGaA
37574 Einbeck (DE)**

(72) Inventors:
• **Breuer, Frank
37574 Einbeck (DE)**
• **Volkmann, Susann
37574 Einbeck (DE)**
• **Gertz, Andreas
37574 Einbeck (DE)**

(74) Representative: **De Clercq & Partners
Edgard Gevaertdreef 10a
9830 Sint-Martens-Latem (BE)**

Remarks:
The complete document including Reference
Table(s) and the Sequence Listing(s) can be
downloaded from the EPO website

(54) **CLUBROOT RESISTANCE AND MARKERS IN BRASSICA**

(57)     The present invention relates to clubroot resistance in Brassicaceae. The present invention in particular relates to a QTL allele conferring clubroot resistance, and the use of such QTL allele for generating clubroot resistant plants. The invention further relates to the identification of clubroot resistant Brassicaceae plants through the identification of the QTL allele, as well as associated markers.

**Description**

**FIELD OF THE INVENTION**

The invention relates to plant breeding, in particular the generation and identification of plants of the genus *Brassica* having resistance to clubroot, such as caused by *Plasmodiophora brassicae.* The present invention further relates to markers useful in the generation and identification of such *Brassica* plants, in particular *Brassica napus* and *Brassica rapa.*

**BACKGROUND OF THE INVENTION**

The clubroot disease of the Brassicaceae plant family comprising oilseed rape is caused by the parasitic protist *Plasmodiophora brassicae.* A *Brassicaceae* suffering from such a protist infection develops chlorosis or leaf yellowing and exhibits a stunted growth. With progressive disease an increased auxin or growth hormone production released from plant and pathogen induces root cell proliferation, causing the formation of galls. Their spindle-like appearance as clubs on the roots were eponym for the clubroot disease. These galls can grow big enough to restrict the xylem tissue and thus inhibit efficient water uptake. Eventually, the roots will rot, and the plant will die. A clubroot infection causes significant yield loss and substantial financial damage. The danger that emanates from this disease lies in the different existing protist races and the rapid adaption of these protist. An aggravating factor is that no control with fungicides is possible.

Breeding *Plasmodiophora brassicae* resistance into *Brassicaceae* is the principal and most effective control method to manage yield loss associated with clubroot disease. The development of molecular genetic markers has facilitated mapping and selection of agriculturally important traits in oilseed rape, and quantitative trait loci (QTL) for clubroot disease were isolated before. Today sold resistant varieties are based on the so-called "Mendel" resistance variety, but a severe breakage of resistance is already seen in the field. A breakage of resistance means that the plants are susceptible to the protist again and because of the rapid rate at which *Plasmodiophora brassicae* overcomes resistance, further sources of resistance are imperative. Therefore, there remains a need for a method to develop and track resistant oilseed rape inbreeds and hybrids through marker assisted breeding, and for commercially acceptable hybrids that are *Plasmodiophora brassicae* resistant. The present invention complies with this need by identifying new genetic sources, which are resistant against Mendel-breaking field isolates derived from symptomatic clubroot galls.

It is an objective of the present invention to address one or more of the above shortcomings.

**SUMMARY OF THE INVENTION**

The present invention relates to clubroot resistance in *Brassicaceae.* Underlying the present invention is a new clubroot resistance QTL/locus. While the locus originally derives from *Brassica rapa,* it is useable as a clubroot resistance source throughout the *Brassicaceae* family, including, but not limited to *Brassica napus* and *Brassica rapa.*

Described herein is a method to map clubroot disease resistance QTL/locus, such as in a DH population. The present invention thus allows tracking of clubroot disease resistance QTL/locus in order to introgress the resistance trait into new plants through traditional breeding. Further, the method allows a later selection of progeny, which contain the genomic background of the agronomically desirable parent and the genomic trait of the clubroot disease resistant donor parent, leading to a *Plasmodiophora brassicae* resistant hybrid.

In certain aspects, the invention relates to a clubroot resistance Polynucleotide, QTL, or locus, as well as markers associated therewith, and uses thereof, such as for the generation or identification of clubroot resistance. The present invention in certain aspects relates to the markers with shown in Tables E and D.

The present inventors have surprisingly found that the clubroot resistance QTL/locus provides widespread resistance, in particular resistance to clubroot isolates which broke through previously known resistance QTLs/loci.

Moreover, the resistance QTL/locus of the present invention does not suffer from linkage drag. Indeed, contrary the previously known clubroot resistance QTLs/loci, which tend to negatively affect yield, the QTL/locus of the present invention when introgressed does not affect yield.

The present invention is in particular captured by any one or any combination of one or more of the below numbered statements 1 to 131, with any other statement and/or embodiments.

1. A method for identifying and/or selecting a plant or plant part of the *Brassicaceae* family, comprising screening for the presence of a QTL allele on a polynucleotide located on a chromosomal interval spanning at most 10 cM, preferably at most 5 cM, more preferably at most 2 cM respectively upstream and downstream of molecular marker (allele) ra74856s01 and ra36444s01.

2. The method according to statement 1, wherein said chromosomal interval is flanked by, and optionally includes molecular marker (allele) ra74856s01 and/or ra36444s01.

3. A method for identifying and/or selecting a plant or plant part of the *Brassicaceae* family, comprising screening for the presence of a QTL allele on a polynucleotide located on a chromosomal interval flanked by and optionally including molecular marker (allele) ra74856s01 and/or ra36444s01.

4. The method according to any of statements 1 to 3, comprising screening for the presence of a polynucleotide comprising one or more molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01 (in the genome of the plant or plant part).

5. A method for identifying and/or selecting a plant or plant part of the *Brassicaceae* family, comprising screening for the presence of a polynucleotide comprising one or more molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01 (in the genome of the plant or plant part).

6. The method according to any of statements 1 to 5, wherein said polynucleic acid comprises at least 5 of said molecular markers (alleles), preferably at least 10, more preferably at least 20, most preferably all.

7. The method according to any of statements 1 to 6, comprising screening for the presence of a polynucleotide comprising one or more molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, and ra36443s01 (in the genome of the plant or plant part).

8. The method according to any of statements 1 to 6, comprising screening for the presence of a polynucleotide comprising one or more molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01 (in the genome of the plant or plant part).

9. The method according to statement 7 or 8, wherein said polynucleic acid comprises at least 5 of said molecular markers (alleles), preferably at least 10, more preferably all.

10. The method according to any of statements 1 to 9, comprising screening for the presence of a polynucleotide comprising one or more molecular marker (allele) selected from ra74862s01, ra74863s01, ra74864s01, ra74910s01, ra74865s01, ra74952s01, and ra74867s01 (in the genome of the plant or plant part).

11. The method according to statement 10, wherein said polynucleic acid comprises at least 5 of said molecular markers (alleles), preferably all.

12. The method according to any of statements 1 to 11, comprising screening for the presence of a polynucleotide comprising one or more molecular marker (allele) selected from ra74862s01 and ra74867s01 (in the genome of the plant or plant part).

13. The method according to statement 12, wherein said polynucleic acid comprises both of said molecular markers (alleles).

14. A method for identifying and/or selecting a plant or plant part of the *Brassicaceae* family, comprising screening for the presence of a polynucleotide comprising one or more molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01 (in the genome of the plant or plant part).

15. A method for identifying and/or selecting a plant or plant part of the *Brassicaceae* family, comprising screening for the presence of a polynucleotide comprising one or more molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01 (in the genome of the plant or plant part).

16. The method according to statement 14 or 15, wherein said polynucleic acid comprises at least 5 of said molecular markers (alleles), preferably at least 10, more preferably all.

17. A method for identifying and/or selecting a plant or plant part of the *Brassicaceae* family, comprising screening for the presence of a polynucleotide comprising one or more molecular marker (allele) selected from ra74862s01, ra74863s01, ra74864s01, ra74910s01, ra74865s01, ra74952s01, and ra74867s01 (in the genome of the plant or plant part).

18. The method according to statement 17, wherein said polynucleic acid comprises at least 5 of said molecular markers (alleles), preferably all.

19. A method for identifying and/or selecting a plant or plant part of the *Brassicaceae* family, comprising screening for the presence of a polynucleotide comprising one or more molecular marker (allele) selected from ra74862s01 and ra74867s01 (in the genome of the plant or plant part).

20. The method according to statement 19, wherein said polynucleic acid comprises both of said molecular markers (alleles).

21. The method according to any of statements 1 to 20, comprising screening for the presence of one or more molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01 (in the genome of the plant or plant part).

22. The method according to any of statements 1 to 21, comprising screening for the presence of one or more molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, and ra36443s01.

23. The method according to any of statements 1 to 21, comprising screening for the presence of one or more molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01.

24. The method according to any of statements 1 to 23, comprising screening for the presence of one or more molecular marker (allele) selected from ra74862s01, ra74863s01, ra74864s01, ra74910s01, ra74865s01, ra74952s01, and ra74867s01.

25. The method according to any of statements 1 to 24, comprising screening for the presence of one or more molecular marker (allele) selected from ra74862s01 and ra74867s01.

26. The method according to any of statements 1 to 25, wherein said polynucleic acid comprises or is comprised in a QTL (allele) (on a chromosomal interval) comprising or flanked by (and optionally including) the molecular marker (allele) ra74856s01 and/or ra36444s01.

27. The method according to any of statements 1 to 26, wherein said polynucleic acid comprises or is comprised in a QTL (allele) (on a chromosomal interval) comprising or flanked by (and optionally including) the molecular marker (allele) ra74856s01 and ra36444s01.

28. The method according to statement 26 or 27, wherein said QTL (allele) is located on chromosome A06 (of *Brassica napus,* or the corresponding chromosome in another species of the *Brassicaceae* family).

29. A method for identifying and/or selecting a plant or plant part of the *Brassicaceae* family, comprising screening for the presence of one or more molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01 (in the genome of the plant or plant part).

30. A method for identifying and/or selecting a plant or plant part of the *Brassicaceae* family, comprising screening for the presence of one or more molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01.

31. A method for identifying and/or selecting a plant or plant part of the *Brassicaceae* family, comprising screening for the presence of one or more molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01.

32. A method for identifying and/or selecting a plant or plant part of the *Brassicaceae* family, comprising screening for the presence of one or more molecular marker (allele) selected from ra74862s01, ra74863s01, ra74864s01, ra74910s01, ra74865s01, ra74952s01, and ra74867s01.

33. A method for identifying and/or selecting a plant or plant part of the *Brassicaceae* family, comprising screening for the presence of one or more molecular marker (allele) selected from ra74862s01 and ra74867s01.

34. A method for identifying and/or selecting a plant or plant part of the *Brassicaceae* family, comprising screening for the presence of a QTL (allele) (on a chromosomal interval) comprising or flanked by (and optionally including) the molecular marker (allele) ra74862s01 and ra74867s01.

35. The method according to statement 34, wherein said QTL (allele) (on a chromosomal interval) comprises or is flanked by (and optionally includes) the molecular marker (allele) ra74856s01 and ra36444s01.

36. A method for identifying and/or selecting a plant or plant part of the *Brassicaceae* family, comprising screening for the presence of a QTL (allele) (on a chromosomal interval) comprising or flanked by (and optionally including) the molecular marker (allele) ra74856s01 and ra36444s01.

37. The method according to any of statements 34 to 36, wherein said QTL (allele) is located on chromosome A06

(of *Brassica napus* or the corresponding chromosome of another species of the *Brassicaceae* family).

38. The method according to any of statements 34 to 37, wherein said QTL (allele) comprises one or more molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01 (in the genome of the plant or plant part).

39. The method according to statement 38, wherein said QTL (allele) comprises at least 5 of said molecular markers (alleles), preferably at least 10, more preferably at least 20, most preferably all.

40. The method according to any of statements 34 to 39, wherein said QTL (allele) comprises one or more molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01 (in the genome of the plant or plant part).

41. The method according to any of statements 34 to 39, wherein said QTL (allele) comprises one or more molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01 (in the genome of the plant or plant part).

42. The method according to statement 40 or 41, wherein said QTL (allele) comprises at least 5 of said molecular markers (alleles), preferably at least 10, more preferably all.

43. The method according to any of statements 34 to 42, wherein said QTL (allele) comprises one or more molecular marker (allele) selected from ra74862s01, ra74863s01, ra74864s01, ra74910s01, ra74865s01, ra74952s01, and ra74867s01 (in the genome of the plant or plant part).

44. The method according to statement 43, wherein said QTL (allele) comprises at least 5 of said molecular markers (alleles), preferably all.

45. The method according to any of statements 34 to 44, wherein said QTL (allele) comprises one or more molecular marker (allele) selected from ra74862s01 and ra74867s01 (in the genome of the plant or plant part).

46. The method according to statement 45, wherein said QTL (allele) comprises both of said molecular markers (alleles).

47. The method according to any of statements 34 to 46, comprising screening for the presence of one or more molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01 (in the genome of the plant or plant part).

48. The method according to any of statements 34 to 47, comprising screening for the presence of one or more molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, and ra36443s01.

49. The method according to any of statements 34 to 47, comprising screening for the presence of one or more molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01.

50. The method according to any of statements 34 to 49, comprising screening for the presence of one or more molecular marker (allele) selected from ra74862s01, ra74863s01, ra74864s01, ra74910s01, ra74865s01, ra74952s01, and ra74867s01.

51. The method according to any of statements 34 to 50, comprising screening for the presence of one or more molecular marker (allele) selected from ra74862s01 and ra74867s01.

52. The method according to any of statements 34 to 50, comprising screening for the presence of one or more molecular marker (allele) selected from ra74856s01 and ra36444s01.

53. The method according to any of statements 1 to 52, wherein molecular marker (allele)

ra74856s01 is or comprises a SNP at a position corresponding to position 6042239 (on chromosome A06) of reference *Brassica napus* genome Darmor-bzh v4.1;

ra74921s01 is or comprises a SNP at a position corresponding to position 6043355 (on chromosome A06) of reference *Brassica napus* genome Darmor-bzh v4.1;

ra36415s01 is or comprises a SNP at a position corresponding to position 6045480 (on chromosome A06) of reference *Brassica napus* genome Darmor-bzh v4.1;

ra74945s01 is or comprises a SNP at a position corresponding to position 6059024 (on chromosome A06) of reference *Brassica napus* genome Darmor-bzh v4.1;

ra74946s01 is or comprises a SNP at a position corresponding to position 6067467 (on chromosome A06) of reference *Brassica napus* genome Darmor-bzh v4.1;

ra74948s01 is or comprises a SNP at a position corresponding to position 6082193 (on chromosome A06) of reference *Brassica napus* genome Darmor-bzh v4.1;

ra36418s01 is or comprises a SNP at a position corresponding to position 6085238 (on chromosome A06) of reference *Brassica napus* genome Darmor-bzh v4.1;

ra05569s01 is or comprises a SNP at a position corresponding to position 6085138 (on chromosome A06) of reference *Brassica napus* genome Darmor-bzh v4.1;

ra36419s01 is or comprises a SNP at a position corresponding to position 6086857 (on chromosome A06) of reference *Brassica napus* genome Darmor-bzh v4.1;

ra74858s01 is or comprises a SNP at a position corresponding to position 6088663 (on chromosome A06) of reference *Brassica napus* genome Darmor-bzh v4.1;

ra74859s01 is or comprises a SNP at a position corresponding to position 6091648 (on chromosome A06) of reference *Brassica napus* genome Darmor-bzh v4.1;

ra36423s01 is or comprises a SNP at a position corresponding to position 6098159 (on chromosome A06) of reference *Brassica napus* genome Darmor-bzh v4.1;

ra74922s01 is or comprises a SNP at a position corresponding to position 6102982 (on chromosome A06) of reference *Brassica napus* genome Darmor-bzh v4.1;

ra74860s01 is or comprises a SNP at a position corresponding to position 6113367 (on chromosome A06) of reference *Brassica napus* genome Darmor-bzh v4.1;

ra36426s01 is or comprises a SNP at a position corresponding to position 6134234 (on chromosome A06) of reference *Brassica napus* genome Darmor-bzh v4.1;

ra36427s01 is or comprises a SNP at a position corresponding to position 6141195 (on chromosome A06) of reference *Brassica napus* genome Darmor-bzh v4.1;

ra36428s01 is or comprises a SNP at a position corresponding to position 6141204 (on chromosome A06) of reference *Brassica napus* genome Darmor-bzh v4.1;

ra36429s01 is or comprises a SNP at a position corresponding to position 6141354 (on chromosome A06) of reference *Brassica napus* genome Darmor-bzh v4.1;

ra36430s01 is or comprises a SNP at a position corresponding to position 6151503 (on chromosome A06) of reference *Brassica napus* genome Darmor-bzh v4.1;

ra36433s01 is or comprises a SNP at a position corresponding to position 6152744 (on chromosome A06) of reference *Brassica napus* genome Darmor-bzh v4.1;

ra36434s01 is or comprises a SNP at a position corresponding to position 6153568 (on chromosome A06) of reference *Brassica napus* genome Darmor-bzh v4.1;

ra74862s01 is or comprises a SNP at a position corresponding to position 6155076 (on chromosome A06) of reference *Brassica napus* genome Darmor-bzh v4.1;

ra36435s01 is or comprises a SNP at a position corresponding to position 6168127 (on chromosome A06) of reference *Brassica napus* genome Darmor-bzh v4.1;

ra74863s01 is or comprises a SNP at a position corresponding to position 6186575 (on chromosome A06) of reference *Brassica napus* genome Darmor-bzh v4.1;

ra74864s01 is or comprises a SNP at a position corresponding to position 6196798 (on chromosome A06) of reference *Brassica napus* genome Darmor-bzh v4.1;

ra74910s01 is or comprises a SNP at a position corresponding to position 6197815 (on chromosome A06) of reference *Brassica napus* genome Darmor-bzh v4.1;

ra74865s01 is or comprises a SNP at a position corresponding to position 6204215 (on chromosome A06) of reference *Brassica napus* genome Darmor-bzh v4.1;

ra74952s01 is or comprises a SNP at a position corresponding to position 6209886 (on chromosome A06) of reference *Brassica napus* genome Darmor-bzh v4.1;

ra36441s01 is or comprises a SNP at a position corresponding to position 6240866 (on chromosome A06) of reference *Brassica napus* genome Darmor-bzh v4.1;

ra74867s01 is or comprises a SNP at a position corresponding to position 6248401 (on chromosome A06) of reference *Brassica napus* genome Darmor-bzh v4.1;

ra36443s01 is or comprises a SNP at a position corresponding to position 6257529 (on chromosome A06) of reference *Brassica napus* genome Darmor-bzh v4.1; and/or

ra36444s01 is or comprises a SNP at a position corresponding to position 6269510 (on chromosome A06) of reference *Brassica napus* genome Darmor-bzh v4.1;

or the complement or reverse complement of any thereof.

54. The method according to statement 53, wherein said molecular marker (allele) comprises at least 15, preferably at least 20 nucleotides.

55. The method according to statement 53 or 54, wherein said molecular marker (allele) comprises contiguous nucleotides 3' and/or 5' of said respective SNP.

56. The method according to any of statements 1 to 55, wherein molecular marker (allele)

ra74856s01 is or comprises a SNP at a position corresponding to position 51 of SEQ ID NO: 247 or a sequence which is at least 90% identical, preferably at least 95% identical, more preferably at least 98% identical, most preferably identical to SEQ ID NO: 247 (and comprising said SNP);

ra74921s01 is or comprises a SNP at a position corresponding to position 51 of SEQ ID NO: 263 or a sequence which is at least 90% identical, preferably at least 95% identical, more preferably at least 98% identical, most preferably identical to SEQ ID NO: 263 (and comprising said SNP);

ra74945s01 is or comprises a SNP at a position corresponding to position 51 of SEQ ID NO: 270 or a sequence which is at least 90% identical, preferably at least 95% identical, more preferably at least 98% identical, most preferably identical to SEQ ID NO: 270 (and comprising said SNP);

ra74946s01 is or comprises a SNP at a position corresponding to position 51 of SEQ ID NO: 271 or a sequence which is at least 90% identical, preferably at least 95% identical, more preferably at least 98% identical, most preferably identical to SEQ ID NO: 271 (and comprising said SNP);

ra74948s01 is or comprises a SNP at a position corresponding to position 51 of SEQ ID NO: 272 or a sequence which is at least 90% identical, preferably at least 95% identical, more preferably at least 98% identical, most preferably identical to SEQ ID NO: 272 (and comprising said SNP);

ra74858s01 is or comprises a SNP at a position corresponding to position 51 of SEQ ID NO: 248 or a sequence which is at least 90% identical, preferably at least 95% identical, more preferably at least 98% identical, most preferably identical to SEQ ID NO: 248 (and comprising said SNP);

ra74859s01 is or comprises a SNP at a position corresponding to position 51 of SEQ ID NO: 249 or a sequence which is at least 90% identical, preferably at least 95% identical, more preferably at least 98% identical, most preferably identical to SEQ ID NO: 249 (and comprising said SNP);

ra74922s01 is or comprises a SNP at a position corresponding to position 51 of SEQ ID NO: 264 or a sequence which is at least 90% identical, preferably at least 95% identical, more preferably at least 98% identical, most preferably identical to SEQ ID NO: 264 (and comprising said SNP);

ra74860s01 is or comprises a SNP at a position corresponding to position 51 of SEQ ID NO: 250 or a sequence which is at least 90% identical, preferably at least 95% identical, more preferably at least 98% identical, most preferably identical to SEQ ID NO: 250 (and comprising said SNP);

ra36428s01 is or comprises a SNP at a position corresponding to position 51 of SEQ ID NO: 231 or a sequence which is at least 90% identical, preferably at least 95% identical, more preferably at least 98% identical, most preferably identical to SEQ ID NO: 231 (and comprising said SNP);

ra74862s01 is or comprises a SNP at a position corresponding to position 51 of SEQ ID NO: 251 or a sequence which is at least 90% identical, preferably at least 95% identical, more preferably at least 98% identical, most preferably identical to SEQ ID NO: 251 (and comprising said SNP);

ra74863s01 is or comprises a SNP at a position corresponding to position 51 of SEQ ID NO: 252 or a sequence which is at least 90% identical, preferably at least 95% identical, more preferably at least 98% identical, most preferably identical to SEQ ID NO: 252 (and comprising said SNP);

ra74864s01 is or comprises a SNP at a position corresponding to position 51 of SEQ ID NO: 253 or a sequence which is at least 90% identical, preferably at least 95% identical, more preferably at least 98% identical, most preferably identical to SEQ ID NO: 253 (and comprising said SNP);

ra74910s01 is or comprises a SNP at a position corresponding to position 51 of SEQ ID NO: 274 or a sequence which is at least 90% identical, preferably at least 95% identical, more preferably at least 98% identical, most preferably identical to SEQ ID NO: 274 (and comprising said SNP);

ra74865s01 is or comprises a SNP at a position corresponding to position 51 of SEQ ID NO: 254 or a sequence which is at least 90% identical, preferably at least 95% identical, more preferably at least 98% identical, most preferably identical to SEQ ID NO: 254 (and comprising said SNP);

ra74952s01 is or comprises a SNP at a position corresponding to position 51 of SEQ ID NO: 273 or a sequence which is at least 90% identical, preferably at least 95% identical, more preferably at least 98% identical, most preferably identical to SEQ ID NO: 273 (and comprising said SNP);

ra74867s01 is or comprises a SNP at a position corresponding to position 51 of SEQ ID NO: 255 or a sequence which is at least 90% identical, preferably at least 95% identical, more preferably at least 98% identical, most preferably identical to SEQ ID NO: 255 (and comprising said SNP); and/or

ra36444s01 is or comprises a SNP at a position corresponding to position 51 of SEQ ID NO: 223 or a sequence which is at least 90% identical, preferably at least 95% identical, more preferably at least 98% identical, most preferably identical to SEQ ID NO: 223 (and comprising said SNP);

or the complement or reverse complement of any thereof.

57. The method according to any of statements 1 to 56, wherein molecular marker (allele)

ra74856s01 has a sequence comprising, consisting of, or comprised in a sequence as set forth in SEQ ID NO: 26 or 100, or a sequence which is at least 90% identical, preferably at least 95% identical, more preferably at least 98% identical, most preferably identical to SEQ ID NO: 26 or 100;

ra74921s01 has a sequence comprising, consisting of, or comprised in a sequence as set forth in SEQ ID NO: 42 or 116, or a sequence which is at least 90% identical, preferably at least 95% identical, more preferably at least 98% identical, most preferably identical to SEQ ID NO: 42 or 116;

ra74945s01 has a sequence comprising, consisting of, or comprised in a sequence as set forth in SEQ ID NO: 49 or 123, or a sequence which is at least 90% identical, preferably at least 95% identical, more preferably at least 98% identical, most preferably identical to SEQ ID NO: 49 or 123;

ra74946s01 has a sequence comprising, consisting of, or comprised in a sequence as set forth in SEQ ID NO: 50 or 124, or a sequence which is at least 90% identical, preferably at least 95% identical, more preferably at least 98% identical, most preferably identical to SEQ ID NO: 50 or 124;

ra74948s01 has a sequence comprising, consisting of, or comprised in a sequence as set forth in SEQ ID NO: 51 or 125, or a sequence which is at least 90% identical, preferably at least 95% identical, more preferably at least 98% identical, most preferably identical to SEQ ID NO: 51 or 125;

ra74858s01 has a sequence comprising, consisting of, or comprised in a sequence as set forth in SEQ ID NO: 27 or 101, or a sequence which is at least 90% identical, preferably at least 95% identical, more preferably at least 98% identical, most preferably identical to SEQ ID NO: 27 or 101;

ra74859s01 has a sequence comprising, consisting of, or comprised in a sequence as set forth in SEQ ID NO: 28 or 102, or a sequence which is at least 90% identical, preferably at least 95% identical, more preferably at least 98% identical, most preferably identical to SEQ ID NO: 28 or 102;

ra74922s01 has a sequence comprising, consisting of, or comprised in a sequence as set forth in SEQ ID NO: 43 or 117, or a sequence which is at least 90% identical, preferably at least 95% identical, more preferably at least 98% identical, most preferably identical to SEQ ID NO: 43 or 117;

ra74860s01 has a sequence comprising, consisting of, or comprised in a sequence as set forth in SEQ ID NO: 29 or 103, or a sequence which is at least 90% identical, preferably at least 95% identical, more preferably at least 98% identical, most preferably identical to SEQ ID NO: 29 or 103;

ra36428s01 has a sequence comprising, consisting of, or comprised in a sequence as set forth in SEQ ID NO: 10 or 84, or a sequence which is at least 90% identical, preferably at least 95% identical, more preferably at least 98% identical, most preferably identical to SEQ ID NO: 10 or 84;

ra74862s01 has a sequence comprising, consisting of, or comprised in a sequence as set forth in SEQ ID NO: 30 or 104, or a sequence which is at least 90% identical, preferably at least 95% identical, more preferably at least 98% identical, most preferably identical to SEQ ID NO: 30 or 104;

ra74863s01 has a sequence comprising, consisting of, or comprised in a sequence as set forth in SEQ ID NO: 31 or 105, or a sequence which is at least 90% identical, preferably at least 95% identical, more preferably at least 98% identical, most preferably identical to SEQ ID NO: 31 or 105;

ra74864s01 has a sequence comprising, consisting of, or comprised in a sequence as set forth in SEQ ID NO: 32 or 106, or a sequence which is at least 90% identical, preferably at least 95% identical, more preferably at least 98% identical, most preferably identical to SEQ ID NO: 32 or 106;

ra74910s01 has a sequence comprising, consisting of, or comprised in a sequence as set forth in SEQ ID NO: 66, or 137, or a sequence which is at least 90% identical, preferably at least 95% identical, more preferably at least 98% identical, most preferably identical to SEQ ID NO: 66 or 137;

ra74865s01 has a sequence comprising, consisting of, or comprised in a sequence as set forth in SEQ ID NO: 33 or 107, or a sequence which is at least 90% identical, preferably at least 95% identical, more preferably at least 98% identical, most preferably identical to SEQ ID NO: 33 or 107;

ra74952s01 has a sequence comprising, consisting of, or comprised in a sequence as set forth in SEQ ID NO: 52 or 126, or a sequence which is at least 90% identical, preferably at least 95% identical, more preferably at least 98% identical, most preferably identical to SEQ ID NO: 52 or 126;

ra74867s01 has a sequence comprising, consisting of, or comprised in a sequence as set forth in SEQ ID NO: 34 or 108, or a sequence which is at least 90% identical, preferably at least 95% identical, more preferably at least 98% identical, most preferably identical to SEQ ID NO: 34 or 108;
and/or

ra36444s01 has a sequence comprising, consisting of, or comprised in a sequence as set forth in SEQ ID NO: 2 or 76, or a sequence which is at least 90% identical, preferably at least 95% identical, more preferably at least 98% identical, most preferably identical to SEQ ID NO: 2 or 76;

or the complement or reverse complement of any thereof.

58. The method according to statement 56 or 57, wherein said molecular marker (allele) comprises at least 15, preferably at least 20 contiguous nucleotides of said respective SEQ ID NO or at least 20 contiguous nucleotides being at least 90% identical, preferably at least 95% identical, more preferably at least 98% identical, most preferably identical to said respective SEQ ID NO, or the complement or reverse complement thereof.

59. The method according to any of statements 56 to 58, wherein said molecular marker (allele) comprises at least the nucleotide at or corresponding to position 51 of said respective SEQ ID NO.

60. A method for identifying and/or selecting a plant or plant part of the *Brassicaceae* family, comprising screening for the presence of (a polynucleotide comprising) one or more SNP at a position corresponding to position 6042239, 6043355, 6045480, 6059024, 6067467, 6082193, 6085238, 6085138, 6086857, 6088663, 6091648, 6098159, 6102982, 6113367, 6134234, 6141195, 6141204, 6141354, 6151503, 6152744, 6153568, 6155076, 6168127, 6186575, 6196798, 6197815, 6204215, 6209886, 6240866, 6248401, 6257529, and/or 6269510 (on chromosome A06) of reference *Brassica napus* genome Darmor-bzh v4.1.

61. A method for identifying and/or selecting a plant or plant part of the *Brassicaceae* family, comprising screening for the presence of (a polynucleotide comprising) one or more SNP at a position corresponding to position 51 of respectively SEQ ID Nos 222 to 274 or to 293, preferably SEQ ID Nos: 247, 263, 270, 271, 272, 248, 249, 264, 250, 231, 251, 252, 253, 274, 254, 273, 255, and/or 223; the complement or reverse complement of any thereof.

62. The method according to any of statements 53 to 61 wherein said SNP is

C in ra74856s01;
G in ra74921s01;
A in ra74945s01;
G in ra74946s01;
T in ra74948s01;
A in ra74858s01;
C in ra74859s01;
G in ra74922s01;
T in ra74860s01;
C in ra36428s01;
T in ra74862s01;
C in ra74863s01;
A in ra74864s01;
A in ra74910s01,
C in ra74865s01;
C in ra74952s01;
C in ra74867s01; and/or
T in ra36444s01.

63. The method according to any of statements 53 to 61 wherein said SNP is not

C in ra74856s01;
G in ra74921s01;
A in ra74945s01;
G in ra74946s01;
T in ra74948s01;
A in ra74858s01;
C in ra74859s01;
G in ra74922s01;
T in ra74860s01;
C in ra36428s01;
T in ra74862s01;
C in ra74863s01;
A in ra74864s01;
A in ra74910s01,

C in ra74865s01;
C in ra74952s01;
C in ra74867s01; and/or
T in ra36444s01.

64. The method according to any of statements 53 to 61 wherein said SNP is

A in ra74856s01;
A in ra74921s01;
C in ra74945s01;
A in ra74946s01;
G in ra74948s01;
T in ra74858s01;
T in ra74859s01;
A in ra74922s01;
G in ra74860s01;
T in ra36428s01;
A in ra74862s01;
A in ra74863s01;
C in ra74864s01;
G in ra74910s01,
T in ra74865s01;
T in ra74952s01;
A in ra74867s01; and/or
C in ra36444s01.

65. The method according to any of statements 1 to 64, which is a method for identifying a plant or plant part of the *Brassicaceae* family having (increased) resistance or tolerance to infection with a parasite from the genus *Plasmodiophora,* such as in comparison to a plant or plant part of the Brassicaceae family not having said polynucleotide, molecular marker (allele), locus (allele), QTL (allele), or SNP (allele) of the invention.

66. The method according to any of statements 1 to 65, which is a method for identifying a plant or plant part of the *Brassicaceae* family having (increased) resistance or tolerance to *Plasmodiophora brassicae,* such as in comparison to a plant or plant part of the Brassicaceae family not having said polynucleotide, molecular marker (allele), locus (allele), QTL (allele), or SNP (allele) of the invention.

67. The method according to any of statements 1 to 66, which is a method for identifying a plant or plant part of the *Brassicaceae* family having (increased) clubroot resistance or tolerance, such as in comparison to a plant or plant part of the Brassicaceae family not having said polynucleotide, molecular marker (allele), locus (allele), QTL (allele), or SNP (allele) of the invention.

68. The method according to any of statements 1 to 67, which is a method for distinguishing between a plant or plant part of the *Brassicaceae* family having (increased) resistance or tolerance to infection with a parasite from the genus *Plasmodiophora* and a plant or plant part of the *Brassicaceae* family not having or lacking (increased) resistance or tolerance to infection with a parasite from the genus *Plasmodiophora,* such as in comparison to a plant or plant part of the Brassicaceae family not having said polynucleotide, molecular marker (allele), locus (allele), QTL (allele), or SNP (allele) of the invention.

69. The method according to any of statements 64 to 68, wherein said plant or plant part of the *Brassicaceae* family is identified as having (increased) resistance or tolerance if said polynucleotide, molecular marker (allele), QTL, or SNP is present in the genome of said plant or plant part, such as in comparison to a plant or plant part of the Brassicaceae family not having said polynucleotide, molecular marker (allele), locus (allele), QTL (allele), or SNP (allele) of the invention.

70. The method according to any of statements 1 to 69, wherein said polynucleotide, molecular marker (allele), QTL, or SNP is homozygous.

71. The method according to any of statements 1 to 69, wherein said polynucleotide, molecular marker (allele), QTL, or SNP is heterozygous.

72. The method according to any of statements 1 to 71, comprising isolating genomic DNA from the plant or plant part.

73. The method according to any of statements 1 to 72, comprising selecting a plant of plant part comprising the polynucleotide, molecular marker (allele), QTL, or SNP.

74. The method according to any of statements 1 to 73, wherein said plant part is a seed.

75. The method according to any of statements 1 to 73, wherein said plant part is not propagation material.

76. The method according to any of statements 1 to 75, wherein said polynucleotide has a length of at most 1 Mb, preferably at most 500 kb, more preferably at most 200 kb, most preferably at most 100 kb, most preferably at most 50 kb.

77. The method according to any of statements 1 to 76, wherein said plant or plant part is of the tribe *Brassiceae*.

78. The method according to any of statements 1 to 77, wherein said plant or plant part is of the genus *Brassica*.

79. The method according to any of statements 1 to 78, wherein said plant or plant part is of the species *Brassica balearica, Brassica carinata, Brassica elongate, Brassica fruticulose, Brassica hilarionis, Brassica juncea, Brassica napus, Brassica narinosa, Brassica nigra, Brassica oleracea, Brassica perviridis, Brassica rapa (syn. B. campestris), Brassica rupestris, Brassica spinescens, Brassica tournefortii, Brassica alba, Brassica hirta, Brassica geniculate, Brassica kaber,* and *Raphanus sativus;* preferably *Brassica napus* or *Brassica rapa.*

80. A plant or plant part of the *Brassicaceae* family comprising the polynucleic acid, molecular marker (allele), QTL, or SNP as defined in any of statements 1 to 79.

81. The plant or plant part according to statement 80, wherein said polynucleic acid, molecular marker (allele), QTL, or SNP is homozygous.

82. The plant or plant part according to statement 80, wherein said polynucleic acid, molecular marker (allele), QTL, or SNP is heterozygous.

83. The plant or plant part according to any of statements 80 to 82, wherein said plant or plant part is mutagenized.

84. The plant or plant part according to any of statements 80 to 83, wherein said plant or plant part is transgenic or gene-edited.

85. The plant or plant part according to any of statements 80 to 82, wherein said plant or plant part is obtained by introgression of said one or more polynucleic acid, molecular marker (allele), QTL, or SNP.

86. The plant or plant part according to any of statements 80 to 85, wherein said plant or plant part is of the tribe *Brassiceae.*

87. The plant or plant part according to any of statements 80 to 86, wherein said plant or plant part is of the genus *Brassica.*

88. The plant or plant part according to any of statements 80 to 87, wherein said plant or plant part is of the species *Brassica balearica, Brassica carinata, Brassica elongate, Brassica fruticulose, Brassica hilarionis, Brassica juncea, Brassica napus, Brassica narinosa, Brassica nigra, Brassica oleracea, Brassica perviridis, Brassica rapa (syn. B. campestris), Brassica rupestris, Brassica spinescens, Brassica tournefortii, Brassica alba, Brassica hirta, Brassica geniculate, Brassica kaber,* and *Raphanus sativus;* preferably *Brassica napus* or *Brassica rapa.*

89. A method for generating or producing a plant or plant part of the *Brassicaceae* family, comprising

> providing a seed mixture harvested from a cross between a first parent plant (population) of the *Brassicaceae* family and a second parent plant (population) of the *Brassicaceae* family, wherein said first and/or second parent plant (population) comprises a polynucleic acid, molecular marker (allele), QTL, or SNP as defined in any of statements 1 to 79;
>
> selecting seeds comprising a polynucleic acid, molecular marker (allele), QTL, or SNP as defined in any of statements 1 to 79.

90. A method for generating or producing a plant or plant part of the *Brassicaceae* family, comprising introducing into the genome of a plant or plant part of the *Brassicaceae* family a polynucleic acid, molecular marker (allele), QTL, or SNP, as defined in any of statements 1 to 79.

91. The method according to statement 90, wherein introducing into the genome comprises introgression.

92. The method according to statement 90 or 91, comprising (a) providing a first plant of the *Brassicaceae* family having the polynucleic acid, molecular marker (allele), QTL, or SNP as defined in any of statements 1 to 79, (b) crossing said first plant with a second plant of the *Brassicaceae* family, and (c) selecting progeny plants or plant parts having the polynucleic acid, molecular marker (allele), QTL, or SNP as defined in any of statements 1 to 79.

93. The method according to statement 92, further comprising (d) harvesting a plant part from said progeny.

94. The method according to statement 90, wherein introducing into the genome comprises mutagenesis.

95. The method according to statement 90, wherein introducing into the genome comprises transgenesis or gene-editing.

96. The method according to statement 90, comprising transforming a plant or plant part, preferably a plant cell, more preferably an immature or mature embryo, an inflorescence, a protoplast or callus, with said polynucleic acid, molecular marker (allele), QTL, or SNP, and optionally regenerating a plant from said plant cell, preferably immature or mature embryo, inflorescence, protoplast or callus.

97. The method according to any of statements 90 to 96, wherein said polynucleotide has a length of at most 1 Mb, preferably at most 500 kb, more preferably at most 300 kb.

98. The method according to any of 89 to 97, wherein said plant part is a plant cell, tissue, organ, or seed.

99. The method according to any of statements 89 to 98, wherein said plant part is an immature or mature embryo, an inflorescence, a protoplast or callus.

100. A plant or plant part of the *Brassicaceae* family obtainable by the method according to any of statements 89 to 99.

101. A (isolated) polynucleotide comprising a polynucleic acid, molecular marker (allele), QTL, or SNP as defined in any of statements 1 to 79, the complement or the reverse complement thereof, or a (unique) fragment thereof.

102. The (isolated) polynucleotide according to statement 101, which comprises or consists of 10 to 500 nucleotides, preferably 15 to 250 nucleotides, more preferably 18 to 250 nucleotides, most preferably 20 to 250 nucleotides.

103. The (isolated) polynucleic acid according to statement 101 or 102, in particular suitable as molecular marker, comprising at least 15, preferably at least 18, more preferably at least 20, contiguous nucleotides of any of SEQ ID NOs: 1 to 221, preferably SEQ ID NOs: 2, 10, 26 to 34, 42-43, 49-52, 66, 76, 84, 100-108, 116-117, 123-126, 137, 147, 155, 171-179, 187-188, 194-197, 208, more preferably SEQ ID NOs: 30-34, 52, 66, 104-108, 126, 137, 175-179, 197, 208; or SEQ ID NOs: 222 to 274 or to 293, preferably SEQ ID NOs: 247, 263, 270, 271, 272, 248, 249, 264, 250, 231, 251, 252, 253, 274, 254, 273, 255, or 223, or complementary to contiguous nucleotides of any of SEQ ID NOs: 1 to 221, preferably SEQ ID NOs: 2, 10, 26 to 34, 42-43, 49-52, 66, 76, 84, 100-108, 116-117, 123-126, 137, 147, 155, 171-179, 187-188, 194-197, 208, more preferably SEQ ID NOs: 30-34, 52, 66, 104-108, 126, 137, 175-179, 197, 208; or SEQ ID NOs: 222 to 274 or to 293, preferably SEQ ID NOs: 247, 263, 270, 271, 272, 248, 249, 264, 250, 231, 251, 252, 253, 274, 254, 273, 255, or 223, or reverse complementary to contiguous nucleotides of any of SEQ ID NOs: 1 to 221, preferably SEQ ID NOs: 2, 10, 26 to 34, 42-43, 49-52, 66, 76, 84, 100-108, 116-117, 123-126, 137, 147, 155, 171-179, 187-188, 194-197, 208, more preferably SEQ ID NOs: 30-34, 52, 66, 104-108, 126, 137, 175-179, 197, 208; or SEQ ID NOs: 222 to 274 or to 293, preferably SEQ ID NOs: 247, 263, 270, 271, 272, 248, 249, 264, 250, 231, 251, 252, 253, 274, 254, 273, 255, or 223.

104. The (isolated) polynucleic acid according to any of statements 101 to 103, in particular suitable as molecular marker, comprising at least the 15 most 3', preferably at least the 18 most 3', more preferably at least the 20 most 3', contiguous nucleotides of any of SEQ ID NO: 1 to 221, or complementary to contiguous nucleotides of any of SEQ ID NO: 1 to 221, or reverse complementary to contiguous nucleotides of any of SEQ ID NO: 1 to 221.

105. The (isolated) polynucleic acid according to any of statements 101 to 104, in particular suitable as molecular marker, comprising at least 15, preferably at least 18, more preferably at least 20, contiguous nucleotides of any of SEQ ID NO: 1 to 145, or complementary to contiguous nucleotides of any of SEQ ID NO: 1 to 145, or reverse complementary to contiguous nucleotides of any of SEQ ID NO: 1 to 145, preferably the most 3' contiguous nucleotides.

106. The (isolated) polynucleic acid according to any of statements 101 to 105, in particular suitable as molecular marker, comprising at least 15, preferably at least 18, more preferably at least 20, contiguous nucleotides of any of SEQ ID NO: 26, 100, 171, 42, 116, 187, 49, 123, 194, 50, 124, 195, 51, 125, 196, 27, 101, 172, 28, 102, 173, 43, 117, 188, 29, 103, 174, 10, 84, 155, 30, 104, 175, 31, 105, 176, 32, 106, 177, 66, 137, 208, 33, 107, 178, 52, 126, 197, 34, 108, 179, 2, 76, or 147, preferably the most 3' contiguous nucleotides.

107. The (isolated) polynucleic acid according to any of statements 101 to 106, in particular suitable as molecular marker, comprising at least 15, preferably at least 18, more preferably at least 20, contiguous nucleotides of any of SEQ ID NO: 26, 100, 42, 116, 49, 123, 50, 124, 51, 125, 27, 101, 28, 102, 43, 117, 29, 103, 10, 84, 30, 104, 31, 105, 32, 106, 66, 137, 33, 107, 52, 126, 34, 108, 2, or 76, preferably the most 3' contiguous nucleotides.

108. The (isolated) polynucleic acid according to any of statements 101 to 107, in particular suitable as molecular marker, comprising at least 15, preferably at least 18, more preferably at least 20, contiguous nucleotides of any of SEQ ID NO: 30, 104, 175, 31, 105, 176, 32, 106, 177, 66, 137, 208, 33, 107, 178, 52, 126, 197, 34, 108, or 179, preferably the most 3' contiguous nucleotides.

109. The (isolated) polynucleic acid according to any of statements 101 to 108, in particular suitable as molecular marker, comprising at least 15, preferably at least 18, more preferably at least 20, contiguous nucleotides of any of SEQ ID NO: 30, 104, 31, 105, 32, 106, 66, 137, 33, 107, 52, 126, 34, or 108, preferably the most 3' contiguous nucleotides.

110. The (isolated) polynucleic acid according to any of statements 101 to 109 comprising a SNP as defined in any of statements 53 to 56 or 60 to 64.

111. An (isolated) polynucleic acid which is the complement or reverse complement of the (isolated) polynucleic acid according to any of statements 101 to 110.

112. A (isolated) polynucleic acid specifically hybridizing with the polynucleotide according to any of statements 1 to 79, the complement or the reverse complement thereof.

113. The (isolated) polynucleic acid according to any of statements 101 to 112, which is a primer or a probe.

114. The (isolated) polynucleic acid according to statement 113, which is an allele-specific primer.

115. The (isolated) polynucleic acid according to any of statements 113 to 114, which is a KASP primer.

116. A primer or probe capable of specifically detecting the polynucleic acid, molecular marker (allele), QTL, or SNP as defined in any of statements 1 to 79.

117. A primer set capable of specifically detecting the polynucleic acid, molecular marker (allele), QTL, or SNP as defined in any of statements 1 to 79.

118. The primer set according to statement 117 comprising at least a first primer and a second primer, each having at least 15, preferably at least 18, more preferably at least 20, contiguous nucleotides of respectively

SEQ ID NO: 26 and 100;
SEQ ID NO: 42 and 116;
SEQ ID NO: 49 and 123;
SEQ ID NO: 50 and 124;
SEQ ID NO: 51 and 125;
SEQ ID NO: 27 and 101;
SEQ ID NO: 28 and 102;
SEQ ID NO: 43 and 117;
SEQ ID NO: 29 and 103;
SEQ ID NO: 10 and 84;
SEQ ID NO: 30 and 104;
SEQ ID NO: 31 and 105;
SEQ ID NO: 32 and 106;
SEQ ID NO: 66 and 137;
SEQ ID NO: 33 and 107;
SEQ ID NO: 52 and 126;
SEQ ID NO: 34 and 108; or
SEQ ID NO: 2 and 76.

119. The primer set according to statement 118 further comprising a third primer.

120. The primer set according to statement 119, wherein said third primer is configured to allow amplification of a polynucleic acid in combination with said first or second primer.

121. The primer set according to statement 117 comprising three primers, each having at least 15, preferably at least 18, more preferably at least 20, contiguous nucleotides of respectively

SEQ ID NO: 26, 100, and 171;
SEQ ID NO: 42, 116, and 187;
SEQ ID NO: 49, 123, and 194;
SEQ ID NO: 50, 124, and 195;
SEQ ID NO: 51, 125, and 196;
SEQ ID NO: 27, 101, and 172;
SEQ ID NO: 28, 102, and 173;
SEQ ID NO: 43, 117, and 188;
SEQ ID NO: 29, 103, and 174;
SEQ ID NO: 10, 84, and 155;
SEQ ID NO: 30, 104, and 175;
SEQ ID NO: 31, 105, and 176;
SEQ ID NO: 32, 106, and 177;
SEQ ID NO: 66, 137, and 208;
SEQ ID NO: 33, 107, and 178;
SEQ ID NO: 52, 126, and 197;
SEQ ID NO: 34, 108, and 179; or
SEQ ID NO: 2, 76, and 147.

122. Use of a polynucleic acid, molecular marker (allele), QTL, or SNP as defined in any of statements 1 to 79, for identifying or selecting a plant or plant part of the Brassicaceae family.

123. Use of a polynucleic acid, molecular marker (allele), QTL, or SNP as defined in any of statements 1 to 79, for generating a plant or plant part of the *Brassicaceae* family.

124. The method according to any of statements 1 to 79, wherein said polynucleic acid is comprised in a chromosomal interval spanning at most 10 cM respectively upstream and downstream of molecular marker (allele) ra74856s01 and ra36444s01.

125. The method according to any of statements 1 to 79, wherein said polynucleic acid is comprised in a chromosomal interval spanning at most 5 cM respectively upstream and downstream of molecular marker (allele) ra74856s01 and ra36444s01.

126. The method according to any of statements 1 to 79, wherein said polynucleic acid is comprised in a chromosomal interval spanning at most 2 cM respectively upstream and downstream of molecular marker (allele) ra74856s01 and ra36444s01.

127. A method for identifying a plant or plant part of the *Brassicaceae* family having (increased) tolerance or resistance to infection with a parasite from the genus *Plasmodiophora (brassicae)*, the method comprising

a) detecting at least one molecular marker (allele) selected from the group consisting of ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01 (in the genome of the plant or plant part); and

b) identifying a plant comprising the at least one molecular marker (allele), thereby identifying a Brassica plant having (increased) tolerance or resistance to infection with a parasite from the genus *Plasmodiophora (brassicae)*, such as in comparison to a plant or plant part of the Brassicaceae family not having said polynucleotide, molecular marker (allele), locus (allele), QTL (allele), or SNP (allele) of the invention.

128. A method for identifying a plant or plant part of the *Brassicaceae* family having (increased) tolerance or resistance to infection with a parasite from the genus *Plasmodiophora (brassicae)*, the method comprising

a) detecting at least one molecular marker (allele) selected from the group consisting of ra36430s01, ra36433s01, ra36434s01, ra74862s01, ra36435s01, ra74863s01, ra74864s01, ra74910s01, ra74865s01, ra74952s01, ra36441s01, ra74867s01, and ra36443s01 (in the genome of the plant or plant part); and

b) identifying a plant comprising the at least one molecular marker (allele), thereby identifying a Brassica plant having (increased) tolerance or resistance to infection with a parasite from the genus *Plasmodiophora (brassicae)*, such as in comparison to a plant or plant part of the Brassicaceae family not having said polynucleotide, molecular marker (allele), locus (allele), QTL (allele), or SNP (allele) of the invention.

129. A method for identifying a plant or plant part of the *Brassicaceae* family having (increased) tolerance or resistance to infection with a parasite from the genus *Plasmodiophora (brassicae)*, the method comprising

a) detecting at least one molecular marker (allele) selected from the group consisting of ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01 (in the genome of the plant or plant part); and

b) identifying a plant comprising the at least one molecular marker (allele), thereby identifying a *Brassica* plant having (increased) tolerance or resistance to infection with a parasite from the genus *Plasmodiophora (brassicae)*, such as in comparison to a plant or plant part of the Brassicaceae family not having said polynucleotide, molecular marker (allele), locus (allele), QTL (allele), or SNP (allele) of the invention.

130. A method for identifying a plant or plant part of the *Brassicaceae* family having (increased) tolerance or resistance to infection with a parasite from the genus *Plasmodiophora (brassicae)*, the method comprising

a) detecting at least one molecular marker (allele) selected from the group consisting of ra74862s01, ra74863s01, ra74864s01, ra74910s01, ra74865s01, ra74952s01, and ra74867s01 (in the genome of the plant or plant part); and

b) identifying a plant comprising the at least one molecular marker (allele), thereby identifying a *Brassica* plant having (increased) tolerance or resistance to infection with a parasite from the genus *Plasmodiophora (brassicae)*, such as in comparison to a plant or plant part of the Brassicaceae family not having said polynucleotide, molecular marker (allele), locus (allele), QTL (allele), or SNP (allele) of the invention.

131. A method for identifying a plant or plant part of the *Brassicaceae* family having (increased) tolerance or resistance to infection with a parasite from the genus *Plasmodiophora (brassicae)*, the method comprising

a) detecting at least one molecular marker (allele) selected from the group consisting of ra74862s01 and ra74867s01 (in the genome of the plant or plant part); and

b) identifying a plant comprising the at least one molecular marker (allele), thereby identifying a *Brassica* plant having (increased) tolerance or resistance to infection with a parasite from the genus *Plasmodiophora (brassicae),* such as in comparison to a plant or plant part of the Brassicaceae family not having said polynucleotide, molecular marker (allele), locus (allele), QTL (allele), or SNP (allele) of the invention.

## DESCRIPTION OF THE DRAWINGS

**Figure 1:** Oilseed rape roots with different degrees of cell proliferation (clubs on the roots).

**Figure 2:** Yield of different *Brassica napus* varieties, among which "Mentor" and "Archimedes" which are resistant to clubroot. The mean of the absolute grain yield of different varieties (Verrechnungs Sorten (VRS)) is determined for different fields.

**Figure 3:** QTL Mapping in BC3S1 (back cross 3 self fertilized) generation with a LOD score of 26 on chromosome A06 of *Brassica napus.*

**Figure 4** Fine mapping in BC3S1 generation of QTL region on chromosome A06 of *Brassica napus,* the 220.000 bp target is marked. Light marked allele calls are in the target region. Dark grey marked allele calls show a phenotypic score marking the end of the target region.

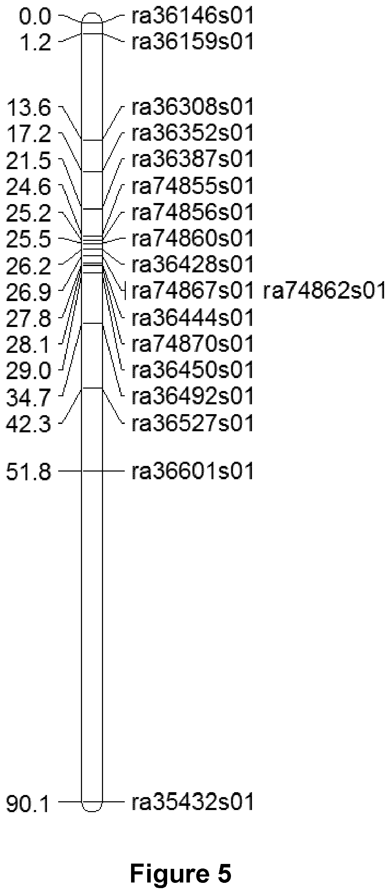

**Figure 5:** SNP position of the markers in reference to the Darmor v4.1 genome and the corresponding position in a genetic map in cM.

## DETAILED DESCRIPTION OF THE INVENTION

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of", as well as the terms "consisting essentially of", "consists essentially" and "consists essentially of". The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-20% or less, preferably +/-10% or less, more preferably +/-5% or less, and still more preferably +/-1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any $\geq3$, $\geq4$, $\geq5$, $\geq6$ or $\geq7$ etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

Standard reference works setting forth the general principles of recombinant DNA technology include Molecular Cloning: A Laboratory Manual, 2nd ed., vol. 1-3, ed. Sambrook et al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates) ("Ausubel et al., 1992"); the series Methods in Enzymology (Academic Press, Inc.); Innis et al., PCR Protocols: A Guide to Methods and Applications, Academic Press: San Diego, 1990; PCR 2: A Practical Approach (M.J. MacPherson, B.D. Hames and G.R. Taylor eds. (1995); Harlow and Lane, eds. (1988) Antibodies, a Laboratory Manual; and Animal Cell Culture (R.I. Freshney, ed. (1987). General principles of microbiology are set forth, for example, in Davis, B. D. et al., Microbiology, 3rd edition, Harper & Row, publishers, Philadelphia, Pa. (1980).

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the invention, and form different embodiments, as would be understood by those in the art. For example, in the appended claims, any of the claimed embodiments can be used in any combination.

In the following detailed description of the invention, reference is made to the accompanying drawings that form a part hereof, and in which are shown by way of illustration only of specific embodiments in which the invention may be practiced. It is to be understood that other embodiments may be utilised, and structural or logical changes may be made without departing from the scope of the present invention. The following detailed description, therefore, is not to be taken in a limiting sense, and the scope of the present invention is defined by the appended claims.

Preferred statements (features) and embodiments of this invention are set herein below. Each of the statements and embodiments of the invention so defined may be combined with any other statement and/or embodiment unless clearly indicated to the contrary. In particular, any feature indicated as being preferred or advantageous may be combined with any other feature or features or statements indicated as being preferred or advantageous.

The term "plant" includes whole plants, including descendants or progeny thereof. As used herein unless clearly indicated otherwise, the term "plant" intends to mean a plant at any developmental stage. The term "plant part" includes any part or derivative of the plant, including particular plant tissues or structures, plant cells, plant protoplast, plant cell or tissue culture from which plants can be regenerated, plant calli, plant clumps and plant cells that are intact in plants or parts of plants, such as seeds, kernels, cobs, flowers, cotyledons, leaves, stems, buds, roots, root tips, stover, and the like. Plant parts may include processed plant parts or derivatives, including flower, oils, extracts etc. "Parts of a plant" are e.g., shoot vegetative organs/structures, e.g., leaves, stems and tubers; roots, flowers and floral organs/structures, e.g., bracts, sepals, petals, stamens, carpels, anthers and ovules; seed, including embryo, endosperm, and seed coat; fruit and the mature ovary; plant tissue, e.g., vascular tissue, ground tissue, and the like; and cells, e.g., guard cells, egg cells, pollen, trichomes and the like; and progeny of the same. Parts of plants may be attached to or separate from a whole intact plant. Such parts of a plant include, but are not limited to, organs, tissues, and cells of a plant, and preferably seeds. A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant. "Plant cell culture" means cultures of plant units such as, for example, protoplasts, cell culture cells, cells in plant tissues, pollen, pollen tubes, ovules, embryo sacs, zygotes and embryos at various stages of development. "Plant material" refers to leaves, stems, roots, flowers or flower parts, fruits, pollen, egg cells, zygotes, seeds, cuttings, cell or tissue cultures, or any other part or product of a plant. This also includes callus or callus tissue as well as extracts (such as extracts from taproots) or samples. A "plant organ" is a distinct and visibly structured and differentiated part of a plant such as a root, stem, leaf, flower bud, or embryo. "Plant tissue" as used herein means a group of plant cells organized into a structural and functional unit. Any tissue of a plant in planta or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue. In certain embodiments, the plant part is a plant organ, tissue, or cell. In certain embodiments, the plant part is seed, pollen, oocyte, protoplast, inflorescence, embryo, or callus.

In certain embodiments, the plant part or derivative is (functional) propagation material, such as germplasm, a seed, or plant embryo or other material from which a plant can be regenerated. In certain embodiments, the plant part or derivative comprises (functional) male and/or female reproductive organs.

In certain embodiments, the plant part or derivative is not (functional) propagation material, such as germplasm, a seed, or plant embryo or other material from which a plant can be regenerated. In certain embodiments, the plant part or derivative does not comprise (functional) male and female reproductive organs. In certain embodiments, the plant part or derivative is or comprises propagation material, but propagation material which does not or cannot be used (anymore) to produce or generate new plants, such as propagation material which have been chemically, mechanically or otherwise rendered non-functional, for instance by heat treatment, acid treatment, compaction, crushing, chopping, etc.

As used herein unless clearly indicated otherwise, the term "plant" intended to mean a plant at any developmental stage.

In certain embodiments, the plant is a crop plant, such as a cash crop, fodder crop, or subsistence crop, such as food or non-food crops, including agriculture, horticulture, floriculture, or industrial crops. The term crop plant has its ordinary meaning as known in the art. By means of further guidance, and without limitation, a crop plant is a plant grown by humans for human or animal food and other resources and can be grown and harvested extensively for profit or subsistence, typically in an agricultural setting or context. In certain embodiments, the plant or plant part is from a (crop) plant of the *Brassicaceae* family. In certain embodiments, the plant or plant part is from a (crop) plant of the *Brassiceae* tribe. In certain embodiments, the plant or plant part is from a (crop) plant of the *Brassica* genus.

As used herein, the term *Brassicaceae* refers to the family of *Brassicaceae,* also known as crucifers or the mustard or cabbage family.

In certain embodiments, the plants or plant parts as referred to herein are from the genus *Brassica* or the genus *Raphanus.* In certain embodiments, the plants or plant parts as referred to herein are from the species *Brassica balearica, Brassica carinata, Brassica elongate, Brassica fruticulose, Brassica hilarionis, Brassica juncea, Brassica napus, Brassica narinosa, Brassica nigra, Brassica oleracea, Brassica perviridis, Brassica rapa (syn. B. campestris), Brassica rupestris, Brassica spinescens, Brassica tournefortii, Brassica alba, Brassica hirta, Brassica geniculate, Brassica kaber,* and *Raphanus sativus.* The skilled person will understand that all lines, cultivars, and varieties of the recited species may be included. However, in certain embodiments, the plant or plant part as referred to herein is not a plant variety, line, or cultivar.

In certain preferred embodiments, the plants or plant parts as referred to herein are from the species *Brassica napus* or *Brassica rapa.*

In certain embodiments, the plants or plant parts as referred to herein are hybrids between different species from the *Brassicaceae* family. In certain embodiments, the plants or plant parts as referred to herein are hybrids between different species from the *Brassica* genus. In certain embodiments, the plants or plant parts as referred to herein are hybrids between different species selected from *Brassica balearica, Brassica carinata, Brassica elongate, Brassica fruticulose, Brassica hilarionis, Brassica juncea, Brassica napus, Brassica narinosa, Brassica nigra, Brassica oleracea, Brassica perviridis, Brassica rapa (syn. B. campestris), Brassica rupestris, Brassica spinescens, Brassica tournefortii, Brassica alba, Brassica hirta, Brassica geniculate, Brassica kaber,* and *Raphanus sativus.* In certain embodiments, the plants or plant parts as referred to herein are hybrids between *Brassica napus* and *Brassica rapa.*

As used herein, the term "plant (part) population" may be used interchangeably with population of plants or plant parts. A plant (part) population preferably comprises a multitude of individual plants (or plant parts thereof), such as preferably at least 10, such as 20, 30, 40, 50, 60, 70, 80, or 90, more preferably at least 100, such as 200, 300, 400, 500, 600, 700, 800, or 900, even more preferably at least 1000, such as at least 10000 or at least 100000.

In certain embodiments, the plant population (or plant parts thereof) is a plant line, strain, or variety. In certain embodiments, the plant population (or plant parts thereof) is not a plant line, strain, or variety. In certain embodiments, the plant population (or plant parts thereof) is an inbred plant line, strain, or variety. In certain embodiments, the plant population (or plant parts thereof) is not an inbred plant line, strain, or variety. In certain embodiments, the plant population (or plant parts thereof) is an outbred plant line, strain, or variety. In certain embodiments, the plant population (or plant parts thereof) is not an outbred plant line, strain, or variety.

The term "elite line" refers to any line that has resulted from breeding and selection for superior agronomic performance. An elite plant is any plant from an elite line.

As used herein, the terms "progeny" and "progeny plant" refer to a plant generated from vegetative or sexual reproduction from one or more parent plants. In gynogenesis-mediated haploid induction, the haploid embryo on the female parent comprises female chromosomes to the exclusion of male chromosomes-thus it is not a progeny of the male haploid-inducing line. The haploid seed typically still has normal triploid endosperm that contains the male genome. The edited haploid progeny and subsequent edited doubled haploid plants and subsequent seed is not the only desired progeny. There is also the seed from the haploid inducer line itself, often carrying the Cas9 transgene, and subsequent plant and seed progeny of the haploid inducing plant. Both the haploid seed and the haploid inducer (self-pollination-derived) seed can be progeny. A progeny plant can be obtained by cloning or selfing a single parent plant, or by crossing two or more parental plants. For instance, a progeny plant can be obtained by cloning or selfing of a parent plant or by crossing two parental plants and include selfings as well as the F1 or F2 or still further generations. An F1 is a first-generation progeny produced from parents at least one of which is used for the first time as donor of a trait, while progeny of second generation (F2) or subsequent generations (F3, F4, and the like) are specimens produced from selfings, intercrosses, backcrosses, and/or other crosses of F1s, F2s, and the like. An F1 can thus be (and in some embodiments is) a hybrid resulting from a cross between two true breeding parents (i.e., parents that are true-breeding are each homozygous for a trait of interest or an allele thereof), while an F2 can be (and in some embodiments is) a progeny resulting from self-pollination of the F1 hybrids. The term "progeny" can in certain embodiments be used interchangeably with "offspring", in particular when the plant or plant material is derived from sexual crossing of parent plants.

Preferably, in crosses between a first plant and a second plant, both plants are from the same genus, preferably from the same species. However, interspecies crosses are also possible.

An "amplicon" is amplified nucleic acid, e.g., a nucleic acid that is produced by amplifying a template nucleic acid by any available amplification method (e.g., PCR, LCR, transcription, or the like).

The term "amplifying" in the context of nucleic acid amplification is any process whereby additional copies of a selected nucleic acid for a transcribed form thereof are produced. Typical amplification methods include various polymerase-based replication methods, including the polymerase chain reaction (PCR), ligase mediated methods such as the ligase chain reaction (LCR) and RNA polymerase-based amplification (e.g., by transcription) methods.

The term "locus" (loci plural) means a specific place or places or a site on a chromosome where for example a QTL, a gene or genetic marker is found. As used herein, the term "quantitative trait locus" or "QTL" has its ordinary meaning known in the art. By means of further guidance, and without limitation, a QTL may refer to a region of DNA that is associated with the differential expression of a quantitative phenotypic trait in at least one genetic background, e.g., in at least one breeding population. The region of the QTL encompasses or is closely linked to the gene or genes that affect the trait in question.

An "allele of a QTL" can comprise multiple genes or other genetic factors within a contiguous genomic region or linkage group, such as a haplotype. An allele of a QTL can denote a haplotype within a specified window wherein said window is a contiguous genomic region that can be defined, and tracked, with a set of one or more polymorphic markers. A haplotype can be defined by the unique fingerprint of alleles at each marker within the specified window. A QTL may encode for one or more alleles that affect the expressivity of a continuously distributed (quantitative) phenotype. In certain embodiments, the QTL as described herein may be homozygous. In certain embodiments, the QTL as described herein may be heterozygous.

As used herein, the term "allele" or "alleles" refers to one or more alternative forms, i.e., different nucleotides or nucleotide sequences, of a locus, such as a gene, marker, QTL, etc.

A "favourable allele" is the allele at a particular locus that confers, or contributes to, a desirable phenotype, e.g., increased clubroot disease resistance as described somewhere else herein, or alternatively, is an allele that allows the identification of plants with decreased clubroot disease resistance that can be removed from a breeding program or planting ("counter-selection"). A favourable allele of a marker is a marker allele that segregates with the favourable phenotype, or alternatively, segregates with the unfavourable plant phenotype, therefore providing the benefit of identifying plants.

As used herein, the term "mutant alleles" or "mutation" of alleles include alleles having one or more mutations, such as insertions, deletions, stop codons, base changes (e.g., transitions or transversions), or alterations in splice junctions, which may or may not give rise to altered gene products. Modifications in alleles may arise in coding or non-coding regions (e.g., promoter regions, exons, introns or splice junctions).

The term "mutation" or "mutated" as used herein refers to a gene or protein product thereof which is altered or modified such that the function normally attributed to the gene or protein product thereof is altered, or alternatively such that the expression, stability, and/or activity normally associated with the gene or protein product thereof is altered. Typically, a mutation as referred to herein results in a phenotypic effect, such as clubroot resistance, as described herein elsewhere. It will be understood that a mutation in a gene or protein product thereof is referred to in comparison with a gene or protein product thereof not having such mutation, such as a wild type or endogenous gene or protein product thereof. Typically, a mutation refers to a modification at the DNA level and includes changes in the genetics and/or epigenetics. An alteration in the genetics may include an insertion, a deletion, an introduction of a stop codon, a base change (e.g., transition or transversion), or an alteration in splice junctions. These alterations may arise in coding or non-coding regions (e.g., promoter regions, exons, introns or splice junctions) of the endogenous DNA sequence. For example, an alteration in the genetics may be the exchange (including insertions, deletions) of at least one nucleotide in the endogenous DNA sequence or in a regulatory sequence of the endogenous DNA sequence. If such a nucleotide exchange takes place in a promoter, for example, this may lead to an altered activity of the promoter, since, for example, cis-regulator elements are modified such that the affinity of a transcription factor to the mutated cis-regulatory elements is altered in comparison to the wild-type promoter, so that the activity of the promoter with the mutated cis-regulatory elements is increased or reduced, depending upon whether the transcription factor is a repressor or inductor, or whether the affinity of the transcription factor to the mutated cis-regulatory elements is intensified or weakened. If such a nucleotide exchange occurs, e.g., in an encoding region of the endogenous DNA sequence, this may lead to an amino acid exchange in the encoded protein, which may produce an alteration in the activity or stability of the protein, in comparison to the wild-type protein. An alteration in the epigenetics may take place via an altered methylation pattern of the DNA. In certain embodiments, a mutation as referred to herein relates to the insertion of one or more nucleotides in a gene. In certain embodiments, a mutation as referred to herein relates to the deletion of one or more nucleotides in a gene. In certain embodiments, the mutation as referred to herein relates to the deletion as well as the insertion of one or more nucleotides. In certain embodiments, certain nucleotide stretches, such as for instance encoding a particular protein domain are deleted. In certain embodiments, certain nucleotide stretches, such as for instance encoding a particular protein domain are deleted and replaced by nucleotide sequences encoding a different protein domain. In certain embodiments, a mutation as referred to herein relates to the exchange of one or more nucleotides in a gene by different nucleotides. In certain embodiments, the mutation is a nonsense mutation (i.e., the mutation results in the generation of a stop codon in a protein encoding sequence). In certain embodiments, the mutation is a frameshift mutation (i.e., an insertion or deletion of one or more nucleotides (not equal to three or a product thereof) in a protein encoding sequence). In certain embodiments, the mutation results in a truncated protein product. In certain embodiments, the mutation results in an N-terminally truncated protein product. In certain embodiments, the mutation results in a C-terminally truncated protein product. In certain embodiments, the mutation results in an N-terminally and C-terminally truncated protein product. In certain embodiments, the mutation results in an altered splice site (such as an altered splice donor and/or splice acceptor site). In certain embodiments, the mutation is in an exon. In certain embodiments, the mutation is in an intron. In certain embodiments, the mutation is in a regulatory sequence, such as a promoter. In certain embodiments, the mutation results in a codon encoding a different amino acid. In certain embodiments, the mutation results in the insertion or deletion of one or more codons (i.e., nucleotide triplets). In certain embodiments, the mutation results in gene deletion. In certain embodiments, the mutation is a knockout mutation. Both frameshift and nonsense mutations can in certain embodiments be considered as knockout mutations, in particular if the mutation is present in an early exon. A knockout mutation (or loss of function mutation) as used herein preferably means that a functional gene product, such as a functional protein, is not produced anymore. In particular, frameshift and nonsense mutations will lead to premature termination of protein translation, such that a truncated protein will result, which often lacks the required stability and/or activity to perform the function naturally attributed to it. In certain embodiments, the mutation is a knockdown mutation. Knockout mutations often, and according to the present invention preferably are recessive. In contrast to a knockout mutation, a knockdown mutation results in a decreased activity, stability, and/or expression (rate) of the native functional gene product, such as a protein, and thereby ultimately in a decreased functionality. For instance, mutations in promoter regions affecting transcriptional activator binding (or other regulatory sequences), in particular reducing transcription rate, can be considered knockdown mutations. Also, mutations negatively affecting protein stability (such as to increase ubiquitination and subsequent protein degradation) can be considered knockdown mutations). In addition, mutations negatively affecting protein activity (such as binding strength or enzymatic activity) can be considered knockdown mutations. It will be understood that the mutations described herein according to the invention confer haploid inducer or inducing activity or capability or enhance haploid inducer or inducing activity or capability, as described herein elsewhere. While mutation described herein may be non-naturally occurring, this need not necessarily be the case.

A "reference sequence" is a defined sequence used as a basis for sequence comparison. The reference sequence is obtained by genotyping a number of lines at the locus, aligning the nucleotide sequences in a sequence alignment program, and obtaining the consensus sequence of the alignment.

In certain embodiments, the reference (genomic) sequence as referred to herein is the *Brassica napus* Darmor reference genome (version 4.1 (v4.1)) as described for instance in Chalhoub et al. (2014) "Early allopolyploid evolution in the post-Neolithic Brassica napus oilseed genome"; Science 345(6199): 950-953 (DOI: 10.1126/science.1253435). The genome can for instance be consulted on the Brassica napus genome browser https://www.genoscope.cns.fr/brassicanapus/ (in particular https://www.genoscope.cns.fr/brassicanapus/data/)

As used herein, reference to Darmor, Darmor-bzh, Darmor v4.1, and Darmor-bzh v4.1 may be with used interchangeably.

In certain embodiments, the polynucleotide, QTL, locus, or markers as referred to herein are homozygous. In certain embodiments, the polynucleotide, QTL, locus, or markers as referred to herein are heterozygous.

Homozygosity may be achieved by breeding and selection, such as crossing heterozygous plants and selecting homozygous offspring, such as backcrossing and selecting for homozygosity. Also, homozygosity may be achieved by (recombinant) introduction of the polynucleotide, QTL, locus, or markers of the invention gene simultaneously or sequentially on both homologous chromosomes. Alternatively, homozygosity may be achieved by doubled haploid technology.

A doubled haploid plant or plant part is one that is developed by the doubling of a haploid set of chromosomes. A plant or seed that is obtained from a doubled haploid plant that is selfed any number of generations may still be identified as a doubled haploid plant. A doubled haploid plant is considered a homozygous plant. A plant is considered to be doubled haploid if it is fertile, even if the entire vegetative part of the plant does not consist of the cells with the doubled set of chromosomes. For example, a plant will be considered a doubled haploid plant if it contains viable gametes, even if it is chimeric. Somatic haploid cells, haploid embryos, haploid seeds, or haploid seedlings produced from haploid seeds can be treated with a chromosome doubling agent. Homozygous plants can be regenerated from haploid cells by contacting the haploid cells, such as embryo cells or callus produced from such cells, with chromosome doubling agents, such as colchicine, pronamide, dithipyr, trifluralin, or another known anti-microtubule agent or anti-microtubule herbicide, or nitrous oxide to create homozygous doubled haploid cells. Treatment of a haploid seed or the resulting seedling generally produces a chimeric plant, partially haploid and partially doubled haploid. It may be beneficial to nick the seedling before treatment with colchicine. When reproductive tissue contains doubled haploid cells, then doubled haploid seed is produced.

In certain embodiments, a wild type/endogenous allele is replaced by a clubroot resistance allele of the invention, preferably all wild type/endogenous alleles are replaced by a clubroot resistance allele of the invention. Replacement can be effected by any means known in the art, as also described herein elsewhere. Replacement, as used herein also includes (direct) mutagenesis of the wild type/endogenous allele(s) at its native genomic locus. Accordingly, in certain embodiments, a wild type/endogenous allele is mutated, as described herein elsewhere, optionally all wild type/endogenous alleles are mutated. The skilled person will understand that only one copy of a wild type/endogenous allele may be mutated, and that homozygosity (if so desired) may be obtained by selfing and subsequent selection. In certain embodiments, a reduced number of wild type/endogenous alleles is present (i.e., the wild type/endogenous allele is heterozygous).

According to the invention, mutations as described herein may be constitutive or conditional or inducible, and/or may be (multiple or single) tissue, organ, or cell (type) specific. The skilled person has ample knowledge how to implement such (differential) mutagenesis.

Mutations as described herein may be introduced by mutagenesis, which may be performed in accordance with any of the techniques known in the art. As used herein, "mutagenization", "mutagenized" or "mutagenesis" includes both conventional mutagenesis and location-specific mutagenesis or "genome editing" or "gene editing". In conventional mutagenesis, modification at the DNA level is not produced in a targeted manner. The plant cell or the plant is exposed to mutagenic conditions, such as TILLING, via UV light exposure or the use of chemical substances (Till et al., 2004). An additional method of random mutagenesis is mutagenesis with the aid of a transposon. Location-specific mutagenesis enables the introduction of modification at the DNA level in a target-oriented manner at predefined locations in the DNA. For example, TALENS, meganucleases, homing endonucleases, zinc finger nucleases, or a CRISPR/Cas system as further described herein may be used for this.

In certain embodiments, a wild type/endogenous allele is knocked out, optionally all wild type/endogenous alleles are knocked out, and a polynucleotide, QTL, locus, marker, or allele as described herein is transgenically introduced, transiently or genomically integrated, preferably genomically integrated. In certain embodiments, a wild type/endogenous allele is knocked out, optionally all wild type/endogenous alleles are knocked out, and is transgenically replaced by a polynucleotide, QTL, locus, marker, or allele as described herein (at the native genomic location of the wild type allele). The skilled person will understand that only one copy of a wild type/endogenous allele may be knocked out and that homozygosity (if so desired) may be obtained by selfing and subsequent selection.

In certain embodiments, the mutations as described herein, are or result in amino acid substitutions (compared to the wild type or unmutated protein, gene, or coding sequence). In certain embodiments, the mutation is a point mutation. Preferably, the mutation is a missense mutation (i.e., the mutation results in a codon encoding a different amino acid). In certain embodiments one or more mutations are present. In certain embodiments, from 1 to 10 mutations are present. In certain embodiments, from 1 to 9 mutations are present. In certain embodiments, from 1 to 8 mutations are present. In certain embodiments, from 1 to 7 mutations are present. In certain embodiments, from 1 to 6 mutations are present. In certain embodiments, from 1 to 5 mutations are present. In certain embodiments, from 1 to 4 mutations are present. In certain embodiments, from 1 to 3 mutations are present. In certain embodiments, from 1 to 2 mutations are present. In certain embodiments, 1 mutation is present. In certain embodiments, from 1 to 10 amino acid substitutions are present in the mutated protein. In certain embodiments, from 1 to 9 amino acid substitutions are present in the mutated protein. In certain embodiments, from 1 to 8 amino acid substitutions are present in the mutated protein. In certain embodiments, from 1 to 7 amino acid substitutions are present in the mutated protein. In certain embodiments, from 1 to 6 amino acid substitutions are present in the mutated protein. In certain embodiments, from 1 to 5 amino acid substitutions are present in the mutated protein. In certain embodiments, from 1 to 4 amino acid substitutions are present in the mutated protein. In certain embodiments, from 1 to 3 amino acid substitutions are present in the mutated protein. In certain embodiments, from 1 to 2 amino acid substitutions are present in the mutated protein. In certain embodiments, 1 amino acid substitution is present in the mutated protein. In certain embodiments, from 1 to 10 point mutations, preferably missense mutations, are present in the mutated gene, allele, or coding sequence. In certain embodiments, from 1 to 9 point mutations, preferably missense mutations, are present in the mutated gene, allele, or coding sequence. In certain embodiments, from 1 to 8 point mutations, preferably missense mutations, are present in the mutated gene, allele, or coding sequence. In certain embodiments, from 1 to 7 point mutations, preferably missense mutations, are present in the mutated gene, allele, or coding sequence. In certain embodiments, from 1 to 6 point mutations, preferably missense mutations, are present in the mutated gene, allele, or coding sequence. In certain embodiments, from 1 to 5 point mutations, preferably missense mutations, are present in the mutated gene, allele, or coding sequence. In certain embodiments, from 1 to 4 point mutations, preferably missense mutations, are present in the mutated gene, allele, or coding sequence. In certain embodiments, from 1 to 3 point mutations, preferably missense mutations, are present in the mutated gene, allele, or coding sequence. In certain embodiments, from 1 to 2 point mutations, preferably missense mutations, are present in the mutated gene, allele, or coding sequence. In certain embodiments, 1 point mutation, preferably missense mutation, is present in the mutated gene, allele, or coding sequence.

As used herein, the terms "introgression", "introgressed" and "introgressing" refer to both a natural and artificial process whereby chromosomal fragments or genes of one species, variety or cultivar are moved into the genome of another species, variety or cultivar, by crossing those species. The process may optionally be completed by backcrossing to the recurrent parent. For example, introgression of a desired allele at a specified locus can be transmitted to at least one progeny via a sexual cross between two parents of the same species, where at least one of the parents has the desired allele in its genome. Alternatively, for example, transmission of an allele can occur by recombination between two donor genomes, e.g., in a fused protoplast, where at least one of the donor protoplasts has the desired allele in its genome. The desired allele can be, e.g., detected by a marker that is associated with a phenotype, at a QTL, a transgene, or the like. In any case, offspring comprising the desired allele can be repeatedly backcrossed to a line having a desired genetic background and selected for the desired allele, to result in the allele becoming fixed in a selected genetic background. The process of "introgressing" is often referred to as "backcrossing" when the process is repeated two or more times. "Introgression fragment" or "introgression segment" or "introgression region" refers to a chromosome fragment (or chromosome part or region) which has been introduced into another plant of the same or related species either artificially or naturally such as by crossing or traditional breeding techniques, such as backcrossing, i.e., the introgressed fragment is the result of breeding methods referred to by the verb "to introgress" (such as backcrossing). It is understood that the term "introgression fragment" never includes a whole chromosome, but only a part of a chromosome. The introgression fragment can be large, e.g., even three quarter or half of a chromosome, but is preferably smaller, such as about 15 Mb or less, such as about 10 Mb or less, about 9 Mb or less, about 8 Mb or less, about 7 Mb or less, about 6 Mb or less, about 5 Mb or less, about 4 Mb or less, about 3 Mb or less, about 2.5 Mb or 2 Mb or less, about 1 Mb (equals 1,000,000 base pairs) or less, or about 0.5 Mb (equals 500,000 base pairs) or less, such as about 200,000 bp (equals 200 kilo base pairs) or less, about 100,000 bp (100 kb) or less, about 50,000 bp (50 kb) or less, about 25,000 bp (25 kb) or less.

In certain embodiments, the polynucleotide, QTL, locus, marker, or allele of the invention is introduced by introgression.

The term "crossed" or "cross" means the fusion of gametes via pollination to produce progeny (e.g., cells, seeds or plants). The term encompasses both sexual crosses (the pollination of one plant by another) and selfing (self-pollination, e.g., when the pollen and ovule are from the same plant). The term "crossing" refers to the act of fusing gametes via pollination to produce progeny.

"Backcrossing" refers to the process whereby the hybrid progeny is repeatedly crossed back to one of the parents. In a backcrossing scheme, the "donor" parent refers to the parental plant with the desired gene or locus to be introgressed. The "recipient" parent (used one or more times) or "recurrent" parent (used two or more times) refers to the parental plant into which the gene or locus is being introgressed.

A genetic element, an introgression fragment, a QTL or a gene or allele conferring a trait is said to be "obtainable from" or can be "obtained from" or "derivable from" or can be "derived from" or "as present in" or "as found in" a plant or plant part as described herein elsewhere if it can be transferred from the plant in which it is present into another plant in which it is not present (such as a line or variety) using traditional breeding techniques without resulting in a phenotypic change of the recipient plant apart from the addition of the trait conferred by the genetic element, locus, introgression fragment, gene or allele. The terms are used interchangeably and the genetic element, locus, introgression fragment, gene or allele can thus be transferred into any other genetic background lacking the trait. Not only pants comprising the genetic element, locus, introgression fragment, gene or allele can be used, but also progeny/descendants from such plants which have been selected to retain the genetic element, locus, introgression fragment, gene or allele, can be used and are encompassed herein. Whether a plant (or genomic DNA, cell or tissue of a plant) comprises the same genetic element, locus, introgression fragment, gene or allele as obtainable from such plant can be determined by the skilled person using one or more techniques known in the art, such as phenotypic assays, whole genome sequencing, molecular marker analysis, trait mapping, chromosome painting, allelism tests and the like, or combinations of techniques. It will be understood that transgenic plants may also be encompassed.

"Introducing" in the meaning of the present invention includes stable or transient integration by means of transformation including Agrobacterium-mediated transformation, transfection, microinjection, biolistic bombardment, insertion using gene editing technology like CRISPR systems (e.g., CRISPR/Cas, in particular CRISPR/Cas9 or CRISPR/Cas12 ), CRISPR/CasX, or CRISPR/CasY), TALENs, zinc finger nucleases or meganucleases, homologous recombination optionally by means of one of the below mentioned gene editing technology including preferably a repair template, modification of endogenous gene using random or targeted mutagenesis like TILLING or above mentioned gene editing technology, etc.

As used herein the terms "genetic engineering", "transformation" and "genetic modification" are all used herein as synonyms for the transfer of isolated and cloned genes into the DNA, usually the chromosomal DNA or genome, of another organism.

"Transgenic" or "genetically modified organisms" (GMOs) as used herein are organisms whose genetic material has been altered using techniques generally known as "recombinant DNA technology". Recombinant DNA technology encompasses the ability to combine DNA molecules from different sources into one molecule ex vivo (e.g., in a test tube). The term "transgenic" here means genetically modified by the introduction of a non-endogenous nucleic acid sequence. Typically, a species-specific nucleic acid sequence is introduced in a form, arrangement, or quantity into the cell in a location where the nucleic acid sequence does not occur naturally in the cell. This terminology generally does not cover organisms whose genetic composition has been altered by conventional cross-breeding or by "mutagenesis" breeding, as these methods predate the discovery of recombinant DNA techniques. "Non-transgenic" as used herein refers to plants and food products derived from plants that are not "transgenic" or "genetically modified organisms" as defined above.

"Transgene", "exogene", or "chimeric gene" refers to a genetic locus comprising a DNA sequence, such as a recombinant gene, which has been introduced into the genome of a plant by transformation, such as *Agrobacterium* mediated transformation. A plant comprising a transgene stably integrated into its genome is referred to as "transgenic plant".

"Gene editing" or "genome editing" refers to genetic engineering in which in which DNA or RNA is inserted, deleted, modified or replaced in the genome of a living organism. Gene editing may comprise targeted or non-targeted (random) mutagenesis. Targeted mutagenesis may be accomplished for instance with designer nucleases, such as for instance with meganucleases, zinc finger nucleases (ZFNs), transcription activator-like effector-based nucleases (TALEN), and the clustered regularly interspaced short palindromic repeats (CRISPR/Cas) system. These nucleases create site-specific double-strand breaks (DSBs) at desired locations in the genome. The induced double-strand breaks are repaired through nonhomologous end-joining (NHEJ) or homologous recombination (HR), resulting in targeted mutations or nucleic acid modifications, such as introduction of the polynucleotide, QTL, locus, marker, or allele as described herein. The use of designer nucleases is particularly suitable for generating gene knockouts or knockdowns. In certain embodiments, designer nucleases are developed which specifically introduce a polynucleic acid, QTL, locus, marker, or induce a mutation, as described herein elsewhere, such as to generate a mutation or a knockout or knockin of a gene. Alternatively, by means of for instance RNA-specific CRISPR/Cas systems, a knockdown can be achieved, as RNA/specific CRISPR/Cas systems (such as Cas13) allow site-directed cleavage of (single-stranded) RNA. Accordingly, in certain embodiments, designer nucleases, in particular RNA-specific CRISPR/Cas systems are developed which specifically target the mRNA, such as to cleave mRNA and generate a knockdown of the gene/mRNA/protein. Delivery and expression systems of designer nuclease systems are well known in the art.

In certain embodiments, the nuclease or targeted/site-specific/homing nuclease is, comprises, consists essentially of, or consists of a (modified) CRISPR/Cas system or complex, a (modified) Cas protein, a (modified) zinc finger, a (modified) zinc finger nuclease (ZFN), a (modified) transcription factor-like effector (TALE), a (modified) transcription factor-like effector nuclease (TALEN), or a (modified) meganuclease. In certain embodiments, said (modified) nuclease or targeted/site-specific/homing nuclease is, comprises, consists essentially of, or consists of a (modified) RNA-guided nuclease. It will be understood that in certain embodiments, the nucleases may be codon optimized for expression in plants. As used herein, the term "targeting" of a selected nucleic acid sequence means that a nuclease or nuclease complex is acting in a nucleotide sequence specific manner. For instance, in the context of the CRISPR/Cas system, the guide RNA is capable of hybridizing with a selected nucleic acid sequence. As uses herein, "hybridization" or "hybridizing" refers to a reaction in which one or more polynucleotides react to form a complex that is stabilized via hydrogen bonding between the bases of the nucleotide residues, i.e., a process in which a single-stranded nucleic acid molecule attaches itself to a complementary nucleic acid strand, i.e., agrees with this base pairing. Standard procedures for hybridization are described, for example, in Sambrook et al., (Molecular Cloning. A Laboratory Manual, Cold Spring Harbor Laboratory Press, 3rd edition 2001). The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PGR, or the cleavage of a polynucleotide by an enzyme. A sequence capable of hybridizing with a given sequence is referred to as the "complement" of the given sequence. Preferably this will be understood to mean an at least 50%, more preferably at least 55%, 60%, 65%, 70%, 75%, 80% or 85%, more preferably 90%, 91%, 92%, 93%, 94%, 95%, 96%, 97%, 98% or 99% of the bases of the nucleic acid strand form base pairs with the complementary nucleic acid strand. The possibility of such binding depends on the stringency of the hybridization conditions.

Gene editing may involve transient, inducible, or constitutive expression of the gene editing components or systems. Gene editing may involve genomic integration or episomal presence of the gene editing components or systems. Gene editing components or systems may be provided on vectors, such as plasmids, which may be delivered by appropriate delivery vehicles, as is known in the art. Preferred vectors are expression vectors.

Gene editing may comprise the provision of recombination templates, to effect homology directed repair (HDR). For instance, a genetic element may be replaced by gene editing in which a recombination template is provided. The DNA may be cut upstream and downstream of a sequence which needs to be replaced. As such, the sequence to be replaced is excised from the DNA. Through HDR, the excised sequence is then replaced by the template. In certain embodiments, the QTL allele of the invention as described herein may be provided on/as a template. By designing the system such that double strand breaks are introduced upstream and downstream of the corresponding region in the genome of a plant, this region is excised and can be replaced with the template comprising the polynucleotide, QTL, locus, marker, or allele as described herein. In this way, introduction of the polynucleotide, QTL, locus, marker, or allele as described herein in a plant need not involve multiple backcrossing, in particular in a plant of specific genetic background.

Similarly, the polynucleic acid of the invention may be provided on/as a template. More advantageously however, the polynucleic acid of the invention may be generated without the use of a recombination template, but solely through the endonuclease action leading to a double strand DNA break which is repaired by NHEJ, resulting in the generation of indels.

In certain embodiments, the nucleic acid modification, such as introduction of the polynucleotide, QTL, locus, marker, or allele as described herein, or mutation is effected by a (modified) transcription activator-like effector nuclease (TALEN) system. Transcription activator-like effectors (TALEs) can be engineered to bind practically any desired DNA sequence. Exemplary methods of genome editing using the TALEN system can be found for example in Cermak T. Doyle EL., Christian M., Wang L., Zhang Y., Schmidt C, et al., Efficient design and assembly of custom TALEN and other TAL effector-based constructs for DNA targeting. Nucleic Acids Res. 2011;39:e82; Zhang F., Cong L., Lodato S., Kosuri S., Church GM., Arlotta P, Efficient construction of sequence-specific TAL effectors for modulating mammalian transcription. Nat Biotechnol. 2011;29:149-153 and US Patent Nos. 8,450,471, 8,440,431 and 8,440,432, all of which are specifically incorporated by reference. By means of further guidance, and without limitation, naturally occurring TALEs or "wild type TALEs" are nucleic acid binding proteins secreted by numerous species of proteobacteria. TALE polypeptides contain a nucleic acid binding domain composed of tandem repeats of highly conserved monomer polypeptides that are predominantly 33, 34 or 35 amino acids in length and that differ from each other mainly in amino acid positions 12 and 13. In advantageous embodiments the nucleic acid is DNA. As used herein, the term "polypeptide monomers", or "TALE monomers" will be used to refer to the highly conserved repetitive polypeptide sequences within the TALE nucleic acid binding domain and the term "repeat variable di-residues" or "RVD" will be used to refer to the highly variable amino acids at positions 12 and 13 of the polypeptide monomers. As provided throughout the disclosure, the amino acid residues of the RVD are depicted using the IUPAC single letter code for amino acids. A general representation of a TALE monomer which is comprised within the DNA binding domain is X1-11-(X12X13)-X14-33 or 34 or 35, where the subscript indicates the amino acid position and X represents any amino acid. X12X13 indicate the RVDs. In some polypeptide monomers, the variable amino acid at position 13 is missing or absent and in such polypeptide monomers, the RVD consists of a single amino acid. In such cases the RVD may be alternatively represented as X*, where X represents X12 and (*) indicates that X13 is absent. The DNA binding domain comprises several repeats of TALE monomers and this may be represented as (X1-11-(X12X13)-X14-33 or 34 or 35)z, where in an advantageous embodiment, z is at least 5 to 40. In a further advantageous embodiment, z is at least 10 to 26. The TALE monomers have a nucleotide binding affinity that is determined by the identity of the amino acids in its RVD. For example, polypeptide monomers with an RVD of NI preferentially bind to adenine (A), polypeptide monomers with an RVD of NG preferentially bind to thymine (T), polypeptide monomers with an RVD of HD preferentially bind to cytosine (C) and polypeptide monomers with an RVD of NN preferentially bind to both adenine (A) and guanine (G). In yet another embodiment of the invention, polypeptide monomers with an RVD of IG preferentially bind to T. Thus, the number and order of the polypeptide monomer repeats in the nucleic acid binding domain of a TALE determines its nucleic acid target specificity. In still further embodiments of the invention, polypeptide monomers with an RVD of NS recognize all four base pairs and may bind to A, T, G or C. The structure and function of TALEs is further described in, for example, Moscou et al., Science 326:1501 (2009); Boch et al., Science 326:1509-1512 (2009); and Zhang et al., Nature Biotechnology 29:149-153 (2011), each of which is incorporated by reference in its entirety.

In certain embodiments, the nucleic acid modification, such as introduction of the polynucleotide, QTL, locus, marker, or allele as described herein, or mutation is effected by a (modified) zinc-finger nuclease (ZFN) system. The ZFN system uses artificial restriction enzymes generated by fusing a zinc finger DNA-binding domain to a DNA-cleavage domain that can be engineered to target desired DNA sequences. Exemplary methods of genome editing using ZFNs can be found for example in U.S. Patent Nos. 6,534,261, 6,607,882, 6,746,838, 6,794,136, 6,824,978, 6,866,997, 6,933,113, 6,979,539, 7,013,219, 7,030,215, 7,220,719, 7,241,573, 7,241,574, 7,585,849, 7,595,376, 6,903,185, and 6,479,626, all of which are specifically incorporated by reference. By means of further guidance, and without limitation, artificial zinc-finger (ZF) technology involves arrays of ZF modules to target new DNA-binding sites in the genome. Each finger module in a ZF array targets three DNA bases. A customized array of individual zinc finger domains is assembled into a ZF protein (ZFP). ZFPs can comprise a functional domain. The first synthetic zinc finger nucleases (ZFNs) were developed by fusing a ZF protein to the catalytic domain of the Type IIS restriction enzyme Fokl. (Kim, Y. G. et al., 1994, Chimeric restriction endonuclease, Proc. Natl. Acad. Sci. U.S.A. 91, 883-887; Kim, Y. G. et al., 1996, Hybrid restriction enzymes: zinc finger fusions to Fok I cleavage domain. Proc. Natl. Acad. Sci. U.S.A. 93, 1156-1160). Increased cleavage specificity can be attained with decreased off target activity by use of paired ZFN heterodimers, each targeting different nucleotide sequences separated by a short spacer. (Doyon, Y. et al., 2011, Enhancing zinc-finger-nuclease activity with improved obligate heterodimeric architectures. Nat. Methods 8, 74-79). ZFPs can also be designed as transcription activators and repressors and have been used to target many genes in a wide variety of organisms.

In certain embodiments, the nucleic acid modification, such as introduction of the polynucleotide, QTL, locus, marker, or allele as described herein is effected by a (modified) meganuclease, which are endodeoxyribonucleases characterized by a large recognition site (double-stranded DNA sequences of 12 to 40 base pairs). Exemplary method for using meganucleases can be found in US Patent Nos: 8,163,514; 8,133,697; 8,021,867; 8,119,361; 8,119,381;

8,124,369; and 8,129,134, which are specifically incorporated by reference.

In certain embodiments, the nucleic acid modification is effected by a (modified) CRISPR/Cas complex or system. With respect to general information on CRISPR/Cas Systems, components thereof, and delivery of such components, including methods, materials, delivery vehicles, vectors, particles, and making and using thereof, including as to amounts and formulations, as well as Cas9CRISPR/Cas-expressing eukaryotic cells, Cas-9 CRISPR/Cas expressing eukaryotes, such as a mouse, reference is made to: Multiplex genome engineering using CRISPR/Cas systems. Cong, L., Ran, F.A., Cox, D., Lin, S., Barretto, R., Habib, N., Hsu, P.D., Wu, X., Jiang, W., Marraffini, L.A., & Zhang, F. Science Feb 15;339(6121):819-23 (2013); RNA-guided editing of bacterial genomes using CRISPR-Cas systems. Jiang W., Bikard D., Cox D., Zhang F, Marraffini LA. Nat Biotechnol Mar;31(3):233-9 (2013); One-Step Generation of Mice Carrying Mutations in Multiple Genes by CRISPR/Cas-Mediated Genome Engineering. Wang H., Yang H., Shivalila CS., Dawlaty MM., Cheng AW., Zhang F., Jaenisch R. Cell May 9;153(4):910-8 (2013); Optical control of mammalian endogenous transcription and epigenetic states. Konermann S, Brigham MD, Trevino AE, Hsu PD, Heidenreich M, Cong L, Platt RJ, Scott DA, Church GM, Zhang F. Nature. 2013 Aug 22;500(7463):472-6. doi: 10.1038/Nature12466. Epub 2013 Aug 23; Double Nicking by RNA-Guided CRISPR Cas9 for Enhanced Genome Editing Specificity. Ran, FA., Hsu, PD., Lin, CY., Gootenberg, JS., Konermann, S., Trevino, AE., Scott, DA., Inoue, A., Matoba, S., Zhang, Y., & Zhang, F. Cell Aug 28. pii: S0092-8674(13)01015-5. (2013); DNA targeting specificity of RNA-guided Cas9 nucleases. Hsu, P., Scott, D., Weinstein, J., Ran, FA., Konermann, S., Agarwala, V., Li, Y., Fine, E., Wu, X., Shalem, O., Cradick, TJ., Marraffini, LA., Bao, G., & Zhang, F. Nat Biotechnol doi:10.1038/nbt.2647 (2013); Genome engineering using the CRISPR-Cas9 system. Ran, FA., Hsu, PD., Wright, J., Agarwala, V., Scott, DA., Zhang, F. Nature Protocols Nov;8(11):2281-308. (2013); Genome-Scale CRISPR-Cas9 Knockout Screening in Human Cells. Shalem, O., Sanjana, NE., Hartenian, E., Shi, X., Scott, DA., Mikkelson, T., Heckl, D., Ebert, BL., Root, DE., Doench, JG., Zhang, F. Science Dec 12. (2013). [Epub ahead of print]; Crystal structure of cas9 in complex with guide RNA and target DNA. Nishimasu, H., Ran, FA., Hsu, PD., Konermann, S., Shehata, SI., Dohmae, N., Ishitani, R., Zhang, F., Nureki, O. Cell Feb 27. (2014). 156(5):935-49; Genomewide binding of the CRISPR endonuclease Cas9 in mammalian cells. Wu X., Scott DA., Kriz AJ., Chiu AC., Hsu PD., Dadon DB., Cheng AW., Trevino AE., Konermann S., Chen S., Jaenisch R., Zhang F., Sharp PA. Nat Biotechnol. (2014) Apr 20. doi: 10.1038/nbt.2889; CRISPR-Cas9 Knockin Mice for Genome Editing and Cancer Modeling, Platt et al., Cell 159(2): 440-455 (2014) DOI: 10.1016/j.cell.2014.09.014; Development and Applications of CRISPR-Cas9 for Genome Engineering, Hsu et al., Cell 157, 1262-1278 (June 5, 2014) (Hsu 2014); Genetic screens in human cells using the CRISPR/Cas9 system, Wang et al., Science. 2014 January 3; 343(6166): 80-84. doi:10.1126/science.1246981; Rational design of highly active sgRNAs for CRISPR-Cas9-mediated gene inactivation, Doench et al., Nature Biotechnology 32(12):1262-7 (2014) published online 3 September 2014; doi:10.1038/nbt.3026, and In vivo interrogation of gene function in the mammalian brain using CRISPR-Cas9, Swiech et al., Nature Biotechnology 33, 102-106 (2015) published online 19 October 2014; doi:10.1038/nbt.3055, Cpf1 Is a Single RNA-Guided Endonuclease of a Class 2 CRISPR-Cas System, Zetsche et al., Cell 163, 1-13 (2015); Discovery and Functional Characterization of Diverse Class 2 CRISPR-Cas Systems, Shmakov et al., Mol Cell 60(3): 385-397 (2015); C2c2 is a single-component programmable RNA-guided RNA-targeting CRISPR effector, Abudayyeh et al., Science (2016) published online June 2, 2016 doi: 10.1126/science.aaf5573. Each of these publications, patents, patent publications, and applications, and all documents cited therein or during their prosecution ("appln cited documents") and all documents cited or referenced in the appln cited documents, together with any instructions, descriptions, product specifications, and product sheets for any products mentioned therein or in any document therein and incorporated by reference herein, are hereby incorporated herein by reference, and may be employed in the practice of the invention. All documents (e.g., these patents, patent publications and applications and the appln cited documents) are incorporated herein by reference to the same extent as if each individual document was specifically and individually indicated to be incorporated by reference.

In certain embodiments, the CRISPR/Cas system or complex is a class 2 CRISPR/Cas system. In certain embodiments, said CRISPR/Cas system or complex is a type II, type V, or type VI CRISPR/Cas system or complex. The CRISPR/Cas system does not require the generation of customized proteins to target specific sequences but rather a single Cas protein can be programmed by an RNA guide (gRNA) to recognize a specific nucleic acid target, in other words the Cas enzyme protein can be recruited to a specific nucleic acid target locus (which may comprise or consist of RNA and/or DNA) of interest using said short RNA guide.

In general, the CRISPR/Cas or CRISPR system is as used herein foregoing documents refers collectively to transcripts and other elements involved in the expression of or directing the activity of CRISPR-associated ("Cas") genes, including sequences encoding a Cas gene and one or more of, a tracr (trans-activating CRISPR) sequence (e.g., tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or "RNA(s)" as that term is herein used (e.g., RNA(s) to guide Cas, such as Cas9, e.g., CRISPR RNA and, where applicable, transactivating (tracr) RNA or a single guide RNA (sgRNA) (chimeric RNA)) or other sequences and transcripts from a CRISPR locus. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides.

In certain embodiments, the gRNA is a chimeric guide RNA or single guide RNA (sgRNA). In certain embodiments, the gRNA comprises a guide sequence and a tracr mate sequence (or direct repeat). In certain embodiments, the gRNA comprises a guide sequence, a tracr mate sequence (or direct repeat), and a tracr sequence. In certain embodiments, the CRISPR/Cas system or complex as described herein does not comprise and/or does not rely on the presence of a tracr sequence (e.g., if the Cas protein is Cpf1).

As used herein, the term "crRNA" or "guide RNA" or "single guide RNA" or "sgRNA" or "one or more nucleic acid components" of a CRISPR/Cas locus effector protein, as applicable, comprises any polynucleotide sequence having sufficient complementarity with a target nucleic acid sequence to hybridize with the target nucleic acid sequence and direct sequence-specific binding of a nucleic acid-targeting complex to the target nucleic acid sequence. In some embodiments, the degree of complementarity, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g., the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). The ability of a guide sequence (within a nucleic acid-targeting guide RNA) to direct sequence-specific binding of a nucleic acid -targeting complex to a target nucleic acid sequence may be assessed by any suitable assay.

A guide sequence, and hence a nucleic acid-targeting guide RNA may be selected to target any target nucleic acid sequence. The target sequence may be DNA. The target sequence may be genomic DNA. The target sequence may be mitochondrial DNA. The target sequence may be any RNA sequence. In some embodiments, the target sequence may be a sequence within an RNA molecule selected from the group consisting of messenger RNA (mRNA), pre-mRNA, ribosomal RNA (rRNA), transfer RNA (tRNA), micro-RNA (miRNA), small interfering RNA (siRNA), small nuclear RNA (snRNA), small nucleolar RNA (snoRNA), double stranded RNA (dsRNA), non-coding RNA (ncRNA), long non-coding RNA (lncRNA), and small cytoplasmatic RNA (scRNA). In some preferred embodiments, the target sequence may be a sequence within an RNA molecule selected from the group consisting of mRNA, pre-mRNA, and rRNA. In some preferred embodiments, the target sequence may be a sequence within an RNA molecule selected from the group consisting of ncRNA, and lncRNA. In some more preferred embodiments, the target sequence may be a sequence within an mRNA molecule or a pre-mRNA molecule.

In certain embodiments, the gRNA comprises a stem loop, preferably a single stem loop. In certain embodiments, the direct repeat sequence forms a stem loop, preferably a single stem loop. In certain embodiments, the spacer length of the guide RNA is from 15 to 35 nt. In certain embodiments, the spacer length of the guide RNA is at least 15 nucleotides. In certain embodiments, the spacer length is from 15 to 17 nt, e.g., 15, 16, or 17 nt, from 17 to 20 nt, e.g., 17, 18, 19, or 20 nt, from 20 to 24 nt, e.g., 20, 21, 22, 23, or 24 nt, from 23 to 25 nt, e.g., 23, 24, or 25 nt, from 24 to 27 nt, e.g., 24, 25, 26, or 27 nt, from 27-30 nt, e.g., 27, 28, 29, or 30 nt, from 30-35 nt, e.g., 30, 31, 32, 33, 34, or 35 nt, or 35 nt or longer. In particular embodiments, the CRISPR/Cas system requires a tracrRNA. The "tracrRNA" sequence or analogous terms includes any polynucleotide sequence that has sufficient complementarity with a crRNA sequence to hybridize. In some embodiments, the degree of complementarity between the tracrRNA sequence and crRNA sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and gRNA sequence are contained within a single transcript, such that hybridization between the two produces a transcript having a secondary structure, such as a hairpin. In an embodiment of the invention, the transcript or transcribed polynucleotide sequence has at least two or more hairpins. In preferred embodiments, the transcript has two, three, four or five hairpins. In a further embodiment of the invention, the transcript has at most five hairpins. In a hairpin structure the portion of the sequence 5' of the final "N" and upstream of the loop may correspond to the tracr mate sequence, and the portion of the sequence 3' of the loop then corresponds to the tracr sequence. In a hairpin structure the portion of the sequence 5' of the final "N" and upstream of the loop may alternatively correspond to the tracr sequence, and the portion of the sequence 3' of the loop corresponds to the tracr mate sequence. In alternative embodiments, the CRISPR/Cas system does not require a tracrRNA, as is known by the skilled person.

In certain embodiments, the guide RNA (capable of guiding Cas to a target locus) may comprise (1) a guide sequence capable of hybridizing to a target locus and (2) a tracr mate or direct repeat sequence (in 5' to 3' orientation, or alternatively in 3' to 5' orientation, depending on the type of Cas protein, as is known by the skilled person). In particular embodiments, the CRISPR/Cas protein is characterized in that it makes use of a guide RNA comprising a guide sequence capable of hybridizing to a target locus and a direct repeat sequence, and does not require a tracrRNA. In particular embodiments, where the CRISPR/Cas protein is characterized in that it makes use of a tracrRNA, the guide sequence, tracr mate, and tracr sequence may reside in a single RNA, i.e., an sgRNA (arranged in a 5' to 3' orientation or alternatively arranged in a 3' to 5' orientation), or the tracr RNA may be a different RNA than the RNA containing the guide and tracr mate sequence. In these embodiments, the tracr hybridizes to the tracr mate sequence and directs the CRISPR/Cas complex to the target sequence.

Typically, in the context of an endogenous nucleic acid-targeting system, formation of a nucleic acid-targeting complex (comprising a guide RNA hybridized to a target sequence and complexed with one or more nucleic acid-targeting effector proteins) results in modification (such as cleavage) of one or both DNA or RNA strands in or near (e.g., within 1, 2, 3, 4, 5, 6, 7, 8, 9, 10, 20, 50, or more base pairs from) the target sequence. As used herein the term "sequence(s) associated with a target locus of interest" refers to sequences near the vicinity of the target sequence (e.g., within 1, 2, 3, 4, 5, 6, 7, 8, 9, 10, 20, 50, or more base pairs from the target sequence, wherein the target sequence is comprised within a target locus of interest). The skilled person will be aware of specific cut sites for selected CRISPR/Cas systems, relative to the target sequence, which as is known in the art may be within the target sequence or alternatively 3' or 5' of the target sequence.

In some embodiments, the unmodified nucleic acid-targeting effector protein may have nucleic acid cleavage activity. In some embodiments, the nuclease as described herein may direct cleavage of one or both nucleic acid (DNA, RNA, or hybrids, which may be single or double stranded) strands at the location of or near a target sequence, such as within the target sequence and/or within the complement of the target sequence or at sequences associated with the target sequence. In some embodiments, the nucleic acid-targeting effector protein may direct cleavage of one or both DNA or RNA strands within about 1, 2, 3, 4, 5, 6, 7, 8, 9, 10, 15, 20, 25, 50, 100, 200, 500, or more base pairs from the first or last nucleotide of a target sequence. In some embodiments, the cleavage may be blunt (e.g., for Cas9, such as SaCas9 or SpCas9). In some embodiments, the cleavage may be staggered (e.g., for Cpf1), i.e., generating sticky ends. In some embodiments, the cleavage is a staggered cut with a 5' overhang. In some embodiments, the cleavage is a staggered cut with a 5' overhang of 1 to 5 nucleotides, preferably of 4 or 5 nucleotides. In some embodiments, the cleavage site is upstream of the PAM. In some embodiments, the cleavage site is downstream of the PAM. In some embodiments, the nucleic acid-targeting effector protein that may be mutated with respect to a corresponding wild-type enzyme such that the mutated nucleic acid-targeting effector protein lacks the ability to cleave one or both DNA or RNA strands of a target polynucleotide containing a target sequence. As a further example, two or more catalytic domains of a Cas protein (e.g., RuvC I, RuvC II, and RuvC III or the HNH domain of a Cas9 protein) may be mutated to produce a mutated Cas protein substantially lacking all DNA cleavage activity. In some embodiments, a nucleic acid-targeting effector protein may be considered to substantially lack all DNA and/or RNA cleavage activity when the cleavage activity of the mutated enzyme is about no more than 25%, 10%, 5%, 1%, 0.1%, 0.01%, or less of the nucleic acid cleavage activity of the non-mutated form of the enzyme; an example can be when the nucleic acid cleavage activity of the mutated form is nil or negligible as compared with the non-mutated form. As used herein, the term "modified" Cas generally refers to a Cas protein having one or more modifications or mutations (including point mutations, truncations, insertions, deletions, chimeras, fusion proteins, etc.) compared to the wild-type Cas protein from which it is derived. By derived is meant that the derived enzyme is largely based, in the sense of having a high degree of sequence homology with, a wildtype enzyme, but that it has been mutated (modified) in some way as known in the art or as described herein.

In certain embodiments, the target sequence should be associated with a PAM (protospacer adjacent motif) or PFS (protospacer flanking sequence or site); that is, a short sequence recognized by the CRISPR complex. The precise sequence and length requirements for the PAM differ depending on the CRISPR enzyme used, but PAMs are typically 2-5 base pair sequences adjacent the protospacer (that is, the target sequence). Examples of PAM sequences are given in the examples section below, and the skilled person will be able to identify further PAM sequences for use with a given CRISPR enzyme. Further, engineering of the PAM Interacting (PI) domain may allow programming of PAM specificity, improve target site recognition fidelity, and increase the versatility of the Cas, e.g., Cas9, genome engineering platform. Cas proteins, such as Cas9 proteins may be engineered to alter their PAM specificity, for example as described in Kleinstiver BP et al., Engineered CRISPR-Cas9 nucleases with altered PAM specificities. Nature. 2015 Jul 23;523(7561):481-5. doi: 10.1038/nature14592. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence. The skilled person will understand that other Cas proteins may be modified analogously.

The Cas protein as referred to herein, such as without limitation Cas9, Cpf1 (Cas12a), C2c1 (Cas12b), C2c2 (Cas13a), C2c3, Cas13b protein, may originate from any suitable source, and hence may include different orthologues, originating from a variety of (prokaryotic) organisms, as is well documented in the art. In certain embodiments, the Cas protein is (modified) Cas9, preferably (modified) Staphylococcus aureus Cas9 (SaCas9) or (modified) Streptococcus pyogenes Cas9 (SpCas9). In certain embodiments, the Cas protein is (modified) Cpf1, preferably Acidaminococcus sp.,

such as Acidaminococcus sp. BV3L6 Cpf1 (AsCpf1) or Lachnospiraceae bacterium Cpf1, such as Lachnospiraceae bacterium MA2020 or Lachnospiraceae bacterium MD2006 (LbCpf1). In certain embodiments, the Cas protein is (modified) C2c2, preferably Leptotrichia wadei C2c2 (LwC2c2) or Listeria newyorkensis FSL M6-0635 C2c2 (LbFSLC2c2). In certain embodiments, the (modified) Cas protein is C2c1. In certain embodiments, the (modified) Cas protein is C2c3. In certain embodiments, the (modified) Cas protein is Cas13b.

Further, gene editing may comprise also the exchange of single nucleotides by means of base editors. A base editor as used herein refers to a protein or a fragment thereof having the capacity to mediate a targeted base modification, i.e., the conversion of a base of interest resulting in a point mutation of interest. Preferably, the at least one base editor in the context of the present invention is temporarily or permanently fused to at least one DSBI enzyme, or optionally to a component of at least one DSBI. The fusion can be covalent and/or non-covalent. Multiple publications have shown targeted base conversion, primarily cytidine (C) to thymine (T), using a CRISPR/Cas9 nickase or non-functional nuclease linked to a cytidine deaminase domain, Apolipoprotein B mRNA-editing catalytic polypeptide (APOBEC1), e.g., APOBEC derived from rat. The deamination of cytosine (C) is catalysed by cytidine deaminases and results in uracil (U), which has the base-pairing properties of thymine (T). Most known cytidine deaminases operate on RNA, and the few examples that are known to accept DNA require single-stranded (ss) DNA. Studies on the dCas9-target DNA complex reveal that at least nine nucleotides (nt) of the displaced DNA strand are unpaired upon formation of the Cas9-guide RNA-DNA 'R-loop' complex (Jore et al., Nat. Struct. Mol. Biol., 18, 529-536 (2011)). Indeed, in the structure of the Cas9 R-loop complex, the first 11 nt of the protospacer on the displaced DNA strand are disordered, suggesting that their movement is not highly restricted. It has also been speculated that Cas9 nickase-induced mutations at cytosines in the non-template strand might arise from their accessibility by cellular cytosine deaminase enzymes. It was reasoned that a subset of this stretch of ssDNA in the R-loop might serve as an efficient substrate for a dCas9-tethered cytidine deaminase to effect direct, programmable conversion of C to U in DNA (Komor et al., supra). Recently, Goudelli et al., ((2017). Programmable base editing of A•T to G•C in genomic DNA without DNA cleavage. Nature, 551(7681), 464.) described adenine base editors (ABEs) that mediate the conversion of A•T to G•C in genomic DNA.

In certain embodiments, the nucleic acid modification is effected by random mutagenesis. The skilled person will understand that identification and selection of suitable mutations may include appropriate selection assays, such as functional selection assays (including genotypic or phenotypic selection assays). In random mutagenesis, cells or organisms may be exposed to mutagens such as UV, X-ray, or gamma ray radiation or mutagenic chemicals (such as for instance such as ethyl methanesulfonate (EMS), ethylnitrosourea (ENU), or dimethylsulfate (DMS), and mutants with desired characteristics are then selected. Mutants can for instance be identified by TILLING (Targeting Induced Local Lesions in Genomes). The method combines mutagenesis, such as mutagenesis using a chemical mutagen such as ethyl methanesulfonate (EMS) with a sensitive DNA screening-technique that identifies single base mutations/point mutations in a target gene. The TILLING method relies on the formation of DNA heteroduplexes that are formed when multiple alleles are amplified by PCR and are then heated and slowly cooled. A "bubble" forms at the mismatch of the two DNA strands, which is then cleaved by a single stranded nuclease. The products are then separated by size, such as by HPLC. See also McCallum et al., "Targeted screening for induced mutations"; Nat Biotechnol. 2000 Apr;18(4):455-7 and McCallum et al., "Targeting Induced Local Lesions IN Genomes (TILLING) for plant functional genomics"; Plant Physiol. 2000 Jun;123(2):439-42, both incorporated by reference in their entirety. By means of further example, and without limitation, the methodologies described in the following publications, incorporated by reference in their entirety, may be adopted according to the present invention, such as in connection with EMS mutagenesis: Till et al., "Discovery of induced point mutations in maize genes by TILLING"; BMC Plant Biol. 2004 Jul 28;4:12; and Weil & Monde "Getting the point-mutations in maize" Crop Sci 2007; 47 S60-S67. The skilled person will understand that depending on the mutagen dose (irradiation of chemical) the (average) mutation density can be varied or fixed. In certain embodiments, the random mutagenesis is single nucleotide mutagenesis. In certain embodiments, the random mutagenesis is chemical mutagenesis, preferably EMS mutagenesis.

"RNA interference" or "RNAi" is a biological process in which RNA molecules inhibit gene expression or translation, by neutralizing targeted mRNA molecules. Two types of small ribonucleic acid (RNA) molecules - microRNA (miRNA) and small interfering RNA (siRNA) - are central to RNA interference. RNAs are the direct products of genes, and these small RNAs can bind to other specific messenger RNA (mRNA) molecules and either increase or decrease their activity, for example by preventing an mRNA from being translated into a protein. The RNAi pathway is found in many eukaryotes, including animals, and is initiated by the enzyme Dicer, which cleaves long double-stranded RNA (dsRNA) molecules into short double-stranded fragments of about 21 nucleotide siRNAs (small interfering RNAs). Each siRNA is unwound into two single-stranded RNAs (ssRNAs), the passenger strand and the guide strand. The passenger strand is degraded, and the guide strand is incorporated into the RNA-induced silencing complex (RISC). Mature miRNAs are structurally similar to siRNAs produced from exogenous dsRNA, but before reaching maturity, miRNAs must first undergo extensive post-transcriptional

modification. A miRNA is expressed from a much longer RNA-coding gene as a primary transcript known as a pri-miRNA which is processed, in the cell nucleus, to a 70-nucleotide stem-loop structure called a pre-miRNA by the microprocessor complex. This complex consists of an RNase III enzyme called Drosha and a dsRNA-binding protein DGCR8. The dsRNA portion of this pre-miRNA is bound and cleaved by Dicer to produce the mature miRNA molecule that can be integrated into the RISC complex; thus, miRNA and siRNA share the same downstream cellular machinery. A short hairpin RNA or small hairpin RNA (shRNA/Hairpin Vector) is an artificial RNA molecule with a tight hairpin turn that can be used to silence target gene expression via RNA interference. The most well-studied outcome is post-transcriptional gene silencing, which occurs when the guide strand pairs with a complementary sequence in a messenger RNA molecule and induces cleavage by Argonaute 2 (Ago2), the catalytic component of the RISC. In will be understood that the RNAi molecules can be applied as such to/in the plant, or can be encoded by appropriate vectors, from which the RNAi molecule is expressed. Delivery and expression systems of RNAi molecules, such as siRNAs, shRNAs or miRNAs are well known in the art.

As used herein, the term "homozygote" refers to an individual cell or plant having the same alleles at one or more or all corresponding loci on hom(e)ologous chromosomes. When the term is used with reference to a specific locus or gene, it means at least that locus or gene has the same alleles. Accordingly, for diploid organisms, the two alleles are identical, for tetraploid organisms, the 4 alleles are identical, etc. As used herein, the term "homozygous" means a genetic condition existing when identical alleles reside at corresponding loci on homologous chromosomes. As used herein, the term "heterozygote" refers to an individual cell or plant having different alleles at one or more or all corresponding loci on hom(e)ologous chromosomes. Accordingly, for diploid organisms, the two alleles are not identical, for tetraploid organisms, the 4 alleles are not identical (i.e., at least one allele is different than the other alleles), etc. When the term is used with reference to a specific locus or gene, it means at least that locus or gene has different alleles. As used herein, the term "heterozygous" means a genetic condition existing when different alleles reside at corresponding loci on homologous chromosomes. In certain embodiments, the proteins, genes, or coding sequences as described herein are heterozygous. In certain embodiments, proteins, genes, or coding sequence alleles as described herein is/are homozygous. In certain embodiments, the proteins, genes, or coding sequence alleles as described herein are heterozygous. It will be understood that homozygosity or heterozygosity preferably relates to at least a gene, i.e., the locus comprising the gene (or coding sequence derived thereof, or protein encoded thereby). However, more specifically, homozygosity or heterozygosity may equally refer to a particular polynucleotide, QTL, locus, marker, or allele, such as a polynucleotide, QTL, locus, marker, or allele described herein. Accordingly, a particular polynucleotide, QTL, locus, marker, or allele can be considered to be homozygous, whereas for instance the remainder of the genome may comprise differences between alleles.

In certain embodiments, the polynucleotide, QTL, locus, marker, and/or allele as defined herein is homozygous. Accordingly, in diploid plants the two alleles are identical (at least with respect to the particular polynucleotide, QTL, locus, or marker), in tetraploid plants the four alleles are identical, and in hexaploid plants the six alleles are identical with respect to the polynucleotide, QTL, locus, or marker. In certain embodiments, the polynucleotide, QTL, locus, or marker as defined herein is heterozygous. Accordingly, in diploid plants the two alleles are not identical, in tetraploid plants the four alleles are not identical (for instance only one, two, or three alleles comprise the specific polynucleotide, QTL, locus, or marker), and in hexaploid plants the six alleles are not identical with respect to the polynucleotide, QTL, locus, or marker (for instance only one, two, three, four or five alleles comprise the specific polynucleotide, QTL, locus, or marker). Similar considerations apply in case of pseudopolyploid pants.

A "marker" is a means of finding or identifying a position on a genetic or physical map, or else linkages among markers and trait loci (loci affecting traits). The position that the marker detects may be known via detection of polymorphic alleles and their genetic mapping, or else by hybridization, sequence match or amplification of a sequence that has been physically mapped. A marker can be a DNA marker (detects DNA polymorphisms), a protein (detects variation at an encoded polypeptide), or a simply inherited phenotype (such as the 'waxy' phenotype). A DNA marker can be developed from genomic nucleotide sequence or from expressed nucleotide sequences (e.g., from a spliced RNA or a cDNA). Depending on the DNA marker technology, the marker may consist of complementary primers flanking the locus and/or complementary probes that hybridize to polymorphic alleles at the locus. The term marker locus is the locus (gene, sequence or nucleotide) that the marker detects. "Marker" or "molecular marker" or "marker locus" or "marker allele" may also be used to denote a nucleic acid or amino acid sequence that is sufficiently unique to characterize a specific locus on the genome. Any detectable polymorphic trait can be used as a marker so long as it is inherited differentially and exhibits linkage disequilibrium with a phenotypic trait of interest.

The development of markers may be based either on quantitative trait loci (QTL) mapping or genome wide association studies (GWAS). A QTL mapping requires two parental lines (according to the present invention, one with having (increased) clubroot resistance or tolerance and one without) that differ genetically for the clubroot resistance or tolerance trait. These two types of mapping are referred to as low resolution mapping due to the fact that the identified chromosomal region (from now on called QTL interval) controlling the trait can contain up to several hundreds of genes within an interval size of 5-30 cM, depending on whether the QTL interval is in the telomeric or pericentromeric region. The reason for such a name is that both QTL mapping as well as GWAS generally only use rather a small size of population (100 to 200 lines), thus the size of the discovered chromosomal interval within which the gene exists becomes very large. Therefore, the markers flanking the identified chromosomal region in 100 percent of cases are not diagnostic, which means the marker score generated does not necessarily match the observed phenotype in the field. For this issue to be resolved, the mapping resolution needs to be increased, and the resolution can be increased by increasing recombination events during meiosis. To accomplish this, one has to increase the population to thousands of lines plus have different generations of selfing or back crossing that take at least four years (and are very expensive and labor-intensive). In reality, the breeder must perform high resolution mapping of fine mapping in order to have a marker nearer to the gene. In the initial screening, the thousands of individuals are screened only for initial flanking markers identified through low-resolution mapping. Such a screening aims to find recombinants. Recombination events between markers and the gene will allow to narrow down the interval and come closer to the gene. The next step is designing additional markers within the targeted interval. These may range for instance from ten to twenty markers, depending on how long an interval is. Then screen the newly identified recombinants with these 10-20 markers. This will help find the closest marker with the least number of recombination events between it and the gene, keeping in mind that the marker is only close to the gene, not within it, so there is still a possibility that the marker score does not match the observed phenotype. So, as you can see the approach requires a lot of time, efforts, and money should be invested. The use of literature-based markers is another possibility. It is more convenient and time-efficient if the information of the closet marker is already published, otherwise the publication is only beneficial to determine the corresponding chromosomal interval responsible for the trait of interest.

Markers that detect genetic polymorphisms between members of a population are well-established in the art. Markers can be defined by the type of polymorphism that they detect and also the marker technology used to detect the polymorphism. Marker types include but are not limited to, e.g., detection of restriction fragment length polymorphisms (RFLP), detection of isozyme markers, randomly amplified polymorphic DNA (RAPD), amplified fragment length polymorphisms (AFLPs), detection of simple sequence repeats (SSRs), detection of amplified variable sequences of the plant genome, detection of self-sustained sequence replication, or detection of single nucleotide polymorphisms (SNPs). SNPs can be detected e.g., via DNA sequencing, PCR-based sequence specific amplification methods, detection of polynucleotide polymorphisms by allele specific hybridization (ASH), dynamic allele-specific hybridization (DASH), molecular beacons, microarray hybridization, oligonucleotide ligase assays, Flap endonucleases, 5' endonucleases, primer extension, single strand conformation polymorphism (SSCP) or temperature gradient gel electrophoresis (TGGE). DNA sequencing, such as the pyrosequencing technology has the advantage of being able to detect a series of linked SNP alleles that constitute a haplotype. Haplotypes tend to be more informative (detect a higher level of polymorphism) than SNPs.

A "marker allele", alternatively an "allele of a marker locus", can refer to one of a plurality of polymorphic nucleotide sequences found at a marker locus in a population. With regard to a SNP marker, allele refers to the specific nucleotide base present at that SNP locus in that individual plant. As used herein, reference to markers or marker alleles refers to markers or marker alleles associated with, linked with, or characteristic of NCLB resistance and/or tolerance, unless explicitly referred to otherwise. Such markers or marker alleles are typically annotated as "donor" markers or marker alleles.

"Fine-mapping" refers to methods by which the position of a QTL can be determined more accurately (narrowed down) and by which the size of the introgression fragment comprising the QTL is reduced. For example, Near Isogenic Lines for the QTL (QTL-NILs) can be made, which contain different, overlapping fragments of the introgression fragment within an otherwise uniform genetic background of the recurrent parent. Such lines can then be used to map on which fragment the QTL is located and to identify a line having a shorter introgression fragment comprising the QTL.

"Marker assisted selection" (of MAS) is a process by which individual plants are selected based on marker genotypes. "Marker assisted counter-selection" is a process by which marker genotypes are used to identify plants that will not be selected, allowing them to be removed from a breeding program or planting. Marker assisted selection uses the presence of molecular markers, which are genetically linked to a particular locus or to a particular chromosome region (e.g., introgression fragment, transgene, polymorphism, mutation, etc), to select plants for the presence of the specific locus or region (introgression fragment, transgene, polymorphism, mutation, etc). For example, a marker allele genetically linked to a QTL or gene as defined herein, can be used to detect and/or select plants comprising the QTL or gene. The closer the genetic linkage of the marker allele to the locus (e.g., about 10 cM, 7 cM, 6 cM, 5 cM, 4 cM, 3 cM, 2 cM, 1 cM, 0.5 cM or less), the less likely it is that the marker is dissociated from the locus through meiotic recombination. Likewise, the closer two markers are linked to each other (e.g., within 10 cM, 7 cM, 5 cM, 4 cM , 3 cM, 2 cM, 1 cM or less) the less likely it is that the two markers will be separated from one another (and the more likely they will co-segregate as a unit). A marker" within 10 cM or within 7 cM or within 5 cM, 3 cM, 2 cM, or 1 cM" of another marker refers to a marker which genetically maps to within the 10 cM or 7 cM or 5 cM, 3 cM, 2 cM, or 1 cM region flanking the marker (i.e., either side of the marker). Similarly, a marker within 10 Mb, 5 Mb, 3 Mb, 2.5 Mb, 2 Mb, 1 Mb, 0.5 Mb, 0.4 Mb, 0.3 Mb, 0.2 Mb, 0.1 Mb, 50 kb, 20 kb, 10kb, 5kb, 2kb, 1 kb or less of another marker refers to a marker which is physically located within the 10 Mb, 5 Mb, 3 Mb, 2.5 Mb, 2 Mb, 1 Mb, 0.5 Mb, 0.4 Mb, 0.3 Mb, 0.2 Mb, 0.1 Mb, 50 kb, 20 kb, 10 kb, 5 kb, 2 kb, 1 kb or less, of the genomic DNA region flanking the marker (i.e., either side of the marker).

"LOD-score" (logarithm (base 10) of odds) refers to a statistical test often used for linkage analysis in animal and plant populations. The LOD score compares the likelihood of obtaining the test data if the two loci (molecular marker loci and/or a phenotypic trait locus) are indeed linked, to the likelihood of observing the same data purely by chance. Positive LOD scores favour the presence of linkage and a LOD score greater than 3.0 is considered evidence for linkage. A LOD score of +3 indicates 1000 to 1 odds that the linkage being observed did not occur by chance.

A "marker haplotype" refers to a combination of alleles at a marker locus.

A "marker locus" is a specific chromosome location in the genome of a species where a specific marker can be found. A marker locus can be used to track the presence of a second linked locus, e.g., one that affects the expression of a phenotypic trait. For example, a marker locus can be used to monitor segregation of alleles at a genetically or physically linked locus.

A "marker probe" is a nucleic acid sequence or molecule that can be used to identify the presence of a marker locus, e.g., a nucleic acid probe that is complementary to a marker locus sequence, through nucleic acid hybridization. Marker probes comprising 30 or more contiguous nucleotides of the marker locus ("all or a portion" of the marker locus sequence) may be used for nucleic acid hybridization. Alternatively, in some aspects, a marker probe refers to a probe of any type that is able to distinguish (i.e., genotype) the particular allele that is present at a marker locus.

The term "molecular marker" may be used to refer to a genetic marker or an encoded product thereof (e.g., a protein) used as a point of reference when identifying a linked locus. A marker can be derived from genomic nucleotide sequences or from expressed nucleotide sequences (e.g., from a spliced RNA, a cDNA, etc.), or from an encoded polypeptide. The term also refers to nucleic acid sequences complementary to or flanking the marker sequences, such as nucleic acids used as probes or primer pairs capable of amplifying the marker sequence. A "molecular marker probe" is a nucleic acid sequence or molecule that can be used to identify the presence of a marker locus, e.g., a nucleic acid probe that is complementary to a marker locus sequence. Alternatively, in some aspects, a marker probe refers to a probe of any type that is able to distinguish (i.e., genotype) the particular allele that is present at a marker locus. Nucleic acids are "complementary" when they specifically hybridize in solution, e.g., according to Watson-Crick base pairing rules. Some of the markers described herein are also referred to as hybridization markers when located on an indel region, such as the non- collinear region described herein. This is because the insertion region is, by definition, a polymorphism vis a vis a plant without the insertion. Thus, the marker need only indicate whether the indel region is present or absent. Any suitable marker detection technology may be used to identify such a hybridization marker, e.g., SNP technology is used in the examples provided herein.

"Genetic markers" are nucleic acids that are polymorphic in a population and where the alleles of which can be detected and distinguished by one or more analytic methods, e.g., RFLP, AFLP, isozyme, SNP, SSR, and the like. The terms "molecular marker" and "genetic marker" are used interchangeably herein. The term also refers to nucleic acid sequences complementary to the genomic sequences, such as nucleic acids used as probes. Markers corresponding to genetic polymorphisms between members of a population can be detected by methods well- established in the art. These include, e.g., PCR-based sequence specific amplification methods, detection of restriction fragment length polymorphisms (RFLP), detection of isozyme markers, detection of polynucleotide polymorphisms by allele specific hybridization (ASH), detection of amplified variable sequences of the plant genome, detection of self-sustained sequence replication, detection of simple sequence repeats (SSRs), detection of single nucleotide polymorphisms (SNPs), or detection of amplified fragment length polymorphisms (AFLPs). Well established methods are also known for the detection of expressed sequence tags (ESTs) and SSR markers derived from EST sequences and randomly amplified polymorphic DNA (RAPD).

A "genetic map" is a description of genetic linkage relationships among loci on one or more chromosomes (or chromosomes) within a given species, generally depicted in a diagrammatic or tabular form. For each genetic map, distances between loci are measured by the recombination frequencies between them, and recombinations between loci can be detected using a variety of molecular genetic markers (also called molecular markers). A genetic map is a product of the mapping population, types of markers used, and the polymorphic potential of each marker between different populations. The order and genetic distances between loci can differ from one genetic map to another.

"Genetic recombination frequency" is the frequency of a crossing over event (recombination) between two genetic loci. Recombination frequency can be observed by following the segregation of markers and/or traits following meiosis.

A "polymorphism" is a variation in the DNA between two or more individuals within a population. A polymorphism typically is a variation in the DNA that is too common to be due merely to new mutation A polymorphism preferably has a frequency of at least 1 % in a population. A useful polymorphism can include a single nucleotide polymorphism (SNP), a simple sequence repeat (SSR), or an insertion/deletion polymorphism, also referred to herein as an "indel". The term "indel" refers to an insertion or deletion, wherein one line may be referred to as having an inserted nucleotide or piece of DNA relative to a second line, or the second line may be referred to as having a deleted nucleotide or piece of DNA relative to the first line.

A "single nucleotide polymorphism (SNP)" is an allelic single nucleotide- A, T, C or G - variation within a DNA sequence representing one locus of at least two individuals of the same species. For example, two sequenced DNA fragments representing the same locus from at least two individuals of the same species, AAGCCTA to AAGCTTA, contain a difference in a single nucleotide.

"Physical distance" between loci (e.g., between molecular markers and/or between phenotypic markers) on the same chromosome is the actually physical distance expressed in bases or base pairs (bp), kilo bases or kilo base pairs (kb) or megabases or mega base pairs (Mb).

"Genetic distance" between loci (e.g., between molecular markers and/or between phenotypic markers) on the same chromosome is measured by frequency of crossing-over, or recombination frequency (RF) and is indicated in centimorgans (cM). One cM corresponds to a recombination frequency of 1% and is equal to a 1% chance that a marker at one genetic locus will be separated from a marker at a second locus due to crossing over in a single generation. If no recombinants can be found, the RF is zero and the loci are either extremely close together physically or they are identical. The further apart two loci are, the higher the RF.

A "physical map" of the genome is a map showing the linear order of identifiable landmarks (including genes, markers, etc.) on chromosome DNA. However, in contrast to genetic maps, the distances between landmarks are absolute (for example, measured in base pairs or isolated and overlapping contiguous genetic fragments) and not based on genetic recombination (that can vary in different populations).

An allele "negatively" correlates with a trait when it is linked to it and when presence of the allele is an indicator that a desired trait or trait form will not occur in a plant comprising the allele. An allele "positively" correlates with a trait when it is linked to it and when presence of the allele is an indicator that the desired trait or trait form will occur in a plant comprising the allele.

A centimorgan ("cM") is a unit of measure of recombination frequency. One cM is equal to a 1 % chance that a marker at one genetic locus will be separated from a marker at a second locus due to crossing over in a single generation.

As used herein, the term "chromosomal interval" designates a contiguous linear span of genomic DNA that resides in planta on a single chromosome. The genetic elements or genes located on a single chromosomal interval are physically linked. The size of a chromosomal interval is not particularly limited. In some aspects, the genetic elements located within a single chromosomal interval are genetically linked, typically with a genetic recombination distance of, for example, less than or equal to 20 cM, or alternatively, less than or equal to 10 cM. That is, two genetic elements within a single chromosomal interval undergo recombination at a frequency of less than or equal to 20% or 10%. The term "chromosomal interval" designates any and all intervals defined by any of the markers set forth in this invention. A chromosomal interval that correlates with increased clubroot disease resistance is provided.

The term "linked" or "closely linked", in the present application, means that recombination between two linked loci occurs with a frequency of equal to or less than about 10% (i.e., are separated on a genetic map by not more than 10 cM), or preferably less than about 5% (i.e., 5 cM), more preferably less than about 1% (i.e., 1 cM). Put another way, the closely linked loci co-segregate at least 90% (or 95% or 99%) of the time. Marker loci are especially useful with respect to the subject matter of the current disclosure when they demonstrate a significant probability of co-segregation (linkage) with a desired trait (e.g., resistance to grey leaf spot). Closely linked loci such as a marker locus and a second locus can display an inter-locus recombination frequency of 10% or less, preferably about 9% or less, still more preferably about 8% or less, yet more preferably about 7% or less, still more preferably about 6% or less, yet more preferably about 5% or less, still more preferably about 4% or less, yet more preferably about 3% or less, and still more preferably about 2% or less. In highly preferred embodiments, the relevant loci display a recombination a frequency of about 1 % or less, e.g., about 0.75% or less, more preferably about 0.5% or less, or yet more preferably about 0.25% or less. Two loci that are localized to the same chromosome, and at such a distance that recombination between the two loci occurs at a frequency of less than 10% (e.g., about 9 %, 8%, 7%, 6%, 5%, 4%, 3%, 2%, 1 %, 0.75%, 0.5%, 0.25%, or less) are also said to be "proximal to" each other. In some cases, two different markers can have the same genetic map coordinates. In that case, the two markers are in such close proximity to each other that recombination occurs between them with such low frequency that it is undetectable.

"Linkage" refers to the tendency for alleles to segregate together more often than expected by chance if their transmission was independent. Typically, linkage refers to alleles on the same chromosome. Genetic recombination occurs with an assumed random frequency over the entire genome. Genetic maps are constructed by measuring the frequency of recombination between pairs of traits or markers. The closer the traits or markers are to each other on the chromosome, the lower the frequency of recombination, and the greater the degree of linkage. Traits or markers are considered herein to be linked if they generally co- segregate. A 1/100 probability of recombination per generation is defined as a genetic map distance of 1.0 centiMorgan (1.0 cM). The term "linkage disequilibrium" refers to a non-random segregation of genetic loci or traits (or both). In either case, linkage disequilibrium implies that the relevant loci are within sufficient physical proximity along a length of a chromosome so that they segregate together with greater than random (i.e., non-random) frequency. Markers that show linkage disequilibrium are considered linked. Linked loci co-segregate more than 50% of the time, e.g., from about 51 % to about 100% of the time. In other words, two markers that co-segregate have a recombination frequency of less than 50% (and by definition, are separated by less than 50 cM on the same linkage group.) As used herein, linkage can be between two markers, or alternatively between a marker and a locus affecting a phenotype. A marker locus can be "associated with" (linked to) a trait. The degree of linkage of a marker locus and a locus affecting a phenotypic trait is measured, e.g., as a statistical probability of co-segregation of that molecular marker with the phenotype (e.g., an F statistic or LOD score).

The "probability value" or "p-value" is the statistical likelihood that the particular combination of a phenotype and the presence or absence of a particular marker allele is random. Thus, the lower the probability score, the greater the likelihood that a phenotype and a particular marker will co-segregate. In this context the probability score is considered "significant" or "nonsignificant". In some embodiments, a probability score of 0.05 (p=0.05, or a 5% probability) of random assortment is considered a significant indication of co-segregation.

The genetic elements or genes located on a single chromosome segment are physically linked. In some embodiments, the two loci are located in close proximity such that recombination between homologous chromosome pairs does not occur between the two loci during meiosis with high frequency, e.g., such that linked loci co-segregate at least about 90% of the time, e.g., 91%, 92%, 93%, 94%, 95%, 96%, 97%, 98%, 99%, 99.5%, 99.75%, or more of the time. The genetic elements located within a chromosomal segment are also "genetically linked", typically within a genetic recombination distance of less than or equal to 50cM, e.g., about 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, 0.75, 0.5, 0.25 cM or less. That is, two genetic elements within a single chromosomal segment undergo recombination during meiosis with each other at a frequency of less than or equal to about 50%, e.g., about 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, 0.75%, 0.5%, 0.25% or less. "Closely linked" markers display a cross over frequency with a given marker of about 10% or less, e.g., 9%, 8%, 7%, 6%, 5%, 4%, 3%, 2%, 1%, 0.75%, 0.5%, 0.25% or less (the given marker locus is within about 10 cM of a closely linked marker locus, e.g., 9, 8, 7, 6, 5, 4, 3, 2, 1, 0.75, 0.5, 0.25 cM or less of a closely linked marker locus). Put another way, closely linked marker loci co- segregate at least about 90% the time, e.g., 91%, 92%, 93%, 94%, 95%, 96%, 97%, 98%, 99%, 99.5%, 99.75%, or more of the time.

As used herein, the term "sequence identity" refers to the degree of identity between any given nucleic acid sequence and a target nucleic acid sequence. Percent sequence identity is calculated by determining the number of matched positions in aligned nucleic acid sequences, dividing the number of matched positions by the total number of aligned nucleotides, and multiplying by 100. A matched position refers to a position in which identical nucleotides occur at the same position in aligned nucleic acid sequences. Percent sequence identity also can be determined for any amino acid sequence. To determine percent sequence identity, a target nucleic acid or amino acid sequence is compared to the identified nucleic acid or amino acid sequence using the BLAST 2 Sequences (Bl2seq) program from the stand-alone version of BLASTZ containing BLASTN and BLASTP. This stand-alone version of BLASTZ can be obtained from Fish & Richardson's web site (World Wide Web at fr.com/blast) or the U.S. government's National Center for Biotechnology Information web site (World Wide Web at ncbi.nlm.nih.gov). Instructions explaining how to use the Bl2seq program can be found in the readme file accompanying BLASTZ. Bl2seq performs a comparison between two sequences using either the BLASTN or BLASTP algorithm. Preferably, BLAST sequence alignments are performed according to the standard (i.e., default) settings (i.e., at the filing date of the present application).

BLASTN is used to compare nucleic acid sequences, while BLASTP is used to compare amino acid sequences. To compare two nucleic acid sequences, the options are set as follows: -i is set to a file containing the first nucleic acid sequence to be compared (e.g., C:\seq I .txt); -j is set to a file containing the second nucleic acid sequence to be compared (e.g., C:\seq2.txt); -p is set to blastn; -o is set to any desired file name (e.g., C :\output.txt); -q is set to - 1 ; -r is set to 2; and all other options are left at their default setting. The following command will generate an output file containing a comparison between two sequences: C:\B12seq -i c:\seqI .txt -j c:\seq2.txt -p blastn -o c:\output.txt - q - 1 -r 2. If the target sequence shares homology with any portion of the identified sequence, then the designated output file will present those regions of homology as aligned sequences. If the target sequence does not share homology with any portion of the identified sequence, then the designated output file will not present aligned sequences. Once aligned, a length is determined by counting the number of consecutive nucleotides from the target sequence presented in alignment with the sequence from the identified sequence starting with any matched position and ending with any other matched position. A matched position is any position where an identical nucleotide is presented in both the target and identified sequences. Gaps presented in the target sequence are not counted since gaps are not nucleotides. Likewise, gaps presented in the identified sequence are not counted since target sequence nucleotides are counted, not nucleotides from the identified sequence. The percent identity over a particular length is determined by counting the number of matched positions over that length and dividing that number by the length followed by multiplying the resulting value by 100. For example, if (i) a 500-base nucleic acid target sequence is compared to a subject nucleic acid sequence, (ii) the Bl2seq program presents 200 bases from the target sequence aligned with a region of the subject sequence where the first and last bases of that 200-base region are matches, and (iii) the number of matches over those 200 aligned bases is 180, then the 500-base nucleic acid target sequence contains a length of 200 and a sequence identity over that length of 90% (i.e. , 180 / 200 x 100 = 90). It will be appreciated that different regions within a single nucleic acid target sequence that aligns with an identified sequence can each have their own percent identity. It is noted that the percent identity value is rounded to the nearest tenth. For example, 78.11, 78.12, 78.13, and 78.14 are rounded down to 78.1, while 78.15, 78.16, 78.17, 78.18, and 78.19 are rounded up to 78.2. It also is noted that the length value will always be an integer.

The term "sequence" when used herein relates to nucleotide sequence(s), polynucleotide(s), nucleic acid sequence(s), nucleic acid(s), nucleic acid molecule, peptides, polypeptides and proteins, depending on the context in which the term "sequence" is used. The terms "polynucleic acid", "nucleotide sequence(s)", "polynucleotide(s)", "nucleic acid sequence(s)", "nucleic acid(s)", "nucleic acid molecule" are used interchangeably herein and refer to nucleotides, either ribonucleotides or deoxyribonucleotides or a combination of both, in a polymeric unbranched form of any length. Nucleic acid sequences include DNA, cDNA, genomic DNA, RNA, synthetic forms and mixed polymers, both sense and antisense strands, or may contain non-natural or derivatized nucleotide bases, as will be readily appreciated by those skilled in the art. Nucleotide designations as used herein are according to the table below.

| A | Adenine |
|---|---|
| C | Cytosine |
| G | Guanine |
| T (or U) | Thymine (or Uracil) |
| R | A or G |
| Y | C orT |
| S | G or C |
| W | A or T |
| K | G orT |
| M | A or C |
| B | C or G or T |
| D | A or G or T |
| H | A or C or T |
| V | A or C or G |
| N | any base |

An "isolated nucleic acid sequence", "isolated polynucleic acid" or "isolated DNA" refers to a nucleic acid sequence which is no longer in the natural environment from which it was isolated, e.g., the nucleic acid sequence in a bacterial host cell or in the plant nuclear or plastid genome. When referring to a "sequence" herein, it is understood that the molecule having such a sequence is referred to, e.g., the nucleic acid molecule. A "host cell" or a "recombinant host cell" or "transformed cell" are terms referring to a new individual cell (or organism) arising as a result of at least one nucleic acid molecule, having been introduced into said cell. The host cell is preferably a plant cell or a bacterial cell. The host cell may contain the nucleic acid as an extra-chromosomally (episomal) replicating molecule, or comprises the nucleic acid integrated in the nuclear or plastid genome of the host cell, or as introduced chromosome, e.g., minichromosome.

When reference is made to a nucleic acid sequence (e.g., DNA or genomic DNA) having "substantial sequence identity to" a reference sequence or having a sequence identity of at least 80%>, e.g., at least 85%, 90%, 95%, 98%> or 99%> nucleic acid sequence identity to a reference sequence, in one embodiment said nucleotide sequence is considered substantially identical to the given nucleotide sequence and can be identified using hybridisation conditions. In another embodiment, the nucleic acid sequence comprises one or more mutations compared to the given nucleotide sequence but still can be identified using stringent hybridisation conditions. "Stringent hybridisation conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridises to a perfectly matched probe. Typically, stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g., 100 nt) are for example those which include at least one wash in 0.2X SSC at 63°C for 20min, or equivalent conditions. Stringent conditions for DNA-DNA hybridisation (Southern blots using a probe of e.g., 100 nt) are for example those which include at least one wash (usually 2) in 0.2X SSC at a temperature of at least 50°C, usually about 55°C, for 20 min, or equivalent conditions. See also Sambrook et al. (1989) and Sambrook and Russell (2001). Examples of high stringent hybridization conditions are conditions under which primarily only those nucleic acid molecules that have at least 90% or at least 95% sequence identity undergo hybridization. Such high stringent hybridization conditions are, for example: 4 x SSC at 65°C and subsequent multiple washes in 0.1 x SSC at 65°C for approximately 1 hour. The term "high stringent hybridization conditions" as used herein may also mean: hybridization at 68°C in 0.25 M sodium phosphate, pH 7.2, 7 % SDS, 1 mM EDTA and 1 % BSA for 16 hours and subsequently washing twice with 2 x SSC and 0.1 % SDS at 68°C. Preferably, hybridization takes place under stringent conditions. Less stringent hybridization conditions are, for example: hybridizing in 4 x SSC at 37 °C and subsequent multiple washing in 1 x SSC at room temperature.

It will be understood that "specifically hybridizing" means that the polynucleic acid hybridises with the (molecular) marker allele (such as under stringent hybridisation conditions, as defined herein elsewhere), but does not (substantially) hybridise with a polynucleic acid not comprising the marker allele or is (substantially) incapable of being used as a PCR primer. By means of example, in a suitable readout, the hybridization signal with the marker allele or PCR amplification of the marker allele is at least 5 times, preferably at least 10 times stronger or more than the hybridisation signal with a non-marker allele, or any other sequence.

"Fragment" is intended to mean a portion of a nucleotide sequence. Fragments can be used as hybridization probes or PCR primers using methods disclosed herein.

When used herein, the term "polypeptide" or "protein" (both terms are used interchangeably herein) means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The term polypeptide also refers to, and does not exclude, modifications of the polypeptide, e.g., glycosylation, acetylation, phosphorylation and the like. Such modifications are well described in basic texts and in more detailed monographs, as well as in the research literature.

Amino acid substitutions encompass amino acid alterations in which an amino acid is replaced with a different naturally-occurring amino acid residue. Such substitutions may be classified as "conservative<1>, in which an amino acid residue contained in the wild-type protein is replaced with another naturally-occurring amino acid of similar character, for example Gly<→Ala, Val<→Ile<→Leu, Asp<→Glu, Lys<→Arg, Asn<→Gln or Phe<→ Trp<→Tyr. Substitutions encompassed by the present invention may also be "non-conservative", in which an amino acid residue which is present in the wild-type protein is substituted with an amino acid with different properties, such as a naturally-occurring amino acid from a different group (e.g., substituting a charged or hydrophobic amino acid with alanine. "Similar amino acids", as used herein, refers to amino acids that have similar amino acid side chains, i.e., amino acids that have polar, non-polar or practically neutral side chains. "Non-similar amino acids", as used herein, refers to amino acids that have different amino acid side chains, for example an amino acid with a polar side chain is non-similar to an amino acid with a non-polar side chain. Polar side chains usually tend to be present on the surface of a protein where they can interact with the aqueous environment found in cells ("hydrophilic" amino acids). On the other hand, "non-polar" amino acids tend to reside within the center of the protein where they can interact with similar non-polar neighbours ("hydrophobic" amino acids"). Examples of amino acids that have polar side chains are arginine, asparagine, aspartate, cysteine, glutamine, glutamate, histidine, lysine, serine, and threonine (all hydrophilic, except for cysteine which is hydrophobic). Examples of amino acids that have non-polar side chains are alanine, glycine, isoleucine, leucine, methionine, phenylalanine, proline, and tryptophan (all hydrophobic, except for glycine which is neutral).

The term "gene" when used herein refers to a polymeric form of nucleotides of any length, either ribonucleotides or desoxyribonucleotides. The term includes double- and single-stranded DNA and RNA. It also includes known types of modifications, for example, methylation, "caps", substitutions of one or more of the naturally occurring nucleotides with an analog. Preferably, a gene comprises a coding sequence encoding the herein defined polypeptide. A "coding sequence" is a nucleotide sequence which is transcribed into mRNA and/or translated into a polypeptide when placed or being under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances.

"Genome" refers to the total DNA, or the entire set of genes, carried by a chromosome or chromosome set.

The term "genotype" is the genetic constitution of an individual (or group of individuals) at one or more genetic loci, as contrasted with the observable trait (the phenotype). Genotype is defined by the allele(s) of one or more known loci that the individual has inherited from its parents. The term genotype can be used to refer to an individual's genetic constitution at a single locus, at multiple led, or, more generally, the term genotype can be used to refer to an individual's genetic make-up for all the genes in its genome.

A "haplotype" is the genotype of an individual at a plurality of genetic loci, i.e., a combination of alleles. Typically, the genetic loci described by a haplotype are physically and genetically linked, i.e., on the same chromosome segment. The term "haplotype" can further refer to sequence, polymorphisms at a particular locus, such as a single marker locus, or sequence polymorphisms at multiple loci along a chromosomal segment in a given genome. The former can also be referred to as "marker haplotypes" or "marker alleles", while the latter can be referred to as "long-range haplotypes".

The terms "phenotype", or "phenotypic trait" or "trait" refers to one or more traits of of a plant, plant part or plant cell. The phenotype can be observable to the naked eye, or by any other means of evaluation known in the art, e.g., pathogen inoculation tests, microscopy, biochemical analysis, or an electromechanical assay. In some cases, a phenotype is directly controlled by a single gene or genetic locus, i.e., a "single gene trait". In other cases, a phenotype is the result of several genes.

The "heritability ($h^2$ of a trait within a population is the proportion of observable differences in a trait between individuals within a population that is due to genetic differences. The h2 value of the QTL is a percentage of variation that is explained by genetics, instead of environment.

"Germplasm" refers to genetic material of or from an individual (e.g., a plant), a group of individuals (e.g., a plant line, variety, or family), or a clone derived from a line, variety, species, or culture. The germplasm can be part of an organism or cell or can be separate from the organism or cell. In general, germplasm provides genetic material with a specific molecular makeup that provides a physical foundation for some, or all of the hereditary qualities of an organism or cell culture. As used herein, germplasm includes cells, seed or tissues from which new plants may be grown, or plant parts, such as leaf's, stems, pollen, or cells that can be cultured into a whole plant.

As used herein, the term "endogenous" refers to a gene or allele which is present in its natural genomic location. The term "endogenous" can be used interchangeably with "native" or "wild-type". This does not however exclude the presence of one or more nucleic acid differences with the wild-type allele. In particular embodiments, the difference with a wild-type allele can be limited to less than 9 preferably less than 6, more particularly less than 3 nucleotide differences, such as 0 nucleotides difference. More particularly, the difference with the wildtype sequence can be in only one nucleotide. Preferably, the endogenous allele encodes a modified protein having less than 9, preferably less than 6, more particularly less than 3 and even more preferably only one or no amino acid difference with the wild-type protein.

A used herein, the term "exogenous polynucleotide" refers to a polynucleotide, such as a gene (or cDNA) or allele which is or has been recombinantly introduced in a cell (or plant). The exogenous polynucleotide may be episomal or genomically integrated. Integration may be random or site-directed. Integration may include replacement of a corresponding endogenous polynucleotide. It will be understood that an exogenous polynucleotide is not naturally present in the cell or plant.

The term "hybrid", "hybrid plant", or hybrid seed" as used in the context of the present invention has its ordinary meaning known in the art. By means of further guidance, and without limitation in the context of the present invention this term refers to the offspring of two (genetically distinct or different) parent plants, which may be different plant lines, cultivars, or varieties. It will be understood that according to the present invention, the parents of a hybrid plant preferably are from the same genus, preferably the same species. Preferably, the parents of a hybrid each are stable populations, having a high degree of homozygosity. The parents typically differ from each other in one or more traits or (agronomic, physiologic, or quality) characteristics. The hybrid therefore typically is heterozygous for such trait or (agronomic, physiologic, or quality) characteristic. According to the present invention, hybrids preferably are the F1 hybrids, i.e., the first generation of offspring resulting from the two parents (e.g., the two parental lines, cultivars, or varieties). The seed produced by crossing two parents is therefore the F1 hybrid seed.

Polynucleotides, QTLs, loci, markers, or alleles can be introduced by any means known in the art, for instance as described herein elsewhere, including CRISPR/Cas, ZFN, TALEN, meganucleases, RNAi, TILLING, etc.).

In certain embodiments, the mutation according to the invention as described herein is introduced by mutagenesis. In certain embodiments, the mutation according to the invention as described herein is introduced by random mutagenesis, preferably TILLING. In certain embodiments, the mutation according to the invention as described herein is introduced by TILLING. In certain embodiments, the mutation according to the invention as described herein is introduced by non-random mutagenesis. In certain embodiments, the mutation according to the invention as described herein is introduced by site-directed mutagenesis. As used herein, site-directed mutagenesis refers to sequence-specific (or sequence-dependent) or target-specific (or target-dependent) mutagenesis, as described herein elsewhere. In certain embodiments, the mutation according to the invention as described herein is introduced by gene-editing. In certain embodiments, the mutation according to the invention as described herein is introduced by genome-editing. In certain embodiments, the mutation according to the invention as described herein is introduced by designer nucleases. In certain embodiments, the mutation according to the invention as described herein is introduced by CRISPR/Cas, zinc finger nucleases, TALEN, or meganucleases, as described herein elsewhere. In certain embodiments, the polynucleotide, QTL, locus, marker, or allele as described herein according to the invention is introduced by CRISPR/Cas.

In certain embodiments, the plants or plant parts according to the invention as described herein, or generated, produced, obtained, identified, and/or selected according to the invention as described herein, are transgenic plants or plant parts.

In certain embodiments, the polynucleotide, QTL, locus, marker, or allele as described herein according to the invention as described herein is knocked-in.

In certain embodiments, the plants or plant parts according to the invention as described herein, or generated, produced, obtained, identified, and/or selected according to the invention as described herein, are from the family of *Brassicaceae.*

In certain embodiments, the plants or plant parts according to the invention as described herein, or generated, produced, obtained, identified, and/or selected according to the invention as described herein are from the genus *Brassica* or the genus *Raphanus.* In certain embodiments, the plants or plant parts according to the invention as described herein, or generated, produced, obtained, identified, and/or selected according to the invention as described herein are from the species *Brassica balearica, Brassica carinata, Brassica elongate, Brassica fruticulose, Brassica hilarionis, Brassica juncea, Brassica napus, Brassica narinosa, Brassica nigra, Brassica oleracea, Brassica perviridis, Brassica rapa (syn. B. campestris), Brassica rupestris, Brassica spinescens, Brassica tournefortii, Brassica alba, Brassica hirta, Brassica geniculate, Brassica kaber,* and *Raphanus sativus.*

In certain preferred embodiments, the plants or plant parts according to the invention as described herein, or generated, produced, obtained, identified, and/or selected according to the invention as described herein are from the species *Brassica napus* or *Brassica rapa.*

In certain embodiments, the plants or plant parts according to the invention as described herein, or generated, produced, obtained, identified, and/or selected according to the invention as described herein are hybrids between different species from the Brassicaceae family. In certain embodiments, the plants or plant parts according to the invention as described herein, or generated, produced, obtained, identified, and/or selected according to the invention as described herein are hybrids between different species from the *Brassica* genus. In certain embodiments, the plants or plant parts according to the invention as described herein, or generated, produced, obtained, identified, and/or selected according to the invention as described herein are hybrids between different species selected from *Brassica balearica, Brassica carinata, Brassica elongate, Brassica fruticulose, Brassica hilarionis, Brassica juncea, Brassica napus, Brassica narinosa, Brassica nigra, Brassica oleracea, Brassica perviridis, Brassica rapa (syn. B. campestris), Brassica rupestris, Brassica spinescens, Brassica tournefortii, Brassica alba, Brassica hirta, Brassica geniculate, Brassica kaber,* and *Raphanus sativus.* In certain embodiments, the plants or plant parts according to the invention as described herein, or generated, produced, obtained, identified, and/or selected according to the invention as described herein are hybrids between *Brassica napus* and *Brassica rapa.*

In certain embodiments, the plant or plant part according to the invention as described herein, or generated, produced, obtained, identified, and/or selected according to the invention as described herein, is a crop plant or crop plant part.

As a reference plant or plant part, an isogenic line may be used, i.e., a plant otherwise identical apart from the (homozygous) clubroot resistance polynucleotide, QTL, locus, or marker(s) as referred to herein elsewhere.

In certain embodiments, the plants or plant parts according to the invention as described herein, or generated, produced, obtained, identified, and/or selected according to the invention as described herein can be used in hybrid breeding or for generating hybrid plants or plant part, such as hybrid seeds. Accordingly, in an aspect, the invention relates to the use of the plants or plant parts according to the invention as described herein, or generated, produced, obtained, identified, and/or selected according to the invention as described herein in hybrid breeding, or for generating hybrid plants or plant parts, such as hybrid seeds.

In an aspect, the invention relates to a method for identifying and/or selecting a plant or plant part of the *Brassicaceae* family, comprising screening for the presence of a QTL (allele) (on a polynucleotide located) on a chromosomal interval spanning at most 10 cM, preferably at most 5 cM, more preferably at most 2 cM respectively upstream and downstream of molecular marker (allele) ra74856s01 and ra36444s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 5 cM, preferably at most 2 cM respectively upstream and downstream of molecular marker (allele) ra74856s01 and ra36444s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 2 cM respectively upstream and downstream of molecular marker (allele) ra74856s01 and ra36444s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 10 Mbp (i.e., 10 mega base pairs), preferably at most 5 Mbp, more preferably at most 2 Mbp respectively upstream and downstream of molecular marker (allele) ra74856s01 and ra36444s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 5 Mbp, preferably at most 2 Mbp respectively upstream and downstream of molecular marker (allele) ra74856s01 and ra36444s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 2 Mbp respectively upstream and downstream of molecular marker (allele) ra74856s01 and ra36444s01 (in the genome of the plant or plant part).

In certain embodiments, the chromosomal interval is flanked by molecular marker (allele) ra74856s01 and ra36444s01.

In certain embodiments, the chromosomal interval is flanked by and includes molecular marker (allele) ra74856s01 and ra36444s01.

In certain embodiments, the chromosomal interval is flanked by and excludes molecular marker (allele) ra74856s01 and ra36444s01.

As referred to herein, a polynucleic acid, such as for instance a QTL (allele) as described herein, is said to be flanked by certain molecular markers or molecular marker alleles if the polynucleic acid is comprised within a polynucleic acid wherein respectively a first marker (allele) is located upstream (i.e., 5') of said polynucleic acid and a second marker (allele) is located downstream (i.e., 3') of said polynucleic acid. Such first and second marker (allele) may border the polynucleic acid. The nucleic acid may equally comprise such first and second marker (allele), such as respectively at or near the 5' and 3' end, for instance respectively within 50 kb of the 5' and 3' end, preferably within 10 kb of the 5' and 3' end, such as within 5 kb of the 5' and 3' end, within 1 kb of the 5' and 3' end, or less.

In an aspect, the invention relates to a method for identifying and/or selecting a plant or plant part of the *Brassicaceae* family, such as a plant or plant part of the *Brassicaceae* family having (increased) clubroot resistance or tolerance, such as in comparison to a plant or plant part of the Brassicaceae family not having said polynucleotide, molecular marker (allele), locus (allele), QTL (allele), or SNP (allele) of the invention, comprising screening for the presence of a QTL (allele) (on a polynucleotide located) on a chromosomal interval comprising, comprised in a polynucleotide comprising, or flanked by molecular marker (allele) ra74856s01 and ra36444s01 (in the genome of the plant or plant part).

In an aspect, the invention relates to a method for identifying and/or selecting a plant or plant part of the *Brassicaceae* family, such as a plant or plant part of the *Brassicaceae* family having (increased) clubroot resistance or tolerance, such as in comparison to a plant or plant part of the Brassicaceae family not having said polynucleotide, molecular marker (allele), locus (allele), QTL (allele), or SNP (allele) of the invention, comprising screening for the presence of a QTL (allele) (on a polynucleotide located) on a chromosomal interval comprising, comprised in a polynucleotide comprising, or flanked by and including molecular marker (allele) ra74856s01 and ra36444s01 (in the genome of the plant or plant part).

In an aspect, the invention relates to a method for identifying and/or selecting a plant or plant part of the *Brassicaceae* family, such as a plant or plant part of the *Brassicaceae* family having (increased) clubroot resistance or tolerance, such as in comparison to a plant or plant part of the Brassicaceae family not having said polynucleotide, molecular marker (allele), locus (allele), QTL (allele), or SNP (allele) of the invention, comprising screening for the presence of a QTL (allele) (on a polynucleotide located) on a chromosomal interval comprising, comprised in a polynucleotide comprising, or flanked by and excluding molecular marker (allele) ra74856s01 and ra36444s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 10 cM, preferably at most 5 cM, more preferably at most 2 cM respectively upstream and downstream of molecular marker (allele) ra74862s01 and ra74867s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 5 cM, preferably at most 2 cM respectively upstream and downstream of molecular marker (allele) ra74862s01 and ra74867s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 2 cM respectively upstream and downstream of molecular marker (allele) ra74862s01 and ra74867s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 10 Mbp, preferably at most 5 Mbp, more preferably at most 2 Mbp respectively upstream and downstream of molecular marker (allele) ra74862s01 and ra74867s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 5 Mpb, preferably at most 2 Mbp respectively upstream and downstream of molecular marker (allele) ra74862s01 and ra74867s01 (in the genome of the plant or plant part).

In an aspect, the invention relates to a method for identifying and/or selecting a plant or plant part of the *Brassicaceae* family, comprising screening for the presence of a QTL (allele) (on a polynucleotide located) on a chromosomal interval spanning at most 2 Mbp respectively upstream and downstream of molecular marker (allele) ra74862s01 and ra74867s01 (in the genome of the plant or plant part).

In certain embodiments, the chromosomal interval is flanked by molecular marker (allele) ra74862s01 and ra74867s01.

In certain embodiments, the chromosomal interval is flanked by and includes molecular marker (allele) ra74862s01 and ra74867s01.

In certain embodiments, the chromosomal interval is flanked by and excludes molecular marker (allele) ra74862s01 and ra74867s01.

In certain embodiments, the chromosomal interval is flanked by molecular marker (allele) ra74856s01 and ra36444s01.

In certain embodiments, the chromosomal interval is flanked by and includes molecular marker (allele) ra74856s01 and ra36444s01.

In certain embodiments, the chromosomal interval is flanked by and excludes molecular marker (allele) ra74856s01 and ra36444s01.

In an aspect, the invention relates to a method for identifying and/or selecting a plant or plant part of the *Brassicaceae* family, such as a plant or plant part of the *Brassicaceae* family having (increased) clubroot resistance or tolerance, comprising screening for the presence of a QTL (allele) (on a polynucleotide located) on a chromosomal interval comprising, comprised in a polynucleotide comprising, or flanked by molecular marker (allele) ra74862s01 and ra74867s01 (in the genome of the plant or plant part).

In an aspect, the invention relates to a method for identifying and/or selecting a plant or plant part of the *Brassicaceae* family, such as a plant or plant part of the *Brassicaceae* family having (increased) clubroot resistance or tolerance, comprising screening for the presence of a QTL (allele) (on a polynucleotide located) on a chromosomal interval comprising, comprised in a polynucleotide comprising, or flanked by and including molecular marker (allele) ra74862s01 and ra74867s01 (in the genome of the plant or plant part).

In an aspect, the invention relates to a method for identifying and/or selecting a plant or plant part of the *Brassicaceae* family, such as a plant or plant part of the *Brassicaceae* family having (increased) clubroot resistance or tolerance, comprising screening for the presence of a QTL (allele) (on a polynucleotide located) on a chromosomal interval comprising, comprised in a polynucleotide comprising, or flanked by and excluding molecular marker (allele) ra74862s01 and ra74867s01 (in the genome of the plant or plant part).

In certain embodiments, the polynucleotide or QTL comprises one or more of molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01.

In certain embodiments, the polynucleotide or QTL comprises five or more of molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01.

In certain embodiments, the polynucleotide or QTL comprises 10 or more of molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01.

In certain embodiments, the polynucleotide or QTL comprises 20 or more of molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01.

In certain embodiments, the polynucleotide or QTL comprises molecular marker (allele) ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01.

In certain embodiments, the polynucleotide or QTL comprises one or more of molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, and ra36443s01.

In certain embodiments, the polynucleotide or QTL comprises 5 or more of molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, and ra36443s01.

In certain embodiments, the polynucleotide or QTL comprises 10 or more of molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, and ra36443s01.

In certain embodiments, the polynucleotide or QTL comprises molecular marker (allele) ra36430s01, ra36433s01, ra36434s01, ra74862s01, ra36435s01, ra74863s01, ra74864s01, ra74910s01, ra74865s01, ra74952s01, ra36441s01, ra74867s01, and ra36443s01.

In certain embodiments, the polynucleotide or QTL comprises one or more of molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01.

In certain embodiments, the polynucleotide or QTL comprises 5 or more of molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01.

In certain embodiments, the polynucleotide or QTL comprises 10 or more of molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01.

In certain embodiments, the polynucleotide or QTL comprises molecular marker (allele) ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01.

In certain embodiments, the polynucleotide or QTL comprises one or more of molecular marker (allele) selected from ra74862s01, ra74863s01, ra74864s01, ra74910s01, ra74865s01, ra74952s01, and ra74867s01.

In certain embodiments, the polynucleotide or QTL comprises 5 or more of molecular marker (allele) selected from ra74862s01, ra74863s01, ra74864s01, ra74910s01, ra74865s01, ra74952s01, and ra74867s01.

In certain embodiments, the polynucleotide or QTL comprises molecular marker (allele) ra74862s01, ra74863s01, ra74864s01, ra74910s01, ra74865s01, ra74952s01, and ra74867s01.

In certain embodiments, the polynucleotide or QTL comprises one or more of molecular marker (allele) selected from ra74862s01 and ra74867s01.

In certain embodiments, the polynucleotide or QTL comprises molecular marker (allele) ra74862s01 and ra74867s01.

In certain embodiments, the method comprises screening for the presence of (a polynucleotide comprising) one or more molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01 (in the genome of the plant or plant part).

In certain embodiments, the method comprises screening for the presence of (a polynucleotide comprising) one or more molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, and ra36443s01 (in the genome of the plant or plant part).

In certain embodiments, the method comprises screening for the presence of (a polynucleotide comprising) one or more molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01 (in the genome of the plant or plant part).

In certain embodiments, the method comprises screening for the presence of (a polynucleotide comprising) one or more molecular marker (allele) selected from ra74862s01, ra74863s01, ra74864s01, ra74910s01, ra74865s01, ra74952s01, and ra74867s01 (in the genome of the plant or plant part).

In certain embodiments, the method comprises screening for the presence of (a polynucleotide comprising) one or more molecular marker (allele) selected from ra74862s01 and ra74867s01 (in the genome of the plant or plant part).

In an aspect, the invention relates to a method for identifying and/or selecting a plant or plant part of the *Brassicaceae* family, such as a plant or plant part of the *Brassicaceae* family having (increased) clubroot resistance or tolerance, comprising screening for the presence of (a polynucleotide comprising) one or more molecular marker (allele) selected from ra74856s01, ra74921s01, ra36415s01, ra74945s01, ra74946s01, ra74948s01, ra36418s01, ra05569s01, ra36419s01, ra74858s01, ra74859s01, ra36423s01, ra74922s01, ra74860s01, ra36426s01, ra36427s01, ra36428s01, ra36429s01, ra36430s01, ra36433s01, ra36434s01, ra74862s01, ra36435s01, ra74863s01, ra74864s01, ra74910s01, ra74865s01, ra74952s01, ra36441s01, ra74867s01, ra36443s01, and ra36444s01 (in the genome of the plant or plant part).

In an aspect, the invention relates to a method for identifying and/or selecting a plant or plant part of the *Brassicaceae* family, such as a plant or plant part of the *Brassicaceae* family having (increased) clubroot resistance or tolerance, comprising screening for the presence of (a polynucleotide comprising) one or more molecular marker (allele) selected from ra36430s01, ra36433s01, ra36434s01, ra74862s01, ra36435s01, ra74863s01, ra74864s01, ra74910s01, ra74865s01, ra74952s01, ra36441s01, ra74867s01, and ra36443s01 (in the genome of the plant or plant part).

In an aspect, the invention relates to a method for identifying and/or selecting a plant or plant part of the *Brassicaceae* family, such as a plant or plant part of the *Brassicaceae* family having (increased) clubroot resistance or tolerance, comprising screening for the presence of (a polynucleotide comprising) one or more molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01 (in the genome of the plant or plant part).

In an aspect, the invention relates to a method for identifying and/or selecting a plant or plant part of the *Brassicaceae* family, such as a plant or plant part of the *Brassicaceae* family having (increased) clubroot resistance or tolerance, comprising screening for the presence of (a polynucleotide comprising) one or more molecular marker (allele) selected from ra74862s01, ra74863s01, ra74864s01, ra74910s01, ra74865s01, ra74952s01, and ra74867s01 (in the genome of the plant or plant part).

In an aspect, the invention relates to a method for identifying and/or selecting a plant or plant part of the *Brassicaceae* family, such as a plant or plant part of the *Brassicaceae* family having (increased) clubroot resistance or tolerance, comprising screening for the presence of (a polynucleotide comprising) one or more molecular marker (allele) selected from ra74862s01 and ra74867s01 (in the genome of the plant or plant part).

In certain embodiments, the polynucleic acid comprises or is comprised in a QTL (allele) (on a polynucleotide located) on a chromosomal interval spanning at most 10 cM, preferably at most 5 cM, more preferably at most 2 cM respectively upstream and downstream of molecular marker (allele) ra74856s01 and ra36444s01 (in the genome of the plant or plant part).

In certain embodiments, the polynucleic acid comprises or is comprised in a QTL (allele) (on a polynucleotide located) on a chromosomal interval spanning at most 5 cM, preferably at most 2 cM respectively upstream and downstream of molecular marker (allele) ra74856s01 and ra36444s01 (in the genome of the plant or plant part).

In certain embodiments, the polynucleic acid comprises or is comprised in a QTL (allele) (on a polynucleotide located) on a chromosomal interval spanning at most 2 cM respectively upstream and downstream of molecular marker (allele) ra74856s01 and ra36444s01 (in the genome of the plant or plant part).

In certain embodiments, the one or more marker(s) are comprised in a QTL (allele) (on a polynucleotide located) on a chromosomal interval spanning at most 10 cM, preferably at most 5 cM, more preferably at most 2 cM respectively upstream and downstream of molecular marker (allele) ra74856s01 and ra36444s01 (in the genome of the plant or plant part).

In certain embodiments, the one or more marker(s) are comprised in a QTL (allele) (on a polynucleotide located) on a chromosomal interval spanning at most 5 cM, preferably at most 2 cM respectively upstream and downstream of molecular marker (allele) ra74856s01 and ra36444s01 (in the genome of the plant or plant part).

In certain embodiments, the one or more marker(s) are comprised in a QTL (allele) (on a polynucleotide located) on a chromosomal interval spanning at most 2 cM respectively upstream and downstream of molecular marker (allele) ra74856s01 and ra36444s01 (in the genome of the plant or plant part).

In certain embodiments, the polynucleic acid comprises or is comprised in a QTL (allele) (on a polynucleotide located) on a chromosomal interval spanning at most 10 Mbp, preferably at most 5 Mbp, more preferably at most 2 Mbp respectively upstream and downstream of molecular marker (allele) ra74856s01 and ra36444s01 (in the genome of the plant or plant part).

In certain embodiments, the polynucleic acid comprises or is comprised in a QTL (allele) (on a polynucleotide located) on a chromosomal interval spanning at most 5 Mbp, preferably at most 2 Mbp respectively upstream and downstream of molecular marker (allele) ra74856s01 and ra36444s01 (in the genome of the plant or plant part).

In certain embodiments, the polynucleic acid comprises or is comprised in a QTL (allele) (on a polynucleotide located) on a chromosomal interval spanning at most 2 Mbp respectively upstream and downstream of molecular marker (allele) ra74856s01 and ra36444s01 (in the genome of the plant or plant part).

In certain embodiments, the one or more marker(s) are comprised in a QTL (allele) (on a polynucleotide located) on a chromosomal interval spanning at most 10 Mbp, preferably at most 5 Mbp, more preferably at most 2 Mbp respectively upstream and downstream of molecular marker (allele) ra74856s01 and ra36444s01 (in the genome of the plant or plant part).

In certain embodiments, the one or more marker(s) are comprised in a QTL (allele) (on a polynucleotide located) on a chromosomal interval spanning at most 5 Mbp, preferably at most 2 Mbp respectively upstream and downstream of molecular marker (allele) ra74856s01 and ra36444s01 (in the genome of the plant or plant part).

In certain embodiments, the one or more marker(s) are comprised in a QTL (allele) (on a polynucleotide located) on a chromosomal interval spanning at most 2 Mbp respectively upstream and downstream of molecular marker (allele) ra74856s01 and ra36444s01 (in the genome of the plant or plant part).

In certain embodiments, the chromosomal interval is flanked by molecular marker (allele) ra74856s01 and ra36444s01.

In certain embodiments, the chromosomal interval is flanked by and includes molecular marker (allele) ra74856s01 and ra36444s01.

In certain embodiments, the chromosomal interval is flanked by and excludes molecular marker (allele) ra74856s01 and ra36444s01.

In certain embodiments, the polynucleic acid comprises or is comprised in a QTL (allele) (on a polynucleotide located) on a chromosomal interval spanning at most 10 cM, preferably at most 5 cM, more preferably at most 2 cM respectively upstream and downstream of molecular marker (allele) ra74862s01 and ra74867s01 (in the genome of the plant or plant part).

In certain embodiments, the polynucleic acid comprises or is comprised in a QTL (allele) (on a polynucleotide located) on a chromosomal interval spanning at most 5 cM, preferably at most 2 cM respectively upstream and downstream of molecular marker (allele) ra74862s01 and ra74867s01 (in the genome of the plant or plant part).

In certain embodiments, the polynucleic acid comprises or is comprised in a QTL (allele) (on a polynucleotide located) on a chromosomal interval spanning at most 2 cM respectively upstream and downstream of molecular marker (allele) ra74862s01 and ra74867s01 (in the genome of the plant or plant part).

In certain embodiments, the one or more marker(s) are comprised in a QTL (allele) (on a polynucleotide located) on a chromosomal interval spanning at most 10 cM, preferably at most 5 cM, more preferably at most 2 cM respectively upstream and downstream of molecular marker (allele) ra74862s01 and ra74867s01 (in the genome of the plant or plant part).

In certain embodiments, the one or more marker(s) are comprised in a QTL (allele) (on a polynucleotide located) on a chromosomal interval spanning at most 5 cM, preferably at most 2 cM respectively upstream and downstream of molecular marker (allele) ra74862s01 and ra74867s01 (in the genome of the plant or plant part).

In certain embodiments, the one or more marker(s) are comprised in a QTL (allele) (on a polynucleotide located) on a chromosomal interval spanning at most 2 cM respectively upstream and downstream of molecular marker (allele) ra74862s01 and ra74867s01 (in the genome of the plant or plant part).

In certain embodiments, the polynucleic acid comprises or is comprised in a QTL (allele) (on a polynucleotide located) on a chromosomal interval spanning at most 10 Mbp, preferably at most 5 Mbp, more preferably at most 2 Mbp respectively upstream and downstream of molecular marker (allele) ra74862s01 and ra74867s01 (in the genome of the plant or plant part).

In certain embodiments, the polynucleic acid comprises or is comprised in a QTL (allele) (on a polynucleotide located) on a chromosomal interval spanning at most 5 Mbp, preferably at most 2 Mbp respectively upstream and downstream of molecular marker (allele) ra74862s01 and ra74867s01 (in the genome of the plant or plant part).

In certain embodiments, the polynucleic acid comprises or is comprised in a QTL (allele) (on a polynucleotide located) on a chromosomal interval spanning at most 2 Mbp respectively upstream and downstream of molecular marker (allele) ra74862s01 and ra74867s01 (in the genome of the plant or plant part).

In certain embodiments, the one or more marker(s) are comprised in a QTL (allele) (on a polynucleotide located) on a chromosomal interval spanning at most 10 Mbp, preferably at most 5 Mbp, more preferably at most 2 Mbp respectively upstream and downstream of molecular marker (allele) ra74862s01 and ra74867s01 (in the genome of the plant or plant part).

In certain embodiments, the one or more marker(s) are comprised in a QTL (allele) (on a polynucleotide located) on a chromosomal interval spanning at most 5 Mbp, preferably at most 2 Mbp respectively upstream and downstream of molecular marker (allele) ra74862s01 and ra74867s01 (in the genome of the plant or plant part).

In certain embodiments, the one or more marker(s) are comprised in a QTL (allele) (on a polynucleotide located) on a chromosomal interval spanning at most 2 Mbp respectively upstream and downstream of molecular marker (allele) ra74862s01 and ra74867s01 (in the genome of the plant or plant part).

In certain embodiments, the chromosomal interval is flanked by molecular marker (allele) ra74862s01 and ra74867s01.

In certain embodiments, the chromosomal interval is flanked by and includes molecular marker (allele) ra74862s01 and ra74867s01.

In certain embodiments, the chromosomal interval is flanked by and excludes molecular marker (allele) ra74862s01 and ra74867s01.

In certain embodiments, the polynucleic acid comprises or is comprised in a QTL (allele) (on a polynucleotide located) on a chromosomal interval comprising, comprised in a polynucleotide comprising, or flanked by molecular marker (allele) ra74856s01 and ra36444s01 (in the genome of the plant or plant part).

In certain embodiments, the polynucleic acid comprises or is comprised in a QTL (allele) (on a polynucleotide located) on a chromosomal interval comprising, comprised in a polynucleotide comprising, or flanked by and including molecular marker (allele) ra74856s01 and ra36444s01 (in the genome of the plant or plant part).

In certain embodiments, the polynucleic acid comprises or is comprised in a QTL (allele) (on a polynucleotide located) on a chromosomal interval comprising, comprised in a polynucleotide comprising, or flanked by and excluding molecular marker (allele) ra74856s01 and ra36444s01 (in the genome of the plant or plant part).

In certain embodiments, the polynucleic acid comprises or is comprised in a QTL (allele) (on a polynucleotide located) on a chromosomal interval comprising, comprised in a polynucleotide comprising, or flanked by molecular marker (allele) ra74862s01 and ra74867s01 (in the genome of the plant or plant part).

In certain embodiments, the polynucleic acid comprises or is comprised in a QTL (allele) (on a polynucleotide located) on a chromosomal interval comprising, comprised in a polynucleotide comprising, or flanked by and including molecular marker (allele) ra74862s01 and ra74867s01 (in the genome of the plant or plant part).

In certain embodiments, the polynucleic acid comprises or is comprised in a QTL (allele) (on a polynucleotide located) on a chromosomal interval comprising, comprised in a polynucleotide comprising, or flanked by and excluding molecular marker (allele) ra74862s01 and ra74867s01 (in the genome of the plant or plant part).

In certain embodiments, the one or more marker(s) are comprised in a QTL (allele) (on a polynucleotide located) on a chromosomal interval comprising, comprised in a polynucleotide comprising, or flanked by molecular marker (allele) ra74856s01 and ra36444s01 (in the genome of the plant or plant part).

In certain embodiments, the one or more marker(s) are comprised in a QTL (allele) (on a polynucleotide located) on a chromosomal interval comprising, comprised in a polynucleotide comprising, or flanked by and including molecular marker (allele) ra74856s01 and ra36444s01 (in the genome of the plant or plant part).

In certain embodiments, the one or more marker(s) are comprised in a QTL (allele) (on a polynucleotide located) on a chromosomal interval comprising, comprised in a polynucleotide comprising, or flanked by and excluding molecular marker (allele) ra74856s01 and ra36444s01 (in the genome of the plant or plant part).

In certain embodiments, the one or more marker(s) are comprised in a QTL (allele) (on a polynucleotide located) on a chromosomal interval comprising, comprised in a polynucleotide comprising, or flanked by molecular marker (allele) ra74862s01 and ra74867s01 (in the genome of the plant or plant part).

In certain embodiments, the one or more marker(s) are comprised in a QTL (allele) (on a polynucleotide located) on a chromosomal interval comprising, comprised in a polynucleotide comprising, or flanked by and including molecular marker (allele) ra74862s01 and ra74867s01 (in the genome of the plant or plant part).

In certain embodiments, the one or more marker(s) are comprised in a QTL (allele) (on a polynucleotide located) on a chromosomal interval comprising, comprised in a polynucleotide comprising, or flanked by and excluding molecular marker (allele) ra74862s01 and ra74867s01 (in the genome of the plant or plant part).

In certain embodiments, the polynucleic acid, QTL, locus, and/or allele of the invention as described herein comprising molecular marker (allele) ra74856s01. In certain embodiments, the polynucleic acid, QTL, locus, and/or allele of the invention as described herein comprising molecular marker (allele) ra74921s01. In certain embodiments, the polynucleic acid, QTL, locus, and/or allele of the invention as described herein comprising molecular marker (allele) ra36415s01. In certain embodiments, the polynucleic acid, QTL, locus, and/or allele of the invention as described herein comprising molecular marker (allele) ra74945s01. In certain embodiments, the polynucleic acid, QTL, locus, and/or allele of the invention as described herein comprising molecular marker (allele) ra74946s01. In certain embodiments, the polynucleic acid, QTL, locus, and/or allele of the invention as described herein comprising molecular marker (allele) ra74948s01. In certain embodiments, the polynucleic acid, QTL, locus, and/or allele of the invention as described herein comprising molecular marker (allele) ra36418s01. In certain embodiments, the polynucleic acid, QTL, locus, and/or allele of the invention as described herein comprising molecular marker (allele) ra05569s01. In certain embodiments, the polynucleic acid, QTL, locus, and/or allele of the invention as described herein comprising molecular marker (allele) ra36419s01. In certain embodiments, the polynucleic acid, QTL, locus, and/or allele of the invention as described herein comprising molecular marker (allele) ra74858s01. In certain embodiments, the polynucleic acid, QTL, locus, and/or allele of the invention as described herein comprising molecular marker (allele) ra74859s01. In certain embodiments, the polynucleic acid, QTL, locus, and/or allele of the invention as described herein comprising molecular marker (allele) ra36423s01. In certain embodiments, the polynucleic acid, QTL, locus, and/or allele of the invention as described herein comprising molecular marker (allele) ra74922s01. In certain embodiments, the polynucleic acid, QTL, locus, and/or allele of the invention as described herein comprising molecular marker (allele) ra74860s01. In certain embodiments, the polynucleic acid, QTL, locus, and/or allele of the invention as described herein comprising molecular marker (allele) ra36426s01. In certain embodiments, the polynucleic acid, QTL, locus, and/or allele of the invention as described herein comprising molecular marker (allele) ra36427s01. In certain embodiments, the polynucleic acid, QTL, locus, and/or allele of the invention as described herein comprising molecular marker (allele) ra36428s01. In certain embodiments, the polynucleic acid, QTL, locus, and/or allele of the invention as described herein comprising molecular marker (allele) ra36429s01. In certain embodiments, the polynucleic acid, QTL, locus, and/or allele of the invention as described herein comprising molecular marker (allele) ra36430s01. In certain embodiments, the polynucleic acid, QTL, locus, and/or allele of the invention as described herein comprising molecular marker (allele) ra36433s01. In certain embodiments, the polynucleic acid, QTL, locus, and/or allele of the invention as described herein comprising molecular marker (allele) ra36434s01. In certain embodiments, the polynucleic acid, QTL, locus, and/or allele of the invention as described herein comprising molecular marker (allele) ra74862s01. In certain embodiments, the polynucleic acid, QTL, locus, and/or allele of the invention as described herein comprising molecular marker (allele) ra36435s01. In certain embodiments, the polynucleic acid, QTL, locus, and/or allele of the invention as described herein comprising molecular marker (allele) ra74863s01. In certain embodiments, the polynucleic acid, QTL, locus, and/or allele of the invention as described herein comprising molecular marker (allele) ra74864s01. In certain embodiments, the polynucleic acid, QTL, locus, and/or allele of the invention as described herein comprising molecular marker (allele) ra74910s01. In certain embodiments, the polynucleic acid, QTL, locus, and/or allele of the invention as described herein comprising molecular marker (allele) ra74865s01. In certain embodiments, the polynucleic acid, QTL, locus, and/or allele of the invention as described herein comprising molecular marker (allele) ra74952s01. In certain embodiments, the polynucleic acid, QTL, locus, and/or allele of the invention as described herein comprising molecular marker (allele) ra36441s01. In certain embodiments, the polynucleic acid, QTL, locus, and/or allele of the invention as described herein comprising molecular marker (allele) ra74867s01. In certain embodiments, the polynucleic acid, QTL, locus, and/or allele of the invention as described herein comprising molecular marker (allele) ra36443s01. In certain embodiments, the polynucleic acid, QTL, locus, and/or allele of the invention as described herein comprising molecular marker (allele) ra36444s01.

In certain embodiments, the plant or plant part is identified if the polynucleotide, QTL, locus, marker(s), and/or allele(s) of the invention as described herein are identified (in the genome of the plant or plant part).

In certain embodiments, the plant or plant part is identified as having (increased) clubroot resistance and/or tolerance, if the polynucleotide, QTL, locus, marker(s), and/or allele(s) of the invention as described herein are identified (in the genome of the plant or plant part).

In certain embodiments, the plant or plant part is identified as having clubroot resistance and/or tolerance, if the polynucleotide, QTL, locus, marker(s), and/or allele(s) of the invention as described herein are identified (in the genome of the plant or plant part).

In certain embodiments, the plant or plant part is identified as having increased clubroot resistance and/or tolerance, if the polynucleotide, QTL, locus, marker(s), and/or allele(s) of the invention as described herein are identified (in the genome of the plant or plant part).

In certain embodiments, the methods for identifying or selecting the plant or plant part of the invention as described herein further comprise selecting the plant or plant part if the polynucleotide, QTL, locus, marker(s), and/or allele(s) of the invention as described herein are identified (in the genome of the plant or plant part).

Preferred molecular markers of the invention as described herein are ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01.

More preferred molecular markers of the invention as described herein are ra74862s01, ra74863s01, ra74864s01, ra74910s01, ra74865s01, ra74952s01, and ra74867s01.

Most preferred molecular markers of the invention as described herein are ra74862s01 and ra74867s01.

The polynucleic acid, QTL, locus, marker(s), and/or allele(s) of the present invention as described herein may be, and preferably are, located on chromosome A06 of *Brassica* napus, or the corresponding chromosome of another *Brassicaceae* species, or another chromosome comprising the corresponding locus in another Brassicaceae species. The skilled person is well aware which chromosomes or chromosomal regions correspond between *Brassicaceae* species, based on the genomic context, as is known in the art. The skilled person will further appreciate that for instance translocations of certain genomic segments during evolution, may result in otherwise corresponding chromosomal regions not necessarily residing anymore on corresponding chromosomes. Nevertheless, the local genetic context may identify such regions (possibly on different chromosomes) as "corresponding".

As used herein the terms "increased pathogen tolerance" and "increased pathogen resistance", or more generally "resistance" or "tolerance" and the like, relate to any relief from, reduced presentation of, improvement of, or any combination thereof of any symptom (such as damage or loss in biomass, in dry matter yield/content or in seed yield) of an infection by a pathogen. Increased pathogen resistance or tolerance as referred to herein may also relate to the ability to which a plant maintains for instance its biomass production (such as harvestable biomass production, such as seed yield) upon or during pathogen infection. A (increased) pathogen resistant or tolerant plant, plant cell or plant part may refer herein to a plant, plant cell or plant part, respectively, having increased resistance/tolerance to a pathogen compared to a (parent) plant from which they are derived (e.g., not comprising the QTL allele or one or more of the molecular marker (allele), SNP (allele), locus (allele), or (resistant conferring) polynucleic acid according to the invention as described herein). Resistance may relate herein to the plant's ability to limit pathogen multiplication. Tolerance may relate herein to a plant's ability to reduce the effect of infection on its fitness regardless of the level of pathogen multiplication. Methods of determining pathogen resistance/tolerance are known to the person of skill in the art, such as visual scoring of pathogen infection or pathogen-induced damage, determination of biomass (yield), etc. As used herein, the terms "increased pathogen tolerance" and "increased pathogen resistance" may be used interchangeably with "reduced sensitivity" or "reduced susceptibility" towards pathogens. Accordingly, a plant, plant part, or plant population according to the invention which is more resistant or more tolerant towards a pathogen is considered less sensitive toward such pathogen. Less sensitive or less susceptible when used herein may be seen as "more tolerant" or "more resistant". Similarly, "more tolerant" or "more resistant" may, vice versa, be seen as "less sensitive" or "less susceptible". More sensitive or more susceptible when used herein may, vice versa, be seen as "less tolerant" or "less resistant". Similarly, "less tolerant" or "less resistant" may, vice versa, be seen as "more sensitive" or "more susceptible". According to the invention, (increased) resistance or tolerance is referred to as (increased) clubroot resistance or tolerance, as described herein elsewhere.

In certain embodiments, pathogen infestation is reduced. In certain embodiments, pathogen infestation is delayed. In certain embodiments, pathogen symptoms are reduced. In certain embodiments, pathogen symptoms are delayed. In certain embodiments, infestation and symptoms are reduced or delayed.

The plants, plant parts or plant populations as described herein having increased pathogen resistance or tolerance can be used to control pathogen infestation or infection. Accordingly, in an aspect, the invention relates to the use of such plants for controlling pathogen infestation or infection. Preferably pathogen infestation or infection is controlled by reduction of pathogen infestation or infection or as reduction in the symptoms of pathogen infestation or infection, at the plant, plant part, or plant population level, such as further described below.

In certain embodiments, an increased resistance or tolerance may present itself as a reduction of infection or infestation (e.g. the amount of pathogens (e.g., per plant area or per plant biomass), the multiplication (rate) or spread (rate)/distribution of pathogens, as well as the speed of pathogen spreading such as at a specific time during the (growth) season) at the (sub) plant level (such as for instance particular cells, organs, or tissues, in particular roots) or at the population level, such as a reduction of at least 5%, preferably at least 10%, such as at least 20%, at least 30%, at least 40%, at least 50%, at least 60%, at least 70%, at least 80%, at least 90%, or (about) 100%. At the population level, an increased resistance or tolerance may present itself as a reduction of infection or infestation as described above, but also for instance as a reduction in the amount of infected plants (or a combination), such as a reduction of at least 5%, preferably at least 10%, such as at least 20%, at least 30%, at least 40%, at least 50%, at least 60%, at least 70%, at least 80%, at least 90%, or (about) 100%. It will be understood that such reduction of infection or infestation can be relative to a reference plant (part) or population (such as a corresponding wild-type plant not according to the invention).

In certain embodiments, an increased resistance or tolerance may present itself as a reduction in the loss of biomass or yield in general or of a particular (harvestable) plant part (such as seed or fruit amount or weight) due to or as a consequence of pathogen infection. In certain embodiments, the resistant or tolerant plants exhibit a loss in biomass production (such as expressed in g/day or kg/ha or kg/ha/day, such as expressed as dry matter for instance expressed as weight percent) under pathogen infection which is at least 1%, preferably at least 2%, such as at least 3%, at least 4%, at least 5%, such as at least 10%, at least 15%, or at least 20% or more, lower than corresponding control plants, such as plants which are less resistant or tolerant, or plants not according to the invention as described herein. In certain embodiments, the resistant or tolerant plants exhibit biomass production (such as expressed in g/day or kg/ha or kg/ha/day, such as expressed as dry matter for instance expressed as weight percent) under pathogen infection which is at least 1%, preferably at least 2%, such as at least 3%, at least 4%, at least 5%, such as at least 10%, at least 15%, or at least 20% or more, higher than corresponding control plants, such as plants which are less resistant or tolerant, or plants not according to the invention as described herein.

As used herein, the term "yield potential" refers to maximum yield obtainable at harvest.

For the evaluation of (increased) clubroot resistance or tolerance, resistance or tolerance may be compared between a plant (or plant part) according to the present invention and a plant or plant part not according to the present invention, i.e., a plant (or plant part) not comprising the polynucleotide, QTL, locus, molecular marker(s), or SNP(s) of the invention. Such plant (or plant part) may for instance be a reference plant (or plant part derived therefrom), such as for instance the Brassica napus-bzh reference genome plant (v4.1) as described herein elsewhere. Such plant may alternatively be a (near) isogenic line (otherwise (substantially) identical to the plant of the invention, apart from lacking the clubroot resistance polynucleotide, QTL, locus, molecular marker(s), or SNP(s) of the invention). A comparison between a Brassica plant (or plant part) comprising the polynucleotide, QTL, locus, molecular marker(s), or SNP(s) of the invention as described herein and a Brassica plant not comprising the polynucleotide, QTL, locus, molecular marker(s), or SNP(s) of the invention e.g., comprising the Brassica napus-bzh reference genome is preferably performed under similar greenhouse or field conditions and preferably with a controlled pathogen contact. Under said conditions the inventive Brassica plant is capable of showing less clubroot formation and less yield loss, as can be seen from the examples described herein. The skilled person will understand that when reference is made to a plant part in connection with clubroot resistance, such is to be interpreted as a plant part comprising (or in the alternative lacking) the polynucleotide, QTL, locus, molecular marker(s), or SNP(s) of the invention and wherein said plant part is derived from or can be used to generate a plant having clubroot resistance (or in the alternative lacking clubroot resistance)

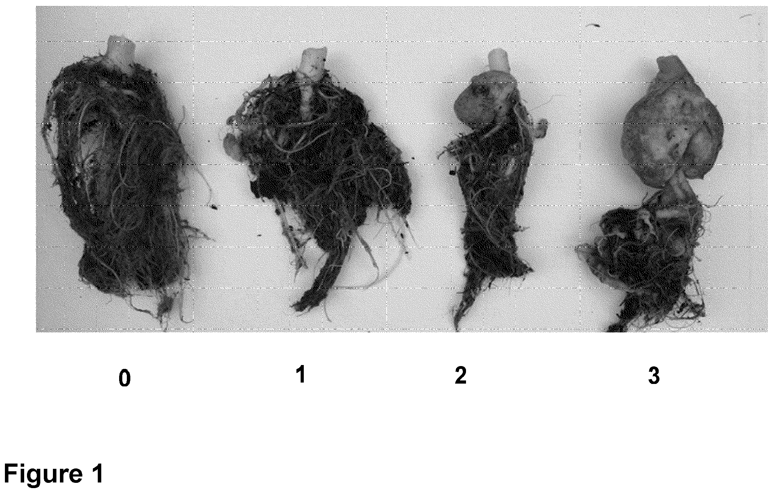

By means of example, and without limitation, an increase of pathogen resistance or tolerance can be evaluated based several criteria, such as (pathogenic) symptoms, which can for instance be classified according to Figure 1. A score can be attributed as follows: 0 = root system without clubs, 1 = a few medium-sized, separate globular clubs >5mm in diameter on lateral roots, 2 = medium-sized clubs 5-10 mm in diameter on the main roots, 3 = severe clubbing, with clubs >10 mm in diameter on the lateral and main roots. An increased resistance or tolerance of the plants or plant parts of the invention, when infected, in certain embodiments entails a reduction of at least 1 point in the above scoring system, compared to plants or plant parts not according to the invention, i.e., plants or plant parts not comprising the polynucleotide, QTL, locus, marker, and/or allele of the invention. In certain embodiments, resistance or tolerance of the plants or plant parts of the invention, when infected, entails a maximum score in the above scoring system of 3, preferably 2, more preferably 1. In certain embodiments, an increased resistance or tolerance of the plants or plant parts of the invention, when infected, entails galls which have an (average) diameter which is at least 20%, preferably at least 50%, more preferably at least 80% smaller than gall diameter of plants not according to the invention. In certain embodiments, an increased resistance or tolerance of the plants or plant parts of the invention, when infected, entails a number of galls which is at least 20%, preferably at least 50%, more preferably at least 80% less than gall diameter of plants not according to the invention. In certain embodiments, an increased resistance or tolerance of the plants or plant parts of the invention, when infected, entails galls which have an (average) diameter which is at least 20%, preferably at least 50%, more preferably at least 80% smaller than gall diameter of plants not according to the invention, and a number of galls which is at least 20%, preferably at least 50%, more preferably at least 80% less than gall diameter of plants not according to the invention. In certain embodiments, resistance or tolerance of the plants or plant parts of the invention, when infected, entails the absence of galls on main roots. The skilled person will understand that for the evaluation of disease burden, such as the number and/or diameter of galls, and in particular comparison of disease burden, plant at similar stages and conditions of infection are compared, e.g., plants of similar size and/or age, infected with similar amounts of pathogen, and/or evaluated at similar time frames after infection. In certain embodiments, an increased resistance or tolerance of the plants or plant parts of the invention, when infected, entails a delay in gall formation, such as a delay of at least 20% in time, preferably at least 50%, more preferably at least 80%, optionally also having reduced gall diameter or amount as described above.

As used herein, "clubroot" refers to a disease of plants of the Brassicaceae family affecting the roots, which is caused by (soilborne) pathogens of the genus Plasmodiophora (also known as Plasmodiaphoromycota) in the phylum of cercozoan, in the class of Phytomyxe, which are eukaryotic protist parasites. Clubroot is characterized by gall formation or distortion on (latent) roots and gives the shape of a club or spindle. Further symptoms include yellowing, wilting, and stunting of the plants. Developing plants may not show any symptoms but as the plants get older, they will start to show symptoms of chlorosis or yellowing, wilting during hot days, and exhibit stunted growth. Below ground, the roots experience cell proliferation due to increased auxin or growth hormone production from the plant as well as the pathogen. This causes the formation of galls that can grow big enough to restrict the xylem tissue inhibiting efficient water uptake by the plant. Galls appear like clubs or spindles on the roots. Eventually the roots will rot, and the plant will die. Preferably, according to certain embodiments of the invention, the *Plasmodiophora* species is *Plasmodiophora brassicae.* As used herein, the term "clubroot resistance/tolerance" and the like are used interchangeably with "Plasmodiophora resistance/tolerance" and the like, such as *Plasmodiophora brassicae* resistance/tolerance.

In certain embodiments, molecular marker (allele) ra74856s01 is or comprises a SNP at a position corresponding to position 6042239 (on chromosome A06) of reference *Brassica napus* genome Darmor-bzh v4.1; or the complement or reverse complement thereof. In certain embodiments, molecular marker (allele) ra74856s01 comprises at least 15 nucleotides, preferably at least 18 nucleotides, more preferably at least 20 nucleotides, such as at least 50 nucleotides. In certain embodiments, molecular marker (allele) ra74856s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74921s01 is or comprises a SNP at a position corresponding to position 6043355 (on chromosome A06) of reference *Brassica napus* genome Darmor-bzh v4.1; or the complement or reverse complement thereof. In certain embodiments, molecular marker (allele) ra74921s01 comprises at least 15 nucleotides, preferably at least 18 nucleotides, more preferably at least 20 nucleotides, such as at least 50 nucleotides.

In certain embodiments, molecular marker (allele) ra74921s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36415s01 is or comprises a SNP at a position corresponding to position 6045480 (on chromosome A06) of reference *Brassica napus* genome Darmor-bzh v4.1; or the complement or reverse complement thereof. In certain embodiments, molecular marker (allele) ra36415s01 comprises at least 15 nucleotides, preferably at least 18 nucleotides, more preferably at least 20 nucleotides, such as at least 50 nucleotides. In certain embodiments, molecular marker (allele) ra36415s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74945s01 is or comprises a SNP at a position corresponding to position 6059024 (on chromosome A06) of reference *Brassica napus* genome Darmor-bzh v4.1; or the complement or reverse complement thereof. In certain embodiments, molecular marker (allele) ra74945s01 comprises at least 15 nucleotides, preferably at least 18 nucleotides, more preferably at least 20 nucleotides, such as at least 50 nucleotides. In certain embodiments, molecular marker (allele) ra74945s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74946s01 is or comprises a SNP at a position corresponding to position 6067467 (on chromosome A06) of reference *Brassica napus* genome Darmor-bzh v4.1; or the complement or reverse complement thereof. In certain embodiments, molecular marker (allele) ra74946s01 comprises at least 15 nucleotides, preferably at least 18 nucleotides, more preferably at least 20 nucleotides, such as at least 50 nucleotides. In certain embodiments, molecular marker (allele) ra74946s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74948s01 is or comprises a SNP at a position corresponding to position 6082193 (on chromosome A06) of reference *Brassica napus* genome Darmor-bzh v4.1; or the complement or reverse complement thereof. In certain embodiments, molecular marker (allele) ra74948s01 comprises at least 15 nucleotides, preferably at least 18 nucleotides, more preferably at least 20 nucleotides, such as at least 50 nucleotides. In certain embodiments, molecular marker (allele) ra74948s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36418s01 is or comprises a SNP at a position corresponding to position 6085238 (on chromosome A06) of reference *Brassica napus* genome Darmor-bzh v4.1; or the complement or reverse complement thereof. In certain embodiments, molecular marker (allele) ra36418s01 comprises at least 15 nucleotides, preferably at least 18 nucleotides, more preferably at least 20 nucleotides, such as at least 50 nucleotides. In certain embodiments, molecular marker (allele) ra36418s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra05569s01 is or comprises a SNP at a position corresponding to position 6085138 (on chromosome A06) of reference *Brassica napus* genome Darmor-bzh v4.1; or the complement or reverse complement thereof. In certain embodiments, molecular marker (allele) ra05569s01 comprises at least 15 nucleotides, preferably at least 18 nucleotides, more preferably at least 20 nucleotides, such as at least 50 nucleotides. In certain embodiments, molecular marker (allele) ra05569s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36419s01 is or comprises a SNP at a position corresponding to position 6086857 (on chromosome A06) of reference *Brassica napus* genome Darmor-bzh v4.1; or the complement or reverse complement thereof. In certain embodiments, molecular marker (allele) ra36419s01 comprises at least 15 nucleotides, preferably at least 18 nucleotides, more preferably at least 20 nucleotides, such as at least 50 nucleotides. In certain embodiments, molecular marker (allele) ra36419s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74858s01 is or comprises a SNP at a position corresponding to position 6088663 (on chromosome A06) of reference *Brassica napus* genome Darmor-bzh v4.1; or the complement or reverse complement thereof. In certain embodiments, molecular marker (allele) ra74858s01 comprises at least 15 nucleotides, preferably at least 18 nucleotides, more preferably at least 20 nucleotides, such as at least 50 nucleotides. In certain embodiments, molecular marker (allele) ra74858s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74859s01 is or comprises a SNP at a position corresponding to position 6091648 (on chromosome A06) of reference *Brassica napus* genome Darmor-bzh v4.1; or the complement or reverse complement thereof. In certain embodiments, molecular marker (allele) ra74859s01 comprises at least 15 nucleotides, preferably at least 18 nucleotides, more preferably at least 20 nucleotides, such as at least 50 nucleotides. In certain embodiments, molecular marker (allele) ra74859s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36423s01 is or comprises a SNP at a position corresponding to position 6098159 (on chromosome A06) of reference *Brassica napus* genome Darmor-bzh v4.1; or the complement or reverse complement thereof. In certain embodiments, molecular marker (allele) ra36423s01 comprises at least 15 nucleotides, preferably at least 18 nucleotides, more preferably at least 20 nucleotides, such as at least 50 nucleotides. In certain embodiments, molecular marker (allele) ra36423s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74922s01 is or comprises a SNP at a position corresponding to position 6102982 (on chromosome A06) of reference *Brassica napus* genome Darmor-bzh v4.1; or the complement or reverse complement thereof. In certain embodiments, molecular marker (allele) ra74922s01 comprises at least 15 nucleotides, preferably at least 18 nucleotides, more preferably at least 20 nucleotides, such as at least 50 nucleotides. In certain embodiments, molecular marker (allele) ra74922s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74860s01 is or comprises a SNP at a position corresponding to position 6113367 (on chromosome A06) of reference *Brassica napus* genome Darmor-bzh v4.1; or the complement or reverse complement thereof. In certain embodiments, molecular marker (allele) ra74860s01 comprises at least 15 nucleotides, preferably at least 18 nucleotides, more preferably at least 20 nucleotides, such as at least 50 nucleotides. In certain embodiments, molecular marker (allele) ra74860s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36426s01 is or comprises a SNP at a position corresponding to position 6134234 (on chromosome A06) of reference *Brassica napus* genome Darmor-bzh v4.1; or the complement or reverse complement thereof. In certain embodiments, molecular marker (allele) ra36426s01 comprises at least 15 nucleotides, preferably at least 18 nucleotides, more preferably at least 20 nucleotides, such as at least 50 nucleotides. In certain embodiments, molecular marker (allele) ra36426s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36427s01 is or comprises a SNP at a position corresponding to position 6141195 (on chromosome A06) of reference *Brassica napus* genome Darmor-bzh v4.1; or the complement or reverse complement thereof. In certain embodiments, molecular marker (allele) ra36427s01 comprises at least 15 nucleotides, preferably at least 18 nucleotides, more preferably at least 20 nucleotides, such as at least 50 nucleotides. In certain embodiments, molecular marker (allele) ra36427s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36428s01 is or comprises a SNP at a position corresponding to position 6141204 (on chromosome A06) of reference *Brassica napus* genome Darmor-bzh v4.1; or the complement or reverse complement thereof. In certain embodiments, molecular marker (allele) ra36428s01 comprises at least 15 nucleotides, preferably at least 18 nucleotides, more preferably at least 20 nucleotides, such as at least 50 nucleotides. In certain embodiments, molecular marker (allele) ra36428s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36429s01 is or comprises a SNP at a position corresponding to position 6141354 (on chromosome A06) of reference *Brassica napus* genome Darmor-bzh v4.1; or the complement or reverse complement thereof. In certain embodiments, molecular marker (allele) ra36429s01 comprises at least 15 nucleotides, preferably at least 18 nucleotides, more preferably at least 20 nucleotides, such as at least 50 nucleotides. In certain embodiments, molecular marker (allele) ra36429s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36430s01 is or comprises a SNP at a position corresponding to position 6151503 (on chromosome A06) of reference *Brassica napus* genome Darmor-bzh v4.1; or the complement or reverse complement thereof. In certain embodiments, molecular marker (allele) ra36430s01 comprises at least 15 nucleotides, preferably at least 18 nucleotides, more preferably at least 20 nucleotides, such as at least 50 nucleotides. In certain embodiments, molecular marker (allele) ra36430s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36433s01 is or comprises a SNP at a position corresponding to position 6152744 (on chromosome A06) of reference *Brassica napus* genome Darmor-bzh v4.1; or the complement or reverse complement thereof. In certain embodiments, molecular marker (allele) ra36433s01 comprises at least 15 nucleotides, preferably at least 18 nucleotides, more preferably at least 20 nucleotides, such as at least 50 nucleotides. In certain embodiments, molecular marker (allele) ra36433s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36434s01 is or comprises a SNP at a position corresponding to position 6153568 (on chromosome A06) of reference *Brassica napus* genome Darmor-bzh v4.1; or the complement or reverse complement thereof. In certain embodiments, molecular marker (allele) ra36434s01 comprises at least 15 nucleotides, preferably at least 18 nucleotides, more preferably at least 20 nucleotides, such as at least 50 nucleotides. In certain embodiments, molecular marker (allele) ra36434s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74862s01 is or comprises a SNP at a position corresponding to position 6155076 (on chromosome A06) of reference *Brassica napus* genome Darmor-bzh v4.1; or the complement or reverse complement thereof. In certain embodiments, molecular marker (allele) ra74862s01 comprises at least 15 nucleotides, preferably at least 18 nucleotides, more preferably at least 20 nucleotides, such as at least 50 nucleotides. In certain embodiments, molecular marker (allele) ra74862s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36435s01 is or comprises a SNP at a position corresponding to position 6168127 (on chromosome A06) of reference *Brassica napus* genome Darmor-bzh v4.1; or the complement or reverse complement thereof. In certain embodiments, molecular marker (allele) ra36435s01 comprises at least 15 nucleotides, preferably at least 18 nucleotides, more preferably at least 20 nucleotides, such as at least 50 nucleotides. In certain embodiments, molecular marker (allele) ra36435s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74863s01 is or comprises a SNP at a position corresponding to position 6186575 (on chromosome A06) of reference *Brassica napus* genome Darmor-bzh v4.1; or the complement or reverse complement thereof. In certain embodiments, molecular marker (allele) ra74863s01 comprises at least 15 nucleotides, preferably at least 18 nucleotides, more preferably at least 20 nucleotides, such as at least 50 nucleotides. In certain embodiments, molecular marker (allele) ra74863s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74864s01 is or comprises a SNP at a position corresponding to position 6196798 (on chromosome A06) of reference *Brassica napus* genome Darmor-bzh v4.1; or the complement or reverse complement thereof. In certain embodiments, molecular marker (allele) ra74864s01 comprises at least 15 nucleotides, preferably at least 18 nucleotides, more preferably at least 20 nucleotides, such as at least 50 nucleotides. In certain embodiments, molecular marker (allele) ra74864s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74910s01 is or comprises a SNP at a position corresponding to position 6197815 (on chromosome A06) of reference *Brassica napus* genome Darmor-bzh v4.1; or the complement or reverse complement thereof. In certain embodiments, molecular marker (allele) ra74910s01 comprises at least 15 nucleotides, preferably at least 18 nucleotides, more preferably at least 20 nucleotides, such as at least 50 nucleotides. In certain embodiments, molecular marker (allele) ra74910s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74865s01 is or comprises a SNP at a position corresponding to position 6204215 (on chromosome A06) of reference *Brassica napus* genome Darmor-bzh v4.1; or the complement or reverse complement thereof. In certain embodiments, molecular marker (allele) ra74865s01 comprises at least 15 nucleotides, preferably at least 18 nucleotides, more preferably at least 20 nucleotides, such as at least 50 nucleotides. In certain embodiments, molecular marker (allele) ra74865s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74952s01 is or comprises a SNP at a position corresponding to position 6209886 (on chromosome A06) of reference *Brassica napus* genome Darmor-bzh v4.1; or the complement or reverse complement thereof. In certain embodiments, molecular marker (allele) ra74952s01 comprises at least 15 nucleotides, preferably at least 18 nucleotides, more preferably at least 20 nucleotides, such as at least 50 nucleotides. In certain embodiments, molecular marker (allele) ra74952s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36441s01 is or comprises a SNP at a position corresponding to position 6240866 (on chromosome A06) of reference *Brassica napus* genome Darmor-bzh v4.1; or the complement or reverse complement thereof. In certain embodiments, molecular marker (allele) ra36441s01 comprises at least 15 nucleotides, preferably at least 18 nucleotides, more preferably at least 20 nucleotides, such as at least 50 nucleotides. In certain embodiments, molecular marker (allele) ra36441s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74867s01 is or comprises a SNP at a position corresponding to position 6248401 (on chromosome A06) of reference *Brassica napus* genome Darmor-bzh v4.1; or the complement or reverse complement thereof. In certain embodiments, molecular marker (allele) ra74867s01 comprises at least 15 nucleotides, preferably at least 18 nucleotides, more preferably at least 20 nucleotides, such as at least 50 nucleotides. In certain embodiments, molecular marker (allele) ra74867s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36443s01 is or comprises a SNP at a position corresponding to position 6257529 (on chromosome A06) of reference *Brassica napus* genome Darmor-bzh v4.1; or the complement or reverse complement thereof. In certain embodiments, molecular marker (allele) ra36443s01 comprises at least 15 nucleotides, preferably at least 18 nucleotides, more preferably at least 20 nucleotides, such as at least 50 nucleotides. In certain embodiments, molecular marker (allele) ra36443s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36444s01 is or comprises a SNP at a position corresponding to position 6269510 (on chromosome A06) of reference *Brassica napus* genome Darmor-bzh v4.1; or the complement or reverse complement thereof. In certain embodiments, molecular marker (allele) ra36444s01 comprises at least 15 nucleotides, preferably at least 18 nucleotides, more preferably at least 20 nucleotides, such as at least 50 nucleotides. In certain embodiments, molecular marker (allele) ra36444s01 comprises at least 15 nucleotides, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74856s01 is or comprises a SNP at a position corresponding to position 51 of SEQ ID NO: 247 or a sequence which is at least 90% identical, preferably at least 95% identical, more preferably at least 98% identical, most preferably identical to SEQ ID NO: 247 (and comprising said SNP); or the complement or reverse complement of any thereof. In certain embodiments, molecular marker (allele) ra74856s01 comprises at least 15 contiguous nucleotides of SEQ ID NO: 247 or a sequence which is at least 90% identical, preferably at least 95% identical, more preferably at least 98% identical, most preferably identical to SEQ ID NO: 247 (and comprising said SNP); or the complement or reverse complement of any thereof, preferably at least 18 nucleotides, more preferably at least 20 nucleotides, such as at least 50 nucleotides. In certain embodiments, molecular marker (allele) ra74856s01 comprises at least 15 contiguous nucleotides of SEQ ID NO: 247 or a sequence which is at least 90% identical, preferably at least 95% identical, more preferably at least 98% identical, most preferably identical to SEQ ID NO: 247 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74921s01 is or comprises a SNP at a position corresponding to position 51 of SEQ ID NO: 263 or a sequence which is at least 90% identical, preferably at least 95% identical, more preferably at least 98% identical, most preferably identical to SEQ ID NO: 263 (and comprising said SNP); or the complement or reverse complement of any thereof. In certain embodiments, molecular marker (allele) ra74921s01 comprises at least 15 contiguous nucleotides of SEQ ID NO: 263 or a sequence which is at least 90% identical, preferably at least 95% identical, more preferably at least 98% identical, most preferably identical to SEQ ID NO: 263 (and comprising said SNP); or the complement or reverse complement of any thereof, preferably at least 18 nucleotides, more preferably at least 20 nucleotides, such as at least 50 nucleotides. In certain embodiments, molecular marker (allele) ra74921s01 comprises at least 15 contiguous nucleotides of SEQ ID NO: 263 or a sequence which is at least 90% identical, preferably at least 95% identical, more preferably at least 98% identical, most preferably identical to SEQ ID NO: 263 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74945s01 is or comprises a SNP at a position corresponding to position 51 of SEQ ID NO: 270 or a sequence which is at least 90% identical, preferably at least 95% identical, more preferably at least 98% identical, most preferably identical to SEQ ID NO: 270 (and comprising said SNP); or the complement or reverse complement of any thereof. In certain embodiments, molecular marker (allele) ra74945s01 comprises at least 15 contiguous nucleotides of SEQ ID NO: 270 or a sequence which is at least 90% identical, preferably at least 95% identical, more preferably at least 98% identical, most preferably identical to SEQ ID NO: 270 (and comprising said SNP); or the complement or reverse complement of any thereof, preferably at least 18 nucleotides, more preferably at least 20 nucleotides, such as at least 50 nucleotides. In certain embodiments, molecular marker (allele) ra74945s01 comprises at least 15 contiguous nucleotides of SEQ ID NO: 270 or a sequence which is at least 90% identical, preferably at least 95% identical, more preferably at least 98% identical, most preferably identical to SEQ ID NO: 270 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74946s01 is or comprises a SNP at a position corresponding to position 51 of SEQ ID NO: 271 or a sequence which is at least 90% identical, preferably at least 95% identical, more preferably at least 98% identical, most preferably identical to SEQ ID NO: 271 (and comprising said SNP); or the complement or reverse complement of any thereof. In certain embodiments, molecular marker (allele) ra74946s01 comprises at least 15 contiguous nucleotides of SEQ ID NO: 271 or a sequence which is at least 90% identical, preferably at least 95% identical, more preferably at least 98% identical, most preferably identical to SEQ ID NO: 271 (and comprising said SNP); or the complement or reverse complement of any thereof, preferably at least 18 nucleotides, more preferably at least 20 nucleotides, such as at least 50 nucleotides. In certain embodiments, molecular marker (allele) ra74946s01 comprises at least 15 contiguous nucleotides of SEQ ID NO: 271 or a sequence which is at least 90% identical, preferably at least 95% identical, more preferably at least 98% identical, most preferably identical to SEQ ID NO: 271 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74948s01 is or comprises a SNP at a position corresponding to position 51 of SEQ ID NO: 272 or a sequence which is at least 90% identical, preferably at least 95% identical, more preferably at least 98% identical, most preferably identical to SEQ ID NO: 272 (and comprising said SNP); or the complement or reverse complement of any thereof. In certain embodiments, molecular marker (allele) ra74948s01 comprises at least 15 contiguous nucleotides of SEQ ID NO: 272 or a sequence which is at least 90% identical, preferably at least 95% identical, more preferably at least 98% identical, most preferably identical to SEQ ID NO: 272 (and comprising said SNP); or the complement or reverse complement of any thereof, preferably at least 18 nucleotides, more preferably at least 20 nucleotides, such as at least 50 nucleotides. In certain embodiments, molecular marker (allele) ra74948s01 comprises at least 15 contiguous nucleotides of SEQ ID NO: 272 or a sequence which is at least 90% identical, preferably at least 95% identical, more preferably at least 98% identical, most preferably identical to SEQ ID NO: 272 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74858s01 is or comprises a SNP at a position corresponding to position 51 of SEQ ID NO: 248 or a sequence which is at least 90% identical, preferably at least 95% identical, more preferably at least 98% identical, most preferably identical to SEQ ID NO: 248 (and comprising said SNP); or the complement or reverse complement of any thereof. In certain embodiments, molecular marker (allele) ra74858s01 comprises at least 15 contiguous nucleotides of SEQ ID NO: 248 or a sequence which is at least 90% identical, preferably at least 95% identical, more preferably at least 98% identical, most preferably identical to SEQ ID NO: 248 (and comprising said SNP); or the complement or reverse complement of any thereof, preferably at least 18 nucleotides, more preferably at least 20 nucleotides, such as at least 50 nucleotides. In certain embodiments, molecular marker (allele) ra74858s01 comprises at least 15 contiguous nucleotides of SEQ ID NO: 248 or a sequence which is at least 90% identical, preferably at least 95% identical, more preferably at least 98% identical, most preferably identical to SEQ ID NO: 248 (and comprising said SNP); or the complement or reverse complement of any thereof, preferably at least 18 nucleotides, such as at least 50 nucleotides including said SNP and contiguous 5', 3', or combined 5' and 3' nucleotides.

In certain embodiments, molecular marker (allele) ra74859s01 is or comprises a SNP at a position corresponding to position 51 of SEQ ID NO: 249 or a sequence which is at least 90% identical, preferably at least 95% identical, more preferably at least 98% identical, most preferably identical to SEQ ID NO: 249 (and comprising said SNP); or the complement or reverse complement of any thereof. In certain embodiments, molecular marker (allele) ra74859s01 comprises at least 15 contiguous nucleotides of SEQ ID NO: 249 or a sequence which is at least 90% identical, preferably at least 95% identical, more preferably at least 98% identical, most preferably identical to SEQ ID NO: 249 (and comprising said SNP); or the complement or reverse complement of any thereof, preferably at least 18 nucleotides, more preferably at least 20 nucleotides, such as at least 50 nucleotides. In certain embodiments, molecular marker (allele) ra74859s01 comprises at least 15 contiguous nucleotides of SEQ ID NO: 249 or a sequence which is at least 90% identical, preferably at least 95% identical, more preferably at least 98% identical, most preferably identical to SEQ ID NO: 249 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74922s01 is or comprises a SNP at a position corresponding to position 51 of SEQ ID NO: 264 or a sequence which is at least 90% identical, preferably at least 95% identical, more preferably at least 98% identical, most preferably identical to SEQ ID NO: 264 (and comprising said SNP); or the complement or reverse complement of any thereof. In certain embodiments, molecular marker (allele) ra74922s01 comprises at least 15 contiguous nucleotides of SEQ ID NO: 264 or a sequence which is at least 90% identical, preferably at least 95% identical, more preferably at least 98% identical, most preferably identical to SEQ ID NO: 264 (and comprising said SNP); or the complement or reverse complement of any thereof, preferably at least 18 nucleotides, more preferably at least 20 nucleotides, such as at least 50 nucleotides. In certain embodiments, molecular marker (allele) ra74922s01 comprises at least 15 contiguous nucleotides of SEQ ID NO: 264 or a sequence which is at least 90% identical, preferably at least 95% identical, more preferably at least 98% identical, most preferably identical to SEQ ID NO: 264 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74860s01 is or comprises a SNP at a position corresponding to position 51 of SEQ ID NO: 250 or a sequence which is at least 90% identical, preferably at least 95% identical, more preferably at least 98% identical, most preferably identical to SEQ ID NO: 250 (and comprising said SNP); or the complement or reverse complement of any thereof. In certain embodiments, molecular marker (allele) ra74860s01 comprises at least 15 contiguous nucleotides of SEQ ID NO: 250 or a sequence which is at least 90% identical, preferably at least 95% identical, more preferably at least 98% identical, most preferably identical to SEQ ID NO: 250 (and comprising said SNP); or the complement or reverse complement of any thereof, preferably at least 18 nucleotides, more preferably at least 20 nucleotides, such as at least 50 nucleotides. In certain embodiments, molecular marker (allele) ra74860s01 comprises at least 15 contiguous nucleotides of SEQ ID NO: 250 or a sequence which is at least 90% identical, preferably at least 95% identical, more preferably at least 98% identical, most preferably identical to SEQ ID NO: 250 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36428s01 is or comprises a SNP at a position corresponding to position 51 of SEQ ID NO: 231 or a sequence which is at least 90% identical, preferably at least 95% identical, more preferably at least 98% identical, most preferably identical to SEQ ID NO: 231 (and comprising said SNP); or the complement or reverse complement of any thereof. In certain embodiments, molecular marker (allele) ra36428s01 comprises at least 15 contiguous nucleotides of SEQ ID NO: 231 or a sequence which is at least 90% identical, preferably at least 95% identical, more preferably at least 98% identical, most preferably identical to SEQ ID NO: 231 (and comprising said SNP); or the complement or reverse complement of any thereof, preferably at least 18 nucleotides, more preferably at least 20 nucleotides, such as at least 50 nucleotides. In certain embodiments, molecular marker (allele) ra36428s01 comprises at least 15 contiguous nucleotides of SEQ ID NO: 231 or a sequence which is at least 90% identical, preferably at least 95% identical, more preferably at least 98% identical, most preferably identical to SEQ ID NO: 231 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74862s01 is or comprises a SNP at a position corresponding to position 51 of SEQ ID NO: 251 or a sequence which is at least 90% identical, preferably at least 95% identical, more preferably at least 98% identical, most preferably identical to SEQ ID NO: 251 (and comprising said SNP); or the complement or reverse complement of any thereof. In certain embodiments, molecular marker (allele) ra74862s01 comprises at least 15 contiguous nucleotides of SEQ ID NO: 251 or a sequence which is at least 90% identical, preferably at least 95% identical, more preferably at least 98% identical, most preferably identical to SEQ ID NO: 251 (and comprising said SNP); or the complement or reverse complement of any thereof, preferably at least 18 nucleotides, more preferably at least 20 nucleotides, such as at least 50 nucleotides. In certain embodiments, molecular marker (allele) ra74862s01 comprises at least 15 contiguous nucleotides of SEQ ID NO: 251 or a sequence which is at least 90% identical, preferably at least 95% identical, more preferably at least 98% identical, most preferably identical to SEQ ID NO: 251 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74863s01 is or comprises a SNP at a position corresponding to position 51 of SEQ ID NO: 252 or a sequence which is at least 90% identical, preferably at least 95% identical, more preferably at least 98% identical, most preferably identical to SEQ ID NO: 252 (and comprising said SNP); or the complement or reverse complement of any thereof. In certain embodiments, molecular marker (allele) ra74863s01 comprises at least 15 contiguous nucleotides of SEQ ID NO: 252 or a sequence which is at least 90% identical, preferably at least 95% identical, more preferably at least 98% identical, most preferably identical to SEQ ID NO: 252 (and comprising said SNP); or the complement or reverse complement of any thereof, preferably at least 18 nucleotides, more preferably at least 20 nucleotides, such as at least 50 nucleotides. In certain embodiments, molecular marker (allele) ra74863s01 comprises at least 15 contiguous nucleotides of SEQ ID NO: 252 or a sequence which is at least 90% identical, preferably at least 95% identical, more preferably at least 98% identical, most preferably identical to SEQ ID NO: 252 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74864s01 is or comprises a SNP at a position corresponding to position 51 of SEQ ID NO: 253 or a sequence which is at least 90% identical, preferably at least 95% identical, more preferably at least 98% identical, most preferably identical to SEQ ID NO: 253 (and comprising said SNP); or the complement or reverse complement of any thereof. In certain embodiments, molecular marker (allele) ra74864s01 comprises at least 15 contiguous nucleotides of SEQ ID NO: 253 or a sequence which is at least 90% identical, preferably at least 95% identical, more preferably at least 98% identical, most preferably identical to SEQ ID NO: 253 (and comprising said SNP); or the complement or reverse complement of any thereof, preferably at least 18 nucleotides, more preferably at least 20 nucleotides, such as at least 50 nucleotides. In certain embodiments, molecular marker (allele) ra74864s01 comprises at least 15 contiguous nucleotides of SEQ ID NO: 253 or a sequence which is at least 90% identical, preferably at least 95% identical, more preferably at least 98% identical, most preferably identical to SEQ ID NO: 253 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74910s01 is or comprises a SNP at a position corresponding to position 51 of SEQ ID NO: 274 or a sequence which is at least 90% identical, preferably at least 95% identical, more preferably at least 98% identical, most preferably identical to SEQ ID NO: 274 (and comprising said SNP); or the complement or reverse complement of any thereof. In certain embodiments, molecular marker (allele) ra74910s01 comprises at least 15 contiguous nucleotides of SEQ ID NO: 274 or a sequence which is at least 90% identical, preferably at least 95% identical, more preferably at least 98% identical, most preferably identical to SEQ ID NO: 274 (and comprising said SNP); or the complement or reverse complement of any thereof, preferably at least 18 nucleotides, more preferably at least 20 nucleotides, such as at least 50 nucleotides. In certain embodiments, molecular marker (allele) ra74910s01 comprises at least 15 contiguous nucleotides of SEQ ID NO: 274 or a sequence which is at least 90% identical, preferably at least 95% identical, more preferably at least 98% identical, most preferably identical to SEQ ID NO: 274 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74865s01 is or comprises a SNP at a position corresponding to position 51 of SEQ ID NO: 254 or a sequence which is at least 90% identical, preferably at least 95% identical, more preferably at least 98% identical, most preferably identical to SEQ ID NO: 254 (and comprising said SNP); or the complement or reverse complement of any thereof. In certain embodiments, molecular marker (allele) ra74865s01 comprises at least 15 contiguous nucleotides of SEQ ID NO: 254 or a sequence which is at least 90% identical, preferably at least 95% identical, more preferably at least 98% identical, most preferably identical to SEQ ID NO: 254 (and comprising said SNP); or the complement or reverse complement of any thereof, preferably at least 18 nucleotides, more preferably at least 20 nucleotides, such as at least 50 nucleotides. In certain embodiments, molecular marker (allele) ra74865s01 comprises at least 15 contiguous nucleotides of SEQ ID NO: 254 or a sequence which is at least 90% identical, preferably at least 95% identical, more preferably at least 98% identical, most preferably identical to SEQ ID NO: 254 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74952s01 is or comprises a SNP at a position corresponding to position 51 of SEQ ID NO: 273 or a sequence which is at least 90% identical, preferably at least 95% identical, more preferably at least 98% identical, most preferably identical to SEQ ID NO: 273 (and comprising said SNP); or the complement or reverse complement of any thereof. In certain embodiments, molecular marker (allele) ra74952s01 comprises at least 15 contiguous nucleotides of SEQ ID NO: 273 or a sequence which is at least 90% identical, preferably at least 95% identical, more preferably at least 98% identical, most preferably identical to SEQ ID NO: 273 (and comprising said SNP); or the complement or reverse complement of any thereof, preferably at least 18 nucleotides, more preferably at least 20 nucleotides, such as at least 50 nucleotides. In certain embodiments, molecular marker (allele) ra74952s01 comprises at least 15 contiguous nucleotides of SEQ ID NO: 273 or a sequence which is at least 90% identical, preferably at least 95% identical, more preferably at least 98% identical, most preferably identical to SEQ ID NO: 273 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74867s01 is or comprises a SNP at a position corresponding to position 51 of SEQ ID NO: 255 or a sequence which is at least 90% identical, preferably at least 95% identical, more preferably at least 98% identical, most preferably identical to SEQ ID NO: 255 (and comprising said SNP); or the complement or reverse complement of any thereof. In certain embodiments, molecular marker (allele) ra74867s01 comprises at least 15 contiguous nucleotides of SEQ ID NO: 255 or a sequence which is at least 90% identical, preferably at least 95% identical, more preferably at least 98% identical, most preferably identical to SEQ ID NO: 255 (and comprising said SNP); or the complement or reverse complement of any thereof, preferably at least 18 nucleotides, more preferably at least 20 nucleotides, such as at least 50 nucleotides. In certain embodiments, molecular marker (allele) ra74867s01 comprises at least 15 contiguous nucleotides of SEQ ID NO: 255 or a sequence which is at least 90% identical, preferably at least 95% identical, more preferably at least 98% identical, most preferably identical to SEQ ID NO: 255 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra36444s01 is or comprises a SNP at a position corresponding to position 51 of SEQ ID NO: 223 or a sequence which is at least 90% identical, preferably at least 95% identical, more preferably at least 98% identical, most preferably identical to SEQ ID NO: 223 (and comprising said SNP); or the complement or reverse complement of any thereof. In certain embodiments, molecular marker (allele) ra36444s01 comprises at least 15 contiguous nucleotides of SEQ ID NO: 223 or a sequence which is at least 90% identical, preferably at least 95% identical, more preferably at least 98% identical, most preferably identical to SEQ ID NO: 223 (and comprising said SNP); or the complement or reverse complement of any thereof, preferably at least 18 nucleotides, more preferably at least 20 nucleotides, such as at least 50 nucleotides. In certain embodiments, molecular marker (allele) ra36444s01 comprises at least 15 contiguous nucleotides of SEQ ID NO: 223 or a sequence which is at least 90% identical, preferably at least 95% identical, more preferably at least 98% identical, most preferably identical to SEQ ID NO: 223 (and comprising said SNP); or the complement or reverse complement of any thereof, preferably at least 18 nucleotides, more preferably at least 20 nucleotides, such as at least 50 nucleotides including said SNP and contiguous 5', 3', or combined 5' and 3' nucleotides.

In certain embodiments, molecular marker (allele) ra74856s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 26 or 100, or a sequence which is at least 90% identical, preferably at least 95% identical, more preferably at least 98% identical, most preferably identical to SEQ ID NO: 26 or 100; or the complement or reverse complement of any thereof.

In certain embodiments, molecular marker (allele) ra74921s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 42 or 116, or a sequence which is at least 90% identical, preferably at least 95% identical, more preferably at least 98% identical, most preferably identical to SEQ ID NO: 42 or 116; or the complement or reverse complement of any thereof.

In certain embodiments, molecular marker (allele) ra74945s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 49 or 123, or a sequence which is at least 90% identical, preferably at least 95% identical, more preferably at least 98% identical, most preferably identical to SEQ ID NO: 49 or 123; or the complement or reverse complement of any thereof.

In certain embodiments, molecular marker (allele) ra74946s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 50 or 124, or a sequence which is at least 90% identical, preferably at least 95% identical, more preferably at least 98% identical, most preferably identical to SEQ ID NO: 50 or 124; or the complement or reverse complement of any thereof.

In certain embodiments, molecular marker (allele) ra74948s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 51 or 125, or a sequence which is at least 90% identical, preferably at least 95% identical, more preferably at least 98% identical, most preferably identical to SEQ ID NO: 51 or 125; or the complement or reverse complement of any thereof.

In certain embodiments, molecular marker (allele) ra74858s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 27 or 101, or a sequence which is at least 90% identical, preferably at least 95% identical, more preferably at least 98% identical, most preferably identical to SEQ ID NO: 27 or 101; or the complement or reverse complement of any thereof.

In certain embodiments, molecular marker (allele) ra74859s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 28 or 102, or a sequence which is at least 90% identical, preferably at least 95% identical, more preferably at least 98% identical, most preferably identical to SEQ ID NO: 28 or 102; or the complement or reverse complement of any thereof.

In certain embodiments, molecular marker (allele) ra74922s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 43 or 117, or a sequence which is at least 90% identical, preferably at least 95% identical, more preferably at least 98% identical, most preferably identical to SEQ ID NO: 43 or 117; or the complement or reverse complement of any thereof.

In certain embodiments, molecular marker (allele) ra74860s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 29 or 103, or a sequence which is at least 90% identical, preferably at least 95% identical, more preferably at least 98% identical, most preferably identical to SEQ ID NO: 29 or 103; or the complement or reverse complement of any thereof.

In certain embodiments, molecular marker (allele) ra36428s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 10 or 84, or a sequence which is at least 90% identical, preferably at least 95% identical, more preferably at least 98% identical, most preferably identical to SEQ ID NO: 10 or 84; or the complement or reverse complement of any thereof.

In certain embodiments, molecular marker (allele) ra74862s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 30 or 104, or a sequence which is at least 90% identical, preferably at least 95% identical, more preferably at least 98% identical, most preferably identical to SEQ ID NO: 30 or 104; or the complement or reverse complement of any thereof.

In certain embodiments, molecular marker (allele) ra74863s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 31 or 105, or a sequence which is at least 90% identical, preferably at least 95% identical, more preferably at least 98% identical, most preferably identical to SEQ ID NO: 31 or 105; or the complement or reverse complement of any thereof.

In certain embodiments, molecular marker (allele) ra74864s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 32 or 106, or a sequence which is at least 90% identical, preferably at least 95% identical, more preferably at least 98% identical, most preferably identical to SEQ ID NO: 32 or 106; or the complement or reverse complement of any thereof.

In certain embodiments, molecular marker (allele) ra74910s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 66 or 137, or a sequence which is at least 90% identical, preferably at least 95% identical, more preferably at least 98% identical, most preferably identical to SEQ ID NO: 66 or 137; or the complement or reverse complement of any thereof.

In certain embodiments, molecular marker (allele) ra74865s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 33 or 107, or a sequence which is at least 90% identical, preferably at least 95% identical, more preferably at least 98% identical, most preferably identical to SEQ ID NO: 33 or 107; or the complement or reverse complement of any thereof.

In certain embodiments, molecular marker (allele) ra74952s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 52 or 126, or a sequence which is at least 90% identical, preferably at least 95% identical, more preferably at least 98% identical, most preferably identical to SEQ ID NO: 52 or 126; or the complement or reverse complement of any thereof.

In certain embodiments, molecular marker (allele) ra74867s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 34 or 108, or a sequence which is at least 90% identical, preferably at least 95% identical, more preferably at least 98% identical, most preferably identical to SEQ ID NO: 34 or 108; or the complement or reverse complement of any thereof.

In certain embodiments, molecular marker (allele) ra36444s01 has a sequence comprising, consisting of, or comprised in (such as at least the 15 most 3' nucleotides, preferably at least the 18 most 3' nucleotides, more preferably at least the 20 most 3' nucleotides), or is capable of being detected by, a polynucleic acid having a sequence as set forth in SEQ ID NO: 2 or 76, or a sequence which is at least 90% identical, preferably at least 95% identical, more preferably at least 98% identical, most preferably identical to SEQ ID NO: 2 or 76; or the complement or reverse complement of any thereof.

In an aspect, the invention relates to a method for identifying and/or selecting a plant or plant part of the *Brassicaceae* family, comprising screening for the presence of (a polynucleotide comprising) one or more SNP at a position corresponding to position 4839277, 4856766, 4884577, 4899658, 4902731, 4911752, 4915604, 4915805, 4915657, 4927351, 4927798, 4944020, 4964195, 4967878, 4967961, 4987049, 5003808, 5007776, 5010462, 5015364, 5015608, 5044742, 5051901, 5056127, 5062564, 5064801, 5079443, 5086034, 5119769, 5138085, 5146309, 5182655, 5185885, 5202213, 5206433, 5218016, 5250035, 5252456, 5254497, 5254635, 5256087, 5256686, 5266053, 5267134, 5289239, 5289964, 5296819, 5297108, 5297397, 5297818, 5297916, 5300782, 5302770, 5310061, 5334804, 5354720, 5364528, 5385366, 5385503, 5388175, 5403991, 5405095, 5419754, 5427345, 5428692, 5429350, 5430360, 5430636, 5430793, 5431324, 5431650, 5439143, 5439416, 5440260, 5441044, 5449534, 5451597, 5452662, 5455275, 5457921, 5460236, 5468640, 5495115, 5499274, 5499251, 5500197, 5501868, 5510178, 5537099, 5542254, 5542333, 5543485, 5555541, 5563828, 5583362, 5590724, 5590984, 5603334, 5603449, 5603932, 5605199, 5605457, 5605497, 5605498, 5605755, 5613935, 5625053, 5625326, 5627883, 5628511, 5632864, 5638550, 5639970, 5641284, 5660335, 5672356, 5690511, 5698241, 5700496, 5705944, 5735389, 5742133, 5754822, 5755109, 5756087, 5758615, 5758728, 5768576, 5780109, 5792363, 5794882, 5848144, 5849412, 5860668, 5863867, 5871964, 5878935, 5879162, 5889764, 5890452, 5891612, 5896863, 5897286, 5897686, 5898065, 5903558, 5911516, 5916939, 5921067, 5924593, 5939740, 5941369, 5943364, 5943520, 5949395, 5951830, 5977944, 5995057, 5997653, 6017553, 6019305, 6022301, 6031226, 6038704, 6042239, 6043355, 6045480, 6059024, 6067467, 6082193, 6085238, 6085138, 6086857, 6088663, 6091648, 6098159, 6102982, 6113367, 6134234, 6141195, 6141204, 6141354, 6151503, 6152744, 6153568, 6155076, 6168127, 6186575, 6196798, 6197815, 6204215, 6209886, 6240866, 6248401, 6257529, 6269510, 6275195, 6275424, 6275538, 6275743, 6276255, 6276300, 6276310, 6276740, 6306470, 6314871, 6315070, 6346195, 6369655, 6373996, 6375256, 6406350, 6410231, 6421616, 6421763, 6425751, 6426888, 6427052, 6427553, 6428706, 6437251, 6454193, 6456013, 6474875, 6474889, 6474995, 6475550, 6496790, 6517587, 6530194, 6544813, 6552185, 6552129, 6556280, 6573500, 6644158, 6647495, 6652312, 6699025, 6705917, 6721459, 6733929, 6758391, 6786083, 6786530, 6795965, 6806634, 6807432, 6848419, 6878047, 6902103, 6938254, 6943320, 6945568, 6948939, 6966982, 6967497, 7008151, 7009992, 7017603, 7060398, 7060302, 7079170, 7083010, 7160147, 7187542, 7210628, 7217480, 7236144, 7240251, 7257713, 7266286, 7268013, 7274483, 7278399, 7278464, 7280606, 7287336, 7292882, 7294136, 7299669, 7301443, 7306347, 7306328, 7331460, 7333695, 7338149, 7356711, 7374019, 7398437, 7399002, 7449905, 7470602, 7513403, 7556119, 7556264, 7560549, 7580680, 7616836, 7629649, 7645380, 7657378, 7657549, 7658143, 7661720, 7661888, 7665850, 7673997, 7677496, 7677988, 7678203, 7682880, 7719606, 7723590, 7723921, 7778970, 7778876, 7779141, 7779681, 7781087, 7807294, 7814278, 7815155, 7818006, 7818067, 7829462, 7834350, 7863191, 7891736, 7900547, 7913396, 7949004, 7951624, 7966871, 7976824, 7979457, 7983003, 7993145, 7998760, 8010638, 8012944, 8014530, 8046270, 8054506, 8058244, 8063823, 8064372, 8070063, 8073587, 8075813, 8101348, 8109398, 8121868, 8128583, 8129591, 8129632, 8146323, 8147266, 8174394, 8174771, and/or 8175263 (on chromosome A06) of reference *Brassica napus* genome Darmor-bzh v4.1.

In an aspect, the invention relates to a method for identifying and/or selecting a plant or plant part of the *Brassicaceae* family, comprising screening for the presence of (a polynucleotide comprising) one or more SNP at a position corresponding to position 4927798, 5543485, 5603449, 5605457, 5605497, 5613935, 5628511, 5632864, 5638550, 5641284, 5660335, 5672356, 5690511, 5698241, 5705944, 5735389, 5742133, 5755109, 5780109, 5848144, 5860668, 5897686, 5898065, 5941369, 5943520, 5995057, 5997653, 6019305, 6022301, 6031226, 6042239, 6043355, 6059024, 6067467, 6082193, 6088663, 6091648, 6102982, 6113367, 6141204, 6155076, 6186575, 6196798, 6197815, 6204215, 6209886, 6248401, 6269510, 6275195, 6275424, 6275538, 6275743, 6276255, 6276300, 6276310, 6276740, 6306470, 6314871, 6346195, 6373996, 6375256, 6425751, 6426888, 6427052, 6427553, 6437251, 6758391, 6786530, 6878047, and/or 7060302 (on chromosome A06) of reference *Brassica napus* genome Darmor-bzh v4.1.

In an aspect, the invention relates to a method for identifying and/or selecting a plant or plant part of the *Brassicaceae* family, comprising screening for the presence of (a polynucleotide comprising) one or more SNP at a position corresponding to position 6042239, 6043355, 6045480, 6059024, 6067467, 6082193, 6085238, 6085138, 6086857, 6088663, 6091648, 6098159, 6102982, 6113367, 6134234, 6141195, 6141204, 6141354, 6151503, 6152744, 6153568, 6155076, 6168127, 6186575, 6196798, 6197815, 6204215, 6209886, 6240866, 6248401, 6257529, and/or 6269510 (on chromosome A06) of reference *Brassica napus* genome Darmor-bzh v4.1.

In an aspect, the invention relates to a method for identifying and/or selecting a plant or plant part of the *Brassicaceae* family, comprising screening for the presence of (a polynucleotide comprising) one or more SNP at a position corresponding to position 6042239, 6043355, 6059024, 6067467, 6082193, 6088663, 6091648, 6102982, 6113367, 6141204, 6155076, 6186575, 6196798, 6197815, 6204215, 6209886, 6248401, and/or 6269510 (on chromosome A06) of reference *Brassica napus* genome Darmor-bzh v4.1.

In an aspect, the invention relates to a method for identifying and/or selecting a plant or plant part of the Brassicaceae family, comprising screening for the presence of (a polynucleotide comprising) one or more SNP at a position corresponding to position 6155076, 6186575, 6196798, 6197815, 6204215, 6209886, and/or 6248401 (on chromosome A06) of reference *Brassica napus* genome Darmor-bzh v4.1.

In an aspect, the invention relates to a method for identifying and/or selecting a plant or plant part of the *Brassicaceae* family, comprising screening for the presence of (a polynucleotide comprising) one or more SNP at a position corresponding to position 6155076 and/or 6248401 (on chromosome A06) of reference *Brassica napus* genome Darmor-bzh v4.1.

In an aspect, the invention relates to a method for identifying and/or selecting a plant or plant part of the *Brassicaceae* family, comprising screening for the presence of (a polynucleotide comprising) one or more SNP at a position corresponding to position 51 of respectively SEQ ID NOs: 222 to 274 or to 293 (in the genome of the plant or plant part); the complement or reverse complement of any thereof.

In an aspect, the invention relates to a method for identifying and/or selecting a plant or plant part of the *Brassicaceae* family, comprising screening for the presence of (a polynucleotide comprising) one or more SNP at a position corresponding to position 51 of respectively SEQ ID NOs: 247, 263, 270, 271, 272, 248, 249, 264, 250, 231, 251, 252, 253, 274, 254, 273, 255, and/or 223 (in the genome of the plant or plant part); the complement or reverse complement of any thereof.

In certain embodiments, the molecular markers according to the invention are SNPs. In certain embodiments, the molecular markers according to the invention comprise at least 15 nucleotides, preferably at least 20 nucleotides. In certain embodiments, the molecular markers according to the invention comprise at least 50 nucleotides. In certain embodiments, the molecular markers according to the invention comprise at most 200 nucleotides. In certain embodiments, the molecular markers according to the invention comprise at most 100 nucleotides. In certain embodiments, the molecular markers according to the invention comprise at least 15 nucleotides, preferably at least 20 nucleotides and at most 200 nucleotides. In certain embodiments, the molecular markers according to the invention comprise at least 50 nucleotides and at most 200 nucleotides. In certain embodiments, the molecular markers according to the invention comprise at least 20 nucleotides and at most 100 nucleotides. In certain embodiments, the molecular markers according to the invention comprise at least 50 nucleotides and at most 100 nucleotides.

It will be understood that when reference is made herein to a (polynucleic acid) sequence comprised in a polynucleic acid sequence, that a fragment thereof is referred to, i.e., a contiguous stretch of nucleotides.

The identity of the SNP as referred to herein differs between the donor allele and the acceptor allele, i.e., differs between the clubroot resistance allele and the susceptible allele. Hence the identity of the SNP, and by extension the molecular markers of the invention as described herein, is capable of distinguishing or discriminating between clubroot resistance and susceptibility. Likewise, the identity of the SNP, and by extension the molecular markers of the invention as described herein, is capable of identifying clubroot resistance or is capable of identifying clubroot susceptibility.

The skilled person will understand that detecting any of the (molecular) marker (allele) or SNP (allele) as described herein may equivalently detect any of the other (molecular) marker (allele) or SNP (allele), given their close linkage. For example, screening for a polynucleotide comprising ra74862s01 and ra74867s01 may be done by screening for these marker (allele) themselves, or alternatively by screening for any of the other marker (allele) of the invention as described herein elsewhere.

The identity of molecular markers of the invention is provided in Table D. Marker allele and SNP allele sequences associated with the resistance allele or alternatively the susceptible alleles are indicated. The respective SNP allele identities for the resistance allele and susceptible allele can be extracted from Tables D and E. The skilled person will understand that these SPN allele identities also apply to the recited genomic positions on the Darmor-bzh v4.1 reference genome as described herein elsewhere.

Suitable preferred markers and SNP identities according to an embodiment of the invention are provided in Table 1 below.

Table 1

| Marker | SNP position | | Resistant SNP identity | Non-resistant/susceptible SNP identity | |
|---|---|---|---|---|---|
| | Position 51 on SEQ ID NO | Position on chromosome A06 of Darmor-bzh v4.1 | | Generic | Specific |
| ra74856s01 | 247 | 6042239 | C | D | A |
| ra74921s01 | 263 | 6043355 | G | H | A |
| ra74945s01 | 270 | 6059024 | A | B | C |
| ra74946s01 | 271 | 6067467 | G | H | A |
| ra74948s01 | 272 | 6082193 | T | V | G |
| ra74858s01 | 248 | 6088663 | A | B | T |
| ra74859s01 | 249 | 6091648 | C | D | T |
| ra74922s01 | 264 | 6102982 | G | H | A |
| ra74860s01 | 250 | 6113367 | T | V | G |
| ra36428s01 | 231 | 6141204 | C | D | T |
| ra74862s01 | 251 | 6155076 | T | V | A |
| ra74863s01 | 252 | 6186575 | C | D | A |
| ra74864s01 | 253 | 6196798 | A | B | C |
| ra74910s01 | 274 | 6197815 | A | B | G |
| ra74865s01 | 254 | 6204215 | C | D | T |
| ra74952s01 | 273 | 6209886 | C | D | T |
| ra74867s01 | 255 | 6248401 | C | D | A |
| ra36444s01 | 223 | 6269510 | T | V | C |

A plant or plant part is identified as resistant to or having increased resistance to clubroot if one or more of the "resistant" SNP alleles is identified or detected.

A plant or plant part is identified as not resistant to/susceptible for or not having increased resistance to clubroot if one or more of the "resistant" SNP alleles is not identified or detected.

A plant or plant part is identified as not resistant to/susceptible for or not having increased resistance to clubroot if one or more of the "non-resistant/susceptible" SNP alleles is identified or detected.

In certain embodiments, the clubroot (increased) resistance allele of molecular marker ra74856s01 comprises a SNP at position 51 of SEQ ID NO: 247 or a sequence which is at least 90% identical, preferably at least 95% identical, more preferably at least 98% identical, most preferably identical to SEQ ID NO: 247, or at a position corresponding to position 6042239 of (on chromosome A06) of reference Brassica napus genome Darmor-bzh v4.1; wherein said SNP is C. In certain embodiments, the clubroot non-(increased) resistance or susceptible allele of molecular marker ra74856s01 comprises a SNP at position 51 of SEQ ID NO: 247 or a sequence which is at least 90% identical, preferably at least 95% identical, more preferably at least 98% identical, most preferably identical to SEQ ID NO: 247, or at a position corresponding to position 6042239 of (on chromosome A06) of reference Brassica napus genome Darmor-bzh v4.1; wherein said SNP is not C. In certain embodiments, the clubroot non-(increased) resistance or susceptible allele of molecular marker ra74856s01 comprises a SNP at position 51 of SEQ ID NO: 247 or a sequence which is at least 90% identical, preferably at least 95% identical, more preferably at least 98% identical, most preferably identical to SEQ ID NO: 247, or at a position corresponding to position 6042239 of (on chromosome A06) of reference Brassica napus genome Darmor-bzh v4.1; wherein said SNP is D. In certain embodiments, the clubroot non-(increased) resistance or susceptible allele of molecular marker ra74856s01 comprises a SNP at position 51 of SEQ ID NO: 247 or a sequence which is at least 90% identical, preferably at least 95% identical, more preferably at least 98% identical, most preferably identical to SEQ ID NO: 247, or at a position corresponding to position 6042239 of (on chromosome A06) of reference Brassica napus genome Darmor-bzh v4.1; wherein said SNP is A.

In certain embodiments, the clubroot (increased) resistance allele of molecular marker ra74921s01 comprises a SNP at position 51 of SEQ ID NO: 263 or a sequence which is at least 90% identical, preferably at least 95% identical, more preferably at least 98% identical, most preferably identical to SEQ ID NO: 263, or at a position corresponding to position 6043355 of (on chromosome A06) of reference Brassica napus genome Darmor-bzh v4.1; wherein said SNP is G. In certain embodiments, the clubroot non-(increased) resistance or susceptible allele of molecular marker ra74921s01 comprises a SNP at position 51 of SEQ ID NO: 263 or a sequence which is at least 90% identical, preferably at least 95% identical, more preferably at least 98% identical, most preferably identical to SEQ ID NO: 263, or at a position corresponding to position 6043355 of (on chromosome A06) of reference Brassica napus genome Darmor-bzh v4.1; wherein said SNP is not G. In certain embodiments, the clubroot non-(increased) resistance or susceptible allele of molecular marker ra74921s01 comprises a SNP at position 51 of SEQ ID NO: 263 or a sequence which is at least 90% identical, preferably at least 95% identical, more preferably at least 98% identical, most preferably identical to SEQ ID NO: 263, or at a position corresponding to position 6043355 of (on chromosome A06) of reference Brassica napus genome Darmor-bzh v4.1; wherein said SNP is H. In certain embodiments, the clubroot non-(increased) resistance or susceptible allele of molecular marker ra74921s01 comprises a SNP at position 51 of SEQ ID NO: 263 or a sequence which is at least 90% identical, preferably at least 95% identical, more preferably at least 98% identical, most preferably identical to SEQ ID NO: 263, or at a position corresponding to position 6043355 of (on chromosome A06) of reference Brassica napus genome Darmor-bzh v4.1; wherein said SNP is A.

In certain embodiments, the clubroot (increased) resistance allele of molecular marker ra74945s01 comprises a SNP at position 51 of SEQ ID NO: 270 or a sequence which is at least 90% identical, preferably at least 95% identical, more preferably at least 98% identical, most preferably identical to SEQ ID NO: 270, or at a position corresponding to position 6059024 of (on chromosome A06) of reference *Brassica napus* genome Darmor-bzh v4.1; wherein said SNP is A. In certain embodiments, the clubroot non-(increased) resistance or susceptible allele of molecular marker ra74945s01 comprises a SNP at position 51 of SEQ ID NO: 270 or a sequence which is at least 90% identical, preferably at least 95% identical, more preferably at least 98% identical, most preferably identical to SEQ ID NO: 270, or at a position corresponding to position 6059024 of (on chromosome A06) of reference *Brassica napus* genome Darmor-bzh v4.1; wherein said SNP is not A. In certain embodiments, the clubroot non-(increased) resistance or susceptible allele of molecular marker ra74945s01 comprises a SNP at position 51 of SEQ ID NO: 270 or a sequence which is at least 90% identical, preferably at least 95% identical, more preferably at least 98% identical, most preferably identical to SEQ ID NO: 270, or at a position corresponding to position 6059024 of (on chromosome A06) of reference *Brassica napus* genome Darmor-bzh v4.1; wherein said SNP is B. In certain embodiments, the clubroot non-(increased) resistance or susceptible allele of molecular marker ra74945s01 comprises a SNP at position 51 of SEQ ID NO: 270 or a sequence which is at least 90% identical, preferably at least 95% identical, more preferably at least 98% identical, most preferably identical to SEQ ID NO: 270, or at a position corresponding to position 6059024 of (on chromosome A06) of reference *Brassica napus* genome Darmor-bzh v4.1; wherein said SNP is C.

In certain embodiments, the clubroot (increased) resistance allele of molecular marker ra74946s01 comprises a SNP at position 51 of SEQ ID NO: 271 or a sequence which is at least 90% identical, preferably at least 95% identical, more preferably at least 98% identical, most preferably identical to SEQ ID NO: 271, or at a position corresponding to position 6067467 of (on chromosome A06) of reference *Brassica napus* genome Darmor-bzh v4.1; wherein said SNP is G. In certain embodiments, the clubroot non-(increased) resistance or susceptible allele of molecular marker ra74946s01 comprises a SNP at position 51 of SEQ ID NO: 271 or a sequence which is at least 90% identical, preferably at least 95% identical, more preferably at least 98% identical, most preferably identical to SEQ ID NO: 271, or at a position corresponding to position 6067467 of (on chromosome A06) of reference *Brassica napus* genome Darmor-bzh v4.1; wherein said SNP is not G. In certain embodiments, the clubroot non-(increased) resistance or susceptible allele of molecular marker ra74946s01 comprises a SNP at position 51 of SEQ ID NO: 271 or a sequence which is at least 90% identical, preferably at least 95% identical, more preferably at least 98% identical, most preferably identical to SEQ ID NO: 271, or at a position corresponding to position 6067467 of (on chromosome A06) of reference *Brassica napus* genome Darmor-bzh v4.1; wherein said SNP is H. In certain embodiments, the clubroot non-(increased) resistance or susceptible allele of molecular marker ra74946s01 comprises a SNP at position 51 of SEQ ID NO: 271 or a sequence which is at least 90% identical, preferably at least 95% identical, more preferably at least 98% identical, most preferably identical to SEQ ID NO: 271, or at a position corresponding to position 6067467 of (on chromosome A06) of reference *Brassica napus* genome Darmor-bzh v4.1; wherein said SNP is A.

In certain embodiments, the clubroot (increased) resistance allele of molecular marker ra74948s01 comprises a SNP at position 51 of SEQ ID NO: 272 or a sequence which is at least 90% identical, preferably at least 95% identical, more preferably at least 98% identical, most preferably identical to SEQ ID NO: 272, or at a position corresponding to position 6082193 of (on chromosome A06) of reference *Brassica napus* genome Darmor-bzh v4.1; wherein said SNP is T. In certain embodiments, the clubroot non-(increased) resistance or susceptible allele of molecular marker ra74948s01 comprises a SNP at position 51 of SEQ ID NO: 272 or a sequence which is at least 90% identical, preferably at least 95% identical, more preferably at least 98% identical, most preferably identical to SEQ ID NO: 272 , or at a position corresponding to position 6082193 of (on chromosome A06) of reference *Brassica napus* genome Darmor-bzh v4.1; wherein said SNP is not T. In certain embodiments, the clubroot non-(increased) resistance or susceptible allele of molecular marker ra74948s01 comprises a SNP at position 51 of SEQ ID NO: 272 or a sequence which is at least 90% identical, preferably at least 95% identical, more preferably at least 98% identical, most preferably identical to SEQ ID NO: 272, or at a position corresponding to position 6082193 of (on chromosome A06) of reference *Brassica napus* genome Darmor-bzh v4.1; wherein said SNP is V. In certain embodiments, the clubroot non-(increased) resistance or susceptible allele of molecular marker ra74948s01 comprises a SNP at position 51 of SEQ ID NO: 272 or a sequence which is at least 90% identical, preferably at least 95% identical, more preferably at least 98% identical, most preferably identical to SEQ ID NO: 272, or at a position corresponding to position 6082193 of (on chromosome A06) of reference *Brassica napus* genome Darmor-bzh v4.1; wherein said SNP is G.

In certain embodiments, the clubroot (increased) resistance allele of molecular marker ra74858s01 comprises a SNP at position 51 of SEQ ID NO: 248 or a sequence which is at least 90% identical, preferably at least 95% identical, more preferably at least 98% identical, most preferably identical to SEQ ID NO: 248, or at a position corresponding to position 6088663 of (on chromosome A06) of reference *Brassica napus* genome Darmor-bzh v4.1; wherein said SNP is A. In certain embodiments, the clubroot non-(increased) resistance or susceptible allele of molecular marker ra74858s01 comprises a SNP at position 51 of SEQ ID NO: 248 or a sequence which is at least 90% identical, preferably at least 95% identical, more preferably at least 98% identical, most preferably identical to SEQ ID NO: 248, or at a position corresponding to position 6088663 of (on chromosome A06) of reference *Brassica napus* genome Darmor-bzh v4.1; wherein said SNP is not A. In certain embodiments, the clubroot non-(increased) resistance or susceptible allele of molecular marker ra74858s01 comprises a SNP at position 51 of SEQ ID NO: 248 or a sequence which is at least 90% identical, preferably at least 95% identical, more preferably at least 98% identical, most preferably identical to SEQ ID NO: 248, or at a position corresponding to position 6088663 of (on chromosome A06) of reference *Brassica napus* genome Darmor-bzh v4.1; wherein said SNP is B. In certain embodiments, the clubroot non-(increased) resistance or susceptible allele of molecular marker ra74858s01 comprises a SNP at position 51 of SEQ ID NO: 248 or a sequence which is at least 90% identical, preferably at least 95% identical, more preferably at least 98% identical, most preferably identical to SEQ ID NO: 248, or at a position corresponding to position 6088663 of (on chromosome A06) of reference *Brassica napus* genome Darmor-bzh v4.1; wherein said SNP is T.

In certain embodiments, the clubroot (increased) resistance allele of molecular marker ra74859s01 comprises a SNP at position 51 of SEQ ID NO: 249 or a sequence which is at least 90% identical, preferably at least 95% identical, more preferably at least 98% identical, most preferably identical to SEQ ID NO: 249, or at a position corresponding to position 6091648 of (on chromosome A06) of reference *Brassica napus* genome Darmor-bzh v4.1; wherein said SNP is C. In certain embodiments, the clubroot non-(increased) resistance or susceptible allele of molecular marker ra74859s01 comprises a SNP at position 51 of SEQ ID NO: 249 or a sequence which is at least 90% identical, preferably at least 95% identical, more preferably at least 98% identical, most preferably identical to SEQ ID NO: 249, or at a position corresponding to position 6091648 of (on chromosome A06) of reference *Brassica napus* genome Darmor-bzh v4.1; wherein said SNP is not C. In certain embodiments, the clubroot non-(increased) resistance or susceptible allele of molecular marker ra74859s01 comprises a SNP at position 51 of SEQ ID NO: 249 or a sequence which is at least 90% identical, preferably at least 95% identical, more preferably at least 98% identical, most preferably identical to SEQ ID NO: 249, or at a position corresponding to position 6091648 of (on chromosome A06) of reference *Brassica napus* genome Darmor-bzh v4.1; wherein said SNP is D. In certain embodiments, the clubroot non-(increased) resistance or susceptible allele of molecular marker ra74859s01 comprises a SNP at position 51 of SEQ ID NO: 249 or a sequence which is at least 90% identical, preferably at least 95% identical, more preferably at least 98% identical, most preferably identical to SEQ ID NO: 249, or at a position corresponding to position 6091648 of (on chromosome A06) of reference *Brassica napus* genome Darmor-bzh v4.1; wherein said SNP is T.

In certain embodiments, the clubroot (increased) resistance allele of molecular marker ra74922s01 comprises a SNP at position 51 of SEQ ID NO: 264 or a sequence which is at least 90% identical, preferably at least 95% identical, more preferably at least 98% identical, most preferably identical to SEQ ID NO: 264, or at a position corresponding to position 6102982 of (on chromosome A06) of reference *Brassica napus* genome Darmor-bzh v4.1; wherein said SNP is G. In certain embodiments, the clubroot non-(increased) resistance or susceptible allele of molecular marker ra74922s01 comprises a SNP at position 51 of SEQ ID NO: 264 or a sequence which is at least 90% identical, preferably at least 95% identical, more preferably at least 98% identical, most preferably identical to SEQ ID NO: 264, or at a position corresponding to position 6102982 of (on chromosome A06) of reference *Brassica napus* genome Darmor-bzh v4.1; wherein said SNP is not G. In certain embodiments, the clubroot non-(increased) resistance or susceptible allele of molecular marker ra74922s01 comprises a SNP at position 51 of SEQ ID NO: 264 or a sequence which is at least 90% identical, preferably at least 95% identical, more preferably at least 98% identical, most preferably identical to SEQ ID NO: 264, or at a position corresponding to position 6102982 of (on chromosome A06) of reference *Brassica napus* genome Darmor-bzh v4.1; wherein said SNP is H. In certain embodiments, the clubroot non-(increased) resistance or susceptible allele of molecular marker ra74922s01 comprises a SNP at position 51 of SEQ ID NO: 264 or a sequence which is at least 90% identical, preferably at least 95% identical, more preferably at least 98% identical, most preferably identical to SEQ ID NO: 264, or at a position corresponding to position 6102982 of (on chromosome A06) of reference *Brassica napus* genome Darmor-bzh v4.1; wherein said SNP is A.

In certain embodiments, the clubroot (increased) resistance allele of molecular marker ra74860s01 comprises a SNP at position 51 of SEQ ID NO: 250 or a sequence which is at least 90% identical, preferably at least 95% identical, more preferably at least 98% identical, most preferably identical to SEQ ID NO: 250, or at a position corresponding to position 6113367 of (on chromosome A06) of reference *Brassica napus* genome Darmor-bzh v4.1; wherein said SNP is T. In certain embodiments, the clubroot non-(increased) resistance or susceptible allele of molecular marker ra74860s01 comprises a SNP at position 51 of SEQ ID NO: 250 or a sequence which is at least 90% identical, preferably at least 95% identical, more preferably at least 98% identical, most preferably identical to SEQ ID NO: 250, or at a position corresponding to position 6113367 of (on chromosome A06) of reference *Brassica napus* genome Darmor-bzh v4.1; wherein said SNP is not T. In certain embodiments, the clubroot non-(increased) resistance or susceptible allele of molecular marker ra74860s01 comprises a SNP at position 51 of SEQ ID NO: 250 or a sequence which is at least 90% identical, preferably at least 95% identical, more preferably at least 98% identical, most preferably identical to SEQ ID NO: 250, or at a position corresponding to position 6113367 of (on chromosome A06) of reference *Brassica napus* genome Darmor-bzh v4.1; wherein said SNP is DV In certain embodiments, the clubroot non-(increased) resistance or susceptible allele of molecular marker ra74860s01 comprises a SNP at position 51 of SEQ ID NO: 250 or a sequence which is at least 90% identical, preferably at least 95% identical, more preferably at least 98% identical, most preferably identical to SEQ ID NO: 250, or at a position corresponding to position 6113367 of (on chromosome A06) of reference *Brassica napus* genome Darmor-bzh v4.1; wherein said SNP is G.

In certain embodiments, the clubroot (increased) resistance allele of molecular marker ra36428s01 comprises a SNP at position 51 of SEQ ID NO: 231 or a sequence which is at least 90% identical, preferably at least 95% identical, more preferably at least 98% identical, most preferably identical to SEQ ID NO: 231, or at a position corresponding to position 6141204 of (on chromosome A06) of reference *Brassica napus* genome Darmor-bzh v4.1; wherein said SNP is C. In certain embodiments, the clubroot non-(increased) resistance or susceptible allele of molecular marker ra36428s01 comprises a SNP at position 51 of SEQ ID NO: 231 or a sequence which is at least 90% identical, preferably at least 95% identical, more preferably at least 98% identical, most preferably identical to SEQ ID NO: 231, or at a position corresponding to position 6141204 of (on chromosome A06) of reference *Brassica napus* genome Darmor-bzh v4.1; wherein said SNP is not C. In certain embodiments, the clubroot non-(increased) resistance or susceptible allele of molecular marker ra36428s01 comprises a SNP at position 51 of SEQ ID NO: 231 or a sequence which is at least 90% identical, preferably at least 95% identical, more preferably at least 98% identical, most preferably identical to SEQ ID NO: 231, or at a position corresponding to position 6141204 of (on chromosome A06) of reference *Brassica napus* genome Darmor-bzh v4.1; wherein said SNP is D. In certain embodiments, the clubroot non-(increased) resistance or susceptible allele of molecular marker ra36428s01 comprises a SNP at position 51 of SEQ ID NO: 231 or a sequence which is at least 90% identical, preferably at least 95% identical, more preferably at least 98% identical, most preferably identical to SEQ ID NO: 231, or at a position corresponding to position 6141204 of (on chromosome A06) of reference *Brassica napus* genome Darmor-bzh v4.1; wherein said SNP is T.

In certain embodiments, the clubroot (increased) resistance allele of molecular marker ra74862s01 comprises a SNP at position 51 of SEQ ID NO: 251 or a sequence which is at least 90% identical, preferably at least 95% identical, more preferably at least 98% identical, most preferably identical to SEQ ID NO: 251, or at a position corresponding to position 6155076 of (on chromosome A06) of reference *Brassica napus* genome Darmor-bzh v4.1; wherein said SNP is T. In certain embodiments, the clubroot non-(increased) resistance or susceptible allele of molecular marker ra74862s01 comprises a SNP at position 51 of SEQ ID NO: 251 or a sequence which is at least 90% identical, preferably at least 95% identical, more preferably at least 98% identical, most preferably identical to SEQ ID NO: 251, or at a position corresponding to position 6155076 of (on chromosome A06) of reference *Brassica napus* genome Darmor-bzh v4.1; wherein said SNP is not T. In certain embodiments, the clubroot non-(increased) resistance or susceptible allele of molecular marker ra74862s01 comprises a SNP at position 51 of SEQ ID NO: 251 or a sequence which is at least 90% identical, preferably at least 95% identical, more preferably at least 98% identical, most preferably identical to SEQ ID NO: 251, or at a position corresponding to position 6155076 of (on chromosome A06) of reference *Brassica napus* genome Darmor-bzh v4.1; wherein said SNP is V. In certain embodiments, the clubroot non-(increased) resistance or susceptible allele of molecular marker ra74862s01 comprises a SNP at position 51 of SEQ ID NO: 251 or a sequence which is at least 90% identical, preferably at least 95% identical, more preferably at least 98% identical, most preferably identical to SEQ ID NO: 251, or at a position corresponding to position 6155076 of (on chromosome A06) of reference *Brassica napus* genome Darmor-bzh v4.1; wherein said SNP is A.

In certain embodiments, the clubroot (increased) resistance allele of molecular marker ra74863s01 comprises a SNP at position 51 of SEQ ID NO: 252 or a sequence which is at least 90% identical, preferably at least 95% identical, more preferably at least 98% identical, most preferably identical to SEQ ID NO: 252, or at a position corresponding to position 6186575 of (on chromosome A06) of reference *Brassica napus* genome Darmor-bzh v4.1; wherein said SNP is C. In certain embodiments, the clubroot non-(increased) resistance or susceptible allele of molecular marker ra74863s01 comprises a SNP at position 51 of SEQ ID NO: 252 or a sequence which is at least 90% identical, preferably at least 95% identical, more preferably at least 98% identical, most preferably identical to SEQ ID NO: 252, or at a position corresponding to position 6186575 of (on chromosome A06) of reference *Brassica napus* genome Darmor-bzh v4.1; wherein said SNP is not C. In certain embodiments, the clubroot non-(increased) resistance or susceptible allele of molecular marker ra74863s01 comprises a SNP at position 51 of SEQ ID NO: 252 or a sequence which is at least 90% identical, preferably at least 95% identical, more preferably at least 98% identical, most preferably identical to SEQ ID NO: 252, or at a position corresponding to position 6186575 of (on chromosome A06) of reference *Brassica napus* genome Darmor-bzh v4.1; wherein said SNP is D. In certain embodiments, the clubroot non-(increased) resistance or susceptible allele of molecular marker ra74863s01 comprises a SNP at position 51 of SEQ ID NO: 252 or a sequence which is at least 90% identical, preferably at least 95% identical, more preferably at least 98% identical, most preferably identical to SEQ ID NO: 252, or at a position corresponding to position 6186575 of (on chromosome A06) of reference *Brassica napus* genome Darmor-bzh v4.1; wherein said SNP is A.

In certain embodiments, the clubroot (increased) resistance allele of molecular marker ra74864s01 comprises a SNP at position 51 of SEQ ID NO: 253 or a sequence which is at least 90% identical, preferably at least 95% identical, more preferably at least 98% identical, most preferably identical to SEQ ID NO: 253, or at a position corresponding to position 6196798 of (on chromosome A06) of reference *Brassica napus* genome Darmor-bzh v4.1; wherein said SNP is A. In certain embodiments, the clubroot non-(increased) resistance or susceptible allele of molecular marker ra74864s01 comprises a SNP at position 51 of SEQ ID NO: 253 or a sequence which is at least 90% identical, preferably at least 95% identical, more preferably at least 98% identical, most preferably identical to SEQ ID NO: 253, or at a position corresponding to position 6196798 of (on chromosome A06) of reference *Brassica napus* genome Darmor-bzh v4.1; wherein said SNP is not A. In certain embodiments, the clubroot non-(increased) resistance or susceptible allele of molecular marker ra74864s01 comprises a SNP at position 51 of SEQ ID NO: 253 or a sequence which is at least 90% identical, preferably at least 95% identical, more preferably at least 98% identical, most preferably identical to SEQ ID NO: 253, or at a position corresponding to position 6196798 of (on chromosome A06) of reference *Brassica napus* genome Darmor-bzh v4.1; wherein said SNP is B. In certain embodiments, the clubroot non-(increased) resistance or susceptible allele of molecular marker ra74864s01 comprises a SNP at position 51 of SEQ ID NO: 253 or a sequence which is at least 90% identical, preferably at least 95% identical, more preferably at least 98% identical, most preferably identical to SEQ ID NO: 253, or at a position corresponding to position 6196798 of (on chromosome A06) of reference *Brassica napus* genome Darmor-bzh v4.1; wherein said SNP is C.

In certain embodiments, the clubroot (increased) resistance allele of molecular marker ra74910s01 comprises a SNP at position 51 of SEQ ID NO: 274 or a sequence which is at least 90% identical, preferably at least 95% identical, more preferably at least 98% identical, most preferably identical to SEQ ID NO: 274, or at a position corresponding to position 6197815 of (on chromosome A06) of reference *Brassica napus* genome Darmor-bzh v4.1; wherein said SNP is A. In certain embodiments, the clubroot non-(increased) resistance or susceptible allele of molecular marker ra74910s01 comprises a SNP at position 51 of SEQ ID NO: 274 or a sequence which is at least 90% identical, preferably at least 95% identical, more preferably at least 98% identical, most preferably identical to SEQ ID NO: 274, or at a position corresponding to position 6197815 of (on chromosome A06) of reference *Brassica napus* genome Darmor-bzh v4.1; wherein said SNP is not A. In certain embodiments, the clubroot non-(increased) resistance or susceptible allele of molecular marker ra74910s01 comprises a SNP at position 51 of SEQ ID NO: 274 or a sequence which is at least 90% identical, preferably at least 95% identical, more preferably at least 98% identical, most preferably identical to SEQ ID NO: 274, or at a position corresponding to position 6197815 of (on chromosome A06) of reference *Brassica napus* genome Darmor-bzh v4.1; wherein said SNP is B. In certain embodiments, the clubroot non-(increased) resistance or susceptible allele of molecular marker ra74910s01 comprises a SNP at position 51 of SEQ ID NO: 274 or a sequence which is at least 90% identical, preferably at least 95% identical, more preferably at least 98% identical, most preferably identical to SEQ ID NO: 274, or at a position corresponding to position 6197815 of (on chromosome A06) of reference *Brassica napus* genome Darmor-bzh v4.1; wherein said SNP is G.

In certain embodiments, the clubroot (increased) resistance allele of molecular marker ra74865s01 comprises a SNP at position 51 of SEQ ID NO: 254 or a sequence which is at least 90% identical, preferably at least 95% identical, more preferably at least 98% identical, most preferably identical to SEQ ID NO: 254, or at a position corresponding to position 6204215 of (on chromosome A06) of reference *Brassica napus* genome Darmor-bzh v4.1; wherein said SNP is C. In certain embodiments, the clubroot non-(increased) resistance or susceptible allele of molecular marker ra74865s01 comprises a SNP at position 51 of SEQ ID NO: 254 or a sequence which is at least 90% identical, preferably at least 95% identical, more preferably at least 98% identical, most preferably identical to SEQ ID NO: 254, or at a position corresponding to position 6204215 of (on chromosome A06) of reference *Brassica napus* genome Darmor-bzh v4.1; wherein said SNP is not C. In certain embodiments, the clubroot non-(increased) resistance or susceptible allele of molecular marker ra74865s01 comprises a SNP at position 51 of SEQ ID NO: 254 or a sequence which is at least 90% identical, preferably at least 95% identical, more preferably at least 98% identical, most preferably identical to SEQ ID NO: 254, or at a position corresponding to position 6204215 of (on chromosome A06) of reference *Brassica napus* genome Darmor-bzh v4.1; wherein said SNP is D. In certain embodiments, the clubroot non-(increased) resistance or susceptible allele of molecular marker ra74865s01 comprises a SNP at position 51 of SEQ ID NO: 254 or a sequence which is at least 90% identical, preferably at least 95% identical, more preferably at least 98% identical, most preferably identical to SEQ ID NO: 254, or at a position corresponding to position 6204215 of (on chromosome A06) of reference *Brassica napus* genome Darmor-bzh v4.1; wherein said SNP is T.

In certain embodiments, the clubroot (increased) resistance allele of molecular marker ra74952s01 comprises a SNP at position 51 of SEQ ID NO: 273 or a sequence which is at least 90% identical, preferably at least 95% identical, more preferably at least 98% identical, most preferably identical to SEQ ID NO: 273, or at a position corresponding to position 6209886 of (on chromosome A06) of reference *Brassica napus* genome Darmor-bzh v4.1; wherein said SNP is C. In certain embodiments, the clubroot non-(increased) resistance or susceptible allele of molecular marker ra74952s01 comprises a SNP at position 51 of SEQ ID NO: 273 or a sequence which is at least 90% identical, preferably at least 95% identical, more preferably at least 98% identical, most preferably identical to SEQ ID NO: 273, or at a position corresponding to position 6209886 of (on chromosome A06) of reference *Brassica napus* genome Darmor-bzh v4.1; wherein said SNP is not C. In certain embodiments, the clubroot non-(increased) resistance or susceptible allele of molecular marker ra74952s01 comprises a SNP at position 51 of SEQ ID NO: 273 or a sequence which is at least 90% identical, preferably at least 95% identical, more preferably at least 98% identical, most preferably identical to SEQ ID NO: 273, or at a position corresponding to position 6209886 of (on chromosome A06) of reference *Brassica napus* genome Darmor-bzh v4.1; wherein said SNP is D. In certain embodiments, the clubroot non-(increased) resistance or susceptible allele of molecular marker ra74952s01 comprises a SNP at position 51 of SEQ ID NO: 273 or a sequence which is at least 90% identical, preferably at least 95% identical, more preferably at least 98% identical, most preferably identical to SEQ ID NO: 273, or at a position corresponding to position 6209886 of (on chromosome A06) of reference *Brassica napus* genome Darmor-bzh v4.1; wherein said SNP is T.

In certain embodiments, the clubroot (increased) resistance allele of molecular marker ra74867s01 comprises a SNP at position 51 of SEQ ID NO: 255 or a sequence which is at least 90% identical, preferably at least 95% identical, more preferably at least 98% identical, most preferably identical to SEQ ID NO: 255, or at a position corresponding to position 6248401 of (on chromosome A06) of reference *Brassica napus* genome Darmor-bzh v4.1; wherein said SNP is C. In certain embodiments, the clubroot non-(increased) resistance or susceptible allele of molecular marker ra74867s01 comprises a SNP at position 51 of SEQ ID NO: 255 or a sequence which is at least 90% identical, preferably at least 95% identical, more preferably at least 98% identical, most preferably identical to SEQ ID NO: 255, or at a position corresponding to position 6248401 of (on chromosome A06) of reference *Brassica napus* genome Darmor-bzh v4.1; wherein said SNP is not C. In certain embodiments, the clubroot non-(increased) resistance or susceptible allele of molecular marker ra74867s01 comprises a SNP at position 51 of SEQ ID NO: 255 or a sequence which is at least 90% identical, preferably at least 95% identical, more preferably at least 98% identical, most preferably identical to SEQ ID NO: 255, or at a position corresponding to position 6248401 of (on chromosome A06) of reference *Brassica napus* genome Darmor-bzh v4.1; wherein said SNP is D. In certain embodiments, the clubroot non-(increased) resistance or susceptible allele of molecular marker ra74867s01 comprises a SNP at position 51 of SEQ ID NO: 255 or a sequence which is at least 90% identical, preferably at least 95% identical, more preferably at least 98% identical, most preferably identical to SEQ ID NO: 255, or at a position corresponding to position 6248401 of (on chromosome A06) of reference *Brassica napus* genome Darmor-bzh v4.1; wherein said SNP is A.

In certain embodiments, the clubroot (increased) resistance allele of molecular marker ra36444s01 comprises a SNP at position 51 of SEQ ID NO: 223 or a sequence which is at least 90% identical, preferably at least 95% identical, more preferably at least 98% identical, most preferably identical to SEQ ID NO: 223, or at a position corresponding to position 6269510 of (on chromosome A06) of reference *Brassica napus* genome Darmor-bzh v4.1; wherein said SNP is T. In certain embodiments, the clubroot non-(increased) resistance or susceptible allele of molecular marker ra36444s01 comprises a SNP at position 51 of SEQ ID NO: 223 or a sequence which is at least 90% identical, preferably at least 95% identical, more preferably at least 98% identical, most preferably identical to SEQ ID NO: 223, or at a position corresponding to position 6269510 of (on chromosome A06) of reference *Brassica napus* genome Darmor-bzh v4.1; wherein said SNP is not T. In certain embodiments, the clubroot non-(increased) resistance or susceptible allele of molecular marker ra36444s01 comprises a SNP at position 51 of SEQ ID NO: 223 or a sequence which is at least 90% identical, preferably at least 95% identical, more preferably at least 98% identical, most preferably identical to SEQ ID NO: 223, or at a position corresponding to position 6269510 of (on chromosome A06) of reference *Brassica napus* genome Darmor-bzh v4.1; wherein said SNP is V. In certain embodiments, the clubroot non-(increased) resistance or susceptible allele of molecular marker ra36444s01 comprises a SNP at position 51 of SEQ ID NO: 223 or a sequence which is at least 90% identical, preferably at least 95% identical, more preferably at least 98% identical, most preferably identical to SEQ ID NO: 223, or at a position corresponding to position 6269510 of (on chromosome A06) of reference *Brassica napus* genome Darmor-bzh v4.1; wherein said SNP is C.

In certain embodiments, the methods for identifying or selecting a plant or plant part as described herein according to the invention are methods for identifying or selecting a plant or plant part having (increased) clubroot resistance and/or tolerance, such as in comparison to a plant or plant part of the Brassicaceae family not having said polynucleotide, molecular marker (allele), locus (allele), QTL (allele), or SNP (allele) of the invention. In certain embodiments, the methods for identifying or selecting a plant or plant part as described herein according to the invention are methods for identifying or selecting a plant or plant part having (increased) resistance and/or tolerance to *Plasmodiophora,* preferably *Plasmodiophora brassicae,* such as in comparison to a plant or plant part of the Brassicaceae family not having said polynucleotide, molecular marker (allele), locus (allele), QTL (allele), or SNP (allele) of the invention.

In certain embodiments, the methods for identifying or selecting a plant or plant part as described herein according to the invention are methods for identifying or selecting a plant or plant part not having (increased) clubroot resistance and/or tolerance, such as in comparison to a plant or plant part of the Brassicaceae family not having said polynucleotide, molecular marker (allele), locus (allele), QTL (allele), or SNP (allele) of the invention. In certain embodiments, the methods for identifying or selecting a plant or plant part as described herein according to the invention are methods for identifying or selecting a plant or plant part not having (increased) resistance and/or tolerance to *Plasmodiophora,* preferably *Plasmodiophora brassicae,* such as in comparison to a plant or plant part of the Brassicaceae family not having said polynucleotide, molecular marker (allele), locus (allele), QTL (allele), or SNP (allele) of the invention. The plant or plant part is identified as such if the corresponding QTL allele, polynucleotide (allele), locus allele, marker allele, or SNP allele is identified or detected, as described herein elsewhere.

In certain embodiments, the methods for identifying or selecting a plant or plant part as described herein according to the invention are methods for distinguishing or discriminating between a plant or plant part having (increased) clubroot resistance and/or tolerance and a plant or plant part not having (increased) clubroot resistance and/or tolerance, such as in comparison to a plant or plant part of the Brassicaceae family not having said polynucleotide, molecular marker (allele), locus (allele), QTL (allele), or SNP (allele) of the invention.

It will be understood that in the context of plant parts, whether or not such plant part is identified or selected as having (increased) clubroot resistance or tolerance relates to resistance/tolerance of the plant from which the plant part is isolated or derived, or alternatively relates to a plant generated from or resulting from such plant part.

In an aspect, the invention relates to a plant or plant part identified and/or selected according to the methods for identifying and/or selecting plants or plant parts according to the invention as described herein.

In certain embodiments, in the plants or plant parts according to the invention as described herein, the QTL (allele), polynucleotide (allele), locus (allele), marker (allele), or SNP (allele) is homozygous.

In certain embodiments, in the plants or plant parts according to the invention as described herein, the QTL allele, polynucleotide allele, locus allele, marker allele, or SNP allele is homozygous.

In certain embodiments, in the plants or plant parts according to the invention as described herein, the QTL (allele), polynucleotide (allele), locus (allele), marker (allele), or SNP (allele) is heterozygous.

In certain embodiments, in the plants or plant parts according to the invention as described herein, the QTL allele, polynucleotide allele, locus allele, marker allele, or SNP allele is heterozygous.

In certain embodiments, in the methods for identifying and/or selecting plants or plant parts according to the invention as described herein, the QTL (allele), polynucleotide (allele), locus (allele), marker (allele), or SNP (allele) is homozygous.

In certain embodiments, in the methods for identifying and/or selecting plants or plant parts according to the invention as described herein, the QTL allele, polynucleotide allele, locus allele, marker allele, or SNP allele is homozygous.

In certain embodiments, in the methods for identifying and/or selecting plants or plant parts according to the invention as described herein, the QTL (allele), polynucleotide (allele), locus (allele), marker (allele), or SNP (allele) is heterozygous.

In certain embodiments, in the methods for identifying and/or selecting plants or plant parts according to the invention as described herein, the QTL allele, polynucleotide allele, locus allele, marker allele, or SNP allele is heterozygous.

In certain embodiments, in the methods for generating plants or plant parts according to the invention as described herein, the QTL (allele), polynucleotide (allele), locus (allele), marker (allele), or SNP (allele) is homozygous.

In certain embodiments, in the methods for generating plants or plant parts according to the invention as described herein, the QTL allele, polynucleotide allele, locus allele, marker allele, or SNP allele is homozygous.

In certain embodiments, in the methods for generating plants or plant parts according to the invention as described herein, the QTL (allele), polynucleotide (allele), locus (allele), marker (allele), or SNP (allele) is heterozygous.

In certain embodiments, in the methods for generating plants or plant parts according to the invention as described herein, the QTL allele, polynucleotide allele, locus allele, marker allele, or SNP allele is heterozygous.

In certain embodiments, in the methods for identifying and/or selecting plants or plant parts according to the invention as described herein, the method comprises isolating genomic DNA from said plant or plant part.

In certain embodiments, in the methods for identifying and/or selecting plants or plant parts according to the invention as described herein, the method comprises selecting said plant or plant part.

In certain embodiments, in the methods for identifying and/or selecting plants or plant parts according to the invention as described herein, the method comprises selecting said plant or plant part if said QTL (allele), polynucleotide (allele), locus (allele) molecular marker (allele), or SNP (allele) is detected or identified.

In certain embodiments, in the methods for identifying and/or selecting plants or plant parts according to the invention as described herein, the method comprises selecting said plant or plant part if said QTL allele, polynucleotide allele, locus allele molecular marker allele, or SNP allele is detected or identified.

In an aspect, the invention relates to Brassica napus hybrid seed designated H9206006, a representative sample of which has been deposited under NCIMB (National Collection of Industrial Food and Marine Bacteria; Ltd. Ferguson Building, Craibstone Estate, Bucksburn, Aberdeen, AB21 9YA Scotland) on February 28, 2022, Accession No. NCIMB 43941, or plants or plant parts grown or obtained therefrom. In an aspect, the invention relates to *Brassica napus* seed as deposited under NCIMB Accession No. NCIMB 43941, or plants or plant parts grown or obtained therefrom. Plants grown or obtained from seed deposited under NCIMB Accession No. NCIMB 43941 exhibit a (increased) clubroot resistance. Seed deposited under NCIMB Accession No. NCIMB 43941 comprises a QTL according to the invention as described herein and conferring (increased) clubroot resistance. Seed deposited under NCIMB Accession No. NCIMB 43941 comprises molecular marker alleles ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01 according to the invention as described herein, associated with (increased) clubroot resistance, such as in comparison to a plant or plant part of the Brassicaceae family not having said polynucleotide, molecular marker (allele), locus (allele), QTL (allele), or SNP (allele) of the invention.

In an aspect, the invention relates to *Brassica napus* hybrid seed designated H9206006, a representative sample of which has been deposited under NCIMB Accession No. NCIMB 43941, or plants or plant parts grown or obtained therefrom. In an aspect, the invention relates to *Brassica napus* hybrid seed H9206006 as deposited under NCIMB Accession No. NCIMB 43941, or plants or plant parts grown or obtained therefrom.

In an aspect, the invention relates to a plant or plant part of the *Brassicaceae* family comprising the QTL (allele), polynucleic acid (allele), locus (allele), molecular marker (allele(s)), or SNP (allele(s)) according to the invention as described herein.

In an aspect, the invention relates to a plant or plant part of the *Brassicaceae* family comprising the QTL allele, polynucleic acid allele, locus allele, molecular marker allele(s), or SNP allele(s) according to the invention as described herein.

In an aspect, the invention relates to a plant or plant part of the *Brassicaceae* family having (increased) clubroot resistance and/or tolerance (, comprising the QTL (allele), polynucleic acid (allele), locus (allele), molecular marker (allele(s)), or SNP (allele(s)) according to the invention as described herein.

In an aspect, the invention relates to a plant or plant part of the *Brassicaceae* family having (increased) clubroot resistance and/or tolerance, comprising the QTL allele, polynucleic acid allele, locus allele, molecular marker allele(s), or SNP allele(s) according to the invention as described herein (such as in comparison to a plant or plant part of the Brassicaceae family not having said polynucleotide, molecular marker (allele), locus (allele), QTL (allele), or SNP (allele) of the invention).

In certain embodiments, the plant or plant part according to the invention as described herein is mutagenized. In certain embodiments, the plant or plant part selected or identified according to the invention as described herein is mutagenized. In certain embodiments, the plant or plant part generated according to the invention as described herein is mutagenized.

In certain embodiments, the plant or plant part according to the invention as described herein is gene-edited. In certain embodiments, the plant or plant part selected or identified according to the invention as described herein is gene-edited. In certain embodiments, the plant or plant part generated according to the invention as described herein is gene-edited.

In certain embodiments, the plant or plant part according to the invention as described herein is transgenic. In certain embodiments, the plant or plant part selected or identified according to the invention as described herein is transgenic. In certain embodiments, the plant or plant part generated according to the invention as described herein is transgenic.

In certain embodiments, the plant or plant part according to the invention as described herein is transformed. In certain embodiments, the plant or plant part selected or identified according to the invention as described herein is transformed. In certain embodiments, the plant or plant part generated according to the invention as described herein is transformed.

In certain embodiments, the plant or plant part according to the invention as described herein is obtained by introgression of a polynucleic acid (allele), QTL (allele), locus (allele), molecular marker (allele(s)), or SNP (allele(s)) of the invention as described herein.

In certain embodiments, the plant or plant part according to the invention as described herein is obtained by transformation of a polynucleic acid (allele), QTL (allele), locus (allele), molecular marker (allele(s)), or SNP (allele(s)) of the invention as described herein.

In certain embodiments, the plant or plant part according to the invention as described herein is obtained by introgression of a polynucleic acid allele, QTL allele, locus allele, molecular marker allele(s), or SNP allele(s) of the invention as described herein.

In certain embodiments, the plant or plant part according to the invention as described herein is obtained by transformation of a polynucleic acid allele, QTL allele, locus allele, molecular marker allele(s), or SNP allele(s) of the invention as described herein.

In certain embodiments, the plant or plant part according to the invention as described herein is obtained by introgression of a clubroot resistance polynucleic acid allele, QTL allele, locus allele, molecular marker allele(s), or SNP allele(s) of the invention as described herein.

In certain embodiments, the plant or plant part according to the invention as described herein is obtained by transformation of a clubroot resistance polynucleic acid allele, QTL allele, locus allele, molecular marker allele(s), or SNP allele(s) of the invention as described herein.

In an aspect, the invention relates to a method for generating, producing, or obtaining a plant or plant part of the *Brassicaceae* family, comprising

> providing a seed mixture harvested from a cross between a first parent plant (population) of the *Brassicaceae* family and a second parent plant (population) of the Brassicaceae family, wherein said first and/or second parent plant (population) comprises a polynucleic acid (allele), molecular marker (allele), locus (allele) QTL (allele), or SNP (allele) according to the invention as described herein;
> selecting seeds comprising a polynucleic acid (allele), molecular marker (allele), QTL (allele), or SNP (allele) according to the invention as described herein.

In an aspect, the invention relates to a method for generating, producing, or obtaining a plant or plant part of the *Brassicaceae* family, comprising

> providing a seed mixture harvested from a cross between a first parent plant (population) of the *Brassicaceae* family and a second parent plant (population) of the Brassicaceae family, wherein said first and/or second parent plant (population) comprises a polynucleic acid allele, molecular marker allele, locus allele, QTL allele, or SNP allele according to the invention as described herein;
> selecting seeds comprising a polynucleic acid allele, molecular marker allele, QTL allele, or SNP allele according to the invention as described herein.

In an aspect, the invention relates to a method for generating, producing, or obtaining a plant or plant part of the *Brassicaceae* family, comprising

> providing a seed mixture harvested from a cross between a first parent plant (population) of the *Brassicaceae* family and a second parent plant (population) of the Brassicaceae family, wherein said first and/or second parent plant (population) comprises a clubroot resistance polynucleic acid allele, molecular marker allele, locus allele, QTL allele, or SNP allele according to the invention as described herein;

selecting seeds comprising a clubroot resistance polynucleic acid allele, molecular marker allele, QTL allele, or SNP allele according to the invention as described herein.

In an aspect, the invention relates to a method for generating, producing, or obtaining a plant or plant part of the *Brassicaceae* family, comprising introducing in a plant or plant part of the *Brassicaceae* family a polynucleic acid (allele), molecular marker (allele), locus (allele), QTL (allele), or SNP (allele) according to the invention as described herein.

In an aspect, the invention relates to a method for generating, producing, or obtaining a plant or plant part of the *Brassicaceae* family, comprising introducing in a plant or plant part of the *Brassicaceae* family a polynucleic acid allele, molecular marker allele, locus allele, QTL allele, or SNP allele according to the invention as described herein.

In an aspect, the invention relates to a method for generating, producing, or obtaining a plant or plant part of the *Brassicaceae* family, comprising introducing in a plant or plant part of the *Brassicaceae* family a clubroot resistance polynucleic acid allele, molecular marker allele, locus allele, QTL allele, or SNP allele according to the invention as described herein.

In an aspect, the invention relates to a method for generating, producing, or obtaining a plant or plant part of the *Brassicaceae* family, comprising introducing in the genome of a plant or plant part of the *Brassicaceae* family a polynucleic acid (allele), molecular marker (allele), locus (allele), QTL (allele), or SNP (allele) according to the invention as described herein.

In an aspect, the invention relates to a method for generating, producing, or obtaining a plant or plant part of the *Brassicaceae* family, comprising introducing in the genome of a plant or plant part of the *Brassicaceae* family a polynucleic acid allele, molecular marker allele, locus allele, QTL allele, or SNP allele according to the invention as described herein.

In an aspect, the invention relates to a method for generating, producing, or obtaining a plant or plant part of the *Brassicaceae* family, comprising introducing in the genome of a plant or plant part of the *Brassicaceae* family a clubroot resistance polynucleic acid allele, molecular marker allele, locus allele, QTL allele, or SNP allele according to the invention as described herein.

In certain embodiments, introducing in (the genome of) a plant or plant part comprised introgression.

In certain embodiments, such methods comprise (a) providing a first plant of the *Brassicaceae* family having the polynucleic acid, molecular marker, locus, QTL, or SNP, (b) crossing said first plant with a second plant of the *Brassicaceae* family, and (c) selecting progeny plants or plant parts having the polynucleic acid, molecular marker allele, locus, QTL, or SNP.

In certain embodiments, introducing in (the genome of) a plant or plant part comprises gene-editing.

In certain embodiments, introducing in (the genome of) a plant or plant part comprises transgenesis.

In certain embodiments, introducing in (the genome of) a plant or plant part comprises transformation.

In certain embodiments, introducing in (the genome of) a plant or plant part comprises mutagenesis.

It will be understood that while the QTL of the present invention originates from *Brassica* rapa, it can be introduced in *Brassica rapa* or alternatively in any other species from the *Brassicaceae* family, such as in particular *Brassica napus.*

In certain embodiments, the methods for generating, producing, or obtaining a plant or plant part comprise harvesting a plant part (such as a seed resulting from a cross between a first and second plan). In certain embodiments, the methods for generating, producing, or obtaining a plant or plant part comprise harvesting a plant part from the progeny plants.

In certain embodiments, the methods for generating, producing, or obtaining a plant or plant part comprise transforming a plant or plant part, preferably a plant cell, more preferably an immature or mature embryo, an inflorescence, a protoplast or callus, with said polynucleic acid, locus, molecular marker allele, QTL, or SNP, and optionally regenerating a plant from said plant cell, preferably immature or mature embryo, inflorescence, protoplast or callus.

In an aspect, the invention relates to a (isolated) polynucleic acid, QTL, locus, or marker according to the invention as described herein, or a (unique) fragment thereof, the complement of any thereof, or the reverse complement of any thereof.

In an aspect, the invention relates to a (isolated) polynucleic acid allele, QTL allele, locus allele, or marker allele according to the invention as described herein, or a (unique) fragment thereof, the complement of any thereof, or the reverse complement of any thereof.

In an aspect, the invention relates to a (isolated) clubroot resistance polynucleic acid allele, QTL allele, locus allele, or marker allele according to the invention as described herein, or a (unique) fragment thereof, the complement of any thereof, or the reverse complement of any thereof.

Fragments preferably are (contiguous) fragments of at least 15, preferably at least 18, more preferably at least 20 (contiguous) nucleotides.

In certain embodiments, the polynucleic acid, QTL, or locus according to the invention as described herein is at most 10 Mbp. In certain embodiments, the polynucleic acid, QTL, or locus according to the invention as described herein is at most 5 Mbp. In certain embodiments, the polynucleic acid, QTL, or locus according to the invention as described herein is at most 2 Mbp. In certain embodiments, the polynucleic acid, QTL, or locus according to the invention as described herein is at most 10 cM. In certain embodiments, the polynucleic acid, QTL, or locus according to the invention as described herein is at most 5 cM. In certain embodiments, the polynucleic acid, QTL, or locus according to the invention as described herein is at most 2 cM.

In certain embodiments, the polynucleic acid, QTL, or locus according to the invention as described herein is at most 1 Mbp. In certain embodiments, the polynucleic acid, QTL, or locus according to the invention as described herein is at most 0.5 Mbp. In certain embodiments, the polynucleic acid, QTL, or locus according to the invention as described herein is at most 0.2 Mbp.

In certain embodiments, the molecular marker or polynucleic acid according to the invention as described herein comprises at most 500 nucleotides. In certain embodiments, the molecular marker or polynucleic acid according to the invention as described herein comprises at most 300 nucleotides. In certain embodiments, the molecular marker or polynucleic acid according to the invention as described herein comprises at most 100 nucleotides. In certain embodiments, the molecular marker or polynucleic acid according to the invention as described herein comprises at least 15 nucleotides. In certain embodiments, the molecular marker or polynucleic acid according to the invention as described herein comprises at least 18 nucleotides. In certain embodiments, the molecular marker or polynucleic acid according to the invention as described herein comprises at least 20 nucleotides. In certain embodiments, the molecular marker or polynucleic acid according to the invention as described herein comprises at least 50 nucleotides.

In certain embodiments, the molecular marker or polynucleic acid according to the invention as described herein comprises at most 500 nucleotides and at least 15 nucleotides. In certain embodiments, the molecular marker or polynucleic acid according to the invention as described herein comprises at most 300 nucleotides and at least 15 nucleotides. In certain embodiments, the molecular marker or polynucleic acid according to the invention as described herein comprises at most 100 nucleotides and at least 15 nucleotides.

In certain embodiments, the molecular marker or polynucleic acid according to the invention as described herein comprises at most 500 nucleotides and at least 18 nucleotides. In certain embodiments, the molecular marker or polynucleic acid according to the invention as described herein comprises at most 300 nucleotides and at least 18 nucleotides. In certain embodiments, the molecular marker or polynucleic acid according to the invention as described herein comprises at most 100 nucleotides and at least 18 nucleotides.

In certain embodiments, the molecular marker or polynucleic acid according to the invention as described herein comprises at most 500 nucleotides and at least 20 nucleotides. In certain embodiments, the molecular marker or polynucleic acid according to the invention as described herein comprises at most 300 nucleotides and at least 20 nucleotides. In certain embodiments, the molecular marker or polynucleic acid according to the invention as described herein comprises at most 100 nucleotides and at least 20 nucleotides.

In certain embodiments, the molecular marker or polynucleic acid according to the invention as described herein comprises at most 500 nucleotides and at least 50 nucleotides. In certain embodiments, the molecular marker or polynucleic acid according to the invention as described herein comprises at most 300 nucleotides and at least 50 nucleotides. In certain embodiments, the molecular marker or polynucleic acid according to the invention as described herein comprises at most 100 nucleotides and at least 50 nucleotides.

In an aspect, the invention relates to a (isolated) polynucleic acid having a sequence as set forth in any of SEQ ID NOs: 1 to 274 or to 293, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 1 to 274 or to 293, the complement thereof, or the reverse complement thereof. The most 3' nucleotide is preferably identical to the most 3' nucleotide of the respective SEQ ID NO (or the complement thereof).

In an aspect, the invention relates to a (isolated) polynucleic acid having a sequence as set forth in any of SEQ ID NOs: 1 to 221, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 1 to 221, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 1 to 221, the complement thereof, or the reverse complement thereof.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 1 to 221, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least the 15, preferably at least 18, more preferably at least 20 most 3' contiguous nucleotides of a sequence as set forth in any of SEQ ID NOs: 1 to 221, the complement thereof, or the reverse complement thereof.

In an aspect, the invention relates to a (isolated) polynucleic acid comprising the at least 15, preferably at least 18, more preferably at least 20 most 3' contiguous nucleotides of a sequence which is at least 90%, preferably at least 95%, more preferably at least 98% identical to a sequence as set forth in any of SEQ ID NOs: 1 to 221, the complement thereof, or the reverse complement thereof. The most 3' nucleotide is preferably identical to the most 3' nucleotide of the respective SEQ ID NO (or the complement thereof).

In an aspect, the invention relates to a (isolated) polynucleic acid having a sequence as set forth in any of SEQ ID NOs: 2, 10, 26 to 34, 42-43, 49-52, 66, 76, 84, 100-108, 116-117, 123-126, 137, 147, 155, 171-179, 187-188, 194-197, 208, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 2, 10, 26 to 34, 42-43, 49-52, 66, 76, 84, 100-108, 116-117, 123-126, 137, 147, 155, 171-179, 187-188, 194-197, 208, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 2, 10, 26 to 34, 42-43, 49-52, 66, 76, 84, 100-108, 116-117, 123-126, 137, 147, 155, 171-179, 187-188, 194-197, 208, the complement thereof, or the reverse complement thereof.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 2, 10, 26 to 34, 42-43, 49-52, 66, 76, 84, 100-108, 116-117, 123-126, 137, 147, 155, 171-179, 187-188, 194-197, 208, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least the 15, preferably at least 18, more preferably at least 20 most 3' contiguous nucleotides of a sequence as set forth in any of SEQ ID NOs: 2, 10, 26 to 34, 42-43, 49-52, 66, 76, 84, 100-108, 116-117, 123-126, 137, 147, 155, 171-179, 187-188, 194-197, 208, the complement thereof, or the reverse complement thereof.

In an aspect, the invention relates to a (isolated) polynucleic acid comprising the at least 15, preferably at least 18, more preferably at least 20 most 3' contiguous nucleotides of a sequence which is at least 90%, preferably at least 95%, more preferably at least 98% identical to a sequence as set forth in any of SEQ ID NOs: 2, 10, 26 to 34, 42-43, 49-52, 66, 76, 84, 100-108, 116-117, 123-126, 137, 147, 155, 171-179, 187-188, 194-197, 208, the complement thereof, or the reverse complement thereof. The most 3' nucleotide is preferably identical to the most 3' nucleotide of the respective SEQ ID NO (or the complement thereof).

In an aspect, the invention relates to a (isolated) polynucleic acid having a sequence as set forth in any of SEQ ID NOs: 30-34, 52, 66, 104-108, 126, 137, 175-179, 197, 208, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 30-34, 52, 66, 104-108, 126, 137, 175-179, 197, 208, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 30-34, 52, 66, 104-108, 126, 137, 175-179, 197, 208, the complement thereof, or the reverse complement thereof.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 30-34, 52, 66, 104-108, 126, 137, 175-179, 197, 208, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least the 15, preferably at least 18, more preferably at least 20 most 3' contiguous nucleotides of a sequence as set forth in any of SEQ ID NOs: 30-34, 52, 66, 104-108, 126, 137, 175-179, 197, 208, the complement thereof, or the reverse complement thereof.

In an aspect, the invention relates to a (isolated) polynucleic acid comprising the at least 15, preferably at least 18, more preferably at least 20 most 3' contiguous nucleotides of a sequence which is at least 90%, preferably at least 95%, more preferably at least 98% identical to a sequence as set forth in any of SEQ ID NOs: 30-34, 52, 66, 104-108, 126, 137, 175-179, 197, 208, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least the 15, preferably at least 18, more preferably at least 20 most 3' contiguous nucleotides of a sequence as set forth in any of SEQ ID NOs: 1 to 145, the complement thereof, or the reverse complement thereof.

In an aspect, the invention relates to a (isolated) polynucleic acid comprising the at least 15, preferably at least 18, more preferably at least 20 most 3' contiguous nucleotides of a sequence which is at least 90%, preferably at least 95%, more preferably at least 98% identical to a sequence as set forth in any of SEQ ID NOs: 1 to 145, the complement thereof, or the reverse complement thereof. The most 3' nucleotide is preferably identical to the most 3' nucleotide of the respective SEQ ID NO (or the complement thereof).

In an aspect, the invention relates to a (isolated) polynucleic acid having a sequence as set forth in any of SEQ ID NOs: 2, 10, 26 to 34, 42-43, 49-52, 66, 76, 84, 100-108, 116-117, 123-126, and 137, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 2, 10, 26 to 34, 42-43, 49-52, 66, 76, 84, 100-108, 116-117, 123-126, and 137, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 2, 10, 26 to 34, 42-43, 49-52, 66, 76, 84, 100-108, 116-117, 123-126, and 137, the complement thereof, or the reverse complement thereof.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 2, 10, 26 to 34, 42-43, 49-52, 66, 76, 84, 100-108, 116-117, 123-126, and 137, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least the 15, preferably at least 18, more preferably at least 20 most 3' contiguous nucleotides of a sequence as set forth in any of SEQ ID NOs: 2, 10, 26 to 34, 42-43, 49-52, 66, 76, 84, 100-108, 116-117, 123-126, and 137, the complement thereof, or the reverse complement thereof.

In an aspect, the invention relates to a (isolated) polynucleic acid comprising the at least 15, preferably at least 18, more preferably at least 20 most 3' contiguous nucleotides of a sequence which is at least 90%, preferably at least 95%, more preferably at least 98% identical to a sequence as set forth in any of SEQ ID NOs: 2, 10, 26 to 34, 42-43, 49-52, 66, 76, 84, 100-108, 116-117, 123-126, and 137, the complement thereof, or the reverse complement thereof. The most 3' nucleotide is preferably identical to the most 3' nucleotide of the respective SEQ ID NO (or the complement thereof).

In an aspect, the invention relates to a (isolated) polynucleic acid having a sequence as set forth in any of SEQ ID NOs: 30-34, 52, 66, 104-108, 126, or 137, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 30-34, 52, 66, 104-108, 126, or 137, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 30-34, 52, 66, 104-108, 126, or 137, the complement thereof, or the reverse complement thereof.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 30-34, 52, 66, 104-108, 126, or 137, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least the 15, preferably at least 18, more preferably at least 20 most 3' contiguous nucleotides of a sequence as set forth in any of SEQ ID NOs: 30-34, 52, 66, 104-108, 126, or 137, the complement thereof, or the reverse complement thereof.

In an aspect, the invention relates to a (isolated) polynucleic acid comprising the at least 15, preferably at least 18, more preferably at least 20 most 3' contiguous nucleotides of a sequence which is at least 90%, preferably at least 95%, more preferably at least 98% identical to a sequence as set forth in any of SEQ ID NOs: 30-34, 52, 66, 104-108, 126, or 137, the complement thereof, or the reverse complement thereof. The most 3' nucleotide is preferably identical to the most 3' nucleotide of the respective SEQ ID NO (or the complement thereof).

In an aspect, the invention relates to a (isolated) polynucleic acid having a sequence as set forth in any of SEQ ID NOs: 26, 100, 171, 42, 116, 187, 49, 123, 194, 50, 124, 195, 51, 125, 196, 27, 101, 172, 28, 102, 173, 43, 117, 188, 29, 103, 174, 10, 84, 155, 30, 104, 175, 31, 105, 176, 32, 106, 177, 66, 137, 208, 33, 107, 178, 52, 126, 197, 34, 108, 179, 2, 76, or 147, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 26, 100, 171, 42, 116, 187, 49, 123, 194, 50, 124, 195, 51, 125, 196, 27, 101, 172, 28, 102, 173, 43, 117, 188, 29, 103, 174, 10, 84, 155, 30, 104, 175, 31, 105, 176, 32, 106, 177, 66, 137, 208, 33, 107, 178, 52, 126, 197, 34, 108, 179, 2, 76, or 147, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 26, 100, 171, 42, 116, 187, 49, 123, 194, 50, 124, 195, 51, 125, 196, 27, 101, 172, 28, 102, 173, 43, 117, 188, 29, 103, 174, 10, 84, 155, 30, 104, 175, 31, 105, 176, 32, 106, 177, 66, 137, 208, 33, 107, 178, 52, 126, 197, 34, 108, 179, 2, 76, or 147, the complement thereof, or the reverse complement thereof.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 26, 100, 171, 42, 116, 187, 49, 123, 194, 50, 124, 195, 51, 125, 196, 27, 101, 172, 28, 102, 173, 43, 117, 188, 29, 103, 174, 10, 84, 155, 30, 104, 175, 31, 105, 176, 32, 106, 177, 66, 137, 208, 33, 107, 178, 52, 126, 197, 34, 108, 179, 2, 76, or 147, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least the 15, preferably at least 18, more preferably at least 20 most 3' contiguous nucleotides of a sequence as set forth in any of SEQ ID NOs: 26, 100, 171, 42, 116, 187, 49, 123, 194, 50, 124, 195, 51, 125, 196, 27, 101, 172, 28, 102, 173, 43, 117, 188, 29, 103, 174, 10, 84, 155, 30, 104, 175, 31, 105, 176, 32, 106, 177, 66, 137, 208, 33, 107, 178, 52, 126, 197, 34, 108, 179, 2, 76, or 147, the complement thereof, or the reverse complement thereof.

In an aspect, the invention relates to a (isolated) polynucleic acid comprising the at least 15, preferably at least 18, more preferably at least 20 most 3' contiguous nucleotides of a sequence which is at least 90%, preferably at least 95%, more preferably at least 98% identical to a sequence as set forth in any of SEQ ID NOs: 26, 100, 171, 42, 116, 187, 49, 123, 194, 50, 124, 195, 51, 125, 196, 27, 101, 172, 28, 102, 173, 43, 117, 188, 29, 103, 174, 10, 84, 155, 30, 104, 175, 31, 105, 176, 32, 106, 177, 66, 137, 208, 33, 107, 178, 52, 126, 197, 34, 108, 179, 2, 76, or 147, the complement thereof, or the reverse complement thereof. The most 3' nucleotide is preferably identical to the most 3' nucleotide of the respective SEQ ID NO (or the complement thereof).

In an aspect, the invention relates to a (isolated) polynucleic acid having a sequence as set forth in any of SEQ ID NOs: 26, 100, 42, 116, 49, 123, 50, 124, 51, 125, 27, 101, 28, 102, 43, 117, 29, 103, 10, 84, 30, 104, 31, 105, 32, 106, 66, 137, 33, 107, 52, 126, 34, 108, 2, or 76, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 26, 100, 42, 116, 49, 123, 50, 124, 51, 125, 27, 101, 28, 102, 43, 117, 29, 103, 10, 84, 30, 104, 31, 105, 32, 106, 66, 137, 33, 107, 52, 126, 34, 108, 2, or 76, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 26, 100, 42, 116, 49, 123, 50, 124, 51, 125, 27, 101, 28, 102, 43, 117, 29, 103, 10, 84, 30, 104, 31, 105, 32, 106, 66, 137, 33, 107, 52, 126, 34, 108, 2, or 76, the complement thereof, or the reverse complement thereof.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 26, 100, 42, 116, 49, 123, 50, 124, 51, 125, 27, 101, 28, 102, 43, 117, 29, 103, 10, 84, 30, 104, 31, 105, 32, 106, 66, 137, 33, 107, 52, 126, 34, 108, 2, or 76, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least the 15, preferably at least 18, more preferably at least 20 most 3' contiguous nucleotides of a sequence as set forth in any of SEQ ID NOs: 26, 100, 42, 116, 49, 123, 50, 124, 51, 125, 27, 101, 28, 102, 43, 117, 29, 103, 10, 84, 30, 104, 31, 105, 32, 106, 66, 137, 33, 107, 52, 126, 34, 108, 2, or 76, the complement thereof, or the reverse complement thereof.

In an aspect, the invention relates to a (isolated) polynucleic acid comprising the at least 15, preferably at least 18, more preferably at least 20 most 3' contiguous nucleotides of a sequence which is at least 90%, preferably at least 95%, more preferably at least 98% identical to a sequence as set forth in any of SEQ ID NOs: 26, 100, 42, 116, 49, 123, 50, 124, 51, 125, 27, 101, 28, 102, 43, 117, 29, 103, 10, 84, 30, 104, 31, 105, 32, 106, 66, 137, 33, 107, 52, 126, 34, 108, 2, or 76, the complement thereof, or the reverse complement thereof. The most 3' nucleotide is preferably identical to the most 3' nucleotide of the respective SEQ ID NO (or the complement thereof).

In an aspect, the invention relates to a (isolated) polynucleic acid having a sequence as set forth in any of SEQ ID NOs: 30, 104, 175, 31, 105, 176, 32, 106, 177, 66, 137, 208, 33, 107, 178, 52, 126, 197, 34, 108, or 179, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 30, 104, 175, 31, 105, 176, 32, 106, 177, 66, 137, 208, 33, 107, 178, 52, 126, 197, 34, 108, or 179, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 30, 104, 175, 31, 105, 176, 32, 106, 177, 66, 137, 208, 33, 107, 178, 52, 126, 197, 34, 108, or 179, the complement thereof, or the reverse complement thereof.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 30, 104, 175, 31, 105, 176, 32, 106, 177, 66, 137, 208, 33, 107, 178, 52, 126, 197, 34, 108, or 179, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least the 15, preferably at least 18, more preferably at least 20 most 3' contiguous nucleotides of a sequence as set forth in any of SEQ ID NOs: 30, 104, 175, 31, 105, 176, 32, 106, 177, 66, 137, 208, 33, 107, 178, 52, 126, 197, 34, 108, or 179, the complement thereof, or the reverse complement thereof.

In an aspect, the invention relates to a (isolated) polynucleic acid comprising the at least 15, preferably at least 18, more preferably at least 20 most 3' contiguous nucleotides of a sequence which is at least 90%, preferably at least 95%, more preferably at least 98% identical to a sequence as set forth in any of SEQ ID NOs: 30, 104, 175, 31, 105, 176, 32, 106, 177, 66, 137, 208, 33, 107, 178, 52, 126, 197, 34, 108, or 179, the complement thereof, or the reverse complement thereof. The most 3' nucleotide is preferably identical to the most 3' nucleotide of the respective SEQ ID NO (or the complement thereof).

In an aspect, the invention relates to a (isolated) polynucleic acid having a sequence as set forth in any of SEQ ID NOs: 30, 104, 31, 105, 32, 106, 66, 137, 33, 107, 52, 126, 34, or 108, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 30, 104, 31, 105, 32, 106, 66, 137, 33, 107, 52, 126, 34, or 108, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 30, 104, 31, 105, 32, 106, 66, 137, 33, 107, 52, 126, 34, or 108, the complement thereof, or the reverse complement thereof.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 30, 104, 31, 105, 32, 106, 66, 137, 33, 107, 52, 126, 34, or 108, the complement thereof, or the reverse complement thereof. The most 3' nucleotide is preferably identical to the most 3' nucleotide of the respective SEQ ID NO (or the complement thereof).

In an aspect, the invention relates to an (isolated) polynucleic acid comprising at least the 15, preferably at least 18, more preferably at least 20 most 3' contiguous nucleotides of a sequence as set forth in any of SEQ ID NOs: 30, 104, 31, 105, 32, 106, 66, 137, 33, 107, 52, 126, 34, or 108, the complement thereof, or the reverse complement thereof.

In an aspect, the invention relates to a (isolated) polynucleic acid comprising the at least 15, preferably at least 18, more preferably at least 20 most 3' contiguous nucleotides of a sequence which is at least 90%, preferably at least 95%, more preferably at least 98% identical to a sequence as set forth in any of SEQ ID NOs: 30, 104, 31, 105, 32, 106, 66, 137, 33, 107, 52, 126, 34, or 108, the complement thereof, or the reverse complement thereof. The most 3' nucleotide is preferably identical to the most 3' nucleotide of the respective SEQ ID NO (or the complement thereof).

Polynucleotide sequences of SEQ ID NOs: 1 to 221 can be used as primers, in particular KASP primers, suitable for identifying the molecular markers of the present invention. The sequences are annotated in Table 2.

Table 2: sequences suitable for detecting the indicated molecular markers. Allele "X" and Allele "Y" designate clubroot resistant or susceptible alleles (or vice versa, as described herein elsewhere). The indicated SEQ ID NOs can be used as allele-specific primers.

"Common primer" refers to the common primer capable of detecting both alleles (in the opposite orientation of the allele-specific primers).

| SEQ ID NO | Description |
| --- | --- |
| SEQ ID NO:1 | ra36387s01 Allele X |
| SEQ ID NO:2 | ra36444s01 Allele X |
| SEQ ID NO:3 | ra36450s01 Allele X |
| SEQ ID NO:4 | ra36451s01 Allele X |
| SEQ ID NO:5 | ra36458s01 Allele X |
| SEQ ID NO:6 | ra36459s01 Allele X |
| SEQ ID NO:7 | ra36460s01 Allele X |
| SEQ ID NO:8 | ra36461s01 Allele X |
| SEQ ID NO:9 | ra36463s01 Allele X |
| SEQ ID NO:10 | ra36428s01 Allele X |
| SEQ ID NO:11 | ra74835s01 Allele X |
| SEQ ID NO:12 | ra74836s01 Allele X |
| SEQ ID NO:13 | ra74837s01 Allele X |
| SEQ ID NO:14 | ra74838s01 Allele X |
| SEQ ID NO:15 | ra74840s01 Allele X |
| SEQ ID NO:16 | ra74841s01 Allele X |
| SEQ ID NO:17 | ra74844s01 Allele X |
| SEQ ID NO:18 | ra74846s01 Allele X |
| SEQ ID NO:19 | ra74847s01 Allele X |
| SEQ ID NO:20 | ra74848s01 Allele X |
| SEQ ID NO:21 | ra74873s01 Allele X |
| SEQ ID NO:22 | ra74851s01 Allele X |
| SEQ ID NO:23 | ra74852s01 Allele X |
| SEQ ID NO:24 | ra74854s01 Allele X |
| SEQ ID NO:25 | ra74855s01 Allele X |
| SEQ ID NO:26 | ra74856s01 Allele X |
| SEQ ID NO:27 | ra74858s01 Allele X |
| SEQ ID NO:28 | ra74859s01 Allele X |
| SEQ ID NO:29 | ra74860s01 Allele X |
| SEQ ID NO:30 | ra74862s01 Allele X |
| SEQ ID NO:31 | ra74863s01 Allele X |
| SEQ ID NO:32 | ra74864s01 Allele X |
| SEQ ID NO:33 | ra74865s01 Allele X |
| SEQ ID NO:34 | ra74867s01 Allele X |
| SEQ ID NO:35 | ra74869s01 Allele X |

(continued)

| SEQ ID NO | Description |
|---|---|
| SEQ ID NO:36 | ra74870s01 Allele X |
| SEQ ID NO:37 | ra74871s01 Allele X |
| SEQ ID NO:38 | ra74915s01 Allele X |
| SEQ ID NO:39 | ra74916s01 Allele X |
| SEQ ID NO:40 | ra74917s01 Allele X |
| SEQ ID NO:41 | ra74919s01 Allele X |
| SEQ ID NO:42 | ra74921s01 Allele X |
| SEQ ID NO:43 | ra74922s01 Allele X |
| SEQ ID NO:44 | ra74938s01 Allele X |
| SEQ ID NO:45 | ra74941s01 Allele X |
| SEQ ID NO:46 | ra74949s01 Allele X |
| SEQ ID NO:47 | ra74932s02 Allele X |
| SEQ ID NO:48 | ra74955s01 Allele X |
| SEQ ID NO:49 | ra74945s01 Allele X |
| SEQ ID NO:50 | ra74946s01 Allele X |
| SEQ ID NO:51 | ra74948s01 Allele X |
| SEQ ID NO:52 | ra74952s01 Allele X |
| SEQ ID NO:56 | ra01867s01 Allele X |
| SEQ ID NO:57 | ra01868s01 Allele X |
| SEQ ID NO:58 | ra13870s01 Allele X |
| SEQ ID NO:59 | ra17237s01 Allele X |
| SEQ ID NO:60 | ra36486s01 Allele X |
| SEQ ID NO:61 | ra36162s01 Allele X |
| SEQ ID NO:62 | ra36410s01 Allele X |
| SEQ ID NO:63 | ra36412s01 Allele X |
| SEQ ID NO:64 | ra36483s01 Allele X |
| SEQ ID NO:65 | ra74872s01 Allele X |
| SEQ ID NO:66 | ra74910s01 Allele X |
| SEQ ID NO:67 | ra74958s01 Allele X |
| SEQ ID NO:68 | ra74958s02 Allele X |
| SEQ ID NO:69 | ra74958s03 Allele X |
| SEQ ID NO:70 | ra74958s04 Allele X |
| SEQ ID NO:71 | ra74958s05 Allele X |
| SEQ ID NO:72 | ra74958s06 Allele X |
| SEQ ID NO:73 | ra74958s07 Allele X |
| SEQ ID NO:74 | ra74958s08 Allele X |
| SEQ ID NO:75 | ra36387s01 Allele Y |
| SEQ ID NO:76 | ra36444s01 Allele Y |

(continued)

| SEQ ID NO | Description |
|---|---|
| SEQ ID NO:77 | ra36450s01 Allele Y |
| SEQ ID NO:78 | ra36451s01 Allele Y |
| SEQ ID NO:79 | ra36458s01 Allele Y |
| SEQ ID NO:80 | ra36459s01 Allele Y |
| SEQ ID NO:81 | ra36460s01 Allele Y |
| SEQ ID NO:82 | ra36461 s01 Allele Y |
| SEQ ID NO:83 | ra36463s01 Allele Y |
| SEQ ID NO:84 | ra36428s01 Allele Y |
| SEQ ID NO:85 | ra74835s01 Allele Y |
| SEQ ID NO:86 | ra74836s01 Allele Y |
| SEQ ID NO:87 | ra74837s01 Allele Y |
| SEQ ID NO:88 | ra74838s01 Allele Y |
| SEQ ID NO:89 | ra74840s01 Allele Y |
| SEQ ID NO:90 | ra74841s01 Allele Y |
| SEQ ID NO:91 | ra74844s01 Allele Y |
| SEQ ID NO:92 | ra74846s01 Allele Y |
| SEQ ID NO:93 | ra74847s01 Allele Y |
| SEQ ID NO:94 | ra74848s01 Allele Y |
| SEQ ID NO:95 | ra74873s01 Allele Y |
| SEQ ID NO:96 | ra74851s01 Allele Y |
| SEQ ID NO:97 | ra74852s01 Allele Y |
| SEQ ID NO:98 | ra74854s01 Allele Y |
| SEQ ID NO:99 | ra74855s01 Allele Y |
| SEQ ID NO:100 | ra74856s01 Allele Y |
| SEQ ID NO:101 | ra74858s01 Allele Y |
| SEQ ID NO:102 | ra74859s01 Allele Y |
| SEQ ID NO:103 | ra74860s01 Allele Y |
| SEQ ID NO:104 | ra74862s01 Allele Y |
| SEQ ID NO:105 | ra74863s01 Allele Y |
| SEQ ID NO:106 | ra74864s01 Allele Y |
| SEQ ID NO:107 | ra74865s01 Allele Y |
| SEQ ID NO:108 | ra74867s01 Allele Y |
| SEQ ID NO:109 | ra74869s01 Allele Y |
| SEQ ID NO:110 | ra74870s01 Allele Y |
| SEQ ID NO:111 | ra74871s01 Allele Y |
| SEQ ID NO:112 | ra74915s01 Allele Y |
| SEQ ID NO:113 | ra74916s01 Allele Y |
| SEQ ID NO:114 | ra74917s01 Allele Y |

(continued)

| SEQ ID NO | Description |
|---|---|
| SEQ ID NO:115 | ra74919s01 Allele Y |
| SEQ ID NO:116 | ra74921s01 Allele Y |
| SEQ ID NO:117 | ra74922s01 Allele Y |
| SEQ ID NO:118 | ra74938s01 Allele Y |
| SEQ ID NO:119 | ra74941s01 Allele Y |
| SEQ ID NO:120 | ra74949s01 Allele Y |
| SEQ ID NO:121 | ra74932s02 Allele Y |
| SEQ ID NO:122 | ra74955s01 Allele Y |
| SEQ ID NO:123 | ra74945s01 Allele Y |
| SEQ ID NO:124 | ra74946s01 Allele Y |
| SEQ ID NO:125 | ra74948s01 Allele Y |
| SEQ ID NO:126 | ra74952s01 Allele Y |
| SEQ ID NO:127 | ra01867s01 Allele Y |
| SEQ ID NO:128 | ra01868s01 Allele Y |
| SEQ ID NO:129 | ra13870s01 Allele Y |
| SEQ ID NO:130 | ra17237s01 Allele Y |
| SEQ ID NO:131 | ra36486s01 Allele Y |
| SEQ ID NO:132 | ra36162s01 Allele Y |
| SEQ ID NO:133 | ra36410s01 Allele Y |
| SEQ ID NO:134 | ra36412s01 Allele Y |
| SEQ ID NO:135 | ra36483s01 Allele Y |
| SEQ ID NO:136 | ra74872s01 Allele Y |
| SEQ ID NO:137 | ra74910s01 Allele Y |
| SEQ ID NO:138 | ra74958s01 Allele Y |
| SEQ ID NO:139 | ra74958s02 Allele Y |
| SEQ ID NO:140 | ra74958s03 Allele Y |
| SEQ ID NO:141 | ra74958s04 Allele Y |
| SEQ ID NO:142 | ra74958s05 Allele Y |
| SEQ ID NO:143 | ra74958s06 Allele Y |
| SEQ ID NO:144 | ra74958s07 Allele Y |
| SEQ ID NO:145 | ra74958s08 Allele Y |
| SEQ ID NO:146 | ra36387s01 common primer |
| SEQ ID NO:147 | ra36444s01 common primer |
| SEQ ID NO:148 | ra36450s01 common primer |
| SEQ ID NO:149 | ra36451s01 common primer |
| SEQ ID NO:150 | ra36458s01 common primer |
| SEQ ID NO:151 | ra36459s01 common primer |
| SEQ ID NO:152 | ra36460s01 common primer |

(continued)

| SEQ ID NO | Description |
|---|---|
| SEQ ID NO:153 | ra36461s01 common primer |
| SEQ ID NO:154 | ra36463s01 common primer |
| SEQ ID NO:155 | ra36428s01 common primer |
| SEQ ID NO:156 | ra74835s01 common primer |
| SEQ ID NO:157 | ra74836s01 common primer |
| SEQ ID NO:158 | ra74837s01 common primer |
| SEQ ID NO:159 | ra74838s01 common primer |
| SEQ ID NO:160 | ra74840s01 common primer |
| SEQ ID NO:161 | ra74841s01 common primer |
| SEQ ID NO:162 | ra74844s01 common primer |
| SEQ ID NO:163 | ra74846s01 common primer |
| SEQ ID NO:164 | ra74847s01 common primer |
| SEQ ID NO:165 | ra74848s01 common primer |
| SEQ ID NO:166 | ra74873s01 common primer |
| SEQ ID NO:167 | ra74851s01 common primer |
| SEQ ID NO:168 | ra74852s01 common primer |
| SEQ ID NO:169 | ra74854s01 common primer |
| SEQ ID NO:170 | ra74855s01 common primer |
| SEQ ID NO:171 | ra74856s01 common primer |
| SEQ ID NO:172 | ra74858s01 common primer |
| SEQ ID NO:173 | ra74859s01 common primer |
| SEQ ID NO:174 | ra74860s01 common primer |
| SEQ ID NO:175 | ra74862s01 common primer |
| SEQ ID NO:176 | ra74863s01 common primer |
| SEQ ID NO:177 | ra74864s01 common primer |
| SEQ ID NO:178 | ra74865s01 common primer |
| SEQ ID NO:179 | ra74867s01 common primer |
| SEQ ID NO:180 | ra74869s01 common primer |
| SEQ ID NO:181 | ra74870s01 common primer |
| SEQ ID NO:182 | ra74871s01 common primer |
| SEQ ID NO:183 | ra74915s01 common primer |
| SEQ ID NO:184 | ra74916s01 common primer |
| SEQ ID NO:185 | ra74917s01 common primer |
| SEQ ID NO:186 | ra74919s01 common primer |
| SEQ ID NO:187 | ra74921s01 common primer |
| SEQ ID NO:188 | ra74922s01 common primer |
| SEQ ID NO:189 | ra74938s01 common primer |
| SEQ ID NO:190 | ra74941s01 common primer |

(continued)

| SEQ ID NO | Description |
|---|---|
| SEQ ID NO:191 | ra74949s01 common primer |
| SEQ ID NO:192 | ra74932s02 common primer |
| SEQ ID NO:193 | ra74955s01 common primer |
| SEQ ID NO:194 | ra74945s01 common primer |
| SEQ ID NO:195 | ra74946s01 common primer |
| SEQ ID NO:196 | ra74948s01 common primer |
| SEQ ID NO:197 | ra74952s01 common primer |
| SEQ ID NO:198 | ra01867s01 common primer |
| SEQ ID NO:199 | ra01868s01 common primer |
| SEQ ID NO:200 | ra13870s01 common primer |
| SEQ ID NO:201 | ra17237s01 common primer |
| SEQ ID NO:202 | ra36486s01 common primer |
| SEQ ID NO:203 | ra36162s01 common primer |
| SEQ ID NO:204 | ra36410s01 common primer |
| SEQ ID NO:205 | ra36412s01 common primer |
| SEQ ID NO:206 | ra36483s01 common primer |
| SEQ ID NO:207 | ra74872s01 common primer |
| SEQ ID NO:208 | ra74910s01 common primer |
| SEQ ID NO:209 | ra74958s01 common primer |
| SEQ ID NO:210 | ra74958s02 common primer |
| SEQ ID NO:211 | ra74958s03 common primer |
| SEQ ID NO:212 | ra74958s04 common primer |
| SEQ ID NO:213 | ra74958s05 common primer |
| SEQ ID NO:214 | ra74958s06 common primer |
| SEQ ID NO:215 | ra74958s07 common primer |
| SEQ ID NO:216 | ra74958s08 common primer |
| SEQ ID NO:217 | ra74958s05 common primer |
| SEQ ID NO:218 | ra74958s06 common primer |
| SEQ ID NO:219 | ra74958s07 common primer |
| SEQ ID NO:220 | ra74958s08 common primer |
| SEQ ID NO:221 | ra74958s08 common primer |

In an aspect, the invention relates to a (isolated) polynucleic acid having a sequence as set forth in any of SEQ ID NOs: 222 to 274 or to 293, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 222 to 274 or to 293, the complement thereof, or the reverse complement thereof. The nucleotide at position 51 is preferably identical to the corresponding nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 222 to 274 or to 293, the complement thereof, or the reverse complement thereof. The nucleotide at position 51 of the respective SEQ ID NO is preferably included.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 222 to 274 or to 293, the complement thereof, or the reverse complement thereof. The nucleotide at position 51 of the respective SEQ ID NO is preferably included and preferably identical to the corresponding nucleotide of the respective SEQ ID NO (or the complement thereof).

In an aspect, the invention relates to a (isolated) polynucleic acid having a sequence as set forth in any of SEQ ID NOs: 247, 263, 270, 271, 272, 248, 249, 264, 250, 231, 251, 252, 253, 274, 254, 273, 255, or 223, the complement thereof, or the reverse complement thereof.

In an aspect, the invention relates to a (isolated) polynucleic acid having a sequence which is at least 90%, preferably at least 95%, more preferably at least 98% identical to a sequence as set forth in any of SEQ ID NOs: 247, 263, 270, 271, 272, 248, 249, 264, 250, 231, 251, 252, 253, 274, 254, 273, 255, or 223, the complement thereof, or the reverse complement thereof. The nucleotide at position 51 is preferably identical to the corresponding nucleotide of the respective SEQ ID NO (or the complement thereof).

In an aspect, the invention relates to an (isolated) polynucleic acid comprising at least 15, preferably at least 18, more preferably at least 20 contiguous nucleotides of a sequence as set forth in any of SEQ ID NOs: 247, 263, 270, 271, 272, 248, 249, 264, 250, 231, 251, 252, 253, 274, 254, 273, 255, or 223, the complement thereof, or the reverse complement thereof. The nucleotide at position 51 of the respective SEQ ID NO is preferably included.

In an aspect, the invention relates to a (isolated) polynucleic acid comprising at least 15, preferably at least 18, more preferably at least 20 contiguous nucleotides of a sequence which is at least 90%, preferably at least 95%, more preferably at least 98% identical to a sequence as set forth in any of SEQ ID NOs: 247, 263, 270, 271, 272, 248, 249, 264, 250, 231, 251, 252, 253, 274, 254, 273, 255, or 223, the complement thereof, or the reverse complement thereof. The nucleotide at position 51 of the respective SEQ ID NO is preferably included and preferably identical to the corresponding nucleotide of the respective SEQ ID NO (or the complement thereof).

In certain embodiments, the polynucleic acids having a sequence of any of SEQ ID NOs: 222 to 274 or to 293 (or the variants having at least 90%, 95%, or 98% sequence identity, or the fragments thereof) comprise the respective clubroot resistance SNP allele, as described herein elsewhere.

In certain embodiments, the polynucleic acids having a sequence of any of SEQ ID NOs: 222 to 274 or to 293 (or the variants having at least 90%, 95%, or 98% sequence identity, or the fragments thereof) lack the respective clubroot resistance SNP allele, as described herein elsewhere.

In certain embodiments, the polynucleic acids having a sequence of any of SEQ ID NOs: 222 to 274 or to 293 (or the variants having at least 90%, 95%, or 98% sequence identity, or the fragments thereof) comprise the respective clubroot susceptibility SNP allele, as described herein elsewhere.

In an aspect, the invention relates to the complement or reverse complement of the polynucleotide of the invention as described herein.

In an aspect, the invention relates to a polynucleic acid specifically hybridizing with the polynucleotide of the invention as described herein.

In an aspect, the invention relates to a polynucleic acid specifically hybridizing with the complement or reverse complement of the polynucleotide of the invention as described herein.

In certain embodiments, the polynucleic acid of the invention as described herein is a primer or a probe. In certain embodiments, the polynucleic acid of the invention as described herein is a primer. In certain embodiments, the polynucleic acid of the invention as described herein is a probe. In certain embodiments, the polynucleic acid of the invention as described herein is an allele-specific primer or a probe. In certain embodiments, the polynucleic acid of the invention as described herein is an allele-specific primer. In certain embodiments, the polynucleic acid of the invention as described herein is an allele-specific probe. In certain embodiments, the polynucleic acid of the invention as described herein is a KASP primer.

In an aspect, the invention relates to a primer or probe capable of specifically detecting the polynucleic acid (allele), QTL (allele), locus (allele), molecular marker (allele), or SNP (allele) according to the invention as described herein.

In an aspect, the invention relates to a primer or probe capable of specifically detecting the polynucleic acid allele, QTL allele, locus allele, molecular marker allele, or SNP allele according to the invention as described herein.

In an aspect, the invention relates to a primer or probe capable of specifically detecting the clubroot resistance polynucleic acid allele, QTL allele, locus allele, molecular marker allele, or SNP allele according to the invention as described herein.

In an aspect, the invention relates to a primer or probe capable of specifically detecting the clubroot susceptibility polynucleic acid allele, QTL allele, locus allele, molecular marker allele, or SNP allele according to the invention as described herein.

In an aspect, the invention relates to an allele-specific primer or probe capable of specifically detecting the polynucleic acid (allele), QTL (allele), locus (allele), molecular marker (allele), or SNP (allele) according to the invention as described herein.

In an aspect, the invention relates to an allele-specific primer or probe capable of specifically detecting the polynucleic acid allele, QTL allele, locus allele, molecular marker allele, or SNP allele according to the invention as described herein.

In an aspect, the invention relates to an allele-specific primer or probe capable of specifically detecting the clubroot resistance polynucleic acid allele, QTL allele, locus allele, molecular marker allele, or SNP allele according to the invention as described herein.

In an aspect, the invention relates to an allele-specific primer or probe capable of specifically detecting the clubroot susceptibility polynucleic acid allele, QTL allele, locus allele, molecular marker allele, or SNP allele according to the invention as described herein.

In an aspect, the invention relates to a (allele-specific) primer capable of specifically detecting the polynucleic acid (allele), QTL (allele), locus (allele), molecular marker (allele), or SNP (allele) according to the invention as described herein.

In an aspect, the invention relates to a (allele-specific) primer capable of specifically detecting the polynucleic acid allele, QTL allele, locus allele, molecular marker allele, or SNP allele according to the invention as described herein.

In an aspect, the invention relates to a (allele-specific) primer capable of specifically detecting the clubroot resistance polynucleic acid allele, QTL allele, locus allele, molecular marker allele, or SNP allele according to the invention as described herein.

In an aspect, the invention relates to a (allele-specific) primer capable of specifically detecting the clubroot susceptibility polynucleic acid allele, QTL allele, locus allele, molecular marker allele, or SNP allele according to the invention as described herein.

In certain embodiments, the primer set comprises at least a first primer and a second primer, both of which preferably are allele-specific primers.

In certain embodiments, the first and second primer respectively comprise at least 15, preferably at least 18, more preferably at least contiguous 20 nucleotides of SEQ ID NO: 26 and 100, preferably the most 3' nucleotides. In certain embodiments, the first and second primer respectively comprise at least 15, preferably at least 18, more preferably at least contiguous 20 nucleotides of SEQ ID NO: 42 and 116, preferably the most 3' nucleotides. In certain embodiments, the first and second primer respectively comprise at least 15, preferably at least 18, more preferably at least contiguous 20 nucleotides of SEQ ID NO: 49 and 123, preferably the most 3' nucleotides. In certain embodiments, the first and second primer respectively comprise at least 15, preferably at least 18, more preferably at least contiguous 20 nucleotides of SEQ ID NO: 50 and 124, preferably the most 3' nucleotides. In certain embodiments, the first and second primer respectively comprise at least 15, preferably at least 18, more preferably at least contiguous 20 nucleotides of SEQ ID NO: 51 and 125, preferably the most 3' nucleotides. In certain embodiments, the first and second primer respectively comprise at least 15, preferably at least 18, more preferably at least contiguous 20 nucleotides of SEQ ID NO: 27 and 101, preferably the most 3' nucleotides. In certain embodiments, the first and second primer respectively comprise at least 15, preferably at least 18, more preferably at least contiguous 20 nucleotides of SEQ ID NO: 28 and 102, preferably the most 3' nucleotides. In certain embodiments, the first and second primer respectively comprise at least 15, preferably at least 18, more preferably at least contiguous 20 nucleotides of SEQ ID NO: 43 and 117, preferably the most 3' nucleotides. In certain embodiments, the first and second primer respectively comprise at least 15, preferably at least 18, more preferably at least contiguous 20 nucleotides of SEQ ID NO: 29 and 103, preferably the most 3' nucleotides. In certain embodiments, the first and second primer respectively comprise at least 15, preferably at least 18, more preferably at least contiguous 20 nucleotides of SEQ ID NO: 10 and 84, preferably the most 3' nucleotides. In certain embodiments, the first and second primer respectively comprise at least 15, preferably at least 18, more preferably at least contiguous 20 nucleotides of SEQ ID NO: 30 and 104, preferably the most 3' nucleotides. In certain embodiments, the first and second primer respectively comprise at least 15, preferably at least 18, more preferably at least contiguous 20 nucleotides of SEQ ID NO: 31 and 105, preferably the most 3' nucleotides. In certain embodiments, the first and second primer respectively comprise at least 15, preferably at least 18, more preferably at least contiguous 20 nucleotides of SEQ ID NO: 32 and 106, preferably the most 3' nucleotides. In certain embodiments, the first and second primer respectively comprise at least 15, preferably at least 18, more preferably at least contiguous 20 nucleotides of SEQ ID NO: 66 and 137, preferably the most 3' nucleotides. In certain embodiments, the first and second primer respectively comprise at least 15, preferably at least 18, more preferably at least contiguous 20 nucleotides of SEQ ID NO: 33 and 107, preferably the most 3' nucleotides. In certain embodiments, the first and second primer respectively comprise at least 15, preferably at least 18, more preferably at least contiguous 20 nucleotides of SEQ ID NO: 52 and 126, preferably the most 3' nucleotides. In certain embodiments, the first and second primer respectively comprise at least 15, preferably at least 18, more preferably at least contiguous 20 nucleotides of SEQ ID NO: 34 and 108, preferably the most 3' nucleotides. In certain embodiments, the first and second primer respectively comprise at least 15, preferably at least 18, more preferably at least contiguous 20 nucleotides of SEQ ID NO: 2 and 76, preferably the most 3' nucleotides.

In certain embodiments, the primer set comprises a third primer (in reverse orientation). In certain embodiments, the third primer is configured to allow amplification of a polynucleic acid in combination with said first or second primer.

In certain embodiments, the first, second, and third primer respectively comprise at least 15, preferably at least 18, more preferably at least contiguous 20 nucleotides of SEQ ID NOs: 26, 100, 171, preferably the most 3' nucleotides. In certain embodiments, the first, second, and third primer respectively comprise at least 15, preferably at least 18, more preferably at least contiguous 20 nucleotides of SEQ ID NOs: 42, 116, 187, preferably the most 3' nucleotides. In certain embodiments, the first, second, and third primer respectively comprise at least 15, preferably at least 18, more preferably at least contiguous 20 nucleotides of SEQ ID NOs: 49, 123, 194, preferably the most 3' nucleotides. In certain embodiments, the first, second, and third primer respectively comprise at least 15, preferably at least 18, more preferably at least contiguous 20 nucleotides of SEQ ID NOs: 50, 124, 195, preferably the most 3' nucleotides. In certain embodiments, the first, second, and third primer respectively comprise at least 15, preferably at least 18, more preferably at least contiguous 20 nucleotides of SEQ ID NOs: 51, 125, 196, preferably the most 3' nucleotides. In certain embodiments, the first, second, and third primer respectively comprise at least 15, preferably at least 18, more preferably at least contiguous 20 nucleotides of SEQ ID NOs: 27, 101, 172, preferably the most 3' nucleotides. In certain embodiments, the first, second, and third primer respectively comprise at least 15, preferably at least 18, more preferably at least contiguous 20 nucleotides of SEQ ID NOs: 28, 102, 173, preferably the most 3' nucleotides. In certain embodiments, the first, second, and third primer respectively comprise at least 15, preferably at least 18, more preferably at least contiguous 20 nucleotides of SEQ ID NOs: 43, 117, 188, preferably the most 3' nucleotides. In certain embodiments, the first, second, and third primer respectively comprise at least 15, preferably at least 18, more preferably at least contiguous 20 nucleotides of SEQ ID NOs: 29, 103, 174, preferably the most 3' nucleotides. In certain embodiments, the first, second, and third primer respectively comprise at least 15, preferably at least 18, more preferably at least contiguous 20 nucleotides of SEQ ID NOs: 10, 84, 155, preferably the most 3' nucleotides. In certain embodiments, the first, second, and third primer respectively comprise at least 15, preferably at least 18, more preferably at least contiguous 20 nucleotides of SEQ ID NOs: 30, 104, 175, preferably the most 3' nucleotides. In certain embodiments, the first, second, and third primer respectively comprise at least 15, preferably at least 18, more preferably at least contiguous 20 nucleotides of SEQ ID NOs: 31, 105, 176, preferably the most 3' nucleotides. In certain embodiments, the first, second, and third primer respectively comprise at least 15, preferably at least 18, more preferably at least contiguous 20 nucleotides of SEQ ID NOs: 32, 106, 177, preferably the most 3' nucleotides. In certain embodiments, the first, second, and third primer respectively comprise at least 15, preferably at least 18, more preferably at least contiguous 20 nucleotides of SEQ ID NOs: 66, 137, 208, preferably the most 3' nucleotides. In certain embodiments, the first, second, and third primer respectively comprise at least 15, preferably at least 18, more preferably at least contiguous 20 nucleotides of SEQ ID NOs: 33, 107, 178, preferably the most 3' nucleotides. In certain embodiments, the first, second, and third primer respectively comprise at least 15, preferably at least 18, more preferably at least contiguous 20 nucleotides of SEQ ID NOs: 52, 126, 197, preferably the most 3' nucleotides. In certain embodiments, the first, second, and third primer respectively comprise at least 15, preferably at least 18, more preferably at least contiguous 20 nucleotides of SEQ ID NOs: 34, 108, 179, preferably the most 3' nucleotides. In certain embodiments, the first, second, and third primer respectively comprise at least 15, preferably at least 18, more preferably at least contiguous 20 nucleotides of SEQ ID NOs: 2, 76, and 147, preferably the most 3' nucleotides.

In an aspect, the invention relates to the use of a polynucleic acid, QTL, locus, marker, or SNP according to the invention as described herein for identifying and/or selecting a plant or plant part of the Brassicaceae family.

In an aspect, the invention relates to the use of a polynucleic acid allele, QTL allele, locus allele, marker allele, or SNP allele according to the invention as described herein for identifying and/or selecting a plant or plant part of the *Brassicaceae* family.

In an aspect, the invention relates to the use of a polynucleic acid allele, QTL allele, locus allele, marker allele, or SNP allele according to the invention as described herein for identifying and/or selecting a plant or plant part of the *Brassicaceae* family having (increased) clubroot resistance and/or tolerance, such as in comparison to a plant or plant part of the Brassicaceae family not having said polynucleotide, molecular marker (allele), locus (allele), QTL (allele), or SNP (allele) of the invention.

In an aspect, the invention relates to the use of a polynucleic acid, QTL, locus, marker, or SNP according to the invention as described herein for generating, producing, or obtaining a plant or plant part of the *Brassicaceae* family.

In an aspect, the invention relates to the use of a polynucleic acid allele, QTL allele, locus allele, marker allele, or SNP allele according to the invention as described herein for generating, producing, or obtaining a plant or plant part of the *Brassicaceae* family.

In an aspect, the invention relates to the use of a polynucleic acid allele, QTL allele, locus allele, marker allele, or SNP allele according to the invention as described herein for generating, producing, or obtaining a plant or plant part of the *Brassicaceae* family having (increased) clubroot resistance and/or tolerance, such as in comparison to a plant or plant part of the Brassicaceae family not having said polynucleotide, molecular marker (allele), locus (allele), QTL (allele), or SNP (allele) of the invention.

The present invention is not to be limited in scope by the specific embodiments described herein. Indeed, various modifications of the invention in addition to those described herein will become apparent to those skilled in the art from the foregoing description and the accompanying figures. Such modifications are intended to fall under the scope of the appended claims. It is further to be understood that all values may include approximates and are provided for description.

The aspects and embodiments of the invention are further supported by the following non-limiting examples. The following examples, including the experiments conducted and the results achieved, are provided for illustrative purposes only and are not constructed as limiting the present invention. The use of these and other examples anywhere in the specification is illustrative only and in no way limits the scope and meaning of the invention or of any exemplified term. Likewise, the invention is not limited to any particular preferred embodiments described here. Indeed, many modifications and variations of the invention may be apparent to those skilled in the art upon reading this specification, and such variations can be made without departing from the invention in spirit or in scope. The invention is therefore to be limited only by the terms of the appended claims along with the full scope of equivalents to which those claims are entitled.

## EXAMPLES

**EXAMPLE 1**: Scoring of gall formation

To induce gall formation an inoculum solution ("isolate") was prepared. Infected roots were collected in the field and crushed in a mixer (as normally used as food processor). The solution is roughly filtered, and the spore concentration is adjusted.

In *Brassica napus* no resistance was expected. To identify new resistance 70 different genetic resources were screened and new resistance sources in *B. rapa* and *B. oleracea* were most promising.

The turnip Aarselia (B. rapa) was tested with the A and C isolate. The turnip showed strong resistance against both, isolate A (Mendel-based varieties show still resistance) and C (Mendel-based varieties are highly susceptible).

A screening was performed in a glasshouse test. Two different isolates were used in the screening, isolate A (Mendel-based varieties show still resistance) and C (Mendel-based varieties are highly susceptible). For the screening (according to E. Diederichsen and M.D. Sacristan, Plant Breeding 115, 5-10 (1996), Disease response of resynthesized Brassica napus L. lines carrying different combinations of resistance to Plasmodiophora brassicae Wor.), plants were grown in sand for 10 to 14 days. The seedlings were washed and dipped for 15 minutes into an inoculum solution. Afterwards the seedlings were potted and grown under greenhouse conditions for approximately 6 weeks. After washing of the roots, the gall formation was scored from 0, meaning no gall formation to 3 strong gall formation (Figure 1).

A disease index (DI) was calculated (according to E. Diederichsen and M.D. Sacristan, Plant Breeding 115, 5-10 (1996), Disease response of resynthesized Brassica napus L. lines carrying different combinations of resistance to Plasmodiophora brassicae Wor.) by using the following formula:

$$DI = \frac{\sum(0n_0 + 1n_1 + 2n_2 + 3n_3)}{3N} \times 100$$

The values $n_0$ to $n_3$ correspond to the number of plants per class and N being the total number of tested plants. Genotypes with a DI<10 were assessed to be highly resistant. A DI between 10-25 leads to an assessment as resistant and genotypes with a DI>25 are rated as susceptible (see Table A).

| Table A: Scoring (from 0, meaning no gall formation to 3 strong gall formation) of max. 20 plants per DH line in clubroot test with C isolate. The variety Sherlock was used as positive control. | | | | | |
|---|---|---|---|---|---|
| | Score | | | | DI |
| | 0 | 1 | 2 | 3 | |
| D3170008-001 | | | | 20 | 100 |
| D3170008-002 | 19 | | | | 0 |
| D3170008-010 | 20 | | | | 0 |
| D3170008-012 | | | | 7 | 100 |

## EP 4 278 891 A1

(continued)

| Table A: Scoring (from 0, meaning no gall formation to 3 strong gall formation) of max. 20 plants per DH line in clubroot test with C isolate. The variety Sherlock was used as positive control. | | | | | |
|---|---|---|---|---|---|
| | Score | | | | DI |
| | 0 | 1 | 2 | 3 | |
| D3170008-013 | | | | 14 | 100 |
| D3170008-015 | | | | 10 | 100 |
| D3170008-018 | | | | 5 | 100 |
| D3170008-021 | | 1 | 1 | 14 | 94 |
| D3170008-023 | 7 | | | | 0 |
| D3170008-025 | | | | 2 | 100 |
| D3170008-026 | 17 | | | | 0 |
| D3170008-028 | | | | 13 | 100 |
| D3170008-030 | | | | 16 | 100 |
| D3170009-001 | 17 | | | | 0 |
| D3170009-002 | | | | 10 | 100 |
| D3170009-003 | | | | 16 | 100 |
| D3170009-004 | 18 | | | | 0 |
| D3170009-005 | 13 | | | | 0 |
| D3170009-006 | 18 | | | | 0 |
| D3170009-007 | | | | 15 | 100 |
| D3170009-008 | | | | 18 | 100 |
| D3170009-009 | | | | 17 | 100 |
| D3170009-010 | 14 | | | | 0 |
| D3170009-011 | 20 | | | | 0 |
| D3170009-012 | | | | 18 | 100 |
| D3170009-013 | | | | 19 | 100 |
| D3170009-014 | 15 | | | | 0 |
| D3170009-015 | | | | 14 | 100 |
| D3170009-016 | 18 | | | | 0 |
| D3170009-018 | | | | 18 | 100 |
| D3170009-020 | | | | 12 | 100 |
| D3170009-021 | | | | 10 | 100 |
| D3170009-025 | | | | 14 | 100 |
| D3170009-026 | | | | 19 | 100 |
| D3170009-027 | | | | 18 | 100 |
| D3170009-029 | | | | 15 | 100 |
| D3170009-030 | | | | 17 | 100 |
| D3170009-031 | 19 | | | | 0 |
| D3170009-032 | 19 | | | | 0 |

(continued)

Table A: Scoring (from 0, meaning no gall formation to 3 strong gall formation) of max. 20 plants per DH line in clubroot test with C isolate. The variety Sherlock was used as positive control.

| | Score | | | | DI |
|---|---|---|---|---|---|
| | **0** | **1** | **2** | **3** | |
| D3170009-033 | | | | 11 | 100 |
| D3170009-034 | 20 | | | | 0 |
| D3170009-035 | 17 | | | 1 | 6 |
| D3170009-037 | 2 | | | 14 | 88 |
| D3170009-038 | 17 | | | | 0 |
| D3170009-039 | 1 | 1 | | 16 | 91 |
| D3170010-001 | 18 | | | | 0 |
| D3170010-002 | 17 | | 1 | | 4 |
| D3170010-004 | 20 | | | | 0 |
| D3170010-006 | | | | 14 | 100 |
| D3170010-008 | 6 | | 1 | 3 | 37 |
| D3170010-009 | | 2 | 2 | 11 | 87 |
| D3170010-010 | 13 | | | | 0 |
| D3170010-014 | | | | 7 | 100 |
| D3170010-016 | | | | 14 | 100 |
| D3170010-019 | 16 | | | | 0 |
| D3170010-026 | | | | 19 | 100 |
| D3170010-027 | 14 | | | | 0 |
| D3170012-001 | 19 | | | | 0 |
| D3170012-002 | | | | 20 | 100 |
| D3170012-004 | | | | 13 | 100 |
| D3170012-005 | | 3 | | 11 | 86 |
| D3170012-006 | 19 | | | | 0 |
| D3170012-007 | 2 | | | | 0 |
| D3170012-008 | | | | 3 | 100 |
| D3170012-009 | | | | 13 | 100 |
| D3170012-011 | 19 | | | | 0 |
| D3170012-012 | | | | 19 | 100 |
| D3170012-014 | 20 | | | | 0 |
| D3170012-015 | 2 | | 1 | 12 | 84 |
| D3170012-019 | | | 1 | 11 | 97 |
| D3170012-020 | | | | 20 | 100 |
| D3170012-021 | 18 | | | | 0 |
| D3170012-022 | | | 1 | 19 | 98 |
| D3170012-023 | 20 | | | | 0 |

(continued)

| Table A: Scoring (from 0, meaning no gall formation to 3 strong gall formation) of max. 20 plants per DH line in clubroot test with C isolate. The variety Sherlock was used as positive control. | | | | | |
|---|---|---|---|---|---|
| | **Score** | | | | **DI** |
| | **0** | **1** | **2** | **3** | |
| D3170012-025 | | | | 17 | 100 |
| D3170012-027 | 20 | | | | 0 |
| D3170012-028 | | | | 17 | 100 |
| D3170014-001 | 5 | | | | 0 |
| D3170014-005 | | | | 16 | 100 |
| D3170014-013 | | | | 15 | 100 |
| D3170014-017 | 17 | | | | 0 |
| D3170014-019 | | | | 17 | 100 |
| D3170014-023 | | | | 14 | 100 |
| D3170015-001 | | | | 7 | 100 |
| D3170015-002 | 20 | | | | 0 |
| D3170015-003 | | | | 16 | 100 |
| D3170015-004 | | | | 17 | 100 |
| D3170015-005 | 18 | | | | 0 |
| D3170015-006 | | 1 | | 14 | 96 |
| D3170015-008 | 19 | | | | 0 |
| D3170015-010 | 20 | | | | 0 |
| D3170015-011 | | | | 18 | 100 |
| D3170015-012 | | | | 19 | 100 |
| D3170015-014 | | | | 16 | 100 |
| D3170015-015 | | | | 19 | 100 |
| D3170015-018 | | | | 17 | 100 |
| D3170015-019 | | | | 16 | 100 |
| D3170015-020 | | | | 16 | 100 |
| D3170015-022 | 17 | | | | 0 |
| D3170015-023 | 20 | | | | 0 |
| D3170015-024 | 19 | | | | 0 |
| D3170015-027 | | | | 18 | 100 |
| D3170015-028 | | | | 19 | 100 |
| D3170019-001 | 17 | | | | 0 |
| D3170019-003 | 18 | | | | 0 |
| D3170019-004 | | | 1 | 14 | 98 |
| D3170019-006 | | | | 19 | 100 |
| D3170019-007 | 2 | | 1 | 9 | 81 |
| D3170019-009 | | | | 14 | 100 |

(continued)

Table A: Scoring (from 0, meaning no gall formation to 3 strong gall formation) of max. 20 plants per DH line in clubroot test with C isolate. The variety Sherlock was used as positive control.

| | Score | | | | DI |
|---|---|---|---|---|---|
| | 0 | 1 | 2 | 3 | |
| D3170019-010 | 17 | | | | 0 |
| D3170019-011 | | | | 16 | 100 |
| D3170019-012 | 17 | | | | 0 |
| D3170019-014 | 13 | | 1 | 4 | 26 |
| D3170019-015 | | | | 16 | 100 |
| D3170019-017 | 20 | | | | 0 |
| D3170019-018 | | 1 | | 11 | 94 |
| D3170019-019 | | | | 14 | 100 |
| D3170019-020 | | | | 18 | 100 |
| D3170019-021 | | | | 18 | 100 |
| D3170019-024 | | | | 18 | 100 |
| D3170019-026 | | | | 19 | 100 |
| D3170019-027 | 20 | | | | 0 |
| D3170019-029 | 18 | | | | 0 |
| D3170019-030 | | | | 17 | 100 |
| Sherlock (susceptible standard) | | | | 13 | 100 |
| Mentor (susceptibile against Isolate C) | | | | 10 | 100 |

As can be derived from Table A DH lines carrying the resistance show a DI of 0 tested with isolate C. Resistant plants from the C isolate test were used for the initiation of a back-crossing (BC) program into restorer breeding material.

The Aarselia resistance locus was crossed until BC4 into a KWS elite breeding line. In each generation the phenotype was checked for selection of resistant heterozygote plants taken for the next back-crossing step. BC4 plants were placed into the greenhouse clubroot test with isolate C for selection of resistant lines (score 0). These lines were used as donor plants for microspore sampling, which is the basis for double haploid production. The resulting DH lines were again screened in the glasshouse because selected BC4 lines are heterozygote for the clubroot trait, so that a 1:1 segregation is expected (Table A). The identified resistant DH lines were the basis for hybrid variety development with an improved resistance.

The resistance level of the DH lines was then compared to the standard varieties 'Mentor' (carrying the Mendel resistance) and Sherlock (no known resistance) tested with 13 field isolates (Table B).

Table B: Resistance reaction of an the DH line (D3170012-001), compared to the standard varieties 'Mentor' (carrying the Mendel resistance) and Sherlock (no known resistance) tested with 13 field isolates. A disease index (DI) <25 implies a clubroot resistance to the respective isolate.

| Genotyp | Origin of tested Clubroot field isolate | DI (0-100, <25 resistant) |
|---|---|---|
| D3170012-001 | Germany, Walkendorf Mecklenburg Vorpommern, 2011, Isolate A | 0 |
| Mentor | Germany, Walkendorf Mecklenburg Vorpommern, 2011, Isolate A | 97 |

(continued)

Table B: Resistance reaction of an the DH line (D3170012-001), compared to the standard varieties 'Mentor' (carrying the Mendel resistance) and Sherlock (no known resistance) tested with 13 field isolates. A disease index (DI) <25 implies a clubroot resistance to the respective isolate.

| Genotyp | Origin of tested Clubroot field isolate | DI (0-100, <25 resistant) |
|---|---|---|
| Sherlock | Germany, Walkendorf Mecklenburg Vorpommern, 2011, Isolate A | 92 |
| D3170012-001 | Germany, Schleswig Holstein, Isolate C | 0 |
| Mentor | Germany, Schleswig Holstein, Isolate C | 88 |
| Sherlock | Germany, Schleswig Holstein, Isolate C | 71 |
| D3170012-001 | Germany, Walkendorf MEK unknown variety, 2015 | 0 |
| Mentor | Germany, Walkendorf MEK unknown variety, 2015 | 54 |
| Sherlock | Germany, Walkendorf MEK unknown variety, 2015 | 80 |
| D3170012-001 | Poland, variety Kadore (Bobrowniki/Korado) | 0 |
| Mentor | Poland, variety Kadore (Bobrowniki/Korado) | 37 |
| Sherlock | Poland, variety Kadore (Bobrowniki/Korado) | 89 |
| D3170012-001 | Poland Mendelson | 0 |
| Mentor | Poland Mendelson | 42 |
| Sherlock | Poland Mendelson | 82 |
| D3170012-001 | Germany, OsterodeNS Isolate B1/B4 | 0 |
| Mentor | Germany, OsterodeNS Isolate B1/B4 | 100 |
| Sherlock | Germany, OsterodeNS Isolate B1/B4 | 89 |
| D3170012-001 | Poland variety Kodiak | 0 |
| Mentor | Poland variety Kodiak | 100 |
| Sherlock | Poland variety Kodiak | 93 |
| D3170012-001 | Poland (Alva ro, Expiro,Atora,Alexa nda r) | 0 |
| Mentor | Poland (Alva ro, Expiro,Atora,Alexa nda r) | 100 |
| Sherlock | Poland (Alva ro, Expiro,Atora,Alexa nda r) | 88 |
| D3170012-001 | France, Isolate Bretagne, Morlaix | 0 |

(continued)

| Table B: Resistance reaction of an the DH line (D3170012-001), compared to the standard varieties 'Mentor' (carrying the Mendel resistance) and Sherlock (no known resistance) tested with 13 field isolates. A disease index (DI) <25 implies a clubroot resistance to the respective isolate. | | |
|---|---|---|
| Genotyp | Origin of tested Clubroot field isolate | DI (0-100, <25 resistant) |
| Mentor | France, Isolate Bretagne, Morlaix | 19 |
| Sherlock | France, Isolate Bretagne, Morlaix | 79 |
| D3170012-001 | France, Isolate Paye de la Loire, Canolé | 0 |
| Mentor | France, Isolate Paye de la Loire, Canolé | 36 |
| Sherlock | France, Isolate Paye de la Loire, Canolé | 89 |
| D3170012-001 | Germany, Isolate Einbeck, Drüber | 0 |
| Mentor | Germany, Isolate Einbeck, Drüber | 59 |
| Sherlock | Germany, Isolate Einbeck, Drüber | 85 |
| D3170012-001 | UK, Isolate Edingburgh | 0 |
| Mentor | UK, Isolate Edingburgh | 32 |
| Sherlock | UK, Isolate Edingburgh | 92 |
| D3170012-001 | Hungary | 0 |
| Mentor | Hungary | 89 |
| Sherlock | Hungary | 87 |

As can be derived from Table A D3170012-001 has a DI of 0 with all tested field isolates. Thus, D3170012-001 has been classified as highly resistant to clubroot caused by Plasmodiophora (brassicae) against these 13 field isolates.

**EXAMPLE 2:** Yield

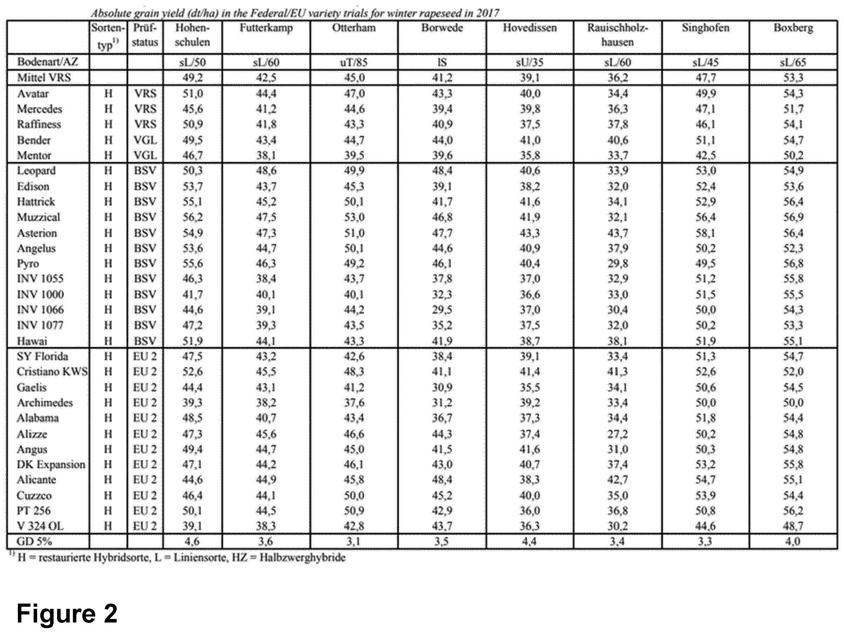

Yield performances of clubroot resistant rape seed varieties in variety trials are regularly at least 10 to 15% lower than yields of non-resistant varieties, which is the reason why resistant varieties are generally only compared to one another. Proof of this can be found in Figure 2 (and https://www.lksh.de/landwirtschaft/ackerkulturen/winterraps/). Especially, the variety "Mentor" decedent from "Mendel" the first clubroot resistant variety shows e.g., a relative grain yield between 33.7% and 50.2%.

In Table C a test hybrid (H6185062) that carries the resistance from D3170012-001 is compared at five different locations in Germany, France and Poland to other non-resistant varieties. The herein described Brassica napus plants carrying the genetic resistance (i.e., the QTL according to the invention) derived from the *B. rapa* (i.e., hybrid (H6185062)) shows no negative agronomic traits. While glucosinolate and oil content remained at a stable level the present clubroot resistant Hybrid variety H6185062 (parent D3170012-001) has a relative yield of 104.5% which is as good and efficient as the yield of non-resistant varieties (Alberto KWS, Bender and Marc KWS, Table C).

**Table C:** Comparison of the new Hybrid H6185062 with other marketed hybrids

| Genotype | Male | Glucosinolates (μmol, measured by NIRS) | Oil content (in % at 91% dry matter, measured by NIRS) | Yield (dt/ha at 91% dry matter relative to trial checks, threshed seeds) |
|---|---|---|---|---|
| H6185062 | D3170012-001 | 14.7 | 44.0 | 102.5 |
| ALBERTO KWS | | 13.5 | 44.3 | 101 |

(continued)

| Genotype | Male | Glucosinolates (μmol, measured by NIRS) | Oil content (in % at 91% dry matter, measured by NIRS) | Yield (dt/ha at 91% dry matter relative to trial checks, threshed seeds) |
|---|---|---|---|---|
| BENDER | | 10.3 | 45.2 | 94.9 |
| MARC KWS | | 11.0 | 43.3 | 97.0 |
| ARCHITEKT | | 13.8 | 44,4 | 107,1 |

**EXAMPLE 3:** QTL Mapping

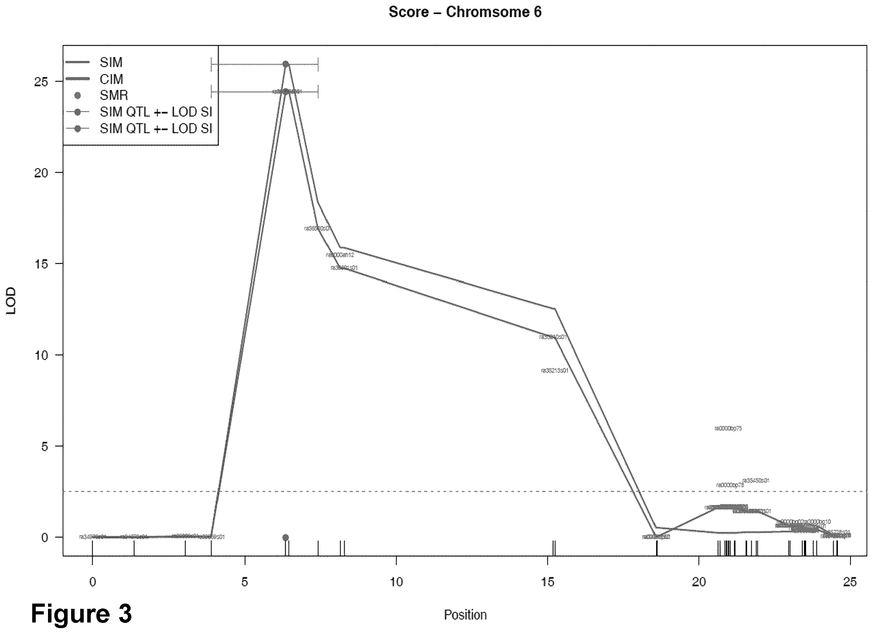

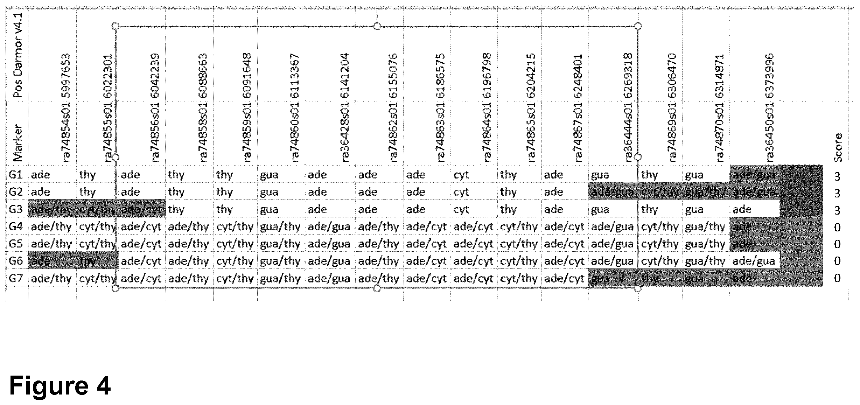

In BC3S1 a first QTL mapping was performed with 360 individual plants using an internal SNP CHIP (17.000 SNPs). A strong QTL with a LOD score of 26 could be detected on chromosome A06 (Figure 3). KASP markers for the target region on A06 were developed based on CHIP SNPs and later re-sequencing data of an Aarselia DH line. The target region could be narrowed down to a region of 220.000 base pairs based on the reference genome called Darmor v4.1 (Chalhoub et al., 2014 Science 345:950-953) (Figure 4). A marker portfolio based on KASP was established comprising:

a) Markers describing the target resistance locus on chromosome A06 comprising 220 kbp on reference genome called Darmor v4.1 needed for clubroot resistance expression.
b) Markers flanking from both sides the target region allowing to introgress the resistance locus from *B. rapa* without transferring any negative agronomic traits into the elite background.
c) Diagnostic haplotype described by markers ra74862s01 (donor allele Y RES, SEQ ID NO: 104) and ra74867s01 (donor allele Y RES, SEQ ID NO: 108).

**Table D**: Sequence information of a selection of markers. SPSs are indicated in the sequences between square brackets [ ]. SNP identity of the donor (Aarselia)/resistance allele and the susceptible allele are labelled as Allele X and Allele Y, whereby the donor (Aarselia)/resistance allele is grey shaded.

| Marker | Allele X | Allele Y | Sequence | Direction | SEQ ID NO |
|---|---|---|---|---|---|
| ra36387s01 | C | T | ctttggaggtytgggcttgtgtgaaggaat cggagacgaatttggagtgg[c/t]agca acagtttgaagctgttgtttccgccaggat gtcatatcttcaacac | F | 222 |
| ra36444s01 | C | T | agaaagtctcattatactttgcttcccttcc ggcgttggaatcactgaaa[c/t]gtttcta aatgtgttacttgaagcattgatttagatatt gaaccttgaac | R | 223 |
| ra36450s01 | A | G | atttgaatctagtttaatatattayaatgact atgaattttgctaaccat[a/g]tgttgaga atctattcttacagggttcctacaactctgc gagtgttttcc | F | 224 |
| ra36451s01 | A | C | cgtgttatcttgtaaaccgtacctagttcag aacgtgcaagacgaatgga[a/c]aga agctctcctcccgcagaaaatttcaccac gtctattagctcstgact | R | 225 |
| ra36458s01 | A | G | agtctggtgagtctcrcgagcattgattga rttcattctgttttcggttt[a/g]catatctttgt | F | 226 |

| | | | | | |
|---|---|---|---|---|---|
| | | | aactctacaaggcttggcgtgtgaacta atgagctttct | | |
| ra36459s01 | A | G | gagatttcgttgctcactgataaaatcgg gtggttacaagataaggtacc[a/g]caa ttgtctaactgacttactcttcaggtttcatat ggaattattattct | F | 227 |
| ra36460s01 | A | G | accatyattctcatacccaattagaacaa agatttccacgccaaaattct[a/g]aaa agaggaagagaagaatcaaggcacct tgtttacgagatttcctcttc | F | 228 |
| ra36461s01 | A | C | aaagcttggttgaagctttctttgcatcgg acactctacatctttaagtt[a/c]caacag caaccatgaaccattttttacctttgtamatt ccagctaattaaa | F | 229 |
| ra36463s01 | C | T | aacagccttttcaaaatattcttttgcctac atttgamgccayattacaa[c/t]ggaac gttagattcataagttcctatattatatccat cgtctaaatatgt | R | 230 |
| ra36428s01 | C | T | tcctctttccatgaaacaacattgttgagct acaatctcttagcaccggc[c/t]ggttctc ggttcaattgagagattagtttgatagaa aaccttccacggtg | R | 231 |
| ra74835s01 | A | G | aaggttcatcatatcattatcaagcattgt ggtccaaatataattcatgt[a/g]tattata tacttggcacccatgcaccaataacgcg tagtaaccacatagt | F | 232 |
| ra74836s01 | A | T | acttctcaaagtttgtaccttttaaaaaaa aaacctttttgttgttctgt[a/t]ttttaatcag acatgtttaaattaacttttaaatgttacag ggaaggtac | R | 233 |
| ra74837s01 | A | C | gaaaaagactagaacatccaacaaat gagagcattaaaagatctgaattt[a/c]t caattaattacagtgatattcgataacgtt gaaaaaagtgaatgatttt | R | 234 |

| | | | | | |
|---|---|---|---|---|---|
| ra74838s01 | C | T | ttattaaagtaagagagagtacgacacc aaactgattgatcaaaattgtt[c/t]gaga tttcacacaacgtgcgatctgaaaaaga cagaaagaggcagagatg | F | 235 |
| ra74840s01 | A | C | gtttgagctttgtacacgtatgagaagcc aaataacggtaaaacgtttga[a/c]actt tgttaaaaaaaaggtccagactttattag tgaaaagtctgcgtat | R | 236 |
| ra74841s01 | C | T | tgctaggtgtgggatgttttgtcaaacaaa gaagctgttgatattgtggc[c/t]tcagct cctagtagaaacactgcggctcgtgcttt agtatgcacagctgt | R | 237 |
| ra74844s01 | C | G | tgtgatgtaagaaactgttaaaaataag atggaaatattgttgtgtaaaa[c/g]taa agaatattttgctattcatccacaaagattt caaatgagtgtctata | F | 238 |
| ra74846s01 | C | T | ctaaccaagtcttctagccacaagtcac atgaccacaacttacctgacca[c/t]aa ctcacctgccaagtctgttcacagctctcc tgaccaagtcattgctga | R | 239 |
| ra74847s01 | C | T | gcaaaagacatgtcacgactcatatcat cataggtactatctcagctatg[c/t]aact gcaacgaaataaaatcccggagatgtc aaaggtagcccaaaaaaca | F | 240 |
| ra74848s01 | A | G | tatatcagtttatttatttgagtctatgtaaca agatgtttaatctaatg[a/g]acaccaac cctttattgtctgctataaatgataaatcctt gtttagaacg | R | 241 |
| ra74873s01 | C | T | ctgtcttttcttgcaaaacatgtatggtctct tcactggtatgatgacc[c/t]ctctttcttctt gcatctttcacaagatgctgctggcagca ttggacaca | R | 242 |
| ra74851s01 | A | G | gtctcacattgctcagccaagcggcggtt cctaagacctactggccgtac[a/g]ctttt | F | 243 |

| | | | | | |
|---|---|---|---|---|---|
| | | | gctactgcagtcattctcataaaccgact acccctctcccgtgctc | | |
| ra74852s01 | A | G | gtgccacttacttgtgtgcgaatcccatttt ccactcccgcatgaaacac[a/g]tcgc gttagactatcatttcatacgcaacaacgt gcaatcgggggctctg | F | 244 |
| ra74854s01 | A | T | caggtacgagctttatttgatcacagtgg ggaacgagttgatgaagctgg[a/t]ccc tccatacctgtacaggtttgttgtttcttaatc ttcctttaaagtca | F | 245 |
| ra74855s01 | C | T | tcagacaagagtcaccagacgaagca agtttcttgtgatcttgtcaatac[c/t]atag ttgactttaagacaaatagtcagtacctta attaatttaatcgttt | R | 246 |
| ra74856s01 | A | C | ttgtaaaggtccttgtttcttgtatttctttctcc taccaagtttgattt[a/c]tatctctttctttctt tttcatcacccacaaaaagttgattttgttt gtt | F | 247 |
| ra74858s01 | A | T | tatagctaaaaataatcaatctccagag aaaataatattctcgttctcca[a/t]ctgctt tctctcatcggagctctcttttttctggttctca aggtaagacaa | R | 248 |
| ra74859s01 | C | T | cggattaacccatgtgttatcccaacaac tagcctgacagttgatgacac[c/t]aaac tgattcttggaggtcaagatagtgatatgg ttgttgcttttagcaa | F | 249 |
| ra74860s01 | G | T | ttcaactttttgtgtgtgattcatgaaaccct ctctgttattgtccttgc[g/t]cttcgctttca cctgatcctctgtttctgttttttggataaagtt cctaaa | F | 250 |
| ra74862s01 | A | T | acgcatgtcaatggttcatctcttatataa gatagagaagtactgaattg[a/t]aaag aagacaagcaatcaaacctgtacgagc ctgttgctacccttgatcc | R | 251 |

| ra74863s01 | A | C | ttctccttttgttctcgctacgcaaccagta attttttttgtcttgagaat[a/c]tcacttttag cattttggtaggcactaaacatgaccagt gatgcataagg | F | 252 |
|---|---|---|---|---|---|
| ra74864s01 | A | C | actttttctcaaaaagtaaatttgatgtttgtg ttatgatttatatatgta[a/c]tgctatttgtttt gatgatattttctctaagcatttatcactga gattag | R | 253 |
| ra74865s01 | C | T | tgcattttctccaagatattcctttgctctcc aaatctccacgagtgcag[c/t]tcagcat cttgactccacatggtcttcaccattcaac atgtcatctgcgt | F | 254 |
| ra74867s01 | A | C | tcatgaacatttagctgtttcagccatgact ccgctgctcttccaagata[a/c]actaac cgctcttgatcaccagattctcgtttccccc acaaacctccttg | R | 255 |
| ra74869s01 | C | T | aaaaaaacaaatctaacactataagtc ccaattttttgataaaagaacaat[c/t]agt gacgaagaaatgatcaaagctagaag aagagagaagaatgattcaaa | F | 256 |
| ra74870s01 | G | T | aaattccatattcactaggaaacttgtccc gtctcacaaatcttcttctt[g/t]ctgagaat cacttgagaggtgaagttccgcctacaat tggcaacctaaac | R | 257 |
| ra74871s01 | G | T | attacgaaggtattcgaggttcgttataca tgggggcaatcaatcatacg[g/t]attcg atggatatgatgtataaaggtgtagacac agagtttccactgatc | F | 258 |
| ra74915s01 | G | T | caaartacagacgtagagacagaatca gggtcaataaggaggagaggaca[g/t] gaactcactctaatgaatactggagaag gctgttcamtgaagacatcttc | F | 259 |
| ra74916s01 | G | T | ckcctcgtgtcgagcttcacacatctccgt cgtacggccattctcaggta[g/t]aaagc | R | 260 |

| | | | gttttctcgtttctcctctrtcgagkttgwgct cgatttgatatg | | |
|---|---|---|---|---|---|
| ra74917s01 | A | G | cgtttgattccttgagaattttttttttttaacct tgagtttgtgtctaa[a/g]ttggattaatgg tgtagaataagatttgaatacgagtattttt attccgt | R | 261 |
| ra74919s01 | A | G | aaaactatgaagagcgtgtggcgtaag ggagatgctgttgcagctgtgga[a/g]a aagttgttaagcaagaacctaaaattgat agtaggggaccacctcagcc | R | 262 |
| ra74921s01 | A | G | aataattaagaactgggtactggttctcat tacctatacacacgttcctt[a/g]cgacg attaaggtcgtgtgttacagttcagttctta attycaaacatatt | R | 263 |
| ra74922s01 | A | G | tttttaatttttaaaagcttwtaaatttgggatg taggtatttgtctctgc[a/g]gagcaattat aaagacatgagtttaaaaaagtgttgta gactttaaaaaa | F | 264 |
| ra74938s01 | A | G | atcctgagctcttgcacagataccggtga cattaggtatgtcaccacatc[a/g]ttcct ggagcccttagtatattcctggtctcatca tccttttggcatat | F | 265 |
| ra74941s01 | G | T | ggagaggagagaggagagtcagtgtt ataaaacgacacgatatcctcacc[g/t] agttacgcctcatttcctctcactccatctct ctctctctgtgttgtg | R | 266 |
| ra74949s01 | G | T | aaatacacataacaaacagaatcttgc attgctacagatactagaaaaac[g/t]a gaaaccacttctttatatacaaatatgtac atgcagttgccttccccct | R | 267 |
| ra74932s02 | C | T | ctctttgacaccgataggaatagtatcatt cgctccaccgctgccttgaa[c/t]tgtttc aatttctcaaactgctctgatcttcgttgcc gcggaagagaacc | R | 268 |

| ra74955s01 | A | T | acgcaaaagaacctgaaggtatgcttta tcctcctcagttgacacaggtc[a/t]ctctt tatagagcttatatgttggtttaaatttgctat acactccataac | R | 269 |
|---|---|---|---|---|---|
| ra74945s01 | A | C | gagagaacaaaagtcataatctggagc tggacatctgggaatctaaatat[a/c]gg aaagaatgttaagttttgatcacataaaa catgatatgatgaaaaaaa | F | 270 |
| ra74946s01 | A | G | ataaacgtttatatcttgcgtagcatttctta actcacacattgcttgtc[a/g]attatttgg atcaggaatcaagttctcagttggcaatg ggagcagctttt | F | 271 |
| ra74948s01 | G | T | atgctgtgtccaggcgtagggttcgtcatc ttgatgttcgtaatgagttg[g/t]ataatgg gatacttaagatgcctccgtctctctattcg tgtgagagattg | R | 272 |
| ra74952s01 | C | T | cccccactgacgcctatactcggtccatc tcttccctgagcggtgacagt[c/t]tctgtg ttcattacttcctcgccgtcatttatgctgct aatgcctgcttc | F | 273 |
| ra74910s01 | A | G | ttaggccacgtaagctgcgagaaagct gtggtcacaactgccttcagtag[a/g]ttt accaatctgggtgtcaggactaacaag aagaagagaatcggtccggt | R | 274 |
| ra01867s01 | G | T | caaaatcagamactgatggagaagaa cataaagtagcaaaggagccagct[g/t ]scaaagctgatggagtagagcgagag tctgtgaaagagccaratgaagaa | F | 275 |
| ra01868s01 | A | C | ttaaagtctactacaaacaagtcaaatg caaaatcaga[a/c]actgatggagaag aacataaagtagcaaaggagccagct kscaaagctga | F | 276 |
| ra13870s01 | A | G | tcawagaaacaaggctgagaatatca gttnaaaaangttacankttatgt[a/g]tg | R | 277 |

| | | | | | |
|---|---|---|---|---|---|
| | | | tccaaagaacaatatgcctcttagtaact cagactaaacaaagttaag | | |
| ra17237s01 | G | T | ggtgttttatgtttttgkctagcttccctcgta gacaccttatagrcytg[g/t]gctacccg gtttaggatcgctaatttctctgaaacgctt tctttagtcac | R | 278 |
| ra36486s01 | G | T | atcaatcggagatataaaaaatgatacc aatagcataataacaaagatat[g/t]tga tttaaaaagaaacttamaaaagtgata grgtatccagatttcacatm | R | 279 |
| ra36162s01 | C | T | tttgcaagttttttctctcattgcgatcccctt tcattcgtacttaccg[c/t]agaaataatg aaattaaaggatttrataactaactaatat yggtcamgta | F | 280 |
| ra36410s01 | A | G | tccacacacacrtacyccactgatccttc caacrttcattaactttttct[a/g]attctcac attctgcagaaaataactttgttgcatcac catgaagtcaaa | R | 281 |
| ra36412s01 | C | T | tatgattggttwatgtttccmtttttcactcc aatctrttgacatgataa[c/t]gctcttact gaatagtttctcatggaacagttctgcata gctctagtgac | F | 282 |
| ra36483s01 | C | T | catccatgatctttctcttctttgtaactttgg cctmctctgtgctcact[c/t]tcatgagttt cttctctgtttccacgcaaagaaccccat attactctgtt | R | 283 |
| ra74872s01 | C | T | gttattacgttttcaaatatagcaaaaatat cacctaatgaggaataata[c/t]ttacttg gagtgaagtaatgatctttccctttgatca aagatattttgt | R | 284 |
| ra74910s01 | A | G | ttaggccacgtaagctgcgagaaagct gtggtcacaactgccttcagtag[a/g]ttt accaatctgggtgtcaggactaacaag aagaagagaatcggtccggt | R | 285 |

| ra74958s01 | C | T | ttgcttctttctcctttactgcaccttgtactct ctctctctctctca[c/t]cattttcgaggtag tgagagagtgagatgcctgaaaccaaa ctcatttct | R | 286 |
|---|---|---|---|---|---|
| ra74958s02 | A | T | gggattttgctgactcacttctcattggga atattcttcatatgtttggc[a/t]tactgaaa tttatgcaatcaactttgcattttaaattaca atcgtgtaat | F | 287 |
| ra74958s03 | T | C | gggaaattcttctgacattcatcagagtg caaacaacattttgtttgg[t/c]aaactat agtctacagcttaatgatacagatcttcctt ggaaggaatata | R | 288 |
| ra74958s04 | C | G | agtatcttactttatccggttcatcactaatt gaaatgcattcattagaa[c/g]accgtgt aaaaaaagattcattcattataattaacgt tttaacagtacca | F | 289 |
| ra74958s05 | A | C | tagatcctctagtgacaacgttgaagcca cctcgtcgaaytggagtat[a/c]gcagct tgctacaaaagaaacaacaatggtttaa aggcgcaattctcaag | R | 290 |
| ra74958s06 | A | C | agtatcgcagcttgctacaaaagaaac aacaatggtttaaaggcgcaatt[a/c]tc aaggtgactttatgcattaagttaaccaa agacgatccacttaccacg | F | 291 |
| ra74958s07 | A | C | cttgctacaaaagaaacaacaatggttt aaaggcgcaattmtcaaggtga[a/c]tt tatgcattaagttaaccaaagacgatcc acttaccacgttcattatat | F | 292 |
| ra74958s08 | C | G | acaatgtcaagatctccttcgaaggggc accaactaacctaaagagagtt[c/g]gt agtaacaaaaattcataagggattcaca aaaaaacaacgaatatctca | R | 293 |

**Table E**: Physical (bp) and genetic (cM) position of the identified markers of the invention on chromosome 6 of the Darmor v4.1 reference genome. Sequence information of KASP primers (for donor/resistant allele, susceptible allele, as well as common primer) for a selection of markers is provided.

| Marker | Chromosome | SNP Position Darmorv 4.1 | SNP Position Genetic Map (cM) | SEQ ID NO Allele X | SEQ ID NO Allele Y | SEQ ID NO common primer |
|---|---|---|---|---|---|---|
| ra36146s01 | A06 | 4839277 | 0 | | | |
| ra36155s01 | A06 | 4856766 | | | | |
| ra61609s01 | A06 | 4884577 | | | | |
| ra36158s01 | A06 | 4899658 | | | | |
| ra36159s01 | A06 | 4902731 | 1,2 | | | |
| ra36160s01 | A06 | 4911752 | | | | |
| ra0000bp10 | A06 | 4915604 | | | | |
| ra0000bp12 | A06 | 4915805 | | | | |
| ra0000bp11 | A06 | 4915657 | | | | |
| ra36161s01 | A06 | 4927351 | | | | |
| ra36162s01 | A06 | 4927798 | | 61 | 132 | 203 |
| ra36163s01 | A06 | 4944020 | | | | |
| ra36164s01 | A06 | 4964195 | | | | |
| ra36165s01 | A06 | 4967878 | | | | |
| ra36166s01 | A06 | 4967961 | | | | |
| ra0000bp13 | A06 | 4987049 | | | | |
| ra0000bp14 | A06 | 5003808 | | | | |
| ra36255s01 | A06 | 5007776 | | | | |
| ra36256s01 | A06 | 5010462 | | | | |
| ra36257s01 | A06 | 5015364 | | | | |
| ra11566s01 | A06 | 5015608 | | | | |
| ra36265s01 | A06 | 5044742 | | | | |
| ra36267s01 | A06 | 5051901 | | | | |
| ra36268s01 | A06 | 5056127 | | | | |
| ra36269s01 | A06 | 5062564 | | | | |

| | | | | | | |
|---|---|---|---|---|---|---|
| ra36270s01 | A06 | 5064801 | | | | |
| ra0000dp13 | A06 | 5079443 | | | | |
| ra36271s01 | A06 | 5086034 | | | | |
| ra36272s01 | A06 | 5119769 | | | | |
| ra36274s01 | A06 | 5138085 | | | | |
| ra0000dp14 | A06 | 5146309 | | | | |
| ra36278s01 | A06 | 5182655 | | | | |
| ra36279s01 | A06 | 5185885 | | | | |
| ra36280s01 | A06 | 5202213 | | | | |
| ra36285s01 | A06 | 5206433 | | | | |
| ra36287s01 | A06 | 5218016 | | | | |
| ra12177s01 | A06 | 5250035 | | | | |
| ra36289s01 | A06 | 5252456 | | | | |
| ra36290s01 | A06 | 5254497 | | | | |
| ra0000dp15 | A06 | 5254635 | | | | |
| ra13899s01 | A06 | 5256087 | | | | |
| ra0000bp15 | A06 | 5256686 | | | | |
| ra36292s01 | A06 | 5266053 | | | | |
| ra36293s01 | A06 | 5267134 | | | | |
| ra0000bp16 | A06 | 5289239 | | | | |
| ra36294s01 | A06 | 5289964 | | | | |
| ra0000bp17 | A06 | 5296819 | | | | |
| ra0000bp18 | A06 | 5297108 | | | | |
| ra0000bp19 | A06 | 5297397 | | | | |
| ra12150s01 | A06 | 5297818 | | | | |
| ra12148s01 | A06 | 5297916 | | | | |
| ra36295s01 | A06 | 5300782 | | | | |
| ra36296s01 | A06 | 5302770 | | | | |
| ra36297s01 | A06 | 5310061 | | | | |
| ra36298s01 | A06 | 5334804 | | | | |
| ra36301s01 | A06 | 5354720 | | | | |
| ra36302s01 | A06 | 5364528 | | | | |
| ra61642s01 | A06 | 5385366 | | | | |

| | | | | | | |
|---|---|---|---|---|---|---|
| ra36306s01 | A06 | 5385503 | | | | |
| ra36308s01 | A06 | 5388175 | 13,6 | | | |
| ra36309s01 | A06 | 5403991 | | | | |
| ra36310s01 | A06 | 5405095 | | | | |
| ra36312s01 | A06 | 5419754 | | | | |
| ra36314s01 | A06 | 5427345 | | | | |
| ra36315s01 | A06 | 5428692 | | | | |
| ra36316s01 | A06 | 5429350 | | | | |
| ra36317s01 | A06 | 5430360 | | | | |
| ra36318s01 | A06 | 5430636 | | | | |
| ra36319s01 | A06 | 5430793 | | | | |
| ra36320s01 | A06 | 5431324 | | | | |
| ra36321s01 | A06 | 5431650 | | | | |
| ra36322s01 | A06 | 5439143 | | | | |
| ra36323s01 | A06 | 5439416 | | | | |
| ra36324s01 | A06 | 5440260 | | | | |
| ra36325s01 | A06 | 5441044 | | | | |
| ra36330s01 | A06 | 5449534 | | | | |
| ra36331s01 | A06 | 5451597 | | | | |
| ra36332s01 | A06 | 5452662 | | | | |
| ra36334s01 | A06 | 5455275 | | | | |
| ra36335s01 | A06 | 5457921 | | | | |
| ra0000dp16 | A06 | 5460236 | | | | |
| ra36336s01 | A06 | 5468640 | | | | |
| ra36337s01 | A06 | 5495115 | | | | |
| ra36338s01 | A06 | 5499274 | | | | |
| ra36339s01 | A06 | 5499251 | | | | |
| ra36340s01 | A06 | 5500197 | | | | |
| ra36341s01 | A06 | 5501868 | | | | |
| ra36342s01 | A06 | 5510178 | | | | |
| ra36345s01 | A06 | 5537099 | | | | |
| ra36346s01 | A06 | 5542254 | | | | |
| ra36347s01 | A06 | 5542333 | | | | |

| | | | | | | |
|---|---|---|---|---|---|---|
| ra74938s01 | A06 | 5543485 | | 44 | 118 | 189 |
| ra36349s01 | A06 | 5555541 | | | | |
| ra36350s01 | A06 | 5563828 | | | | |
| ra36352s01 | A06 | 5583362 | 17,2 | | | |
| ra0000bp23 | A06 | 5590724 | | | | |
| ra0000bp24 | A06 | 5590984 | | | | |
| ra36353s01 | A06 | 5603334 | | | | |
| ra74915s01 | A06 | 5603449 | | 38 | 112 | 183 |
| ra36354s01 | A06 | 5603932 | | | | |
| ra36355s01 | A06 | 5605199 | | | | |
| ra01868s01 | A06 | 5605457 | | 57 | 128 | 199 |
| ra01867s01 | A06 | 5605497 | | 56 | 127 | 198 |
| ra01866s01 | A06 | 5605498 | | | | |
| ra36356s01 | A06 | 5605755 | | | | |
| ra74835s01 | A06 | 5613935 | | 11 | 85 | 156 |
| ra36357s01 | A06 | 5625053 | | | | |
| ra36358s01 | A06 | 5625326 | | | | |
| ra36359s01 | A06 | 5627883 | | | | |
| ra74836s01 | A06 | 5628511 | | 12 | 86 | 157 |
| ra74837s01 | A06 | 5632864 | | 13 | 87 | 158 |
| ra74941s01 | A06 | 5638550 | | 45 | 119 | 190 |
| ra0000dp17 | A06 | 5639970 | | | | |
| ra74838s01 | A06 | 5641284 | | 14 | 88 | 159 |
| ra74840s01 | A06 | 5660335 | | 15 | 89 | 160 |
| ra74841s01 | A06 | 5672356 | | 16 | 90 | 161 |
| ra74916s01 | A06 | 5690511 | | 39 | 113 | 184 |
| ra74949s01 | A06 | 5698241 | | 46 | 120 | 191 |
| ra36363s01 | A06 | 5700496 | | | | |
| ra74932s02 | A06 | 5705944 | | 47 | 121 | 192 |
| ra74872s01 | A06 | 5735389 | | 65 | 136 | 207 |
| ra74844s01 | A06 | 5742133 | | 17 | 91 | 162 |
| ra05134s01 | A06 | 5754822 | | | | |
| ra74955s01 | A06 | 5755109 | | 48 | 122 | 193 |

| | | | | | | |
|---|---|---|---|---|---|---|
| ra36364s01 | A06 | 5756087 | | | | |
| ra36365s01 | A06 | 5758615 | | | | |
| ra36366s01 | A06 | 5758728 | | | | |
| ra36367s01 | A06 | 5768576 | | | | |
| ra74846s01 | A06 | 5780109 | | 18 | 92 | 163 |
| ra36368s01 | A06 | 5792363 | | | | |
| ra36369s01 | A06 | 5794882 | | | | |
| ra74847s01 | A06 | 5848144 | | 19 | 93 | 164 |
| ra36377s01 | A06 | 5849412 | | | | |
| ra74917s01 | A06 | 5860668 | | 40 | 114 | 185 |
| ra74848s01 | A06 | 5863867 | | | | |
| ra36379s01 | A06 | 5871964 | | | | |
| ra36381s01 | A06 | 5878935 | | | | |
| ra0000dp18 | A06 | 5879162 | | | | |
| ra36383s01 | A06 | 5889764 | | | | |
| ra36384s01 | A06 | 5890452 | | | | |
| ra36385s01 | A06 | 5891612 | | | | |
| ra36386s01 | A06 | 5896863 | | | | |
| ra0000bp25 | A06 | 5897286 | | | | |
| ra36387s01 | A06 | 5897686 | 21,5 | 1 | 75 | 146 |
| ra74873s01 | A06 | 5898065 | | 21 | 95 | 166 |
| ra36388s01 | A06 | 5903558 | | | | |
| ra0000dp19 | A06 | 5911516 | | | | |
| ra36389s01 | A06 | 5916939 | | | | |
| ra36390s01 | A06 | 5921067 | | | | |
| ra36392s01 | A06 | 5924593 | | | | |
| ra36396s01 | A06 | 5939740 | | | | |
| ra74851s01 | A06 | 5941369 | | 22 | 96 | 167 |
| ra0000dp20 | A06 | 5943364 | | | | |
| ra74852s01 | A06 | 5943520 | | 23 | 97 | 168 |
| ra36397s01 | A06 | 5949395 | | | | |
| ra36398s01 | A06 | 5951830 | | | | |
| ra14651s01 | A06 | 5977944 | | | | |

| | | | | | | |
|---|---|---|---|---|---|---|
| ra74919s01 | A06 | 5995057 | | 41 | 115 | 186 |
| ra74854s01 | A06 | 5997653 | | 24 | 98 | 169 |
| ra36409s01 | A06 | 6017553 | | | | |
| ra36410s01 | A06 | 6019305 | | 62 | 133 | 204 |
| ra74855s01 | A06 | 6022301 | 24,6 | 25 | 99 | 170 |
| ra36412s01 | A06 | 6031226 | | 63 | 134 | 205 |
| ra36414s01 | A06 | 6038704 | | | | |
| ra74856s01 | A06 | 6042239 | 25,2 | 26 | 100 | 171 |
| ra74921s01 | A06 | 6043355 | | 42 | 116 | 187 |
| ra36415s01 | A06 | 6045480 | | | | |
| ra74945s01 | A06 | 6059024 | | 49 | 123 | 194 |
| ra74946s01 | A06 | 6067467 | | 50 | 124 | 195 |
| ra74948s01 | A06 | 6082193 | | 51 | 125 | 196 |
| ra36418s01 | A06 | 6085238 | | | | |
| ra05569s01 | A06 | 6085138 | | | | |
| ra36419s01 | A06 | 6086857 | | | | |
| ra74858s01 | A06 | 6088663 | | 27 | 101 | 172 |
| ra74859s01 | A06 | 6091648 | | 28 | 102 | 173 |
| ra36423s01 | A06 | 6098159 | | | | |
| ra74922s01 | A06 | 6102982 | | 43 | 117 | 188 |
| ra74860s01 | A06 | 6113367 | 25,5 | 29 | 103 | 174 |
| ra36426s01 | A06 | 6134234 | | | | |
| ra36427s01 | A06 | 6141195 | | | | |
| ra36428s01 | A06 | 6141204 | 26,2 | 10 | 84 | 155 |
| ra36429s01 | A06 | 6141354 | | | | |
| ra36430s01 | A06 | 6151503 | | | | |
| ra36433s01 | A06 | 6152744 | | | | |
| ra36434s01 | A06 | 6153568 | | | | |
| ra74862s01 | A06 | 6155076 | 26,9 | 30 | 104 | 175 |
| ra36435s01 | A06 | 6168127 | | | | |
| ra74863s01 | A06 | 6186575 | | 31 | 105 | 176 |
| ra74864s01 | A06 | 6196798 | | 32 | 106 | 177 |
| ra74910s01 | A06 | 6197815 | | 66 | 137 | 208 |

| ra74865s01 | A06 | 6204215 | | 33 | 107 | 178 |
|---|---|---|---|---|---|---|
| ra74952s01 | A06 | 6209886 | | 52 | 126 | 197 |
| ra36441s01 | A06 | 6240866 | | | | |
| ra74867s01 | A06 | 6248401 | 26,9 | 34 | 108 | 179 |
| ra36443s01 | A06 | 6257529 | | | | |
| ra36444s01 | A06 | 6269510 | 27,8 | 2 | 76 | 147 |
| ra74958s01 | A06 | 6275195 | | 67 | 138 | 209 |
| ra74958s02 | A06 | 6275424 | | 68 | 139 | 210 |
| ra74958s03 | A06 | 6275538 | | 69 | 140 | 211 |
| ra74958s04 | A06 | 6275743 | | 70 | 141 | 212 |
| ra74958s05 | A06 | 6276255 | | 71 | 142 | 213 |
| ra74958s06 | A06 | 6276300 | | 72 | 143 | 214 |
| ra74958s07 | A06 | 6276310 | | 73 | 144 | 215 |
| ra74958s08 | A06 | 6276740 | | 74 | 145 | 216 |
| ra74869s01 | A06 | 6306470 | | 35 | 109 | 180 |
| ra74870s01 | A06 | 6314871 | 28,1 | 36 | 110 | 181 |
| ra36447s01 | A06 | 6315070 | | | | |
| ra74871s01 | A06 | 6346195 | | 37 | 111 | 182 |
| ra36449s01 | A06 | 6369655 | | | | |
| ra36450s01 | A06 | 6373996 | 29 | 3 | 77 | 148 |
| ra36451s01 | A06 | 6375256 | | 4 | 78 | 149 |
| ra36453s01 | A06 | 6406350 | | | | |
| ra36454s01 | A06 | 6410231 | | | | |
| ra50627s01 | A06 | 6421616 | | | | |
| ra36457s01 | A06 | 6421763 | | | | |
| ra36458s01 | A06 | 6425751 | | 5 | 79 | 150 |
| ra36459s01 | A06 | 6426888 | | 6 | 80 | 151 |
| ra36460s01 | A06 | 6427052 | | 7 | 81 | 152 |
| ra36461s01 | A06 | 6427553 | | 8 | 82 | 153 |
| ra50626s01 | A06 | 6428706 | | | | |
| ra36463s01 | A06 | 6437251 | | 9 | 83 | 154 |
| ra36467s01 | A06 | 6454193 | | | | |
| ra50623s01 | A06 | 6456013 | | | | |

| | | | | | | |
|---|---|---|---|---|---|---|
| ra36469s01 | A06 | 6474875 | | | | |
| ra36470s01 | A06 | 6474889 | | | | |
| ra36471s01 | A06 | 6474995 | | | | |
| ra0000bp26 | A06 | 6475550 | | | | |
| ra50619s01 | A06 | 6496790 | | | | |
| ra0000dp21 | A06 | 6517587 | | | | |
| ra0000bp27 | A06 | 6530194 | | | | |
| ra36474s01 | A06 | 6544813 | | | | |
| ra07603s02 | A06 | 6552185 | | | | |
| ra07603s01 | A06 | 6552129 | | | | |
| ra36475s01 | A06 | 6556280 | | | | |
| ra36476s01 | A06 | 6573500 | | | | |
| ra36477s01 | A06 | 6644158 | | | | |
| ra0000dp22 | A06 | 6647495 | | | | |
| ra36478s01 | A06 | 6652312 | | | | |
| ra50605s01 | A06 | 6699025 | | | | |
| ra0000dp23 | A06 | 6705917 | | | | |
| ra36481s01 | A06 | 6721459 | | | | |
| ra36482s01 | A06 | 6733929 | | | | |
| ra36483s01 | A06 | 6758391 | | 64 | 135 | 206 |
| ra36485s01 | A06 | 6786083 | | | | |
| ra36486s01 | A06 | 6786530 | | 60 | 131 | 202 |
| ra36490s01 | A06 | 6795965 | | | | |
| ra36492s01 | A06 | 6806634 | 34,7 | | | |
| ra36493s01 | A06 | 6807432 | | | | |
| ra0000dp24 | A06 | 6848419 | | | | |
| ra17237s01 | A06 | 6878047 | | 59 | 130 | 201 |
| ra0000dp25 | A06 | 6902103 | | | | |
| ra36499s01 | A06 | 6938254 | | | | |
| ra36500s01 | A06 | 6943320 | | | | |
| ra36501s01 | A06 | 6945568 | | | | |
| ra36502s01 | A06 | 6948939 | | | | |
| ra36503s01 | A06 | 6966982 | | | | |

| | | | | | | |
|---|---|---|---|---|---|---|
| ra36504s01 | A06 | 6967497 | | | | |
| ra36506s01 | A06 | 7008151 | | | | |
| ra36507s01 | A06 | 7009992 | | | | |
| ra0000dp26 | A06 | 7017603 | | | | |
| ra36509s01 | A06 | 7060398 | | | | |
| ra13870s01 | A06 | 7060302 | | 58 | 129 | 200 |
| ra46770s01 | A06 | 7079170 | | | | |
| ra0000dp27 | A06 | 7083010 | | | | |
| ra0000dp28 | A06 | 7160147 | | | | |
| ra0000av87 | A06 | 7187542 | | | | |
| ra36511s01 | A06 | 7210628 | | | | |
| ra36512s01 | A06 | 7217480 | | | | |
| ra00169s01 | A06 | 7236144 | | | | |
| ra0000dp29 | A06 | 7240251 | | | | |
| ra36516s01 | A06 | 7257713 | | | | |
| ra36517s01 | A06 | 7266286 | | | | |
| ra36518s01 | A06 | 7268013 | | | | |
| ra36519s01 | A06 | 7274483 | | | | |
| ra36520s01 | A06 | 7278399 | | | | |
| ra36521s01 | A06 | 7278464 | | | | |
| ra36522s01 | A06 | 7280606 | | | | |
| ra36523s01 | A06 | 7287336 | | | | |
| ra36524s01 | A06 | 7292882 | | | | |
| ra36525s01 | A06 | 7294136 | | | | |
| ra00695s01 | A06 | 7299669 | | | | |
| ra36526s01 | A06 | 7301443 | | | | |
| ra36527s01 | A06 | 7306347 | 42,3 | | | |
| ra36528s01 | A06 | 7306328 | | | | |
| ra36529s01 | A06 | 7331460 | | | | |
| ra36530s01 | A06 | 7333695 | | | | |
| ra36531s01 | A06 | 7338149 | | | | |
| ra36532s01 | A06 | 7356711 | | | | |
| ra0000dp30 | A06 | 7374019 | | | | |

| | | | | | |
|---|---|---|---|---|---|
| ra0000ah03 | A06 | 7398437 | | | |
| ra36533s01 | A06 | 7399002 | | | |
| ra0000dp31 | A06 | 7449905 | | | |
| ra0000dp32 | A06 | 7470602 | | | |
| ra0000dp33 | A06 | 7513403 | | | |
| ra36536s01 | A06 | 7556119 | | | |
| ra36537s01 | A06 | 7556264 | | | |
| ra36539s01 | A06 | 7560549 | | | |
| ra36540s01 | A06 | 7580680 | | | |
| ra36541s01 | A06 | 7616836 | | | |
| ra36545s01 | A06 | 7629649 | | | |
| ra36548s01 | A06 | 7645380 | | | |
| ra0000aa06 | A06 | 7657378 | | | |
| ra12096s01 | A06 | 7657549 | | | |
| ra36549s01 | A06 | 7658143 | | | |
| ra36550s01 | A06 | 7661720 | | | |
| ra36551s01 | A06 | 7661888 | | | |
| ra36553s01 | A06 | 7665850 | | | |
| ra36554s01 | A06 | 7673997 | | | |
| ra36555s01 | A06 | 7677496 | | | |
| ra36556s01 | A06 | 7677988 | | | |
| ra36557s01 | A06 | 7678203 | | | |
| ra36558s01 | A06 | 7682880 | | | |
| ra0000dp34 | A06 | 7719606 | | | |
| ra36559s01 | A06 | 7723590 | | | |
| ra36560s01 | A06 | 7723921 | | | |
| ra0000ah05 | A06 | 7778970 | | | |
| ra0000ah06 | A06 | 7778876 | | | |
| ra36566s01 | A06 | 7779141 | | | |
| ra36567s01 | A06 | 7779681 | | | |
| ra36568s01 | A06 | 7781087 | | | |
| ra0000ab35 | A06 | 7807294 | | | |
| ra36572s01 | A06 | 7814278 | | | |

| | | | | | | |
|---|---|---|---|---|---|---|
| ra36573s01 | A06 | 7815155 | | | | |
| ra0000ah07 | A06 | 7818006 | | | | |
| ra0000ah08 | A06 | 7818067 | | | | |
| ra36575s01 | A06 | 7829462 | | | | |
| ra36576s01 | A06 | 7834350 | | | | |
| ra0000dp35 | A06 | 7863191 | | | | |
| ra0000dp36 | A06 | 7891736 | | | | |
| ra0000ah09 | A06 | 7900547 | | | | |
| ra0000dp37 | A06 | 7913396 | | | | |
| ra0000ah10 | A06 | 7949004 | | | | |
| ra36579s01 | A06 | 7951624 | | | | |
| ra36580s01 | A06 | 7966871 | | | | |
| ra36581s01 | A06 | 7976824 | | | | |
| ra36582s01 | A06 | 7979457 | | | | |
| ra36583s01 | A06 | 7983003 | | | | |
| ra36584s01 | A06 | 7993145 | | | | |
| ra0000ah11 | A06 | 7998760 | | | | |
| ra0000ah12 | A06 | 8010638 | | | | |
| ra0000ah13 | A06 | 8012944 | | | | |
| ra36586s01 | A06 | 8014530 | | | | |
| ra36589s01 | A06 | 8046270 | | | | |
| ra36590s01 | A06 | 8054506 | | | | |
| ra36593s01 | A06 | 8058244 | | | | |
| ra36595s01 | A06 | 8063823 | | | | |
| ra36596s01 | A06 | 8064372 | | | | |
| ra0000dp38 | A06 | 8070063 | | | | |
| ra40565s01 | A06 | 8073587 | | | | |
| ra71771s01 | A06 | 8075813 | | | | |
| ra36597s01 | A06 | 8101348 | | | | |
| ra36598s01 | A06 | 8109398 | | | | |
| ra36599s01 | A06 | 8121868 | | | | |
| ra36600s01 | A06 | 8128583 | | | | |
| ra36601s01 | A06 | 8129591 | 51,8 | | | |

| ra36602s01 | A06 | 8129632 | | | | |
|---|---|---|---|---|---|---|
| ra36604s01 | A06 | 8146323 | | | | |
| ra36605s01 | A06 | 8147266 | | | | |
| ra36606s01 | A06 | 8174394 | | | | |
| ra36607s01 | A06 | 8174771 | | | | |
| ra36608s01 | A06 | 8175263 | | | | |

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims. It is further to be understood that all values are approximate and are provided for description.
Patents, patent applications, publications, product descriptions, and protocols are cited throughout this application, the disclosures of which are incorporated herein by reference in their entireties for all purposes.

**Claims**

1. A method for identifying and/or selecting a plant or plant part of the Brassicaceae family, comprising screening for the presence of a QTL allele on a polynucleotide located on a chromosomal interval spanning at most 10 cM, preferably at most 5 cM, more preferably at most 2 cM respectively upstream and downstream of molecular marker (allele) ra74856s01 and ra36444s01.

2. The method according to claims 1, comprising screening for the presence of a polynucleotide comprising one or more molecular marker (allele) selected from ra74856s01, ra74921s01, ra74945s01, ra74946s01, ra74948s01, ra74858s01, ra74859s01, ra74922s01, ra74860s01, ra36428s01, ra74862s01, ra74863s01, ra74864s01, ra74910s01, ra74865s01, ra74952s01, ra74867s01, and ra36444s01 (in the genome of the plant or plant part).

3. The method according to claim 1 or 2, wherein molecular marker (allele)

   ra74856s01 is or comprises a SNP at a position corresponding to position 6042239 (on chromosome A06) of reference Brassica napus genome Darmor-bzh v4.1;
   ra74921s01 is or comprises a SNP at a position corresponding to position 6043355 (on chromosome A06) of reference Brassica napus genome Darmor-bzh v4.1;
   ra36415s01 is or comprises a SNP at a position corresponding to position 6045480 (on chromosome A06) of reference Brassica napus genome Darmor-bzh v4.1;
   ra74945s01 is or comprises a SNP at a position corresponding to position 6059024 (on chromosome A06) of reference Brassica napus genome Darmor-bzh v4.1;
   ra74946s01 is or comprises a SNP at a position corresponding to position 6067467 (on chromosome A06) of reference Brassica napus genome Darmor-bzh v4.1;
   ra74948s01 is or comprises a SNP at a position corresponding to position 6082193 (on chromosome A06) of reference Brassica napus genome Darmor-bzh v4.1;
   ra36418s01 is or comprises a SNP at a position corresponding to position 6085238 (on chromosome A06) of reference Brassica napus genome Darmor-bzh v4.1;
   ra05569s01 is or comprises a SNP at a position corresponding to position 6085138 (on chromosome A06) of reference Brassica napus genome Darmor-bzh v4.1;
   ra36419s01 is or comprises a SNP at a position corresponding to position 6086857 (on chromosome A06) of reference Brassica napus genome Darmor-bzh v4.1;
   ra74858s01 is or comprises a SNP at a position corresponding to position 6088663 (on chromosome A06) of reference Brassica napus genome Darmor-bzh v4.1;
   ra74859s01 is or comprises a SNP at a position corresponding to position 6091648 (on chromosome A06) of reference Brassica napus genome Darmor-bzh v4.1;
   ra36423s01 is or comprises a SNP at a position corresponding to position 6098159 (on chromosome A06) of reference Brassica napus genome Darmor-bzh v4.1;

ra74922s01 is or comprises a SNP at a position corresponding to position 6102982 (on chromosome A06) of reference Brassica napus genome Darmor-bzh v4.1;

ra74860s01 is or comprises a SNP at a position corresponding to position 6113367 (on chromosome A06) of reference Brassica napus genome Darmor-bzh v4.1;

ra36426s01 is or comprises a SNP at a position corresponding to position 6134234 (on chromosome A06) of reference Brassica napus genome Darmor-bzh v4.1;

ra36427s01 is or comprises a SNP at a position corresponding to position 6141195 (on chromosome A06) of reference Brassica napus genome Darmor-bzh v4.1;

ra36428s01 is or comprises a SNP at a position corresponding to position 6141204 (on chromosome A06) of reference Brassica napus genome Darmor-bzh v4.1;

ra36429s01 is or comprises a SNP at a position corresponding to position 6141354 (on chromosome A06) of reference Brassica napus genome Darmor-bzh v4.1;

ra36430s01 is or comprises a SNP at a position corresponding to position 6151503 (on chromosome A06) of reference Brassica napus genome Darmor-bzh v4.1;

ra36433s01 is or comprises a SNP at a position corresponding to position 6152744 (on chromosome A06) of reference Brassica napus genome Darmor-bzh v4.1;

ra36434s01 is or comprises a SNP at a position corresponding to position 6153568 (on chromosome A06) of reference Brassica napus genome Darmor-bzh v4.1;

ra74862s01 is or comprises a SNP at a position corresponding to position 6155076 (on chromosome A06) of reference Brassica napus genome Darmor-bzh v4.1;

ra36435s01 is or comprises a SNP at a position corresponding to position 6168127 (on chromosome A06) of reference Brassica napus genome Darmor-bzh v4.1;

ra74863s01 is or comprises a SNP at a position corresponding to position 6186575 (on chromosome A06) of reference Brassica napus genome Darmor-bzh v4.1;

ra74864s01 is or comprises a SNP at a position corresponding to position 6196798 (on chromosome A06) of reference Brassica napus genome Darmor-bzh v4.1;

ra74910s01 is or comprises a SNP at a position corresponding to position 6197815 (on chromosome A06) of reference Brassica napus genome Darmor-bzh v4.1;

ra74865s01 is or comprises a SNP at a position corresponding to position 6204215 (on chromosome A06) of reference Brassica napus genome Darmor-bzh v4.1;

ra74952s01 is or comprises a SNP at a position corresponding to position 6209886 (on chromosome A06) of reference Brassica napus genome Darmor-bzh v4.1;

ra36441s01 is or comprises a SNP at a position corresponding to position 6240866 (on chromosome A06) of reference Brassica napus genome Darmor-bzh v4.1;

ra74867s01 is or comprises a SNP at a position corresponding to position 6248401 (on chromosome A06) of reference Brassica napus genome Darmor-bzh v4.1;

ra36443s01 is or comprises a SNP at a position corresponding to position 6257529 (on chromosome A06) of reference Brassica napus genome Darmor-bzh v4.1; and/or

ra36444s01 is or comprises a SNP at a position corresponding to position 6269510 (on chromosome A06) of reference Brassica napus genome Darmor-bzh v4.1;

or the complement or reverse complement of any thereof.

4. The method according to any of claims 1 to 3, wherein molecular marker (allele)

ra74856s01 is or comprises a SNP at a position corresponding to position 51 of SEQ ID NO: 247 or a sequence which is at least 90% identical, preferably at least 95% identical, more preferably at least 98% identical, most preferably identical to SEQ ID NO: 247 (and comprising said SNP);

ra74921s01 is or comprises a SNP at a position corresponding to position 51 of SEQ ID NO: 263 or a sequence which is at least 90% identical, preferably at least 95% identical, more preferably at least 98% identical, most preferably identical to SEQ ID NO: 263 (and comprising said SNP);

ra74945s01 is or comprises a SNP at a position corresponding to position 51 of SEQ ID NO: 270 or a sequence which is at least 90% identical, preferably at least 95% identical, more preferably at least 98% identical, most preferably identical to SEQ ID NO: 270 (and comprising said SNP);

ra74946s01 is or comprises a SNP at a position corresponding to position 51 of SEQ ID NO: 271 or a sequence which is at least 90% identical, preferably at least 95% identical, more preferably at least 98% identical, most preferably identical to SEQ ID NO: 271 (and comprising said SNP);

ra74948s01 is or comprises a SNP at a position corresponding to position 51 of SEQ ID NO: 272 or a sequence which is at least 90% identical, preferably at least 95% identical, more preferably at least 98% identical, most preferably identical to SEQ ID NO: 272 (and comprising said SNP);

ra74858s01 is or comprises a SNP at a position corresponding to position 51 of SEQ ID NO: 248 or a sequence which is at least 90% identical, preferably at least 95% identical, more preferably at least 98% identical, most preferably identical to SEQ ID NO: 248 (and comprising said SNP);

ra74859s01 is or comprises a SNP at a position corresponding to position 51 of SEQ ID NO: 249 or a sequence which is at least 90% identical, preferably at least 95% identical, more preferably at least 98% identical, most preferably identical to SEQ ID NO: 249 (and comprising said SNP);

ra74922s01 is or comprises a SNP at a position corresponding to position 51 of SEQ ID NO: 264 or a sequence which is at least 90% identical, preferably at least 95% identical, more preferably at least 98% identical, most preferably identical to SEQ ID NO: 264 (and comprising said SNP);

ra74860s01 is or comprises a SNP at a position corresponding to position 51 of SEQ ID NO: 250 or a sequence which is at least 90% identical, preferably at least 95% identical, more preferably at least 98% identical, most preferably identical to SEQ ID NO: 250 (and comprising said SNP);

ra36428s01 is or comprises a SNP at a position corresponding to position 51 of SEQ ID NO: 231 or a sequence which is at least 90% identical, preferably at least 95% identical, more preferably at least 98% identical, most preferably identical to SEQ ID NO: 231 (and comprising said SNP);

ra74862s01 is or comprises a SNP at a position corresponding to position 51 of SEQ ID NO: 251 or a sequence which is at least 90% identical, preferably at least 95% identical, more preferably at least 98% identical, most preferably identical to SEQ ID NO: 251 (and comprising said SNP);

ra74863s01 is or comprises a SNP at a position corresponding to position 51 of SEQ ID NO: 252 or a sequence which is at least 90% identical, preferably at least 95% identical, more preferably at least 98% identical, most preferably identical to SEQ ID NO: 252 (and comprising said SNP);

ra74864s01 is or comprises a SNP at a position corresponding to position 51 of SEQ ID NO: 253 or a sequence which is at least 90% identical, preferably at least 95% identical, more preferably at least 98% identical, most preferably identical to SEQ ID NO: 253 (and comprising said SNP);

ra74910s01 is or comprises a SNP at a position corresponding to position 51 of SEQ ID NO: 274 or a sequence which is at least 90% identical, preferably at least 95% identical, more preferably at least 98% identical, most preferably identical to SEQ ID NO: 274 (and comprising said SNP);

ra74865s01 is or comprises a SNP at a position corresponding to position 51 of SEQ ID NO: 254 or a sequence which is at least 90% identical, preferably at least 95% identical, more preferably at least 98% identical, most preferably identical to SEQ ID NO: 254 (and comprising said SNP);

ra74952s01 is or comprises a SNP at a position corresponding to position 51 of SEQ ID NO: 273 or a sequence which is at least 90% identical, preferably at least 95% identical, more preferably at least 98% identical, most preferably identical to SEQ ID NO: 273 (and comprising said SNP);

ra74867s01 is or comprises a SNP at a position corresponding to position 51 of SEQ ID NO: 255 or a sequence which is at least 90% identical, preferably at least 95% identical, more preferably at least 98% identical, most preferably identical to SEQ ID NO: 255 (and comprising said SNP); and/or

ra36444s01 is or comprises a SNP at a position corresponding to position 51 of SEQ ID NO: 223 or a sequence which is at least 90% identical, preferably at least 95% identical, more preferably at least 98% identical, most preferably identical to SEQ ID NO: 223 (and comprising said SNP);

or the complement or reverse complement of any thereof.

5. The method according to claim 4, wherein said molecular marker (allele) comprises at least 15, preferably at least 20 contiguous nucleotides of said respective SEQ ID NO or at least 20 contiguous nucleotides being at least 90% identical, preferably at least 95% identical, more preferably at least 98% identical, most preferably identical to said respective SEQ ID NO, or the complement or reverse complement thereof.

6. The method according to any of claims 3 to 5 wherein said SNP is

C in ra74856s01;
G in ra74921s01;
A in ra74945s01;
G in ra74946s01;
T in ra74948s01;
A in ra74858s01;
C in ra74859s01;
G in ra74922s01;
T in ra74860s01;

C in ra36428s01;
T in ra74862s01;
C in ra74863s01;
A in ra74864s01;
C in ra74865s01;
C in ra74952s01;
C in ra74867s01; and/or
T in ra36444s01.

7. The method according to any of claims 1 to 6, which is a method for identifying a plant or plant part of the Brassicaceae family having an (increased) resistance or tolerance to infection with a parasite from the genus *Plasmodiophora*, such as in comparison to a plant or plant part of the Brassicaceae family not having said polynucleotide, molecular marker (allele), QTL (allele), or SNP (allele).

8. The method according to claim 7, wherein said plant or plant part of the Brassicaceae family is identified as having an (increased) resistance or tolerance if said polynucleotide, molecular marker (allele), QTL, or SNP is present in the genome of said plant or plant part.

9. A method for generating or producing a plant or plant part of the Brassicaceae family, comprising

   providing a seed mixture harvested from a cross between a first parent plant (population) of the Brassicaceae family and a second parent plant (population) of the Brassicaceae family, wherein said first and/or second parent plant (population) comprises a polynucleic acid, molecular marker (allele), QTL, or SNP as defined in any of claims 1 to 8;
   selecting seeds comprising a polynucleic acid, molecular marker (allele), QTL (allele), or SNP (allele) as defined in any of claims 1 to 8.

10. A method for generating or producing a plant or plant part of the Brassicaceae family, comprising introducing into the genome of a plant or plant part of the Brassicaceae family a polynucleic acid, molecular marker (allele), QTL (allele), or SNP (allele), as defined in any of claims 1 to 8.

11. A plant or plant part of the Brassicaceae family identified by or obtainable by the method according to any of claims 1 to 10.

12. A (isolated) polynucleotide comprising a polynucleic acid, molecular marker (allele), QTL, or SNP as defined in any of claims 1 to 8, the complement or the reverse complement thereof, or a (unique) fragment thereof.

13. The (isolated) polynucleic acid according to claim 12, in particular suitable as molecular marker, comprising at least 15, preferably at least 18, more preferably at least 20, contiguous nucleotides of any of SEQ ID NOs: 1 to 221, preferably SEQ ID NOs: 2, 10, 26 to 34, 42-43, 49-52, 66, 76, 84, 100-108, 116-117, 123-126, 137, 147, 155, 171-179, 187-188, 194-197, 208, more preferably SEQ ID NOs: 30-34, 52, 66, 104-108, 126, 137, 175-179, 197, 208; or SEQ ID NOs: 222 to 274 or to 293, preferably SEQ ID NOs: 247, 263, 270, 271, 272, 248, 249, 264, 250, 231, 251, 252, 253, 274, 254, 273, 255, or 223, or complementary to contiguous nucleotides of any of SEQ ID NOs: 1 to 221, preferably SEQ ID NOs: 2, 10, 26 to 34, 42-43, 49-52, 66, 76, 84, 100-108, 116-117, 123-126, 137, 147, 155, 171-179, 187-188, 194-197, 208, more preferably SEQ ID NOs: 30-34, 52, 66, 104-108, 126, 137, 175-179, 197, 208; or SEQ ID NOs: 222 to 274 or to 293, preferably SEQ ID NOs: 247, 263, 270, 271, 272, 248, 249, 264, 250, 231, 251, 252, 253, 274, 254, 273, 255, or 223, or reverse complementary to contiguous nucleotides of any of SEQ ID NOs: 1 to 221, preferably SEQ ID NOs: 2, 10, 26 to 34, 42-43, 49-52, 66, 76, 84, 100-108, 116-117, 123-126, 137, 147, 155, 171-179, 187-188, 194-197, 208, more preferably SEQ ID NOs: 30-34, 52, 66, 104-108, 126, 137, 175-179, 197, 208; or SEQ ID NOs: 222 to 274 or to 293, preferably SEQ ID NOs: 247, 263, 270, 271, 272, 248, 249, 264, 250, 231, 251, 252, 253, 274, 254, 273, 255, or 223.

14. A (isolated) polynucleic acid specifically hybridizing with the polynucleotide according to any of claims 1 to 8, the complement or the reverse complement thereof.

15. A Brassica napus seed as deposited under NCIMB Accession No. NCIMB 43941, or plants or plant parts grown or obtained therefrom.

Figure 1

*Absolute grain yield (dt/ha) in the Federal/EU variety trials for winter rapeseed in 2017*

| | Sorten-typ[1] | Prüf-status | Hohen-schulen | Futterkamp | Otterham | Borwede | Hovedissen | Rauischholz-hausen | Singhofen | Boxberg |
|---|---|---|---|---|---|---|---|---|---|---|
| Bodenart/AZ | | | sL/50 | sL/60 | uT/85 | lS | sU/35 | sL/60 | sL/45 | sL/65 |
| Mittel VRS | | | 49,2 | 42,5 | 45,0 | 41,2 | 39,1 | 36,2 | 47,7 | 53,3 |
| Avatar | H | VRS | 51,0 | 44,4 | 47,0 | 43,3 | 40,0 | 34,4 | 49,9 | 54,3 |
| Mercedes | H | VRS | 45,6 | 41,2 | 44,6 | 39,4 | 39,8 | 36,3 | 47,1 | 51,7 |
| Raffiness | H | VRS | 50,9 | 41,8 | 43,3 | 40,9 | 37,5 | 37,8 | 46,1 | 54,1 |
| Bender | H | VGL | 49,5 | 43,4 | 44,7 | 44,0 | 41,0 | 40,6 | 51,1 | 54,7 |
| Mentor | H | VGL | 46,7 | 38,1 | 39,5 | 39,6 | 35,8 | 33,7 | 42,5 | 50,2 |
| Leopard | H | BSV | 50,3 | 48,6 | 49,9 | 48,4 | 40,6 | 33,9 | 53,0 | 54,9 |
| Edison | H | BSV | 53,7 | 43,7 | 45,3 | 39,1 | 38,2 | 32,0 | 52,4 | 53,6 |
| Hattrick | H | BSV | 55,1 | 45,2 | 50,1 | 41,7 | 41,6 | 34,1 | 52,9 | 56,4 |
| Muzzical | H | BSV | 56,2 | 47,5 | 53,0 | 46,8 | 41,9 | 32,1 | 56,4 | 56,9 |
| Asterion | H | BSV | 54,9 | 47,3 | 51,0 | 47,7 | 43,3 | 43,7 | 58,1 | 56,4 |
| Angelus | H | BSV | 53,6 | 44,7 | 50,1 | 44,6 | 40,9 | 37,9 | 50,2 | 52,3 |
| Pyro | H | BSV | 55,6 | 46,3 | 49,2 | 46,1 | 40,4 | 29,8 | 49,5 | 56,8 |
| INV 1055 | H | BSV | 46,3 | 38,4 | 43,7 | 37,8 | 37,0 | 32,9 | 51,2 | 55,8 |
| INV 1000 | H | BSV | 41,7 | 40,1 | 40,1 | 32,3 | 36,6 | 33,0 | 51,5 | 55,5 |
| INV 1066 | H | BSV | 44,6 | 39,1 | 44,2 | 29,5 | 37,0 | 30,4 | 50,0 | 54,3 |
| INV 1077 | H | BSV | 47,2 | 39,3 | 43,5 | 35,2 | 37,5 | 32,0 | 50,2 | 53,3 |
| Hawai | H | BSV | 51,9 | 44,1 | 43,3 | 41,9 | 38,7 | 38,1 | 51,9 | 55,1 |
| SY Florida | H | EU 2 | 47,5 | 43,2 | 42,6 | 38,4 | 39,1 | 33,4 | 51,3 | 54,7 |
| Cristiano KWS | H | EU 2 | 52,6 | 45,5 | 48,3 | 41,1 | 41,4 | 41,3 | 52,6 | 52,0 |
| Gaelis | H | EU 2 | 44,4 | 43,1 | 41,2 | 30,9 | 35,5 | 34,1 | 50,6 | 54,5 |
| Archimedes | H | EU 2 | 39,3 | 38,2 | 37,6 | 31,2 | 39,2 | 33,4 | 50,0 | 50,0 |
| Alabama | H | EU 2 | 48,5 | 40,7 | 43,4 | 36,7 | 37,3 | 34,4 | 51,8 | 54,4 |
| Alizze | H | EU 2 | 47,3 | 45,6 | 46,6 | 44,3 | 37,4 | 27,2 | 50,2 | 54,8 |
| Angus | H | EU 2 | 49,4 | 44,7 | 45,0 | 41,5 | 41,6 | 31,0 | 50,3 | 54,8 |
| DK Expansion | H | EU 2 | 47,1 | 44,2 | 46,1 | 43,0 | 40,7 | 37,4 | 53,2 | 55,8 |
| Alicante | H | EU 2 | 44,6 | 44,9 | 45,8 | 48,4 | 38,3 | 42,7 | 54,7 | 55,1 |
| Cuzzco | H | EU 2 | 46,4 | 44,1 | 50,0 | 45,2 | 40,0 | 35,0 | 53,9 | 54,4 |
| PT 256 | H | EU 2 | 50,1 | 44,5 | 50,9 | 42,9 | 36,0 | 36,8 | 50,8 | 56,2 |
| V 324 OL | H | EU 2 | 39,1 | 38,3 | 42,8 | 43,7 | 36,3 | 30,2 | 44,6 | 48,7 |
| GD 5% | | | 4,6 | 3,6 | 3,1 | 3,5 | 4,4 | 3,4 | 3,3 | 4,0 |

[1] H = restaurierte Hybridsorte, L = Liniensorte, HZ = Halbzwerghybride

# Figure 2

Figure 3

| Marker | ra7485401 | ra7485501 | ra7485601 | ra7485801 | ra7485901 | ra7486001 | ra3642801 | ra7485201 | ra7486301 | ra7486401 | ra7486501 | ra7486701 | ra3644401 | ra7486901 | ra7487001 | ra3645001 | Score |
|---|---|---|---|---|---|---|---|---|---|---|---|---|---|---|---|---|---|
| Pos Darmor v4.1 | 5997653 | 6022301 | 6042239 | 6088663 | 6091648 | 6113367 | 6141204 | 6155076 | 6186575 | 6196798 | 6204215 | 6248401 | 6269318 | 6306470 | 6314871 | 6373996 | |
| G1 | ade | thy | ade | thy | thy | gua | ade | ade | ade | cyt | thy | ade | gua | thy | gua | ade/gua | 3 |
| G2 | ade | thy | ade | thy | thy | gua | ade | ade | ade | cyt | thy | ade | | | gua/thy | ade/gua | 3 |
| G3 | ade/thy | cyt/thy | ade/cyt | thy | thy | gua | ade | ade | ade | cyt | thy | ade | gua | thy | gua | ade | 3 |
| G4 | ade/thy | cyt/thy | ade/cyt | ade/thy | cyt/thy | gua/thy | ade/gua | ade/thy | ade/cyt | ade/cyt | cyt/thy | ade/cyt | ade/gua | cyt/thy | gua/thy | ade | 0 |
| G5 | ade/thy | cyt/thy | ade/cyt | ade/thy | cyt/thy | gua/thy | ade/gua | ade/thy | ade/cyt | ade/cyt | cyt/thy | ade/cyt | ade/gua | cyt/thy | gua/thy | ade/gua | 0 |
| G6 | ade | thy | ade/cyt | ade/thy | cyt/thy | gua/thy | ade/gua | ade/thy | ade/cyt | ade/cyt | cyt/thy | ade/cyt | ade/gua | cyt/thy | gua/thy | ade/gua | 0 |
| G7 | ade/thy | cyt/thy | ade/cyt | ade/thy | cyt/thy | gua/thy | ade/gua | ade/thy | ade/cyt | ade/cyt | cyt/thy | ade/cyt | gua | thy | gua | ade | 0 |

Figure 4

Figure 5

Europäisches
Patentamt

European
Patent Office

Office européen
des brevets

## EUROPEAN SEARCH REPORT

Application Number

EP 22 17 4669

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/226806 A1 (WUHAN GREENFAFA INST OF NOVEL GENECHIP R&D CO LTD [CN] ET AL.) 18 November 2021 (2021-11-18) * SEQ ID NO: 9519 discloses a Brassica SNP marker having 97% identity to SEQ ID NO:231; SEQ ID NO: 5574, 8128, 1056, 18476 disclose Brassica SNP markers having 100% identity to SEQ ID NO:151, 153, 202, and 206 over 96-121 nucleotides; * -& Anonymous: "GSN:BKE62833", , 23 December 2021 (2021-12-23), pages 1-1, XP55975409, Retrieved from the Internet: URL:http://ibis.internal.epo.org/exam/dbfetch.jsp?id=GSN:BKE62833 [retrieved on 2022-10-27] ----- | 1-5,9-14 | INV. A01H1/04 A01H1/00 A01H5/10 A01H6/20 C12Q1/6895 |
| X | AU 2020 100 975 A4 (BEIJING ACAD AGRIC & FORESTRY) 27 August 2020 (2020-08-27) * B. rapa SNP primer showing 100% identity to SEQ ID NO: 56 over 29 nucleotides; derived frrom chromosome A06 * -& Anonymous: "GSN:BIG39026", , 29 October 2020 (2020-10-29), pages 1-1, XP055975723, Retrieved from the Internet: URL:http://ibis.internal.epo.org/exam/dbfetch.jsp?id=GSN:BIG39026 [retrieved on 2022-10-28] ----- -/-- | 10-12 | TECHNICAL FIELDS SEARCHED (IPC) A01H C12Q |

The present search report has been drawn up for all claims

| Place of search | Date of completion of the search | Examiner |
|---|---|---|
| Munich | 28 October 2022 | Kania, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 4**

## EUROPEAN SEARCH REPORT

Application Number

EP 22 17 4669

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHANG HUI ET AL: "Identification and Fine-Mapping of Clubroot (Plasmodiophora brassicae) Resistant QTL in Brassica rapa", HORTICULTURAE, vol. 8, no. 1, 11 January 2022 (2022-01-11), page 66, XP55975384, DOI: 10.3390/horticulturae8010066 ----- | 1-15 | |
| A | SHAH NADIL ET AL: "Genetic variation analysis of field isolates of clubroot and their responses tolines containing resistant genesandand their combination in homozygous and heterozygous state", MOLECULAR BREEDING, SPRINGER NETHERLANDS, DORDRECHT, vol. 39, no. 10-11, 12 November 2019 (2019-11-12), XP037184535, ISSN: 1380-3743, DOI: 10.1007/S11032-019-1075-3 [retrieved on 2019-11-12] ----- | 1-15 | |
| A | NGUYEN M L ET AL: "The New Clubroot Resistance Locus Is Located on Chromosome A05 in Chinese Cabbage (Brassica rapaL.)", RUSSIAN JOURNAL OF GENETICS, MOSCOW, RU, vol. 54, no. 3, 28 March 2018 (2018-03-28), pages 296-304, XP036467745, ISSN: 1022-7954, DOI: 10.1134/S1022795418030080 [retrieved on 2018-03-28] ----- | 1-15 | TECHNICAL FIELDS SEARCHED (IPC) |
| A | CN 114 381 463 A (AGRICULTURAL UNIV IN CHINA) 22 April 2022 (2022-04-22) ----- | 1-15 | |
| | -/-- | | |

The present search report has been drawn up for all claims

| Place of search | Date of completion of the search | Examiner |
|---|---|---|
| Munich | 28 October 2022 | Kania, Thomas |

EPO FORM 1503 03.82 (P04C01)

Europäisches Patentamt

European Patent Office

Office européen des brevets

## EUROPEAN SEARCH REPORT

**Application Number**

EP 22 17 4669

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FENGQUN YU ET AL: "Genotyping-by-sequencing reveals three QTL for clubroot resistance to six pathotypes of Plasmodiophora brassicae in Brassica rapa", SCIENTIFIC REPORTS, vol. 7, no. 1, 3 July 2017 (2017-07-03), XP055496842, DOI: 10.1038/s41598-017-04903-2 ----- | 1-15 | |
| A | WO 2021/231098 A1 (CARGILL INC [US]) 18 November 2021 (2021-11-18) ----- | 1-15 | |
| A | HUANG Z ET AL: "Fine mapping of a clubroot resistance gene from turnip using SNP markers identified from bulked segregant RNA-Seq", MOLECULAR BREEDING, SPRINGER NETHERLANDS, DORDRECHT, vol. 39, no. 9, 29 August 2019 (2019-08-29), XP036914090, ISSN: 1380-3743, DOI: 10.1007/S11032-019-1038-8 [retrieved on 2019-08-29] ----- | 1-15 | |
| | | | **TECHNICAL FIELDS SEARCHED (IPC)** |
| A | CN 112 725 523 A (OIL CROPS RES INSTITUTE CHINESE ACADEMY OF AGRICULTURAL SCIENCES) 30 April 2021 (2021-04-30) ----- | 1-15 | |
| T | WO 2022/136239 A1 (NPZ INNOVATION GMBH [DE]) 30 June 2022 (2022-06-30) * SEQ ID NO: 23 shows 98% identity to present SEQ ID NO: 223 as well as the identical SNP at the corresponding position * -/-- | | |

The present search report has been drawn up for all claims

| Place of search | Date of completion of the search | Examiner |
|---|---|---|
| Munich | 28 October 2022 | Kania, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## EUROPEAN SEARCH REPORT

Application Number

EP 22 17 4669

### DOCUMENTS CONSIDERED TO BE RELEVANT

| Category | Citation of document with indication, where appropriate, of relevant passages | Relevant to claim |
|---|---|---|
| | -& Anonymous:  "GSN:BLH43528",<br><br>,<br>11 August 2022 (2022-08-11), pages 1-1,<br>XP055975414,<br>Retrieved from the Internet:<br>URL:http://ibis.internal.epo.org/exam/dbfe<br>tch.jsp?id=GSN:BLH43528<br>[retrieved on 2022-10-27]<br>-& Anonymous:  "GSN:BLH43520",<br><br>,<br>11 August 2022 (2022-08-11), pages 1-1,<br>XP055975765,<br>Retrieved from the Internet:<br>URL:http://ibis.internal.epo.org/exam/dbfe<br>tch.jsp?id=GSN:BLH43520<br>[retrieved on 2022-10-28]<br>----- | |

**CLASSIFICATION OF THE APPLICATION (IPC)**

**TECHNICAL FIELDS SEARCHED (IPC)**

The present search report has been drawn up for all claims

| Place of search | Date of completion of the search | Examiner |
|---|---|---|
| Munich | 28 October 2022 | Kania, Thomas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 17 4669

This annex lists the patent family members relating to the patent documents cited in the above-mentioned European search report.
The members are as contained in the European Patent Office EDP file on
The European Patent Office is in no way liable for these particulars which are merely given for the purpose of information.

28-10-2022

| Patent document cited in search report | | Publication date | Patent family member(s) | Publication date |
|---|---|---|---|---|
| WO 2021226806 | A1 | 18-11-2021 | NONE | |
| AU 2020100975 | A4 | 27-08-2020 | NONE | |
| CN 114381463 | A | 22-04-2022 | NONE | |
| WO 2021231098 | A1 | 18-11-2021 | NONE | |
| CN 112725523 | A | 30-04-2021 | NONE | |
| WO 2022136239 | A1 | 30-06-2022 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8450471 B
- US 8440431 B
- US 8440432 B
- US 6534261 B
- US 6607882 B
- US 6746838 B
- US 6794136 B
- US 6824978 B
- US 6866997 B
- US 6933113 B
- US 6979539 B
- US 7013219 B
- US 7030215 B
- US 7220719 B
- US 7241573 B
- US 7241574 B
- US 7585849 B
- US 7595376 B
- US 6903185 B
- US 6479626 B
- US 8163514 B
- US 8133697 B
- US 8021867 B
- US 8119361 B
- US 8119381 B
- US 8124369 B
- US 8129134 B

**Non-patent literature cited in the description**

- Molecular Cloning: A Laboratory Manual. Cold Spring Harbor Laboratory Press, 1989, vol. 1-3
- Current Protocols in Molecular Biology. Greene Publishing and Wiley-Interscience, 1992
- Methods in Enzymology. Academic Press, Inc,
- INNIS et al. PCR Protocols: A Guide to Methods and Applications. Academic Press, 1990
- PCR 2: A Practical Approach. 1995
- Antibodies, a Laboratory Manual. 1988
- Animal Cell Culture. 1987
- DAVIS, B. D. et al. Microbiology. Harper & Row, publishers, 1980
- CHALHOUB et al. Early allopolyploid evolution in the post-Neolithic Brassica napus oilseed genome. *Science,* 2014, vol. 345 (6199), 950-953
- SAMBROOK et al. Molecular Cloning. A Laboratory Manual. Cold Spring Harbor Laboratory Press, 2001
- CERMAK T ; DOYLE EL. ; CHRISTIAN M. ; WANG L. ; ZHANG Y. ; SCHMIDT C et al. Efficient design and assembly of custom TALEN and other TAL effector-based constructs for DNA targeting. *Nucleic Acids Res.,* 2011, vol. 39, e82
- ZHANG F. ; CONG L. ; LODATO S. ; KOSURI S. ; CHURCH GM. ; ARLOTTA P. Efficient construction of sequence-specific TAL effectors for modulating mammalian transcription. *Nat Biotechnol.,* 2011, vol. 29, 149-153
- MOSCOU et al. *Science,* 2009, vol. 326, 1501
- BOCH et al. *Science,* 2009, vol. 326, 1509-1512
- ZHANG et al. *Nature Biotechnology,* 2011, vol. 29, 149-153
- KIM, Y. G. et al. Chimeric restriction endonuclease. *Proc. Natl. Acad. Sci. U.S.A.,* 1994, vol. 91, 883-887
- KIM, Y. G. et al. Hybrid restriction enzymes: zinc finger fusions to Fok I cleavage domain. *Proc. Natl. Acad. Sci. U.S.A.,* 1996, vol. 93, 1156-1160
- DOYON, Y. et al. Enhancing zinc-finger-nuclease activity with improved obligate heterodimeric architectures. *Nat. Methods,* 2011, vol. 8, 74-79
- CONG, L. ; RAN, F.A. ; COX, D. ; LIN, S. ; BARRETTO, R. ; HABIB, N. ; HSU, P.D. ; WU, X. ; JIANG, W. ; MARRAFFINI, L.A. *Science,* 15 February 2013, vol. 339 (6121), 819-23
- JIANG W. ; BIKARD D. ; COX D. ; ZHANG F ; MARRAFFINI LA. *Nat Biotechnol,* March 2013, vol. 31 (3), 233-9
- WANG H. ; YANG H. ; SHIVALILA CS. ; DAWLATY MM. ; CHENG AW. ; ZHANG F. ; JAENISCH R. *Cell,* 09 May 2013, vol. 153 (4), 910-8
- KONERMANN S ; BRIGHAM MD ; TREVINO AE ; HSU PD ; HEIDENREICH M ; CONG L ; PLATT RJ ; SCOTT DA ; CHURCH GM ; ZHANG F. *Nature,* 22 August 2013, vol. 500 (7463), 472-6
- RAN, FA. ; HSU, PD. ; LIN, CY. ; GOOTENBERG, JS. ; KONERMANN, S. ; TREVINO, AE. ; SCOTT, DA. ; INOUE, A. ; MATOBA, S. ; ZHANG, Y. *Cell,* 28 August 2013

- **HSU, P. ; SCOTT, D. ; WEINSTEIN, J. ; RAN, FA. ; KONERMANN, S. ; AGARWALA, V. ; LI, Y. ; FINE, E. ; WU, X. ; SHALEM, O.** *Nat Biotechnol,* 2013
- **RAN, FA. ; HSU, PD. ; WRIGHT, J. ; AGARWALA, V. ; SCOTT, DA. ; ZHANG, F.** *Nature Protocols,* November 2013, vol. 8 (11), 2281-308
- **SHALEM, O. ; SANJANA, NE. ; HARTENIAN, E. ; SHI, X. ; SCOTT, DA. ; MIKKELSON, T. ; HECKL, D. ; EBERT, BL. ; ROOT, DE. ; DOENCH, JG.** *Science,* 12 December 2013
- **NISHIMASU, H. ; RAN, FA. ; HSU, PD. ; KONERMANN, S. ; SHEHATA, SI. ; DOHMAE, N. ; ISHITANI, R. ; ZHANG, F. ; NUREKI, O.** *Cell,* vol. 156 (5), 935-49
- **WU X. ; SCOTT DA. ; KRIZ AJ. ; CHIU AC. ; HSU PD. ; DADON DB. ; CHENG AW. ; TREVINO AE. ; KONERMANN S. ; CHEN S.** *Nat Biotechnol.,* 20 April 2014
- **PLATT et al.** *Cell,* 2014, vol. 159 (2), 440-455
- **HSU et al.** *Cell,* 05 June 2014, vol. 157, 1262-1278
- **WANG et al.** *Science,* 03 January 2014, vol. 343 (6166), 80-84
- **DOENCH et al.** *Nature Biotechnology,* 03 September 2014, vol. 32 (12), 1262-7
- **SWIECH et al.** *Nature Biotechnology,* 19 October 2014, vol. 33, 102-106
- **ZETSCHE et al.** *Cell,* 2015, vol. 163, 1-13
- **SHMAKOV et al.** *Mol Cell,* 2015, vol. 60 (3), 385-397
- **ABUDAYYEH et al.** *Science,* 02 June 2016
- **KLEINSTIVER BP et al.** Engineered CRISPR-Cas9 nucleases with altered PAM specificities. *Nature,* 23 July 2015, vol. 523 (7561), 481-5
- **JORE et al.** *Nat. Struct. Mol. Biol.,* 2011, vol. 18, 529-536
- **GOUDELLI et al.** Nature. *Programmable base editing of A•T to G•C in genomic DNA without DNA cleavage,* 2017, vol. 551 (7681), 464
- **MCCALLUM et al.** Targeted screening for induced mutations. *Nat Biotechnol.,* April 2000, vol. 18 (4), 455-7
- **MCCALLUM et al.** Targeting Induced Local Lesions IN Genomes (TILLING) for plant functional genomics. *Plant Physiol.,* June 2000, vol. 123 (2), 439-42
- **TILL et al.** Discovery of induced point mutations in maize genes by TILLING. *BMC Plant Biol.,* 28 July 2004, vol. 4, 12
- **WEIL ; MONDE.** Getting the point-mutations in maize. *Crop Sci,* 2007, vol. 47, S60-S67
- **E. DIEDERICHSEN ; M.D. SACRISTAN.** *Plant Breeding,* 1996, vol. 115, 5-10
- **CHALHOUB et al.** *Science,* 2014, vol. 345, 950-953